# EUROPEAN PATENT APPLICATION

(11) **EP 4 434 979 A1**
(43) Date of publication of application: **25.09.2024**
(21) Application number: 22894970.7
(22) Date of filing: 18.11.2022
(51) Int. Cl.: C07D 401/12, C07D 401/14, C07D 405/14, C07D 413/14, A61K 31/444, A61P 35/00

(54) **KIF18A INHIBITOR**

(30) Priority: 19.11.2021 CN 202111399876; 09.03.2022 CN 202210225663; 13.06.2022 CN 202210667619
(71) Applicant: Wigen Biomedicine Technology (Shanghai) Co., Ltd., Shanghai 201203 (CN)
(72) Inventor: XIE, Yuli, Shanghai 201203 (CN); WU, Yingming, Shanghai 201203 (CN); QIAN, Lihui, Shanghai 201203 (CN)
(74) Representative: Willett, Christopher David
(86) International application number: PCT/CN2022/132954
(87) International publication number: WO 2023/088441

(57) **Abstract**

The present invention discloses a KIF18A inhibitor. Specifically, the present invention relates to a compound of general formula (1), a method for preparing same, and use of the compound of general formula (1) or an isomer, a crystalline form, a pharmaceutically acceptable salt, a hydrate or a solvate thereof as a KIF18A inhibitor in preparing an anti-tumor medicament.

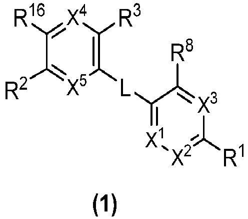

## Description

The present application claims priority to Chinese Patent Application No. 202111399876.4 filed on November 19, 2022, Chinese Patent Application No. 202210225663.8 filed on March 9, 2022, and Chinese Patent Application No. 202210667619.2 filed on June 13, 2022, which are incorporated herein by reference in their entireties.

### TECHNICAL FIELD

The present invention relates to the field of pharmaceutical chemistry, and particularly to a compound with an inhibitory effect on KIF18A protein, a method for preparing same, and use of the compound in preparing an anti-tumor medicament.

### BACKGROUND

Genomic instability is a common feature of most tumor cells. Most tumor cells exhibit abnormal gain or deletion of chromosomes. The chromosome instability of tumor cells leads to the interaction of abnormal chromosomes with mitotic spindle microtubules, which in turn causes chromosome segregation errors. Cells with chromosome instability develop increased spindle microtubule polymerization and reduced spindle microtubule-centromere contact turnover, as compared to cells with normal chromosomes. Therefore, anti-mitotic therapies directed against the microtubule backbone may be particularly effective for cells with chromosome instability.

Kinesins are a class of molecular motors that play important roles in cell division and intracellular vesicle and organelle transport. Mitotic kinesins play important roles in several aspects such as spindle assembly, chromosome segregation, centrosome segregation, and dynamics. Human kinesins are classified into 14 subfamilies based on differences in amino acid sequences of motor domains, and the ATPase activity located in the motor domains drives the proteins to move unidirectionally along the microtubules. The non-motor domains of these proteins are responsible for interacting with substrates, and a variety of different membranous organelles, signal transduction scaffold systems, and chromosomes serve as substrates with which the non-motor domains can interact. The kinesins gain energy through ATP hydrolysis, moving the substrates along polarized microtubules. Therefore, kinesins are commonly referred to as "plus-end" or "minus-end" directed motors.

KIF18A protein belongs to the kinesin-8 subfamily. KIF18A protein is overexpressed in various types of cancers, such as lung, ovarian, cervical, breast, pancreatic, prostate, colon, and bladder cancers. Studies suggest that KIF18A affects the dynamics of the plus-ends of the centromere microtubules so as to control correct chromosome positioning and spindle tension. In tumor cells with chromosome instability, abnormal microtubule dynamics make such cells particularly dependent on KIF18A protein to reduce spindle microtubule-centromere contact turnover and to limit microtubule growth (Nat Commun. 2021, 12, 1213). When KIF18A protein is deleted from tumor cells with chromosome instability, centrosomes of the cells are fragmented and mitotic progression is slowed or stopped. However, these phenomena do not occur in cells with normal chromosomes. Therefore, the activity of KIF18A protein does not have a significant effect on the proliferation of normal cells but is very critical for the growth of chromosomally unstable tumors.

Accordingly, the development of KIF18A inhibitors is a new promising approach against tumors with chromosome instability.

### SUMMARY

The present invention provides a compound of general formula (1), or an isomer, a crystalline form, a pharmaceutically acceptable salt, a hydrate or a solvate thereof: 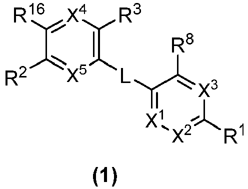 wherein, in general formula (1):
X¹ is -CR⁵= or N;
X² is -CR⁶= or N;
X³ is -CR⁷= or N;
X⁴ is -CR⁴= or N;
X⁵ is -CR¹⁵= or N;
when X⁵ is -CR¹⁵= and X⁴ is -CR⁴=, R¹⁶ is -C₃₋₈ cycloalkyl, -OR¹⁷, -SR¹⁸, -NR¹⁸R¹⁹, or -NO₂;
when X⁵ is -CR¹⁵= and X⁴ is N, R¹⁶ is -O-C₁₋₈ hydrocarbyl, -C₃₋₈ cycloalkyl, -OR¹⁷, -SR¹⁸, -NR²⁰R²¹, or -NO₂;
when X⁵ is N, R¹⁶ is -O-C₁₋₈ hydrocarbyl, -C₃₋₈ cycloalkyl, -OR¹⁷, -SR¹⁸, -NR²⁰R²¹, or -NO₂;
L is -(C=O)-NR⁹-* or -NR⁹-(C=O)-*; and no more than four of X¹, X², X³, X⁴, and X⁵ are N;
* represents a position linking to 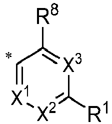 terminal;
R¹⁷ is H, -C₁₋₈ halohydrocarbyl, -C₃₋₈ cycloalkyl, or -C₃₋₈ halocycloalkyl, wherein the -C₁₋₈ halohydrocarbyl, - C₃₋₈ cycloalkyl, or -C₃₋₈ halocycloalkyl may be optionally substituted with 0, 1, 2, or 3 of the following groups: H, halogen, and -C₁₋₄ hydrocarbyl;
R¹⁸ and R¹⁹ are each independently H, -C₁₋₈ hydrocarbyl, -C₁₋₈ halohydrocarbyl, -C₃₋₈ cycloalkyl, or -C₃₋₈ halocycloalkyl, wherein the -C₁₋₈ hydrocarbyl, -C₁₋₈ halohydrocarbyl, -C₃₋₈ cycloalkyl, or -C₃₋₈ halocycloalkyl may be optionally substituted with 0, 1, 2, or 3 of the following groups: H, halogen, and -C₁₋₄ hydrocarbyl;
R²⁰ and R²¹ are each independently H, -C₁₋₈ hydrocarbyl, -C₁₋₈ halohydrocarbyl, -C₃₋₈ cycloalkyl, or -C₃₋₈ halocycloalkyl, wherein the -C₁₋₈ hydrocarbyl, -C₁₋₈ halohydrocarbyl, -C₃₋₈ cycloalkyl, or -C₃₋₈ halocycloalkyl may be optionally substituted with 0, 1, 2, or 3 of the following groups: H, halogen, and -C₁₋₄ hydrocarbyl; or
R²⁰ and R²¹ may be combined with the nitrogen atom to which they are each connected to form a saturated or partially saturated 3-, 4-, 5-, or 6-membered monocyclic ring or 4-, 5-, 6-, 7-, 8-, 9-, 10-, 11-, or 12-membered bicyclic ring containing 0, 1, 2, or 3 N atoms and 0, 1, or 2 atoms selected from O and S;
R¹ is -CN or -Z-R¹⁰, wherein Z is a chemical bond, -C₀₋₄ hydrocarbyl-, -NR¹¹-, -NR¹¹SO₂-, -SO₂NR¹¹-, -NR¹¹-S(=O)(=NH)-, -S(=O)(=NH)-, -S-, -S(=O)-, -SO₂-, -C₀₋₄ hydrocarbyl-O-, -(C=O)-, -(C=O)NR¹¹-, -C(=N-OH)-, or -NR¹¹(C=O)-; or the group -Z-R¹⁰ is -N=S(=O)-(R¹⁰)₂, wherein two R¹⁰ may be combined with the sulfur atom to which they are each connected to form a saturated or partially saturated 3-, 4-, 5-, or 6-membered monocyclic ring containing 0, 1, 2, or 3 N atoms and 0, 1, or 2 atoms selected from O and S;
R² is halogen or a group -Y-R¹², wherein Y is a chemical bond, -C₀₋₄ hydrocarbyl-, -N(C₀₋₁ hydrocarbyl)-C₀₋₄ hydrocarbyl-, -C(=O)NR^{a}R^{a} (C₁₋₄ hydrocarbyl)-, -O-C₀₋₄ hydrocarbyl-, -S-, -S(=O)-, -SO₂-, -SO₂NR¹²-, or - S(=O)(=NH)-;
R³ is H, halogen, C₁₋₈ hydrocarbyl, or C₁₋₄ halohydrocarbyl;
R⁴ is H, halogen, R^{4a}, or R^{4b};
R⁵ is H, halogen, C₁₋₈ alkyl, or C₁₋₄ haloalkyl;
R⁶ is H, halogen, C₁₋₈ alkyl, C₁₋₄ haloalkyl, -OH, -O-R^{6a}, or -O-R^{6b};
R⁷ is H, halogen, C₁₋₈ hydrocarbyl, or C₁₋₄ halohydrocarbyl;
R⁸ is selected from the group consisting of: 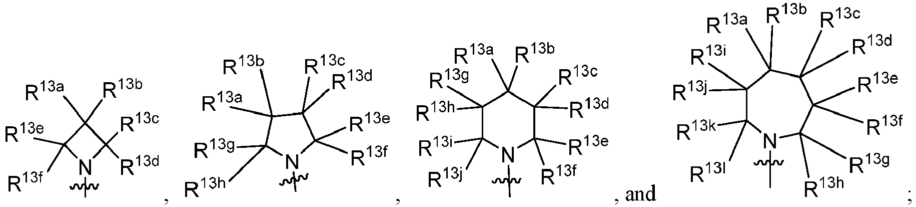
R^{13a}, R^{13b}, R^{13c}, R^{13d}, R^{13e}, R^{13f}, R^{13g}, R^{13h}, R¹³ⁱ, R^{13j}, R^{13k}, and R^{13l} are each independently H, halogen, R^{13m}, or R¹³ⁿ; or each of the pairs of R^{13a}/R^{13b}, R^{13c}/R^{13d}, R^{13e}/R^{13f}, R^{13g}/R^{13h}, R¹³ⁱ/R^{13j}, and R^{13k}/R^{13l} may independently form, with the carbon atom to which they are each connected, a saturated or partially saturated 3-, 4-, 5-, or 6-membered monocyclic ring spiro-linked to R⁸ ring, wherein the 3-, 4-, 5-, or 6-membered monocyclic ring contains 0, 1, 2, or 3 N atoms and 0, 1, or 2 atoms selected from O and S, and further, the 3-, 4-, 5-, or 6-membered monocyclic ring is substituted with 0, 1, 2, or 3 groups selected from: F, Cl, Br, C_{I-6} hydrocarbyl, C₁₋₄ halohydrocarbyl, -OR^{a}, -OC₁₋₄ halohydrocarbyl, CN, -NR^{a}R^{a}, and oxo;
R⁹ is H or C₁₋₆ hydrocarbyl;
R¹⁰ is H, R^{10a}, R^{10b}, or R^{10c};
R¹¹ is H, R^{11a}, or R^{11b};
R¹² is R^{12a} or R^{12b};
R¹⁵ is H, halogen, C₁₋₈ hydrocarbyl, C₁₋₄ halohydrocarbyl, -O-C₁₋₈ hydrocarbyl, or -O-R^{15a}, wherein R^{15a} is a saturated or partially saturated 3-, 4-, 5-, or 6-membered monocyclic ring containing 0, 1, 2, or 3 N atoms and 0, 1, or 2 atoms selected from O and S;
R^{4a}, R^{6a}, R^{10a}, R^{11a}, R^{12a}, or R^{13m}, in each case, is independently selected from: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0, 1 or 2 atoms selected from O and S, wherein the monocyclic ring and bicyclic ring may be each independently and optionally substituted with 0, 1, 2, or 3 of the following groups: F, Cl, Br, C_{I-6} hydrocarbyl, C₁₋₄ halohydrocarbyl, -OR^{a}, -OC₁₋₄ halohydrocarbyl, CN, -C(=O)R^{b}, - C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, R¹⁴, and oxo;
R^{4b}, R^{6b}, R^{10b}, R^{11b}, R^{12b}, or R¹³ⁿ, in each case, is independently selected from: C₁₋₆ hydrocarbyl, wherein the hydrocarbyl may be optionally substituted with 0, 1, 2, 3, 4, or 5 of the following groups: F, Cl, Br, -R^{a}, -OR^{a}, -OC₁₋₄ halohydrocarbyl, and CN;
R^{10c}, in each case, is independently selected from: C_{I-6} hydrocarbyl, wherein the hydrocarbyl may be optionally substituted with 0, 1, 2, 3, 4, or 5 of the following groups: F, Cl, Br, -R^{a}, -R^{c}, -OR^{a}, -OC₁₋₄ halohydrocarbyl, and CN;
R¹⁴, in each case, is independently selected from: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 of the following groups: F, Cl, Br, C_{I-6} hydrocarbyl, C₁₋₄ halohydrocarbyl, -OR^{a}, -OC₁₋₄ halohydrocarbyl, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, - S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, - N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, and oxo;
R^{a}, in each case, is independently H or R^{b};
R^{b}, in each case, is independently C_{I-6} hydrocarbyl, phenyl, or benzyl, wherein the hydrocarbyl may be optionally substituted with 0, 1, 2, or 3 of the following groups: halogen, -OH, -OC₁₋₄ hydrocarbyl, -NH₂, - NHC₁₋₄ hydrocarbyl, -OC(=O)C₁₋₄ hydrocarbyl, and -N(C₁₋₄ hydrocarbyl) C₁₋₄ hydrocarbyl; and the phenyl and benzyl may be each independently and optionally substituted with 0, 1, 2, or 3 of the following groups: halogen, C₁₋₄ hydrocarbyl, C₁₋₃ halohydrocarbyl, -OH, -OC₁₋₄ hydrocarbyl, -NH₂, -NHC₁₋₄ hydrocarbyl, - OC(=O)C₁₋₄ hydrocarbyl, and -N(C₁₋₄ hydrocarbyl) C₁₋₄ hydrocarbyl; and
R^{c}, in each case, is independently -OC(=O)C₁₋₅ hydrocarbyl, wherein the hydrocarbyl may be optionally substituted with 1, 2, or 3 of the following groups: -OH and -NH₂.

In another preferred embodiment, general formula (1) has the following structure: 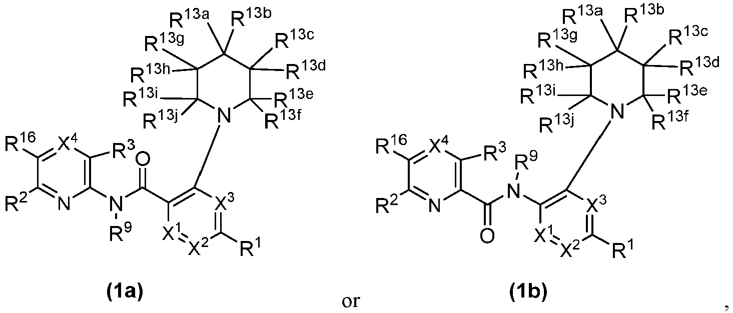 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂.

In another preferred embodiment, general formula (1) has the following structure: 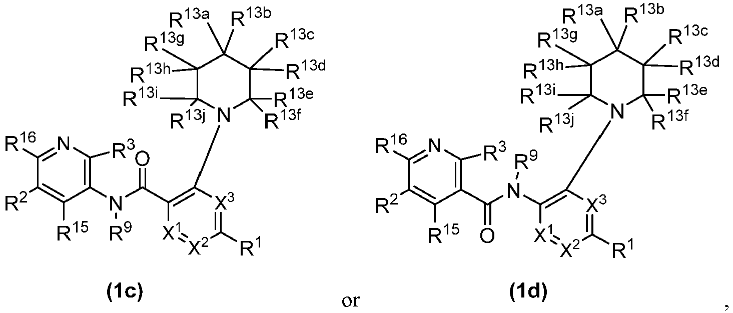 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂.

In another preferred embodiment, general formula (1) has the following structure: 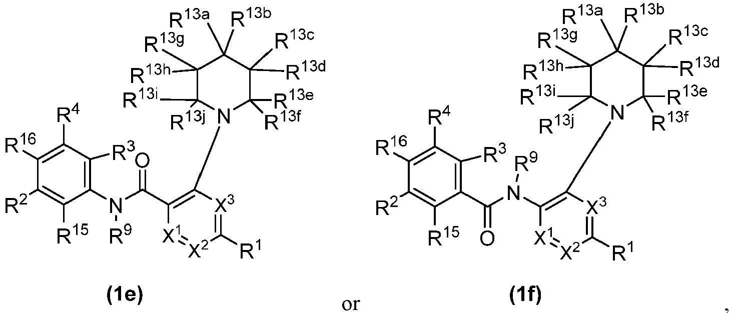 wherein R¹⁶ is -C₃₋₆ cycloalkyl, -OH, -O-C₁₋₄ halohydrocarbyl, -O-C₃₋₆ cycloalkyl, -O-C₃₋₆ halocycloalkyl, - SH, -S-C₁₋₆ hydrocarbyl, -S-C₁₋₄ halohydrocarbyl, -S-C₃₋₆ cycloalkyl, -S-C₃₋₆ halocycloalkyl, -NR¹⁸R¹⁹, or - NO₂.

In another preferred embodiment, in general formula (1), R¹⁶ is -OH, -OCF₃, -OCH₂F, -OCHF₂, -OCH₂CF₃, - OCF₂CF₃, -OCF₂Cl, -OCFCl₂, 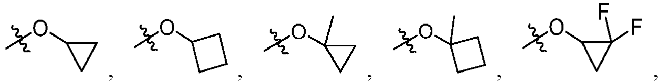 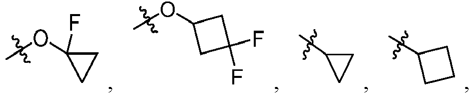 -SH, -SCH₃, -SCH₂CH₃, 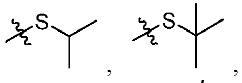 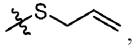 -SCF₃, -SCH₂CF₃, -SCF₂CF₃, -SCF₂Cl, -SCFCl₂, 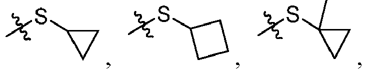 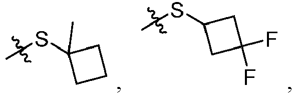 -NH₂, 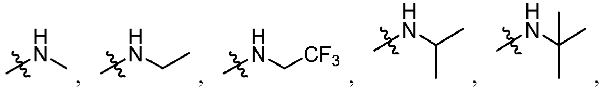 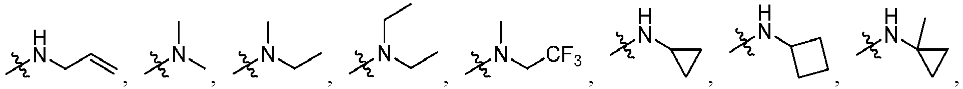 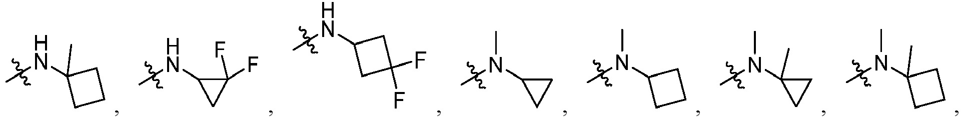 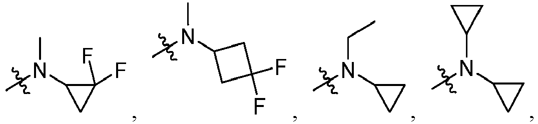 or -NO₂, preferably -OCF₃, -OCH₂F, -OCHF₂, 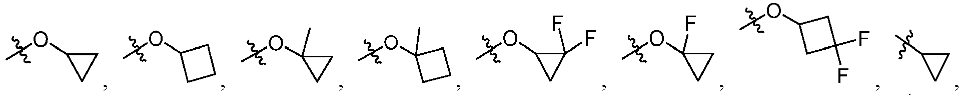 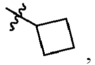 -SCH₃, -SCF₃, -SCF₂Cl, -SCFCl₂, 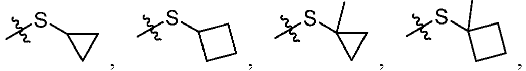 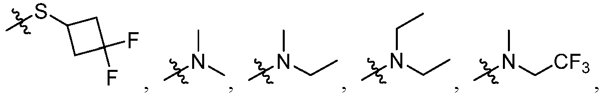 or -NO₂, and more preferably -OCF₃, - OCH₂F, -OCHF₂, 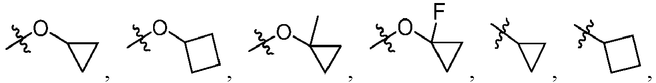 -SCH₃, -SCF₃, 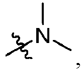 or -NO₂.

In another preferred embodiment, in general formula (1), R¹⁶ is -OCH₃, -OCH₂CH₃, -OCH₂CH₂CH₃, 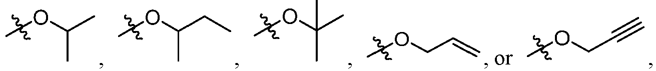 preferably -OCH₃.

In another preferred embodiment, in general formula (1), R⁹ is H, methyl, or ethyl, preferably H.

In another preferred embodiment, in general formula (1), R^{13c}, R^{13d}, R^{13e}, R^{13f}, R^{13g}, R^{13h}, R¹³ⁱ, R^{13j}, R^{13k}, and R^{13l} are each independently H, halogen, C₁₋₆ hydrocarbyl, or C₁₋₄ halohydrocarbyl; and R^{13a} and R^{13b} in the pair of R^{13a}/R^{13b} may be combined with the carbon atom to which they are each connected to form a saturated 3-, 4-, or 5-membered monocyclic ring spiro-linked to R⁸ ring, wherein the monocyclic ring contains 0, 1, 2, or 3 N atoms and 0, 1, or 2 atoms selected from O and S; preferably, R^{13c}, R^{13d}, R^{13e}, R^{13f}, R^{13g}, R^{13h}, R¹³ⁱ, R^{13j}, R^{13k}, and R^{13l} are each independently H, methyl, or ethyl; and R^{13a} and R^{13b} in the pair of R^{13a}/R^{13b} may be combined with the carbon atom to which they are each connected to form a cyclopropyl, cyclobutyl, or cyclopentyl ring spiro-linked to R⁸ ring.

In another preferred embodiment, in general formula (1), structural unit 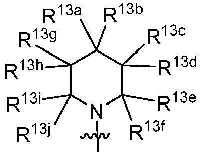 is: 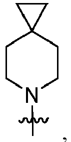 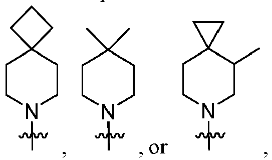 preferably

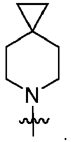

In another preferred embodiment, in general formula (1), Z is a chemical bond, -NH-, -NHSO₂-, -SO₂NH-, - S(=O)(=NH)-, -S-, -S(=O)-, -SO₂-, -(C=O)-, -(C=O)NH-, or -NH(C=O)-.

In another preferred embodiment, in general formula (1), R¹⁰ is selected from: (a) H; (b) C₁₋₆ hydrocarbyl, wherein the hydrocarbyl may be optionally substituted with 0, 1, 2, or 3 of the following groups: F, Cl, Br, - OH, and -OCH₃; (c) a group such that when the group -Z-R¹⁰ is -N=S(=O)-(R¹⁰)₂, two R¹⁰ may be combined with the sulfur atom to which they are each connected to form a saturated, partially saturated, or unsaturated 3-, 4-, 5-, 6-, or 7-membered monocyclic ring containing 0, 1, 2, or 3 N atoms and 0 or 1 atom selected from O and S, wherein the monocyclic ring is substituted with 0, 1, 2, or 3 groups selected from: F, Cl, Br, C₁₋₆ hydrocarbyl, C₁₋₄ halohydrocarbyl, -C₁₋₆ hydrocarbyl OH, -OH, -OCH₃, -NH₂, and oxo; and (d) C₁₋₆ hydrocarbyl, wherein the C₁₋₆ hydrocarbyl may be optionally substituted with 1, 2, or 3 of the following group: -OC(=O)C₁₋₅ hydrocarbyl, wherein the C₁₋₅ hydrocarbyl may be optionally substituted with 1 or 2 of the following groups: -OH and -NH₂; and the C₁₋₆ hydrocarbyl may be optionally substituted with 0, 1, 2, or 3 of the following groups: F, Cl, Br, -OH, and -OCH₃.

In another preferred embodiment, in general formula (1), R¹ is -CN or a group -Z-R¹⁰, wherein Z is a chemical bond, -NH-, -NHSO₂-, -SO₂NH-, -S(=O)(=NH)-, -S-, -S(=O)-, -SO₂-, -(C=O)-, -(C=O)NH-, or -NH(C=O)-; and R¹⁰ is selected from:
(a) H;
(b) cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 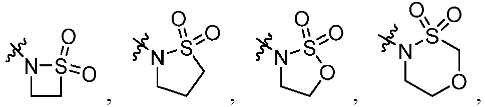 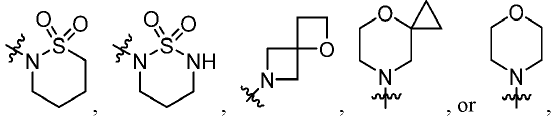 wherein the rings may be each independently and optionally substituted with 0, 1, 2, or 3 of the following groups: OH, F, methyl, -CH₂OH, -C(=O)OCH₃, - C(=O)OC(CH₃)₃, NH₂, CN, and oxo; and oxetanyl and cyclopropyl are preferred;
(c) C₁₋₆ hydrocarbyl substituted with 0, 1, 2, or 3 OH, F, -C(=O)OCH₃, -NH₂, -NH(CH₃), or -N(CH₃)₂, preferably C₁₋₆ hydrocarbyl substituted with 0, 1, 2, or 3 OH groups, and more preferably C₁₋₆ hydrocarbyl substituted with 1 OH group; and
(d) C₁₋₆ hydrocarbyl, wherein the C₁₋₆ hydrocarbyl may be optionally substituted with 1, 2, or 3 of the following groups: 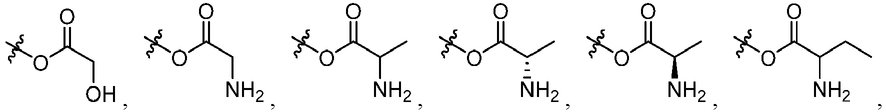 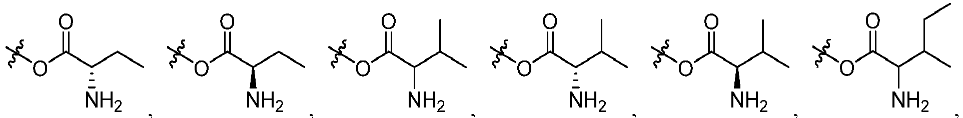 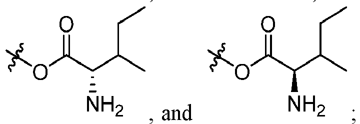 and the C₁₋₆ hydrocarbyl may be optionally substituted with 0, 1, 2, or 3 of the following groups: F, Cl, Br, -OH, and -OCH₃.

In another preferred embodiment, in general formula (1), the group -Z-R¹⁰ is -N=S(=O)-(R¹⁰)₂, wherein two R¹⁰ may be combined with the sulfur atom to which they are each connected to form a saturated or partially saturated 3-, 4-, 5-, or 6-membered monocyclic ring containing 0, 1, 2, or 3 N atoms and 0, 1 or 2 atoms selected from O and S; preferably, the group -Z-R¹⁰ is selected from:

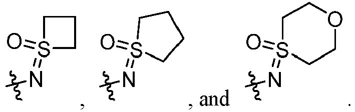

In another preferred embodiment, in general formula (1), R¹ is a group -Z-R¹⁰, wherein Z is -NHSO₂- or - SO₂NH-; and R¹⁰ is oxetanyl or cyclopropyl, or R¹⁰ is C₁₋₆ hydrocarbyl substituted with 0, 1, 2, or 3 OH groups; or R¹⁰ is C₁₋₆ hydrocarbyl, wherein the C₁₋₆ hydrocarbyl may be optionally substituted with 1, 2, or 3 of the following groups:

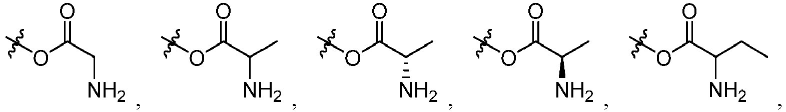

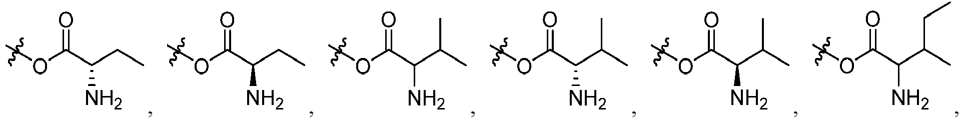

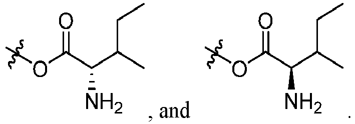

In another preferred embodiment, in general formula (1), R¹⁰ is selected from C₁₋₆ hydrocarbyl, wherein the hydrocarbyl may be optionally substituted with 0, 1, 2, or 3 of the following groups: 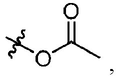 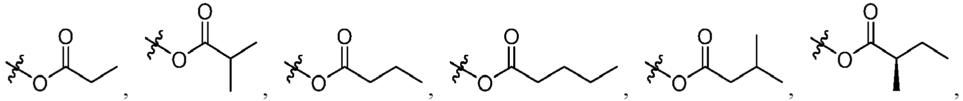 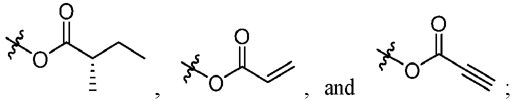 the hydrocarbyl is preferably substituted with 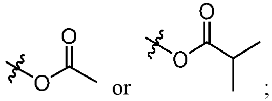 Z is -NHSO₂- or -SO₂NH-; Z is preferably -NHSO₂-.

In another preferred embodiment, in general formula (1), R¹⁰ is selected from C₁₋₆ hydrocarbyl, wherein the hydrocarbyl may be optionally substituted with 1, 2, or 3 of the following groups: 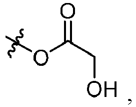 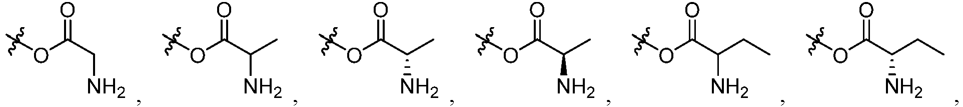 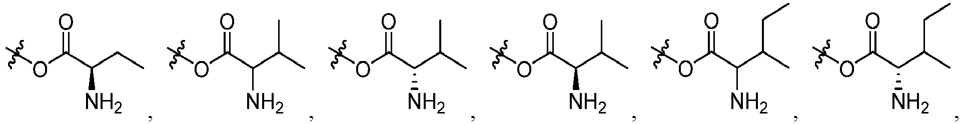 and 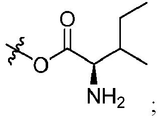 Z is -NHSO₂- or -SO₂NH-.

In another preferred embodiment, in general formula (1), R² is halogen or a group -Y-R¹², wherein Y is a chemical bond, -NH-, -NH-(CH₂)₀₋₄-, or -O-(CH₂)₀₋₄-; and R¹² is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 of the following groups: F, Cl, Br, C₁₋₆ hydrocarbyl, C₁₋₄ halohydrocarbyl, -OH, -OC₁₋₄ halohydrocarbyl, CN, R¹⁴, and oxo; or R¹² is C₁₋₆ hydrocarbyl, wherein the hydrocarbyl may be optionally substituted with 0, 1, 2, 3, 4, or 5 of the following groups: F, Cl, Br, -OH, -OC₁₋₄ halohydrocarbyl, and CN.

In another preferred embodiment, in general formula (1), R² is a saturated 5- or 6-membered monocyclic ring, wherein the ring contains 0, 1 or 2 N atoms and 0 or 1 O atom, and the ring is substituted with 0, 1, 2, or 3 groups selected from: F, Cl, Br, C₁₋₆ hydrocarbyl, C₁₋₄ halohydrocarbyl, -OH, -OC₁₋₄ halohydrocarbyl, CN, R¹⁴, and oxo.

In another preferred embodiment, in general formula (1), R² is: (a) halogen; (b) a group -Y-R¹², wherein Y is a chemical bond; and R¹² is morpholinyl, piperidinyl, azetidinyl, pyrrolidinyl, cyclopropyl, cyclobutyl, cyclopentyl, cyclohexyl, piperazinyl, tetrahydrofuranyl, 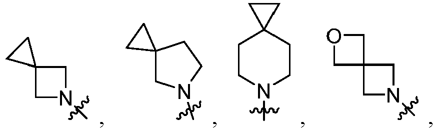 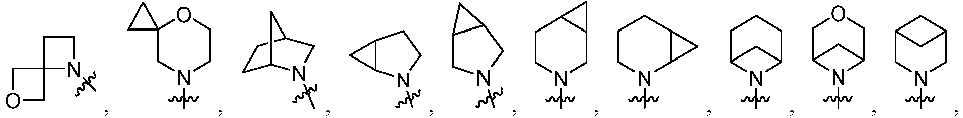 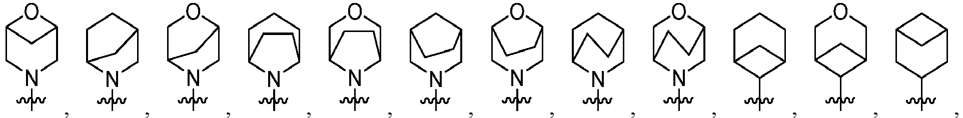 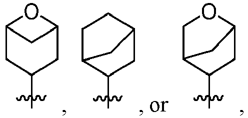 wherein each of the rings is substituted with 0, 1, 2, or 3 groups selected from: F, Cl, Br, methyl, CF₃, -OH, -OCHF₂, CN, and oxo; or (c) a group -Y-R¹², wherein Y is -NH-, -O-, -O-(CH₂)-, - O-(CH₂)-(CH₂)-, or -O-(CH₂)-(CH₂)-(CH₂)-, and R¹² is 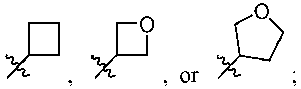 or R¹² is C₁₋₆ hydrocarbyl, wherein the hydrocarbyl may be optionally substituted with 0, 1, 2, 3, 4, or 5 of the following groups: F, Cl, Br, methyl, CF₃, -OH, and CN.

In another preferred embodiment, in general formula (1), R² is morpholinyl or piperidinyl, wherein the morpholinyl and piperidinyl may be optionally substituted with 0, 1, 2, or 3 of the following groups: F, Cl, Br, methyl, CF₃, -OH, -OCHF₂, and CN.

In another preferred embodiment, in general formula (1), R² is piperidinyl substituted with 1, 2, or 3 fluorine groups.

In another preferred embodiment, in general formula (1), R² is:

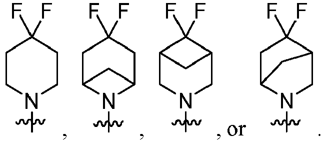

In another preferred embodiment, in general formula (1), R² is morpholinyl substituted with 1, 2, or 3 methyl groups.

In another preferred embodiment, in general formula (1), R² is

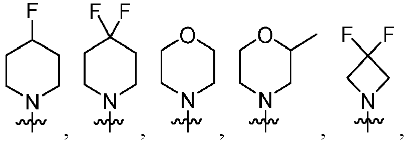

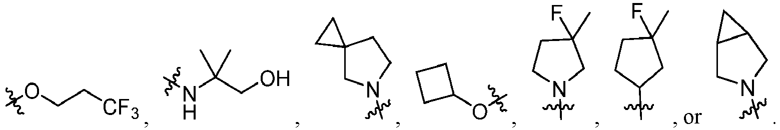

In another preferred embodiment, in general formula (1), R¹⁰ is selected from cyclopropyl, cyclobutyl, cyclopentyl, oxetanyl, azetidinyl, tetrahydrofuranyl, and 1,3,4-oxathiazinanyl.

In another preferred embodiment, in general formula (1), R³ is H.

In another preferred embodiment, in general formula (1), R⁴ is selected from: (a) H; (b) C₁₋₆ hydrocarbyl substituted with 0, 1, 2, or 3 OH groups; (c) cyclopropyl; and (d) F; R⁴ is preferably H, F, or methyl; R⁴ is more preferably H.

In another preferred embodiment, in general formula (1), R⁵ is H or F, preferably H.

In another preferred embodiment, in general formula (1), R⁶ is H or F, preferably H.

In another preferred embodiment, in general formula (1), R⁷ is H.

In another preferred embodiment, in general formula (1), R¹⁵ is H or F, preferably H.

In another preferred embodiment, general formula (1) has the following structure: 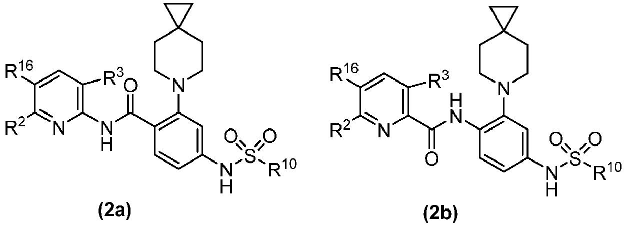 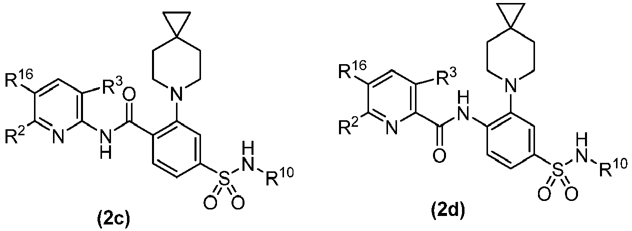 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂, wherein R², R³, R¹⁰, R²⁰, and R²¹ are defined as previously described and exemplified in specific examples.

In another preferred embodiment, general formula (1) has the following structure: 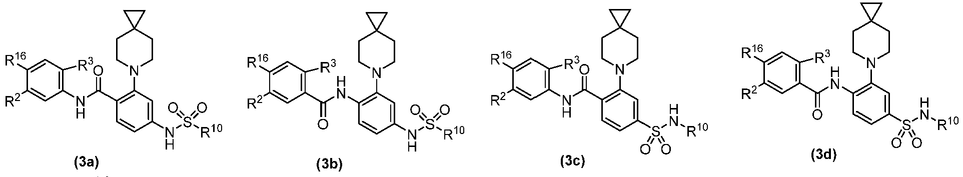 wherein R¹⁶ is -C₃₋₆ cycloalkyl, -OH, -O-C₁₋₄ halohydrocarbyl, -O-C₃₋₆ cycloalkyl, -O-C₃₋₆ halocycloalkyl, - SH, -S-C₁₋₆ hydrocarbyl, -S-C₁₋₄ halohydrocarbyl, -S-C₃₋₆ cycloalkyl, -S-C₃₋₆ halocycloalkyl, -NR¹⁸R¹⁹, or - NO₂, wherein R², R³, R¹⁰, R¹⁸, and R¹⁹ are defined as previously described and exemplified in specific examples.

In another preferred embodiment general formula (1) has the following structure: 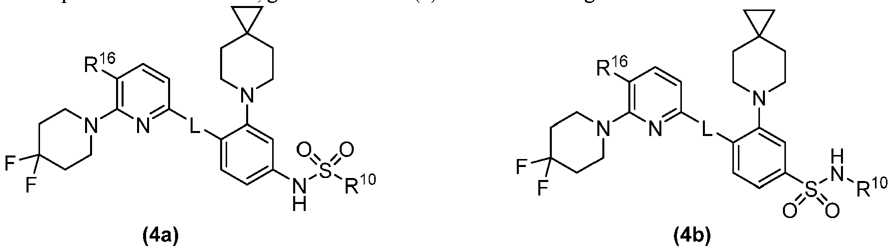 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂, wherein L, R¹⁰, R²⁰, and R²¹ are defined as previously described and exemplified in specific examples.

In another preferred embodiment, general formula (1) has the following structure: 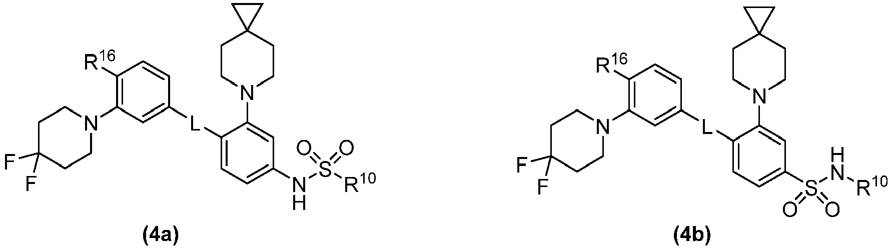 wherein R¹⁶ is -C₃₋₆ cycloalkyl, -OH, -O-C₁₋₄ halohydrocarbyl, -O-C₃₋₆ cycloalkyl, -O-C₃₋₆ halocycloalkyl, - SH, -S-C₁₋₆ hydrocarbyl, -S-C₁₋₄ halohydrocarbyl, -S-C₃₋₆ cycloalkyl, -S-C₃₋₆ halocycloalkyl, -NR¹⁸R¹⁹, or - NO₂, wherein L, R¹⁰, R¹⁸, and R¹⁹ are defined as previously described and exemplified in specific examples.

In various different embodiments of the present invention, the compound of general formula (1) has one of the following structures: 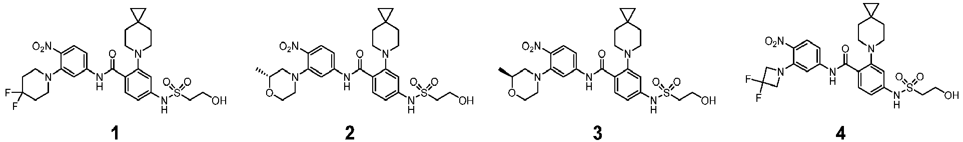 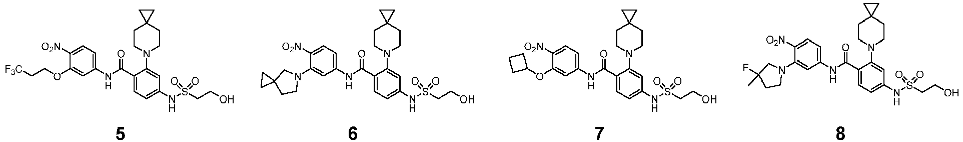 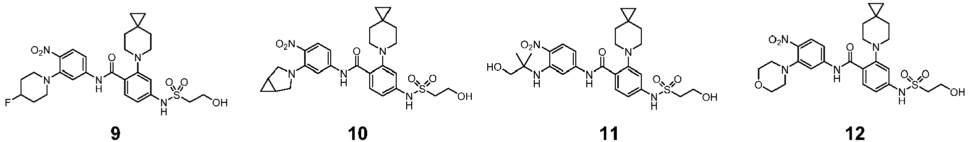 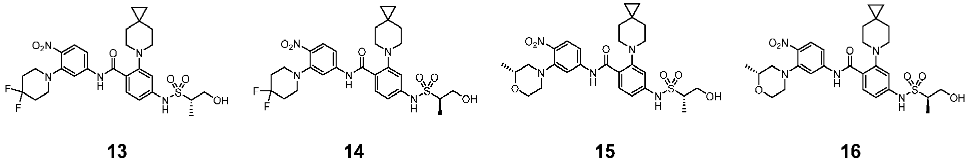 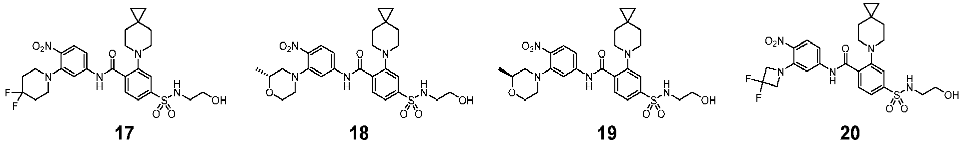 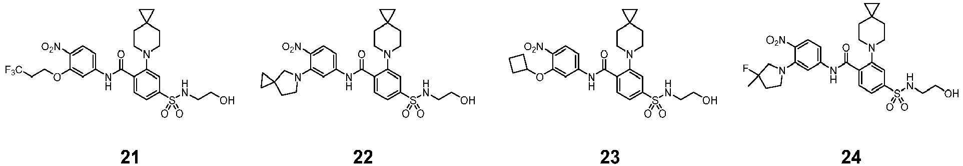 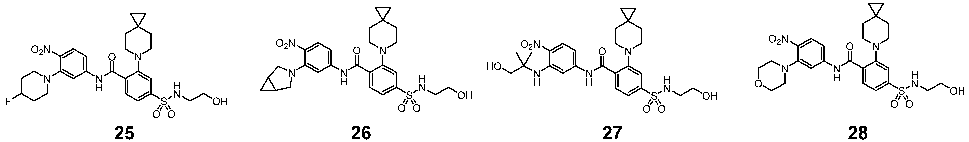 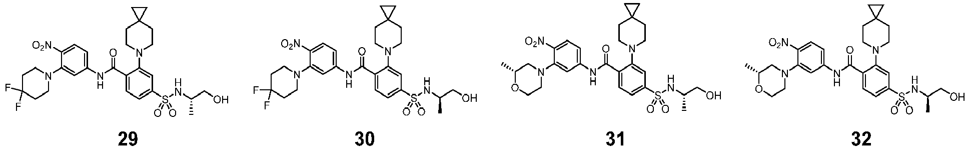 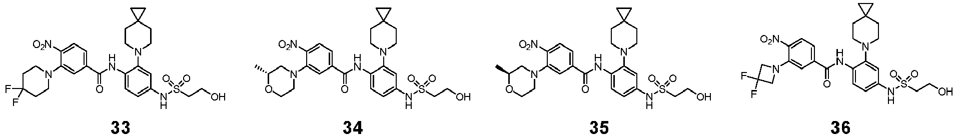 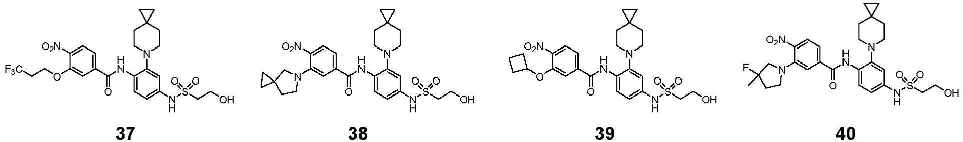 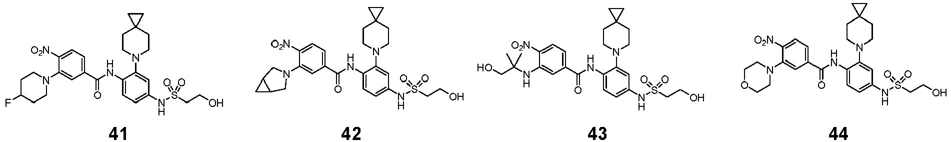 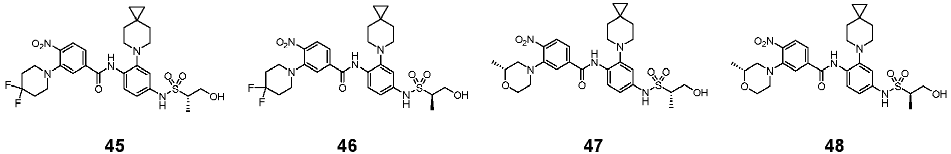 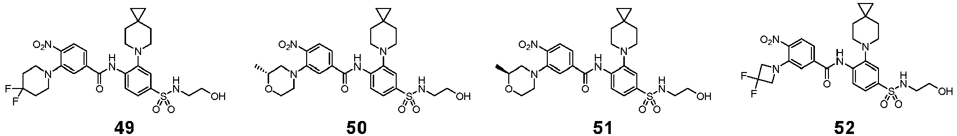 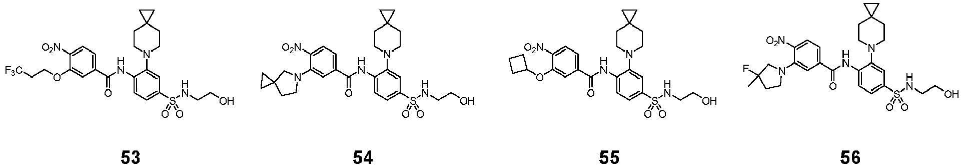 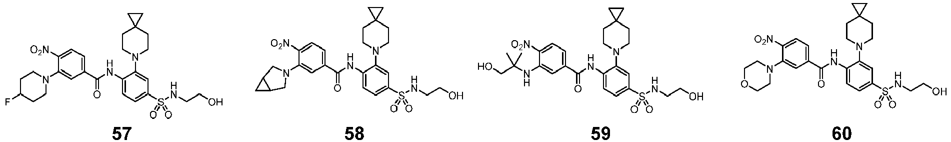 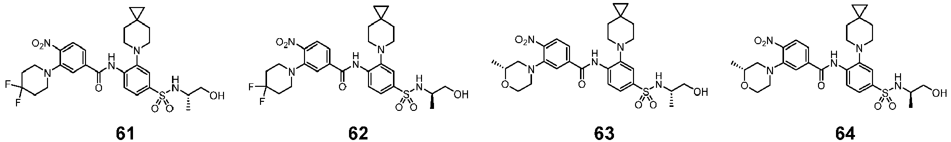 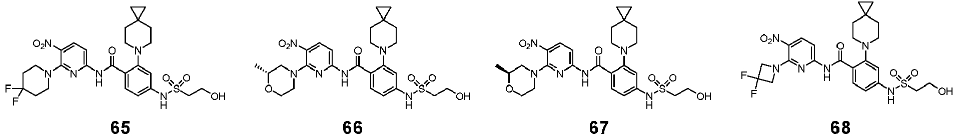 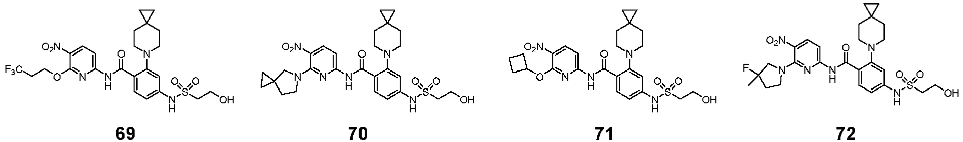 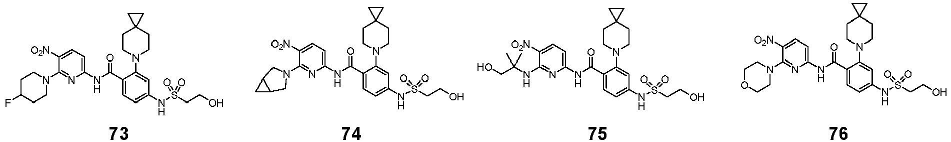 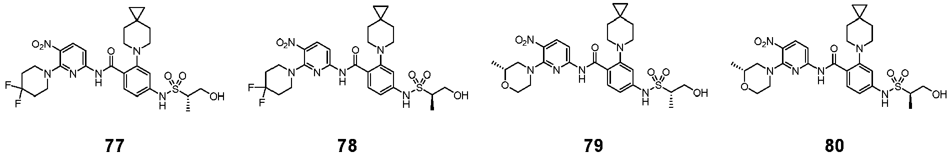 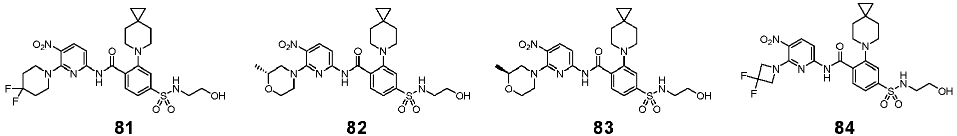 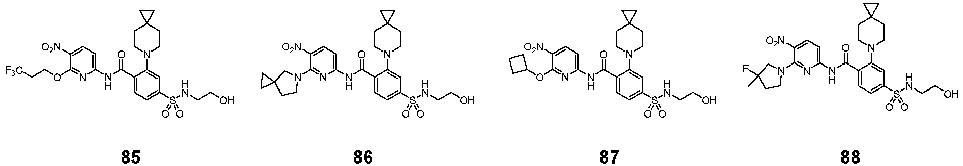 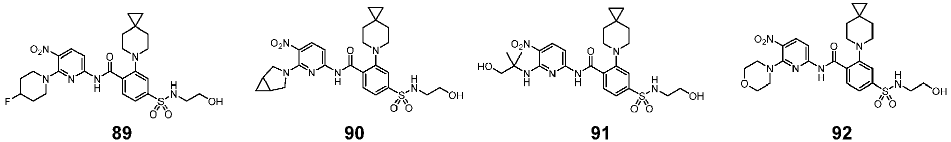 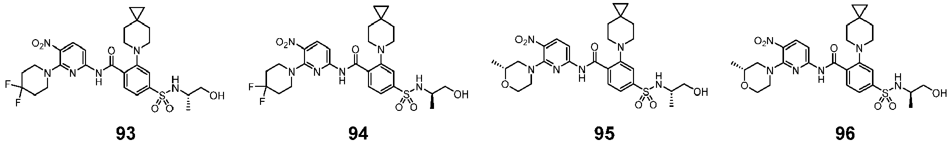 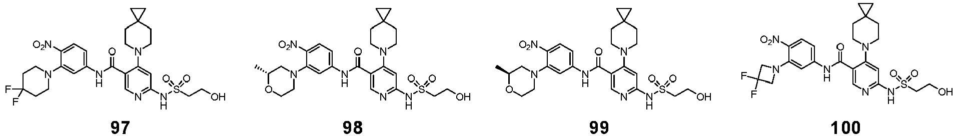 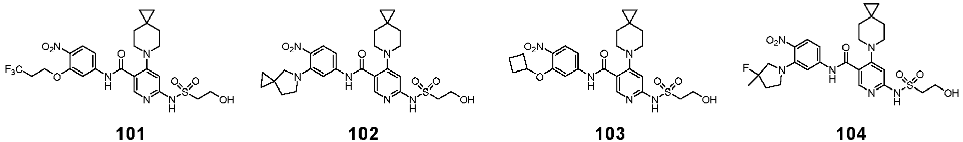 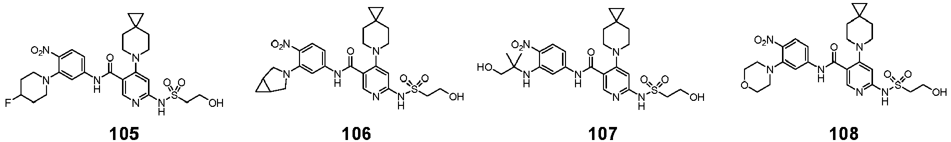 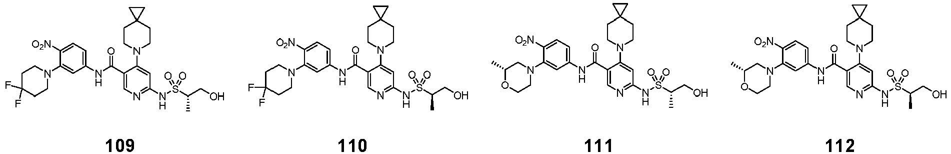 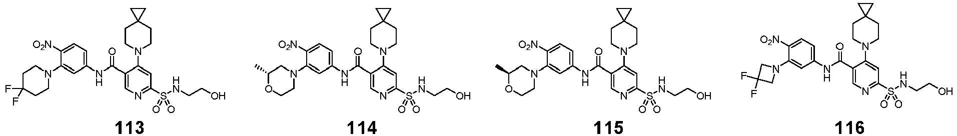 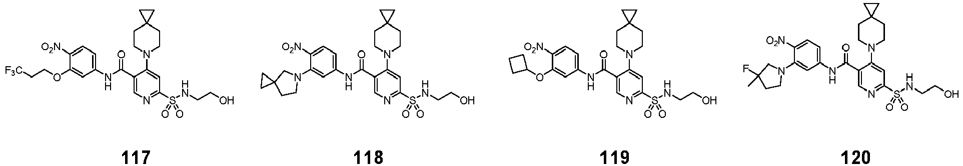 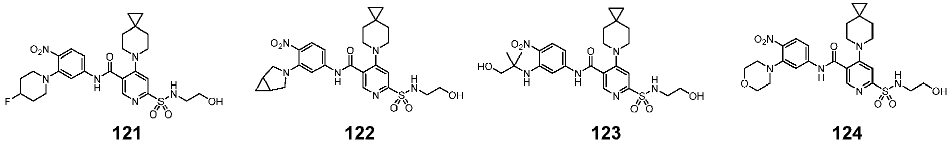 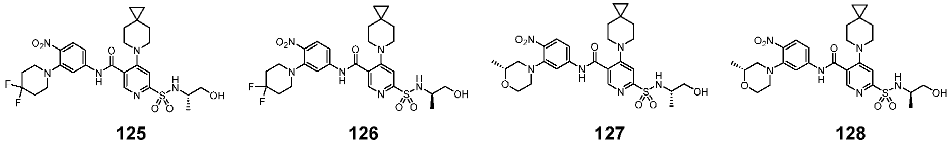 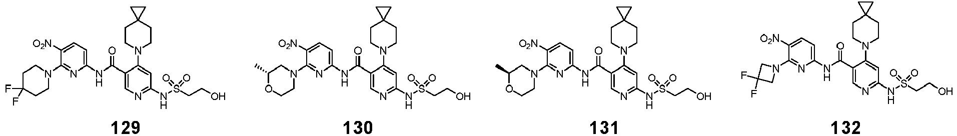 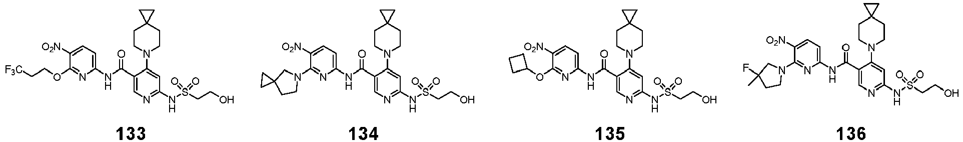 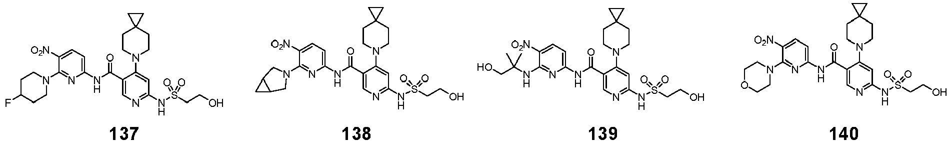 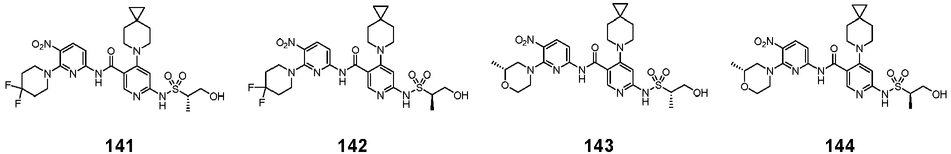 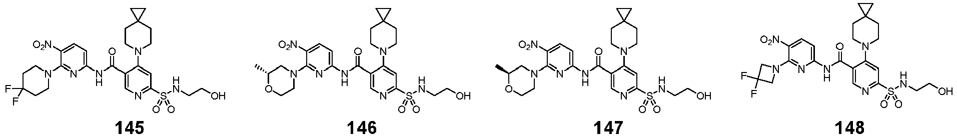 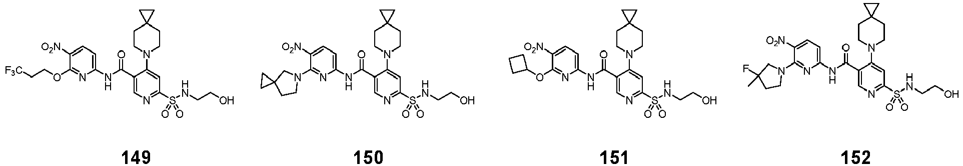 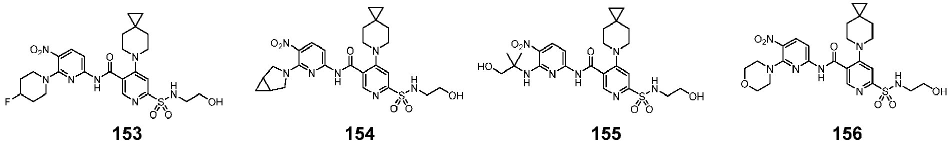 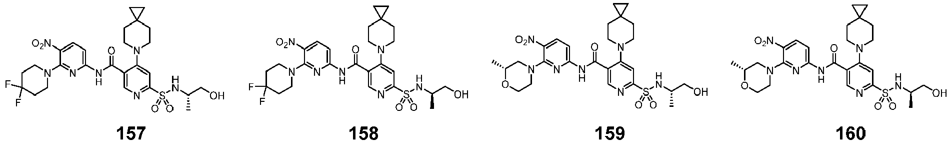 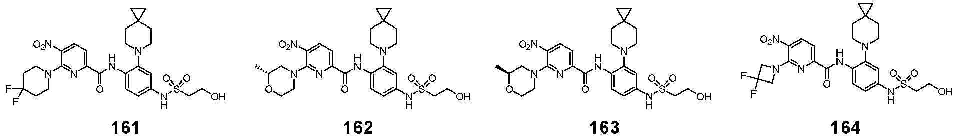 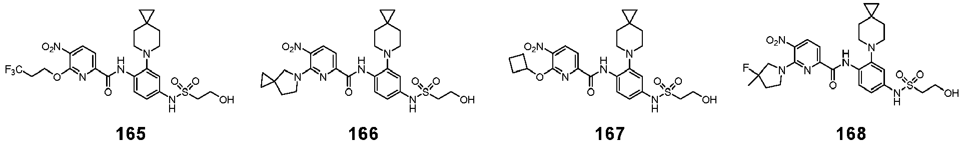 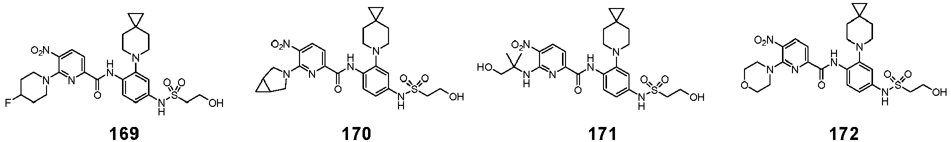 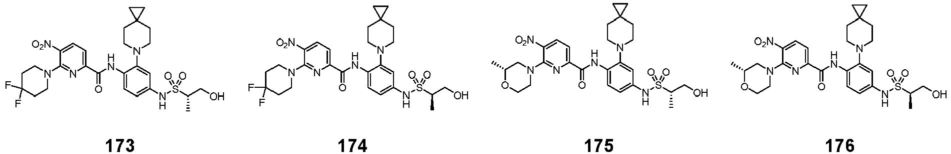 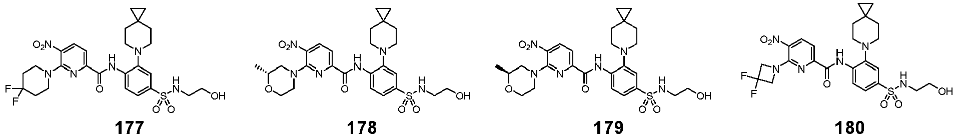 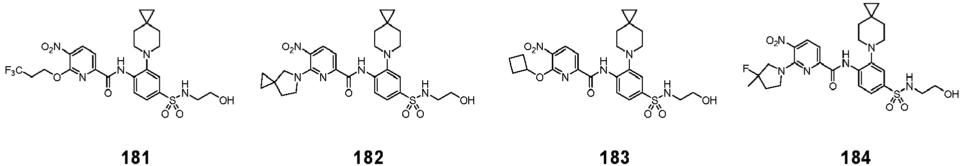 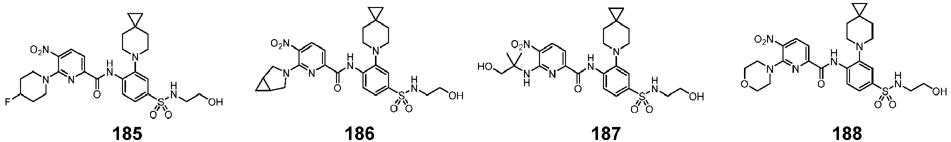 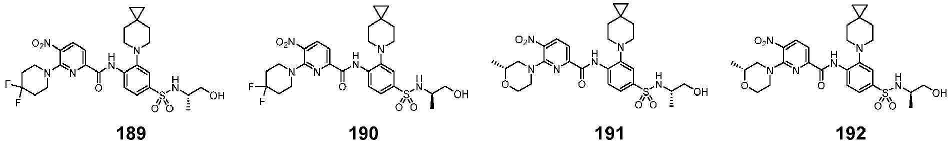 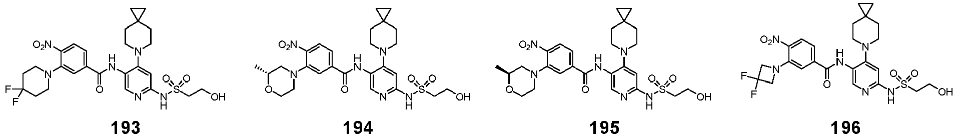 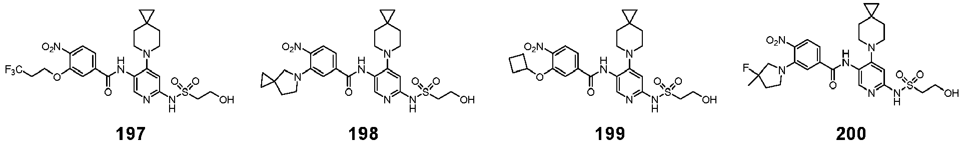 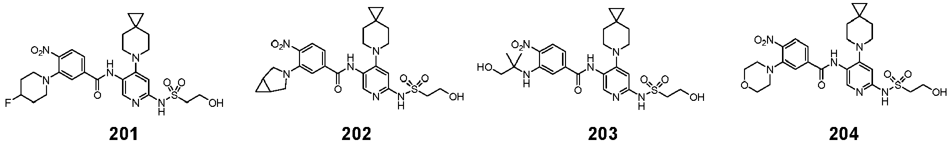 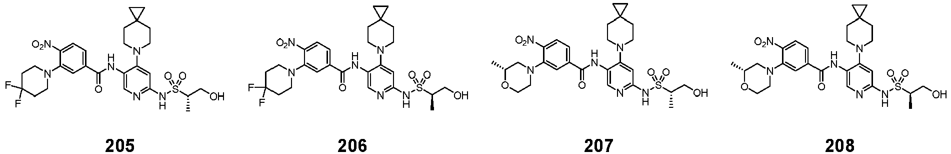 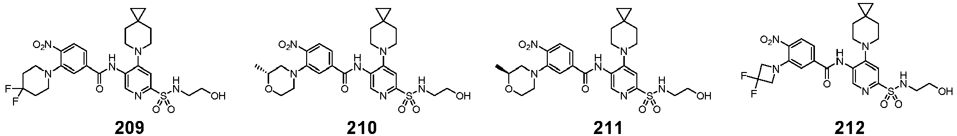 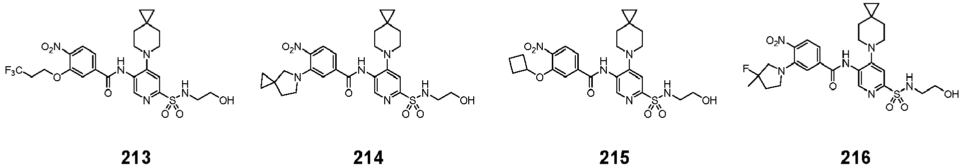 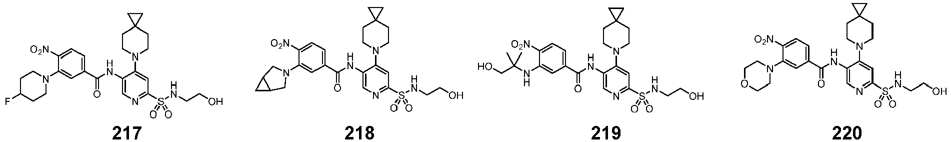 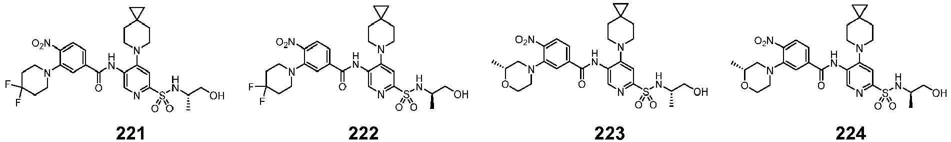 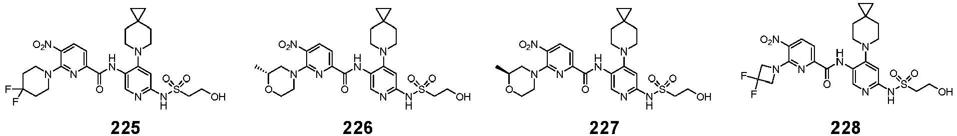 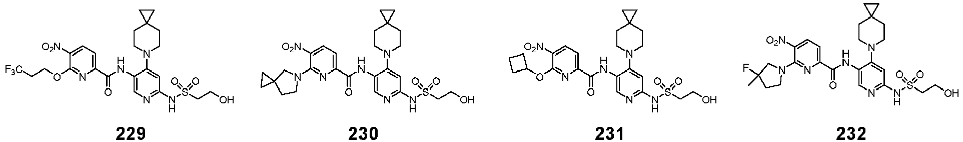 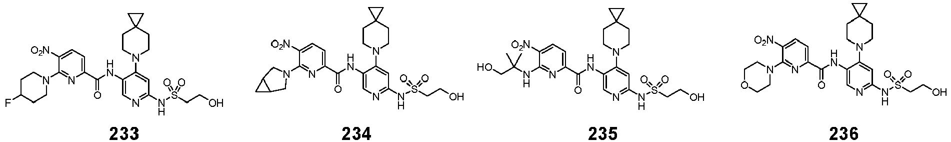 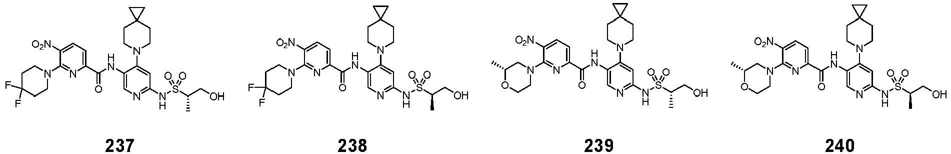 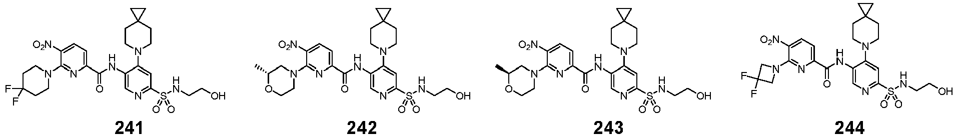 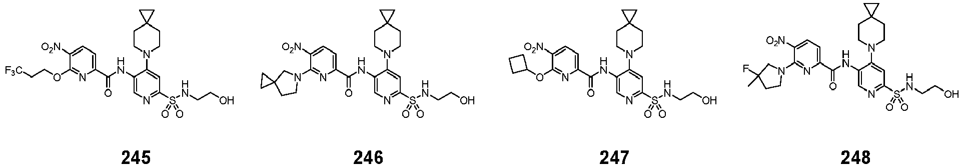 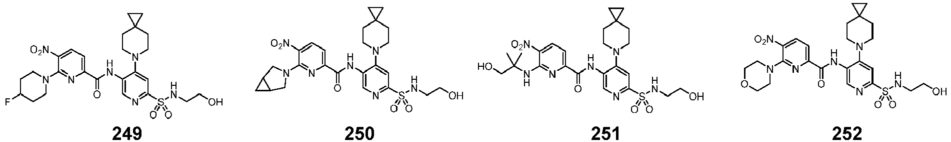 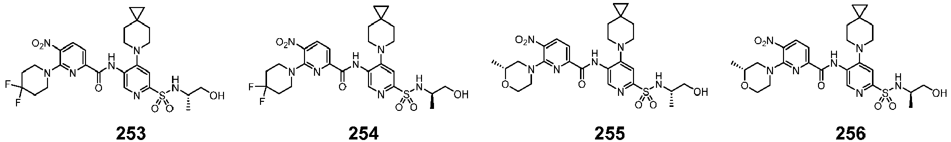 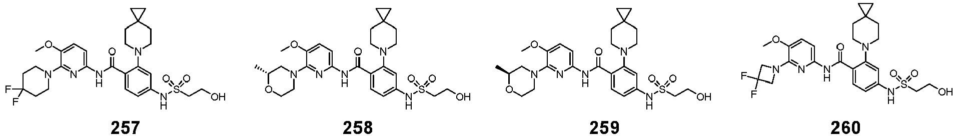 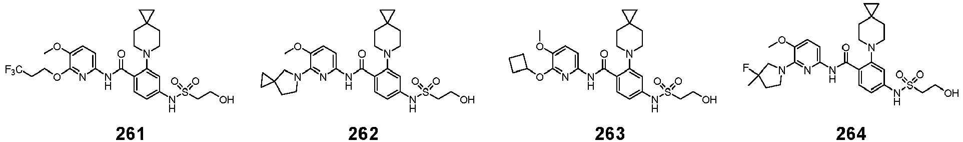 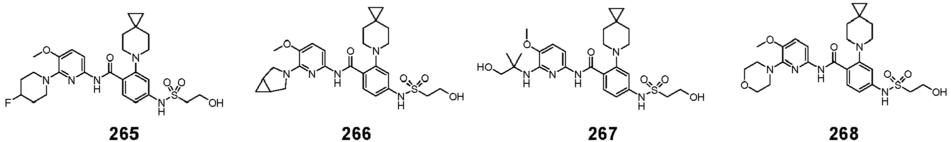 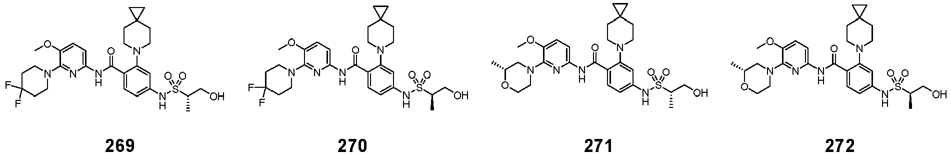 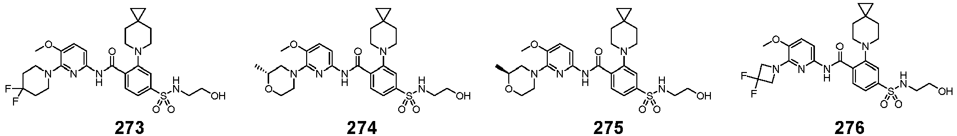 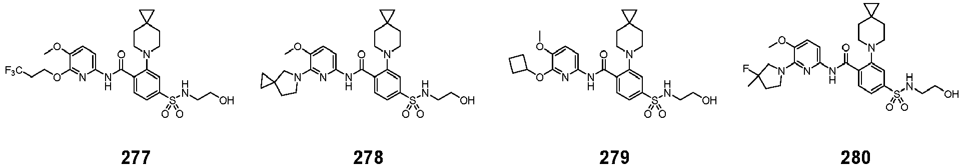 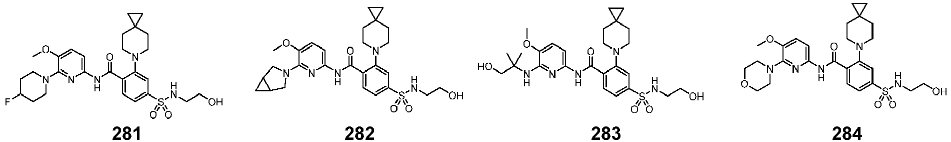 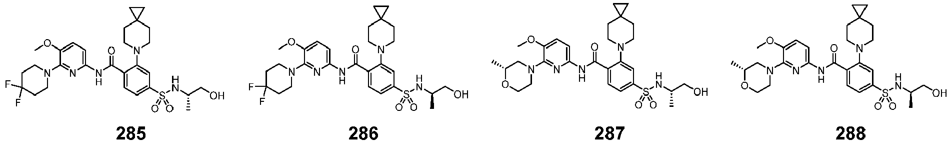 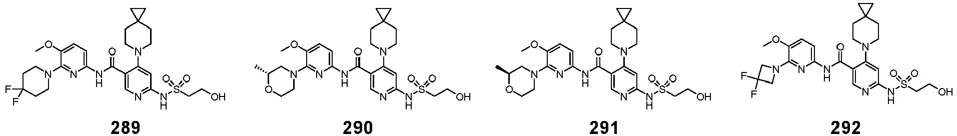 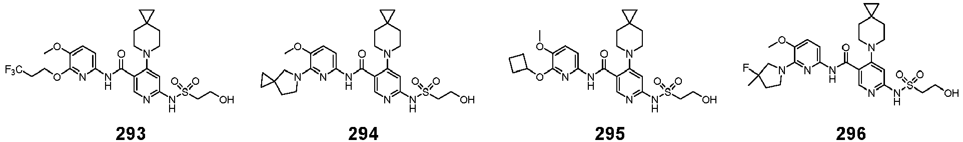 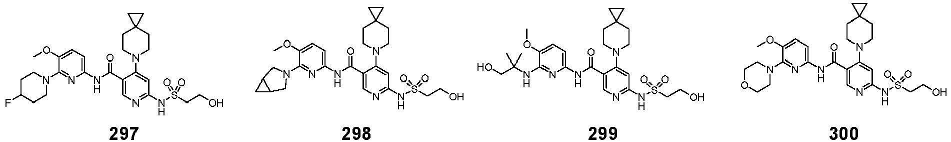 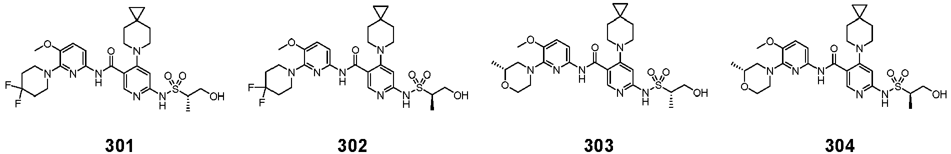 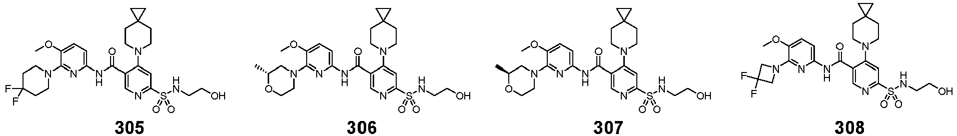 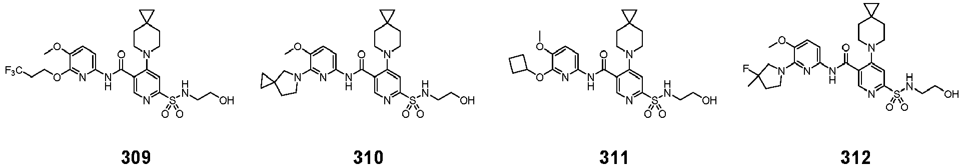 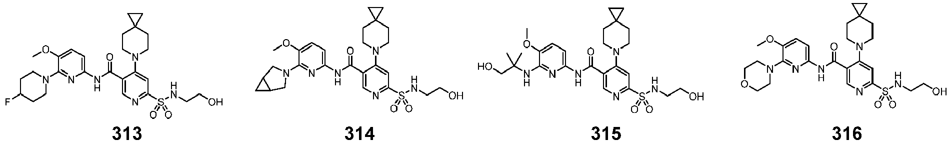 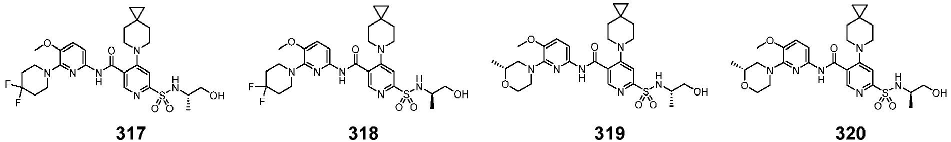 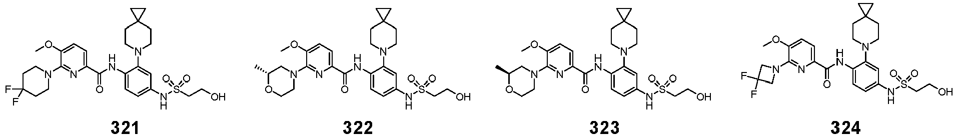 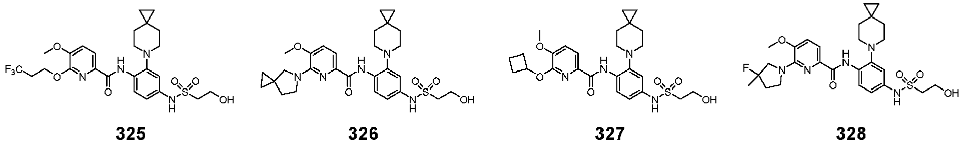 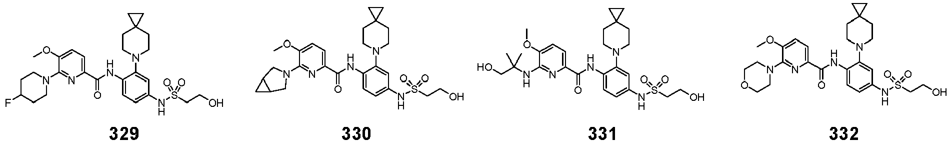 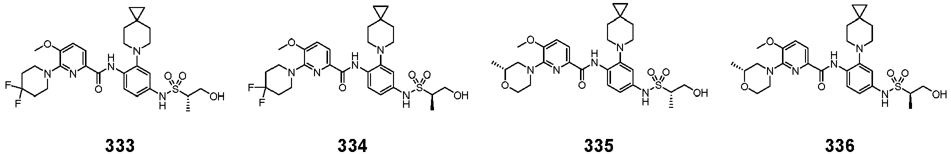 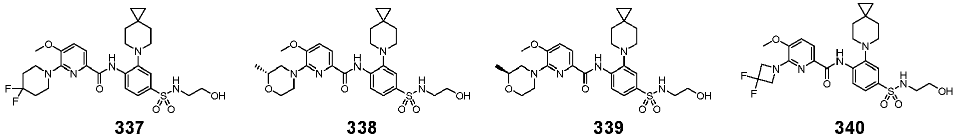 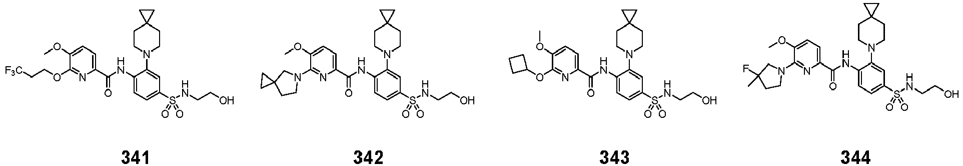 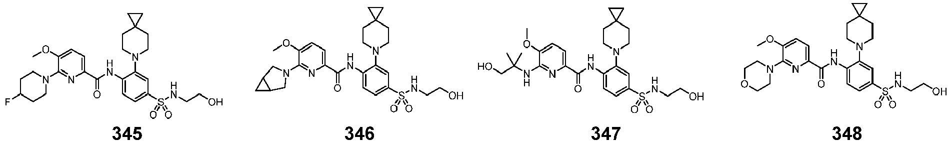 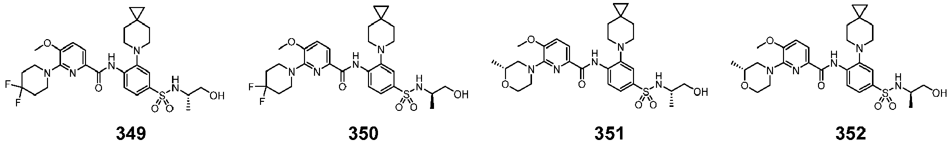 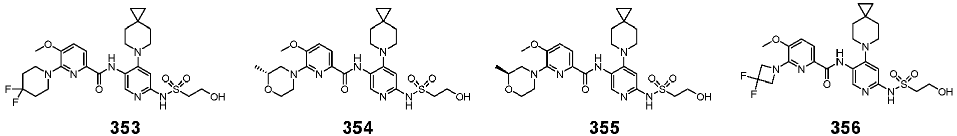 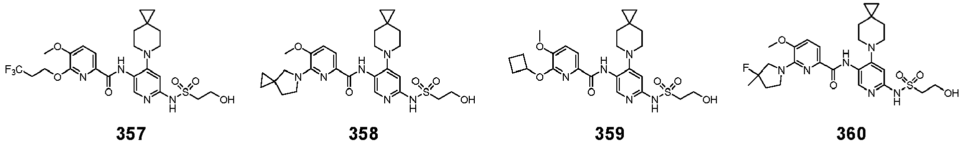 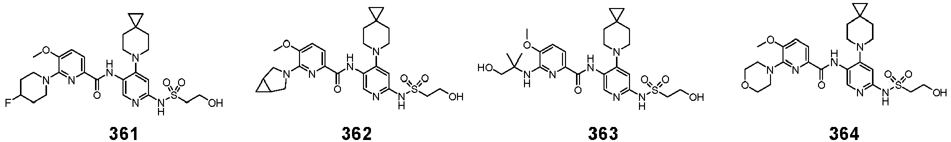 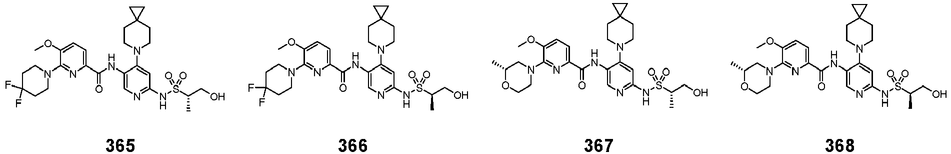 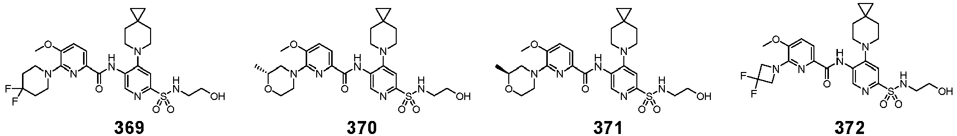 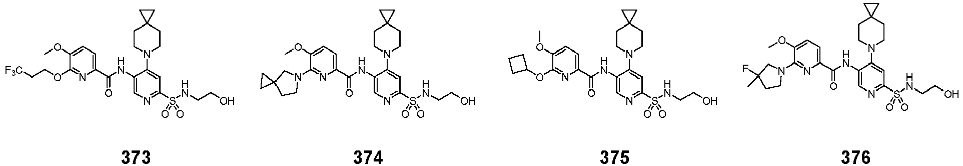 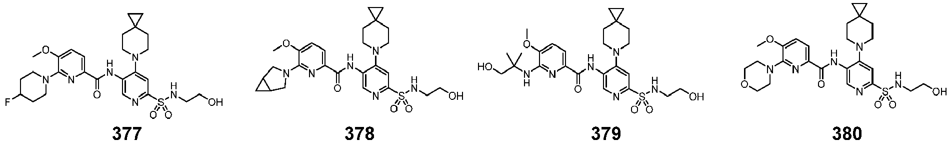 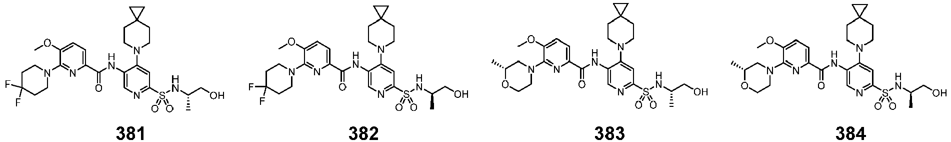 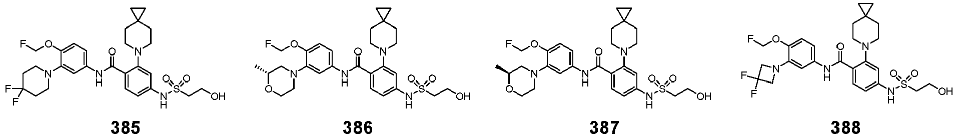 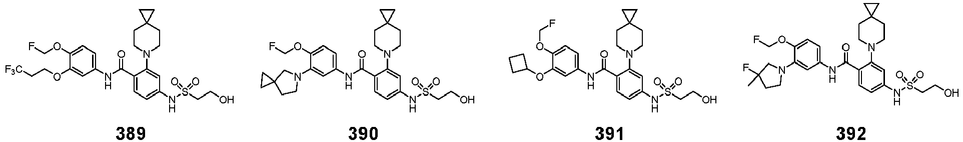 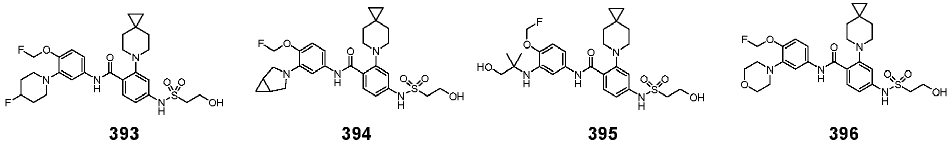 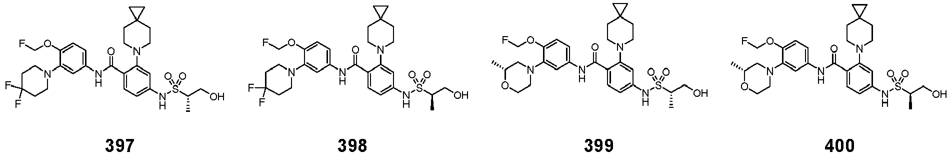 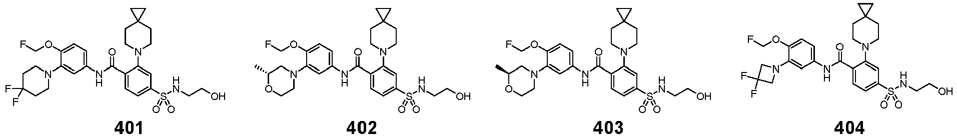 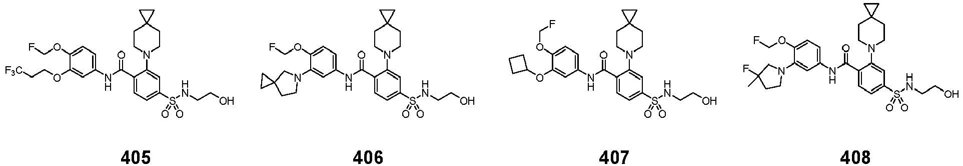 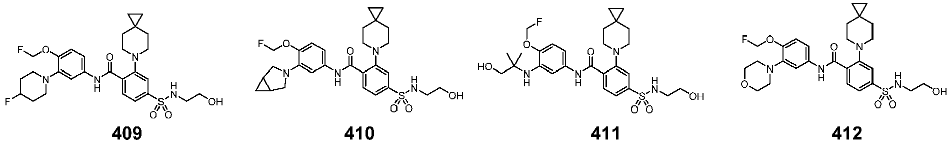 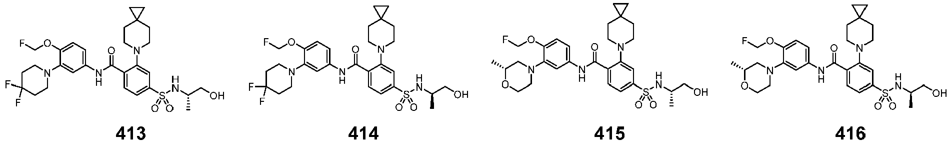 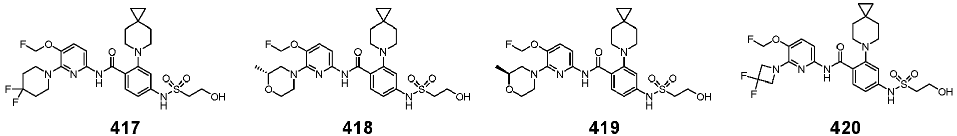 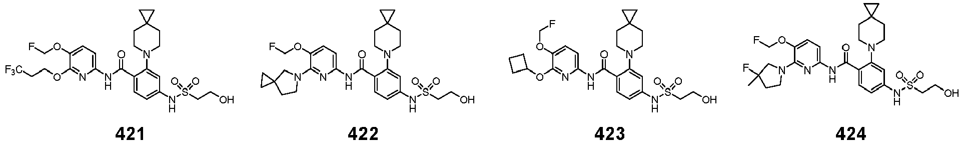 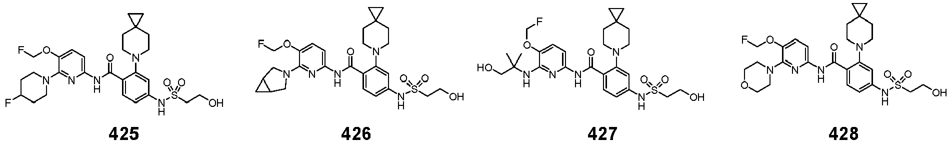 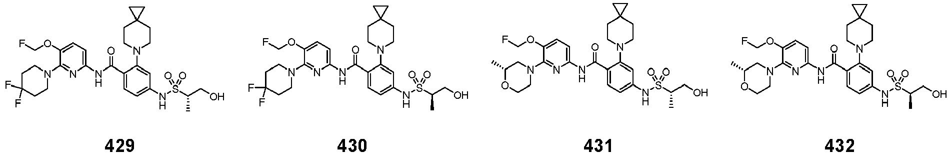 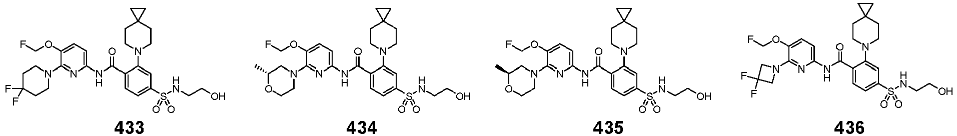 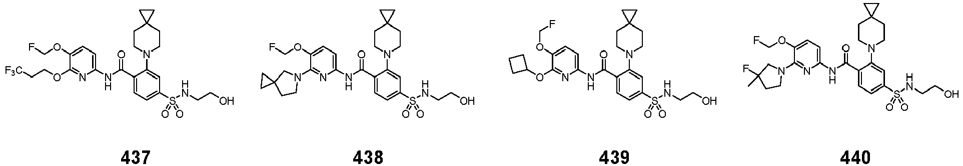 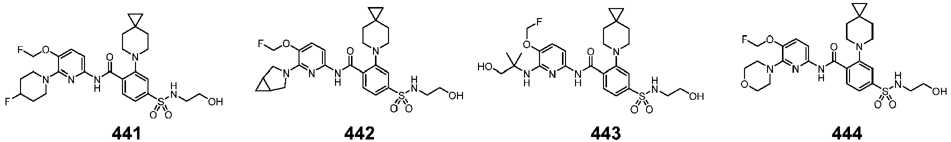 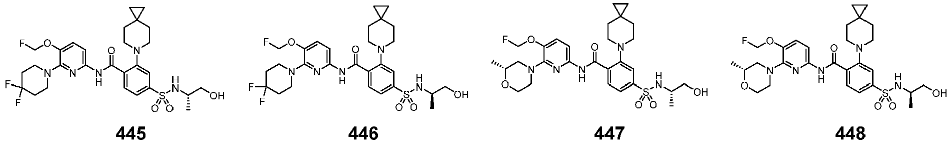 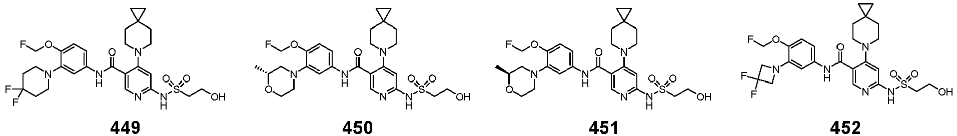 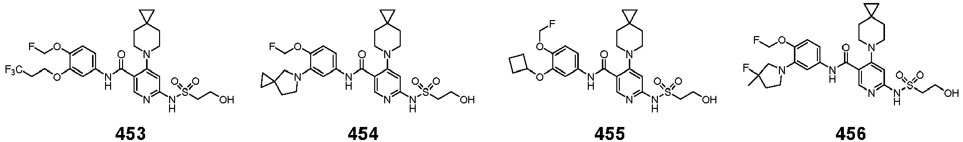 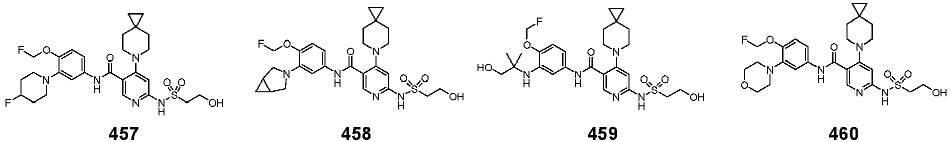 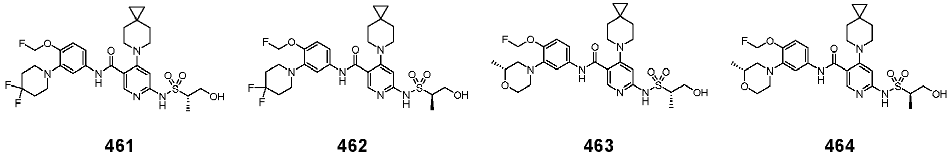 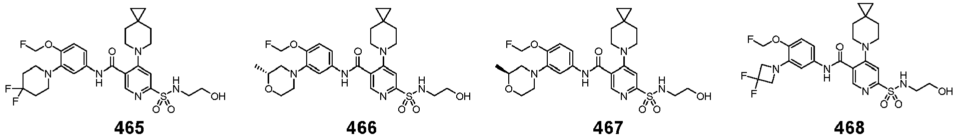 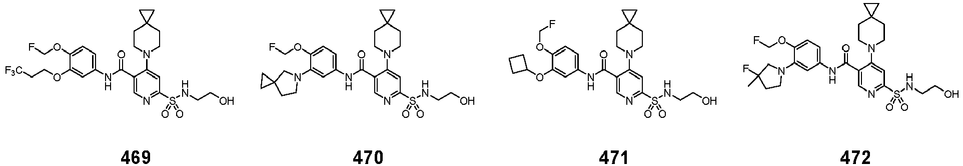 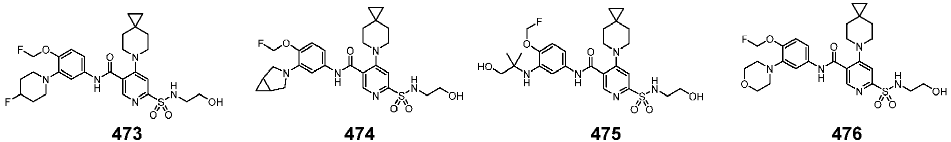 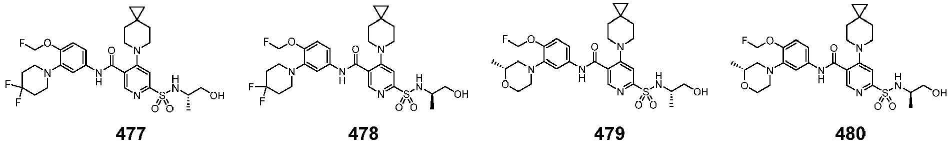 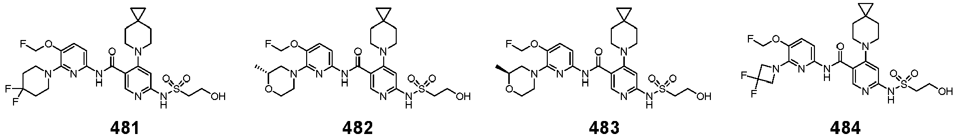 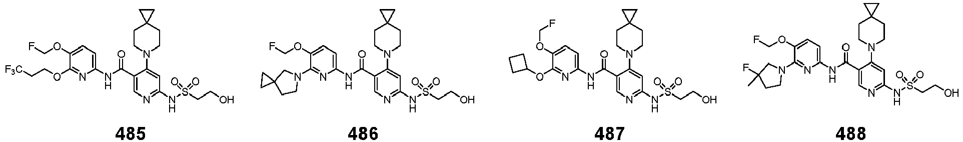 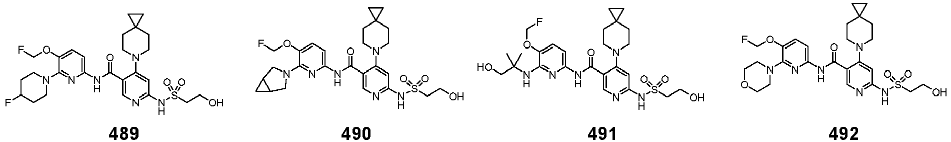 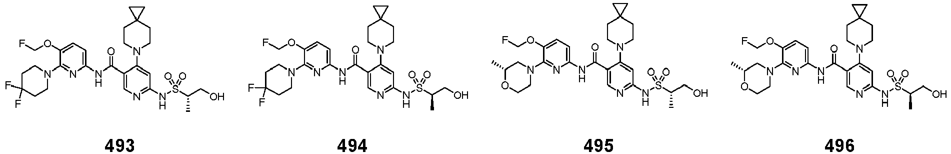 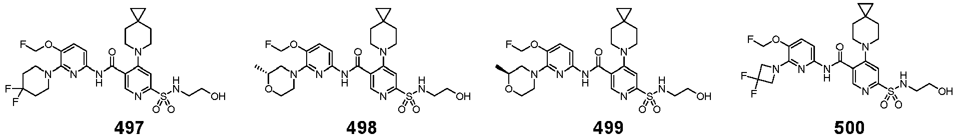 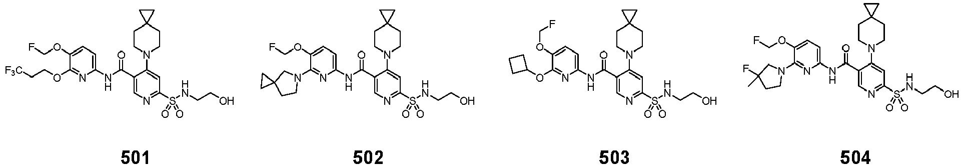 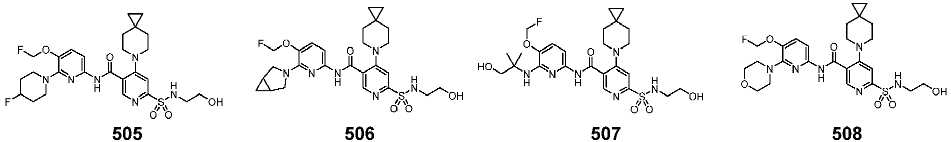 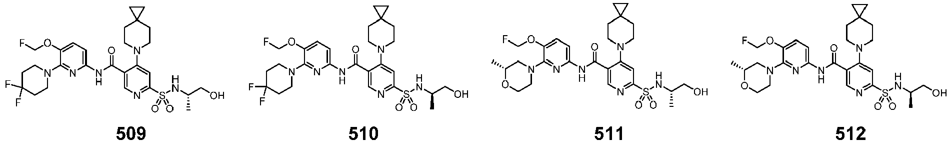 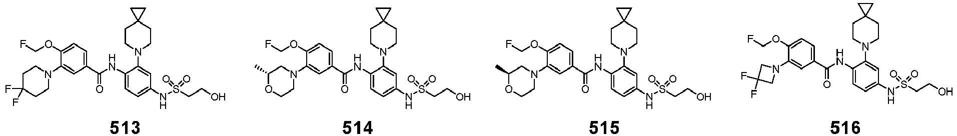 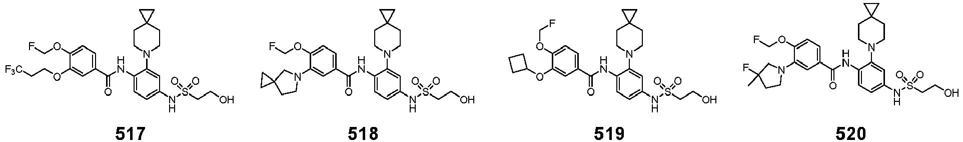 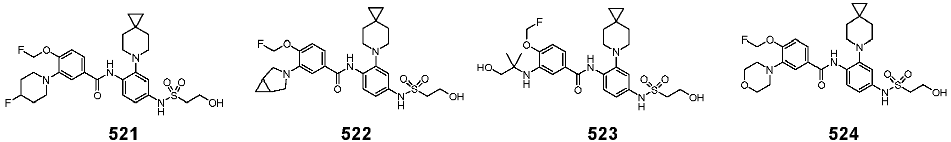 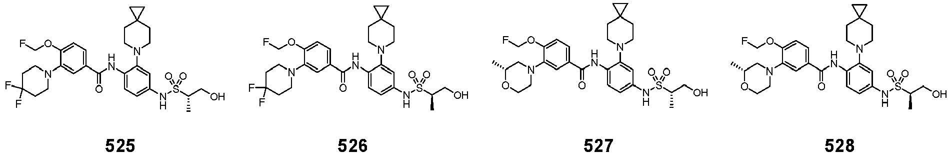 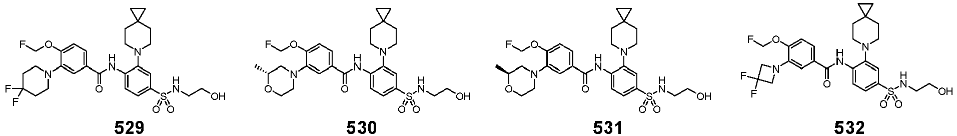 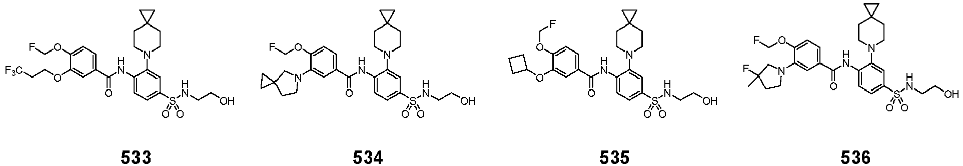 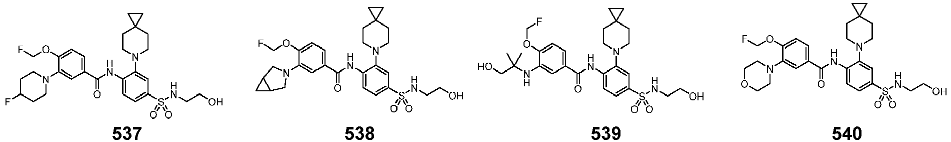 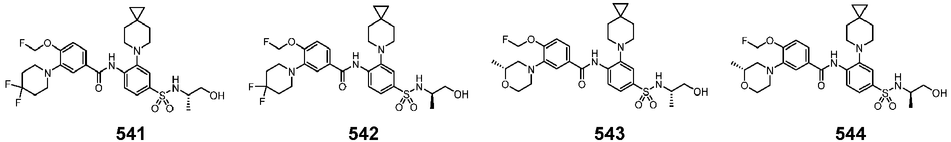 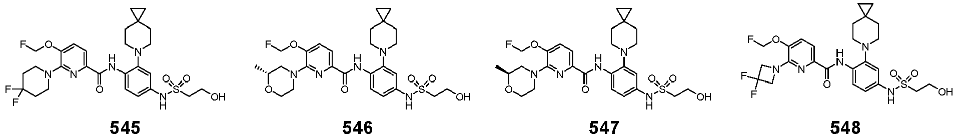 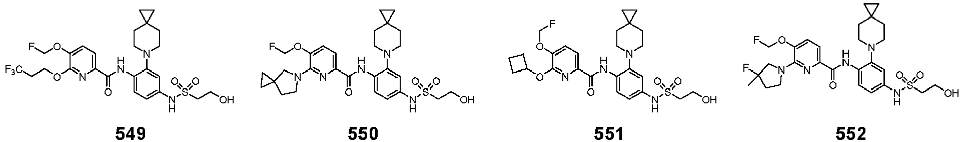 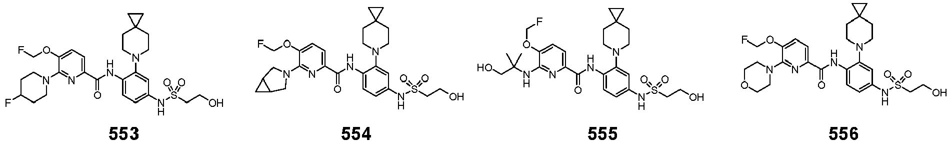 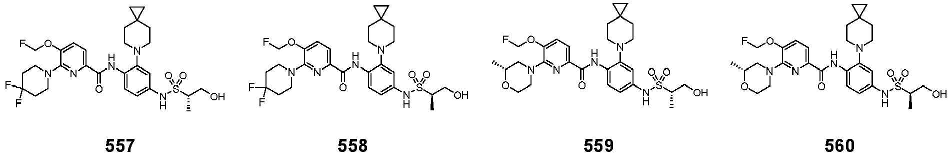 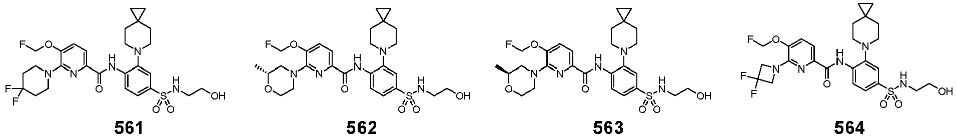 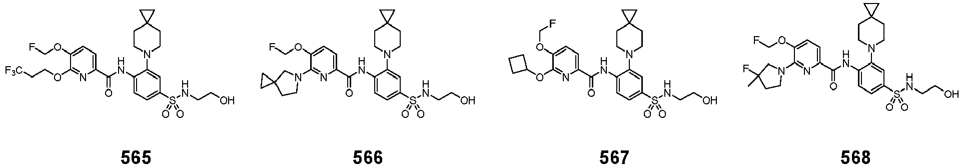 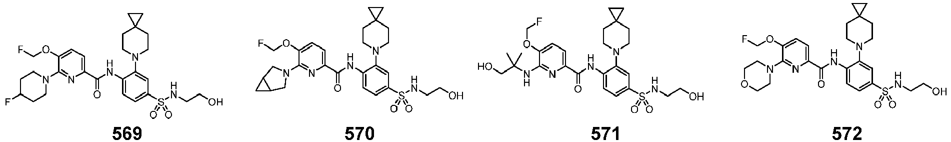 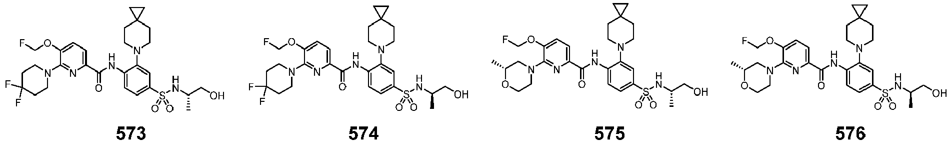 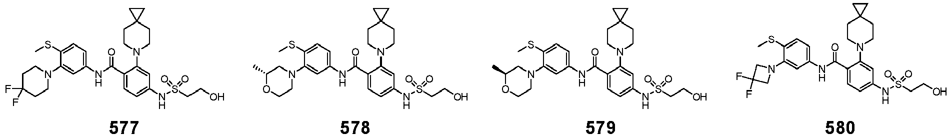 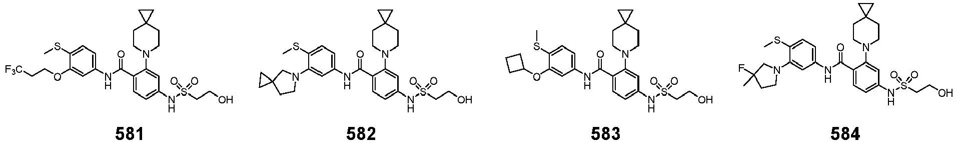 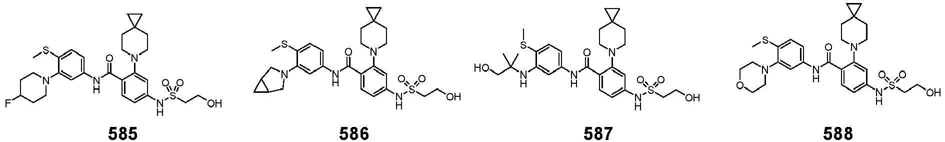 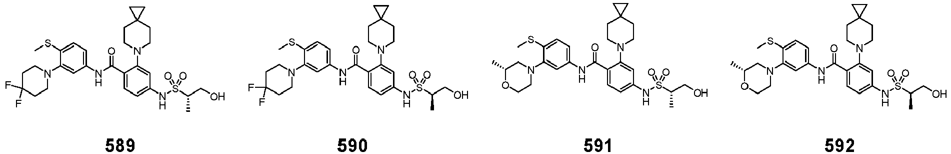 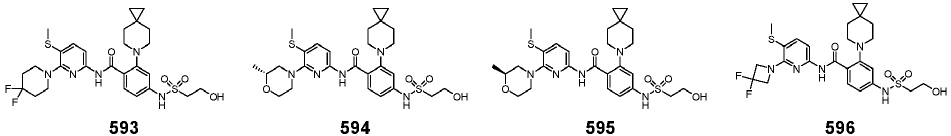 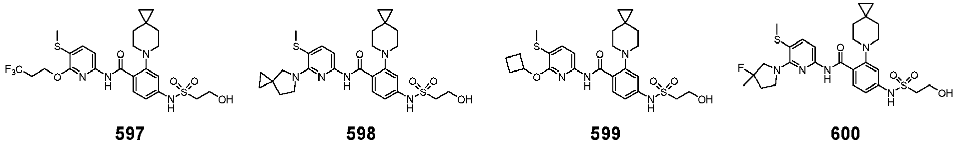 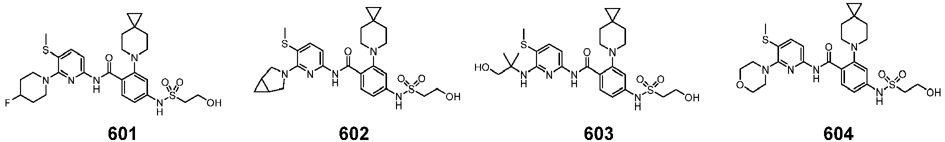 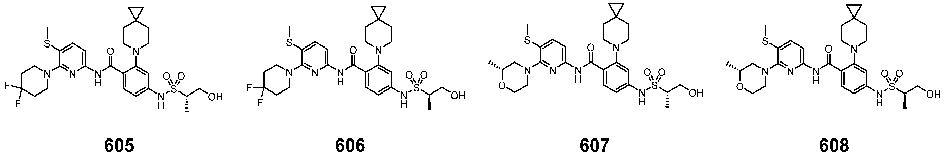 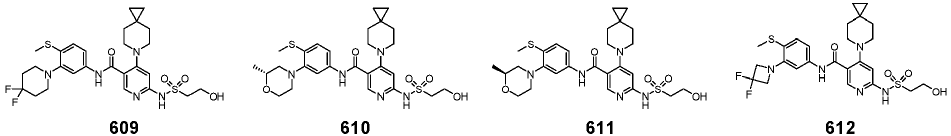 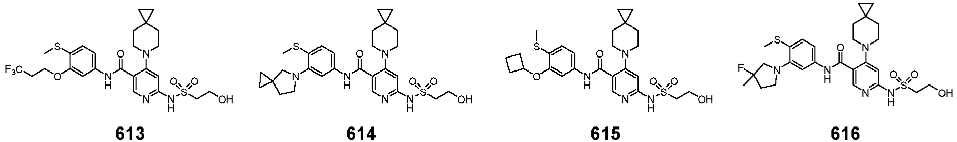 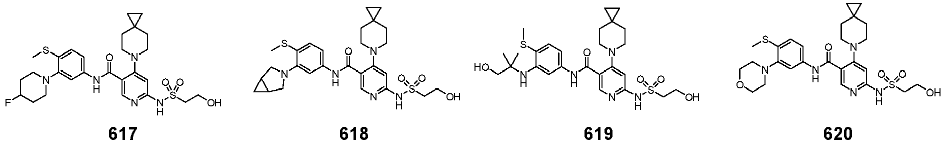 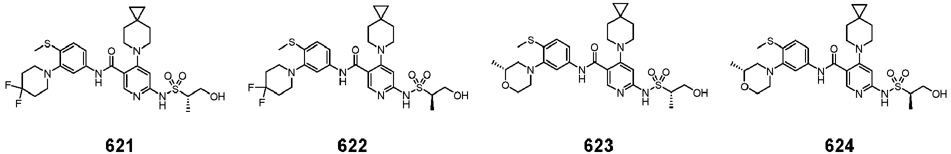 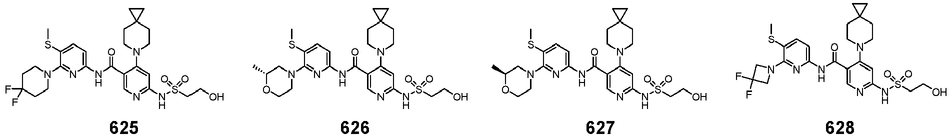 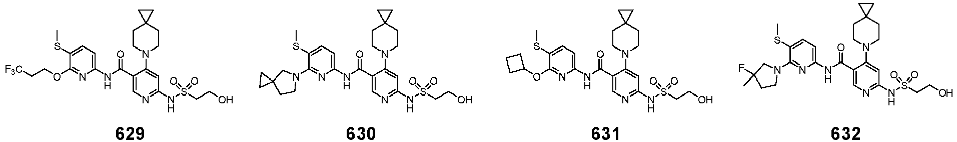 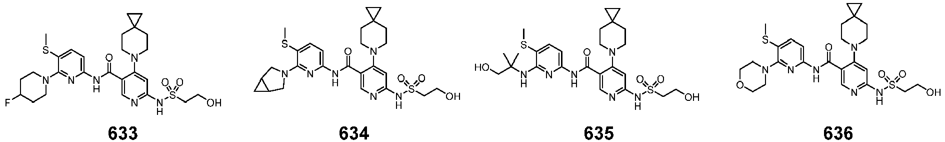 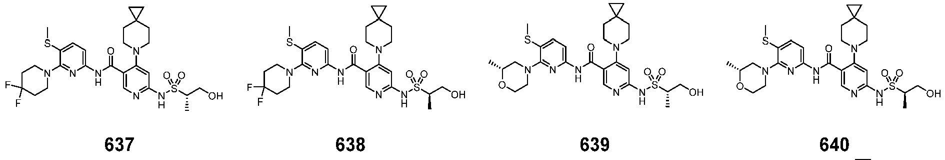 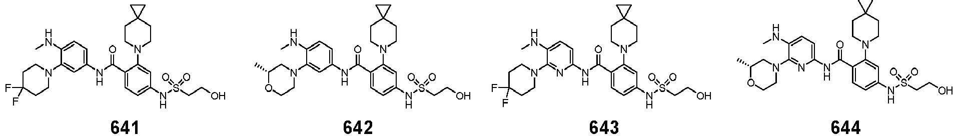 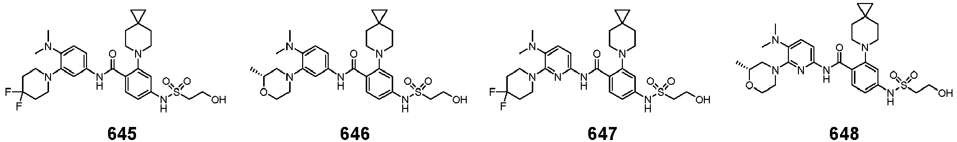 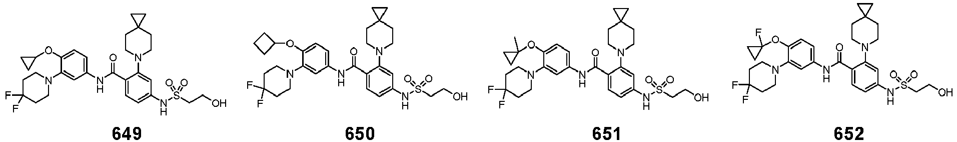 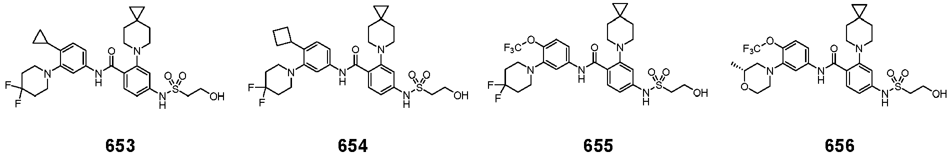 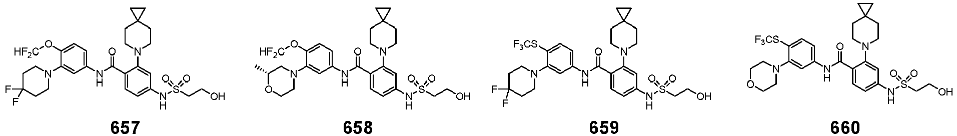 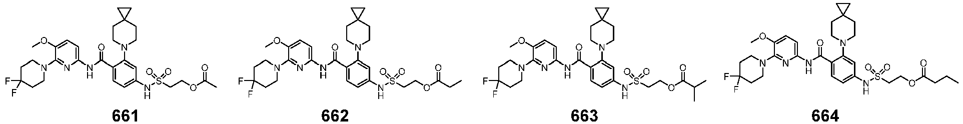 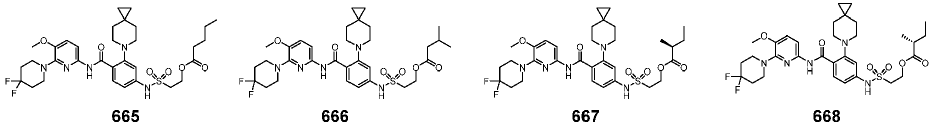 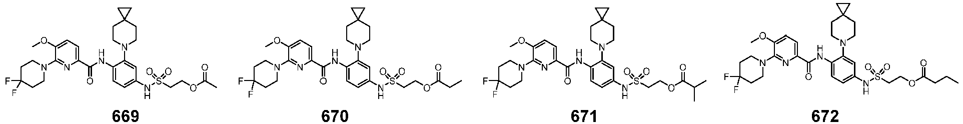 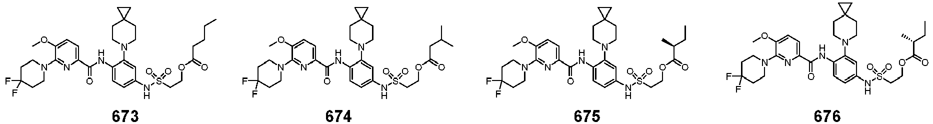 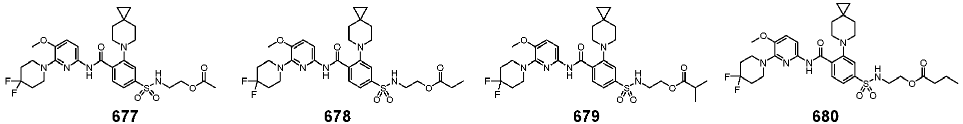 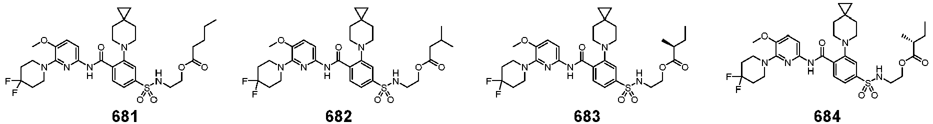 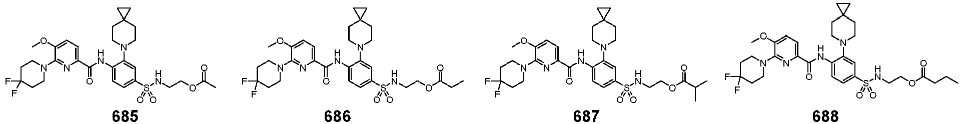 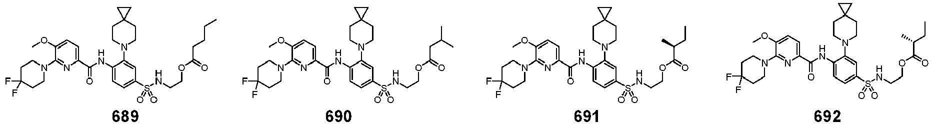 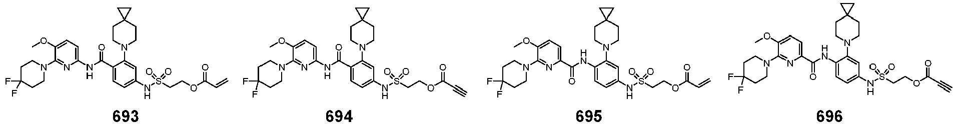 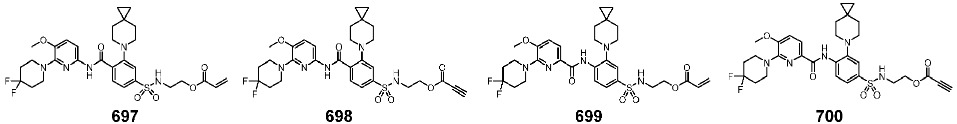 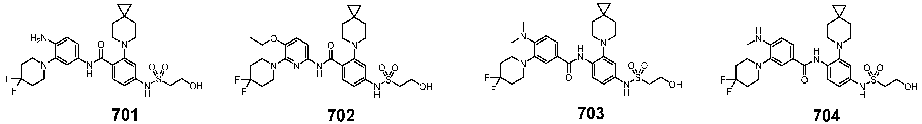 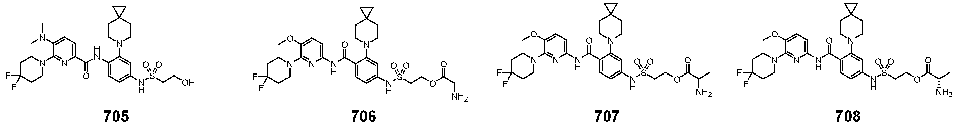 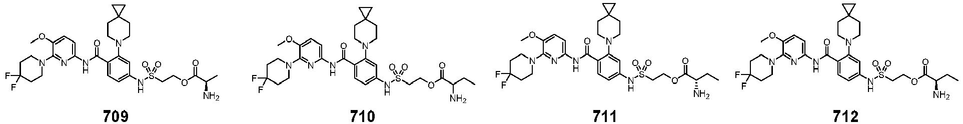 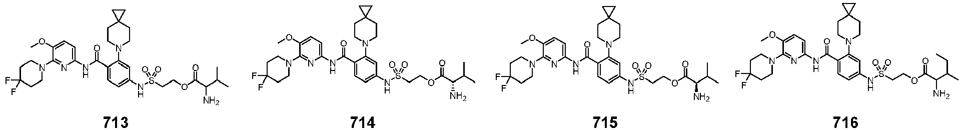 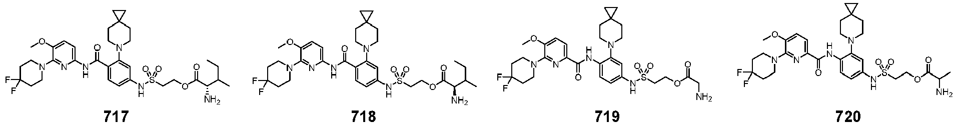 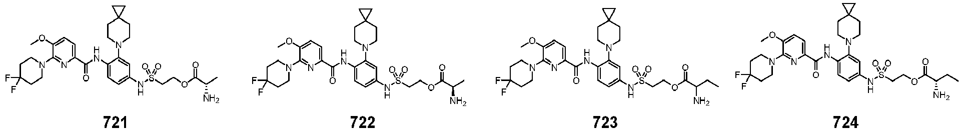 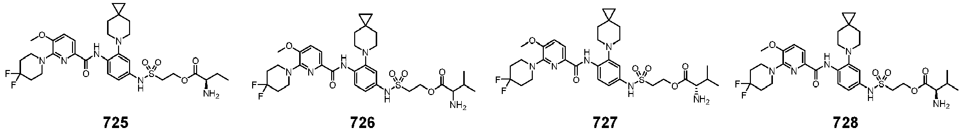 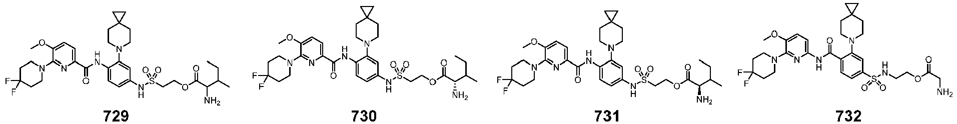 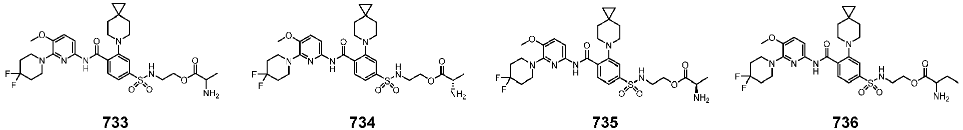 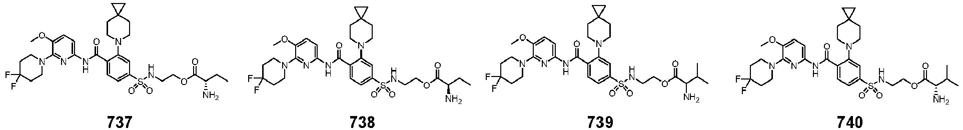 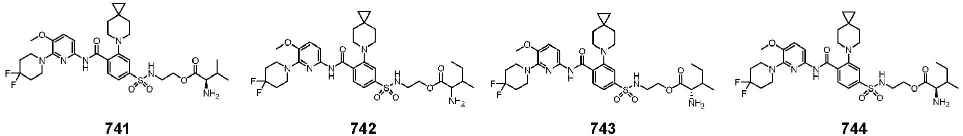 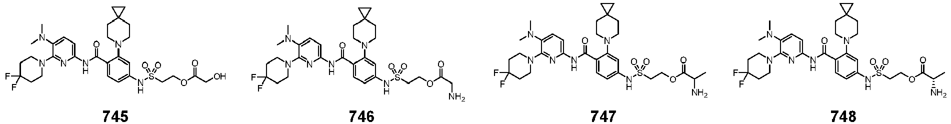 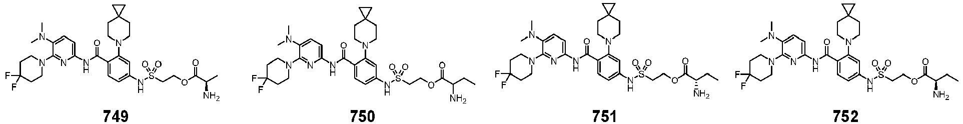 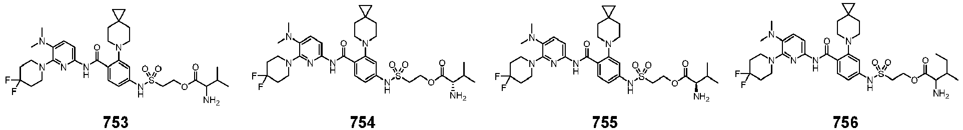 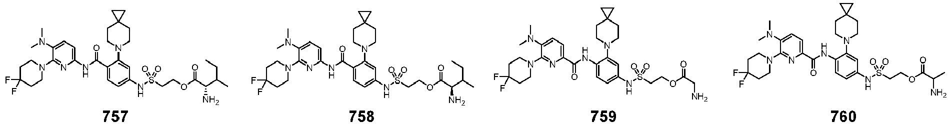 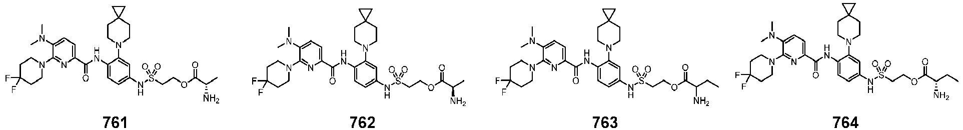 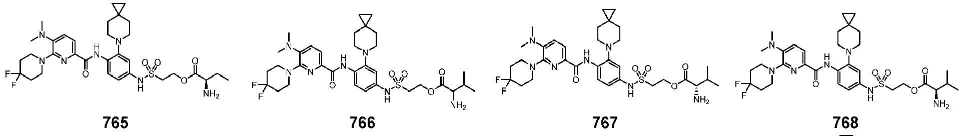 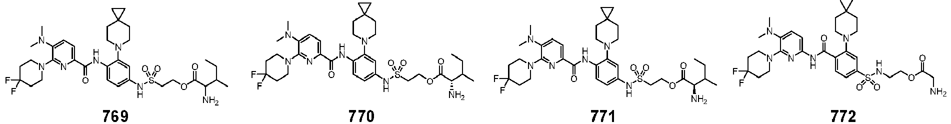 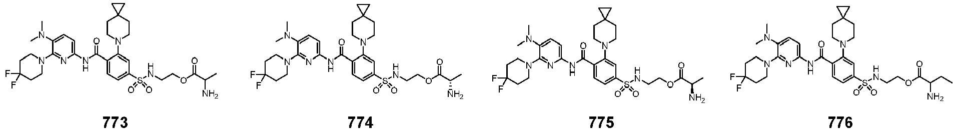 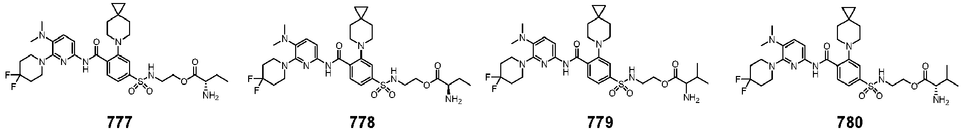 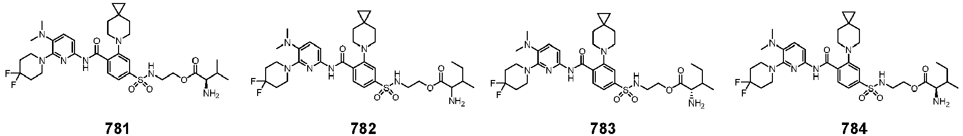 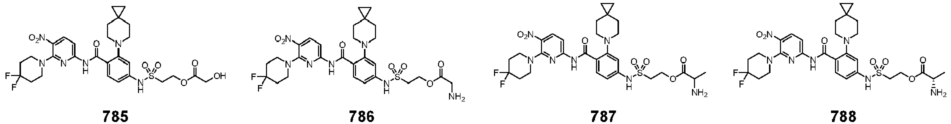 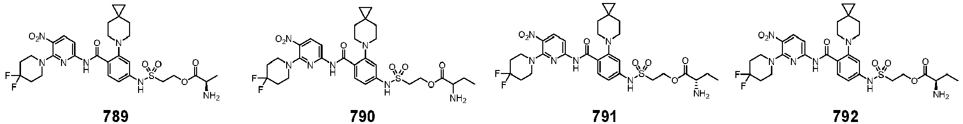 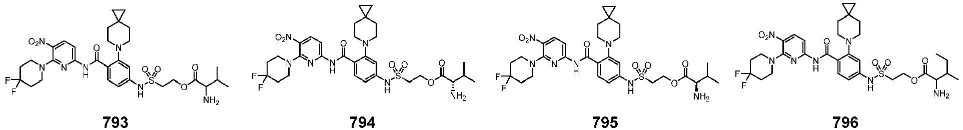 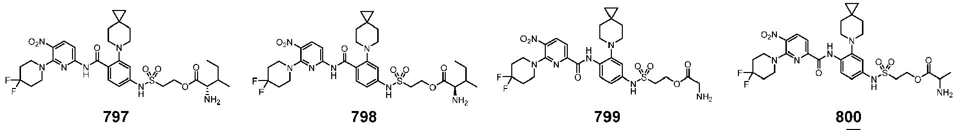 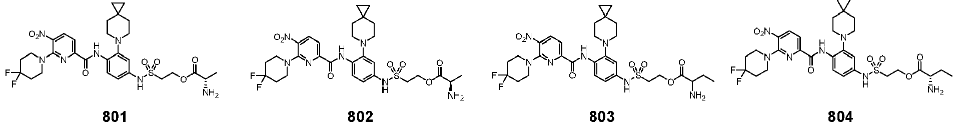 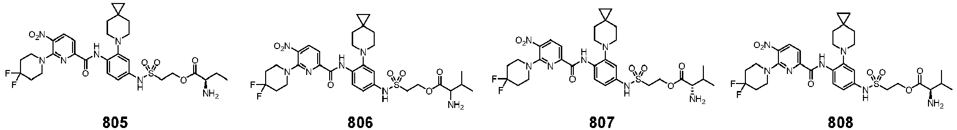 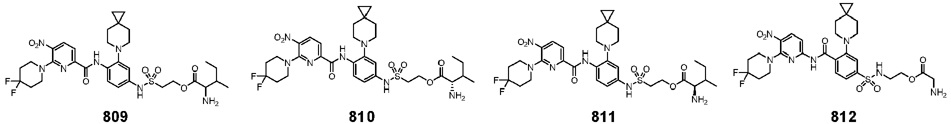 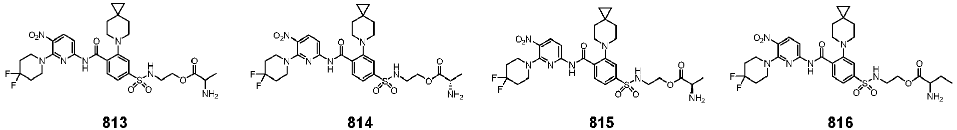 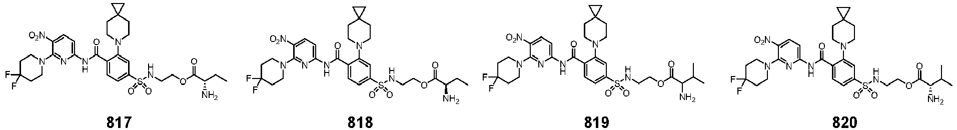 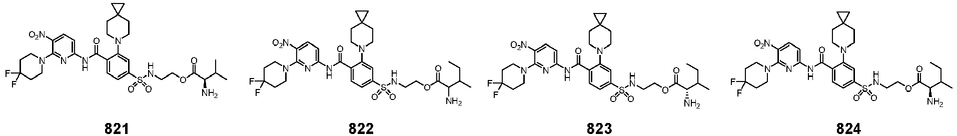 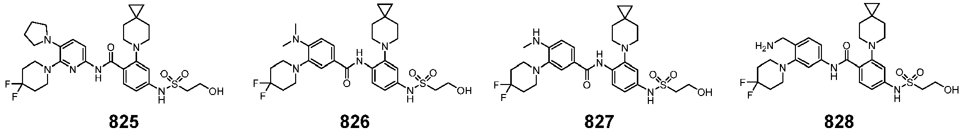 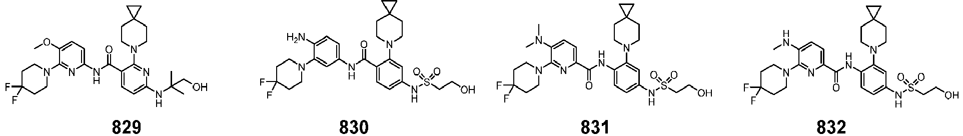 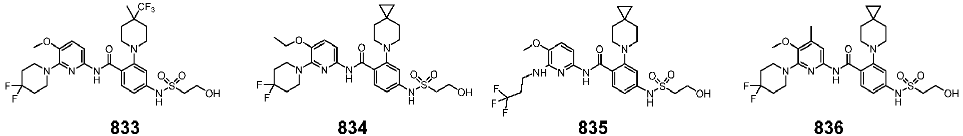 or

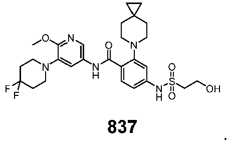

The present invention is further intended to provide a pharmaceutical composition comprising a pharmaceutically acceptable carrier, diluent, and/or excipient, and the compound of general formula (1) or the isomer, the crystalline form, the pharmaceutically acceptable salt, the hydrate or the solvate thereof disclosed herein as an active ingredient.

The present invention is still further intended to provide use of the compound of general formula (1) or the isomer, the crystalline form, the pharmaceutically acceptable salt, the hydrate or the solvate thereof disclosed herein, or the above pharmaceutical composition in preparing a medicament for treating, regulating, or preventing a disease related to KIF18A protein, wherein the disease is preferably a cancer, and the cancer is a hematologic cancer or a solid tumor.

The present invention is even further intended to provide a method for treating, regulating, or preventing a disease related to KIF18A protein, comprising: administering to a subject a therapeutically effective amount of the compound of general formula (1) or the isomer, the crystalline form, the pharmaceutically acceptable salt, the hydrate or the solvate disclosed herein, or the above pharmaceutical composition.

Through synthesis and careful studies of various classes of novel compounds with KIF18A protein inhibitory effects, the inventors have discovered that the compound of general formula (1) has surprisingly strong KIF18A protein inhibitory activity.

It should be understood that both the above general description and the following detailed description of the present invention are exemplary and explanatory, and are intended to provide further explanation of the present invention claimed.

### Synthesis of Compound

Methods for preparing the compounds disclosed herein are specifically described below, which, however, are not intended to limit the present invention in any way.

The compounds described above may be synthesized using standard synthetic techniques or well-known techniques in combination with the methods described herein. In addition, solvents, temperatures, and other reaction conditions mentioned herein may vary. Starting materials for the synthesis of the compounds may be obtained synthetically or from commercial sources, such as, but not limited to, Aldrich Chemical Co. (Milwaukee, Wis.) or Sigma Chemical Co. (St. Louis, Mo.). The compounds described herein and other related compounds having various substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing a compound can be changed by using appropriate reagents and conditions for introducing different groups into the formulas provided herein.

In one aspect, the compounds described herein are prepared according to methods well known in the art. However, the conditions involved in the methods, such as reactants, solvent, base, amount of the compound used, reaction temperature, and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention further provides a method for preparing the compounds described herein, wherein the compound of general formula (1) may be prepared by the following general reaction scheme 1, 2, 3, or 4.

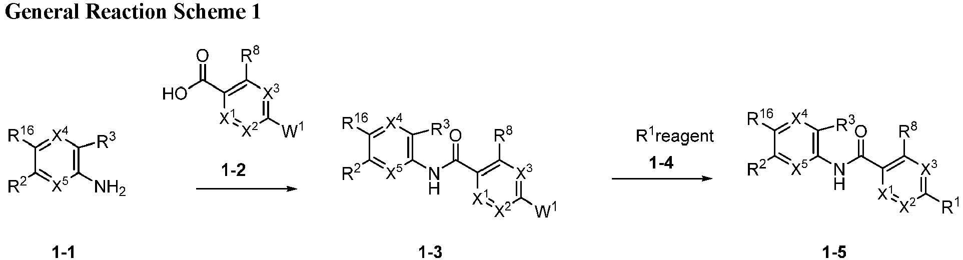

Embodiments of the compound of general formula (1) may be prepared according to general reaction scheme 1, wherein R¹, R², R³, R⁸, R¹⁶, X¹, X², X³, X⁴, and X⁵ are as defined above; W¹ represents fluorine, chlorine, bromine, or iodine; H represents hydrogen; N represents nitrogen; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropane-1-ol, (9) 2-mercapto-2-methylpropan-1-ol, (10) 2-aminoethyl-1-ol, or (11) cyclopropanethiol. As shown in general reaction scheme 1, compound 1-1 and compound 1-2 are subjected to an amidation reaction to generate compound 1-3, and compound 1-3 reacts with R¹ reagent 1-4 to generate compound 1-5.

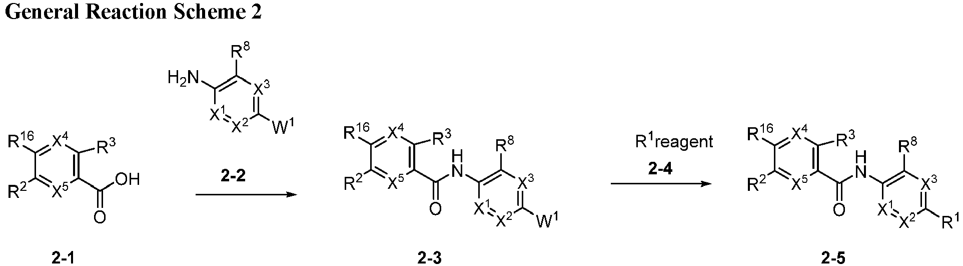

Embodiments of the compound of general formula (1) may be prepared according to general reaction scheme 2, wherein R¹, R², R³, R⁸, R¹⁶, X¹, X², X³, X⁴, and X⁵ are as defined above; W¹ represents fluorine, chlorine, bromine, or iodine; H represents hydrogen; N represents nitrogen; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropane-1-ol, (9) 2-mercapto-2-methylpropan-1-ol, (10) 2-aminoethyl-1-ol, or (11) cyclopropanethiol. As shown in general reaction scheme 2, compound 2-1 and compound 2-2 are subjected to an amidation reaction to generate compound 2-3, and compound 2-3 reacts with R¹ reagent 2-4 to generate compound 2-5.

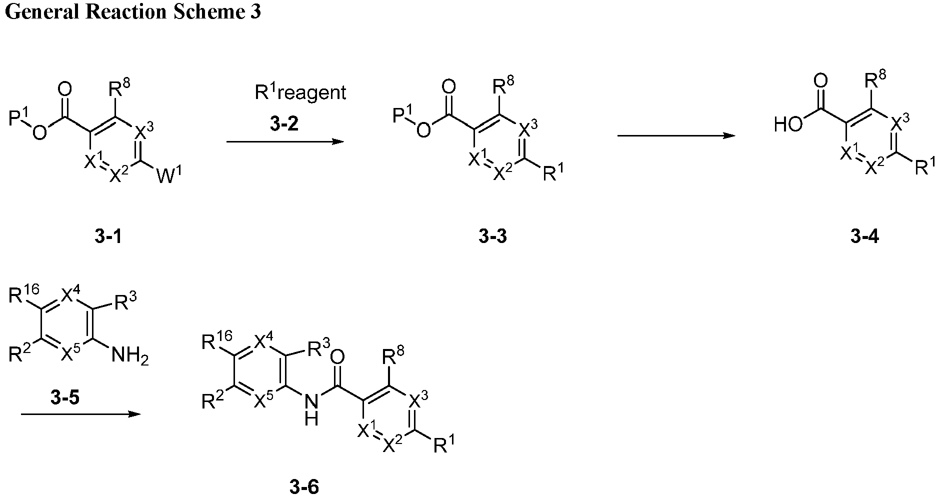

Embodiments of the compound of general formula (1) may be prepared according to general reaction scheme 3, wherein R¹, R², R³, R⁸, R¹⁶, X¹, X², X³, X⁴, and X⁵ are as defined above; W¹ represents fluorine, chlorine, bromine, or iodine; H represents hydrogen; N represents nitrogen; P¹ is a ester group-protecting group; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropane-1-ol, (9) 2-mercapto-2-methylpropan-1-ol, (10) 2-aminoethyl-1-ol, or (11) cyclopropanethiol. As shown in general reaction scheme 3, compound 3-1 reacts with R¹ reagent 3-2 to generate compound 3-3, compound 3-3 removes the ester group-protecting group P¹ to give compound 3-4, and compound 3-4 and compound 3-5 are subjected to an amidation reaction to generate compound 3-6.

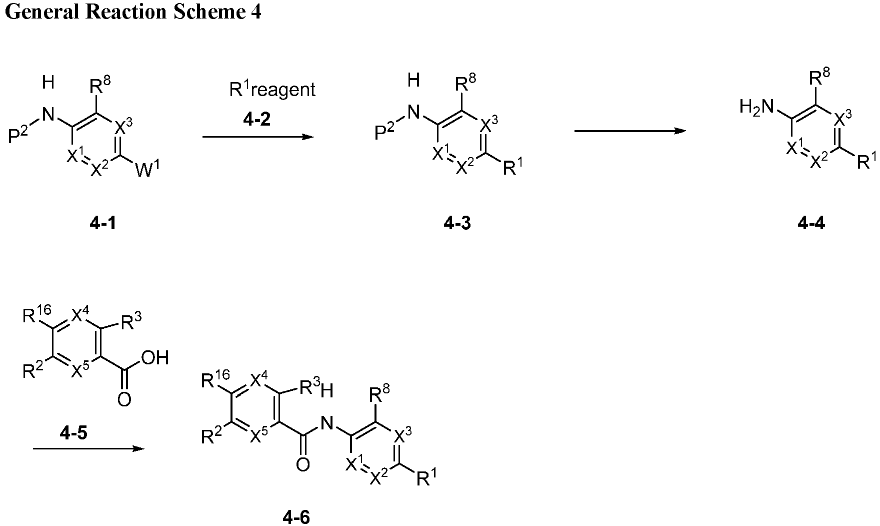

Embodiments of the compound of general formula (1) may be prepared according to general reaction scheme 4, wherein R¹, R², R³, R⁸, X¹, X², X³, X⁴, and X⁵ are as defined above; W¹ represents fluorine, chlorine, bromine, or iodine; H represents hydrogen; N represents nitrogen; P² is a amino-protecting group; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropane-1-ol, (9) 2-mercapto-2-methylpropan-1-ol, (10) 2-aminoethyl-1-ol, or (11) cyclopropanethiol. As shown in general reaction scheme 4, compound 4-1 reacts with R¹ reagent 4-2 to generate compound 4-3, compound 4-3 removes the amino-protecting group P² to give compound 4-4, and compound 4-4 and compound 4-5 are subjected to an amidation reaction to generate compound 4-6.

### Further Forms of Compounds

"Pharmaceutically acceptable" herein refers to a substance, such as a carrier or diluent, which will not lead to loss of biological activity or properties in a compound and is relatively non-toxic. For example, when an individual is given a substance, such substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed. (Wiley, 2002).

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol, or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized, and nanoparticle forms. In addition, the compound of general formula (1) includes crystalline forms, but may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability, and solubility. Different factors such as recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer, and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by common hydrogen and carbon, and compared with an undeuterated medicament, the deuterated medicament generally has the advantages of reduced adverse effects, increased medicament stability, enhanced efficacy, prolonged *in vivo* half-life and the like. All isotopic variations of the compound of the present invention, whether radioactive or not, are intended to be encompassed within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques, and pharmacology are used. As used herein, "or" or "and" refers to "and/or" unless otherwise stated.

Unless otherwise specified, "C_{α-β} hydrocarbyl" means a hydrocarbyl group containing a minimum of α and a maximum of β carbon atoms in a branched or linear relationship, wherein α and β represent integers. The hydrocarbyl described in this section may also contain one or two double or triple bonds. A designation of C₀ hydrocarbyl represents a direct bond. Examples of the C₁₋₆ hydrocarbyl include, but are not limited to, the following:

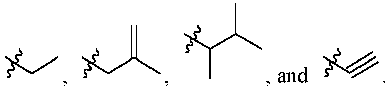

Unless otherwise specified, "C_{α-β} halohydrocarbyl" means a hydrocarbyl group, as described above, in which any number (at least one) of the hydrogen atoms attached to the hydrocarbyl chain are replaced by F, Cl, Br, or I.

Unless otherwise specified, "oxo" and "thio" represent =O (e.g., carbonyl) and =S (e.g., thiocarbonyl), respectively.

Unless otherwise specified, "halo" or "halogen" means a halogen atom selected from F, Cl, Br, and I.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, sec-butoxy, and tert-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO, and ^{t-}BuO.

Unless otherwise specified, "cycloalkyl" refers to a monocyclic non-aromatic hydrocarbon ring system. The ring carbon atoms of the cycloalkyl may optionally be oxidized to form an oxo or sulfido group. The cycloalkyl further includes cycloalkylene. In some embodiments, the cycloalkyl contains 0, 1, or 2 double bonds. In some embodiments, the cycloalkyl contains 1 or 2 double bonds (partially unsaturated cycloalkyl). Examples of the cycloalkyl include, but are not limited to, cyclopropyl, cyclobutyl, cyclopentyl, cyclohexyl, cycloheptyl, cyclopentenyl, cyclohexenyl, cyclohexadienyl, cycloheptatrienyl, and the like.

Unless otherwise specified, "bicyclic ring" means a group that features two connecting rings. The bicyclic ring may be a carbocyclic ring (all ring atoms are carbon atoms) or a heterocyclic ring (ring atoms include, for example, 1, 2, or 3 heteroatoms, such as N, O, or S, in addition to carbon atoms). These two rings may be aliphatic (e.g., decalin and norbornane), or may be aromatic (e.g., naphthalene), or a combination of aliphatic and aromatic (e.g., tetralin). Bicyclic rings include: (a) spirocyclic compounds, wherein the two rings share only one single atom (the spiro atom, which is typically a quaternary carbon); examples of the spirocyclic compounds include, but are not limited to: 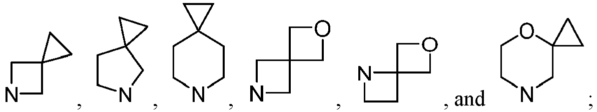
(b) fused bicyclic compounds, wherein the two rings share two adjacent atoms, that is, the rings share a covalent bond, i.e., the bridgehead atoms are directly connected (e.g., α-thujene and decalin); examples of the fused bicyclic rings include, but are not limited to: 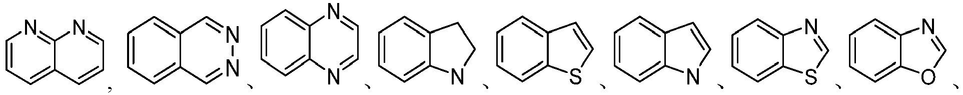 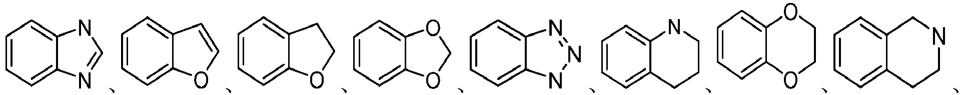 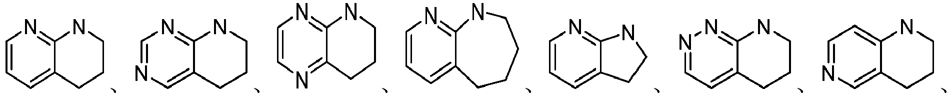 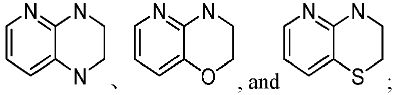 and
(c) bridged bicyclic compounds, wherein the two rings share three or more atoms and the two bridgehead atoms are separated by a bridge containing at least one atom; for example, norbornane, also known as bicyclo[2.2.1]heptane, can be thought of as a pair of cyclopentane rings, each sharing three of their five carbon atoms; examples of the bridged bicyclic rings include, but are not limited to:

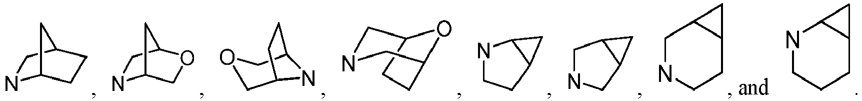

Unless otherwise specified, "carbocyclic ring" or "carbocyclic", by itself or in combination with other terms, represents a cyclic form of "C_{α-β} hydrocarbyl". Examples of the carbocyclic ring include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcamyl, bicyclo[1.1.1]pentyl, bicyclo[2.1.1]hexyl, and the like.

Unless otherwise specified, "heterocyclic ring" or "heterocyclic" means a ring containing at least one carbon atom and at least one other atom selected from N, O, and S. Examples of the heterocyclic ring that may be found in the claims include, but are not limited to, the following:

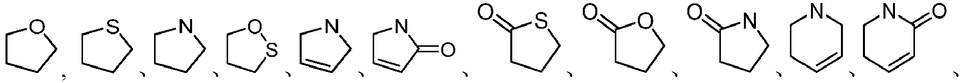

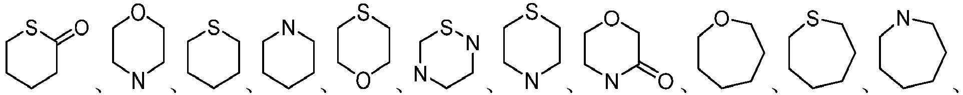

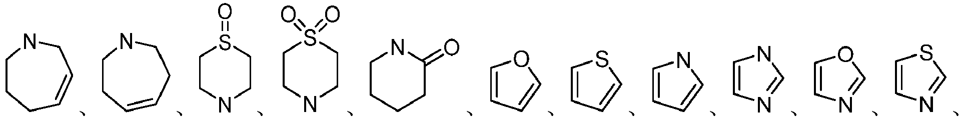

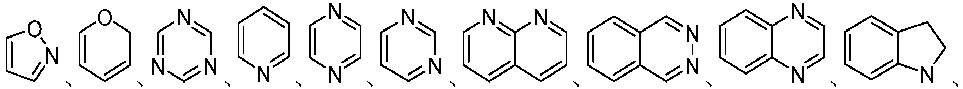

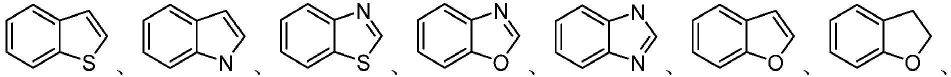

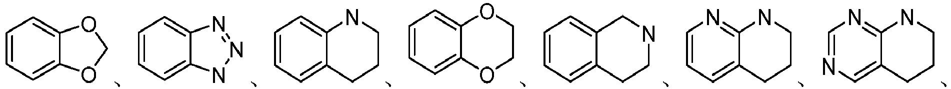

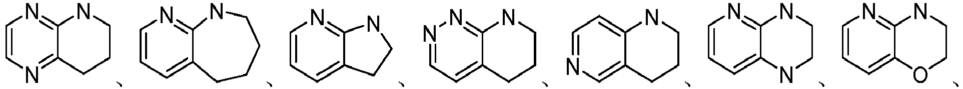

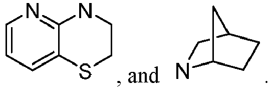

"Optional" or "optionally" means that the subsequently described event or circumstance may, but does not necessarily, occur, and the description includes instances where the event or circumstance occurs and instances where it does not.

"Saturated, partially saturated, or unsaturated" includes substituents saturated with hydrogen, substituents completely unsaturated with hydrogen, and substituents partially saturated with hydrogen.

When one of the variables is selected from a chemical bond, it means that the two groups linked by this variable are linked directly. For example, when L in X-L-Y represents a chemical bond, it means that the structure is actually X-Y.

When the number of a group is 0, such as -N(C₀ hydrocarbyl)-C₀₋₄ hydrocarbyl-, it means that the linker group is -NH-C₀₋₄ hydrocarbyl-.

When the number of a linker group is 0, such as -(CH₂)₀-, it means that the linker group is a chemical bond.

Unless otherwise stated, the absolute configuration of a stereogenic center is represented by a wedged solid bond 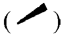 and a wedged dashed bond 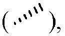 and the relative configuration of a stereogenic center is represented by a straight solid bond 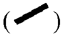 and a straight dashed bond 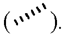 A wavy line 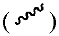 represents a wedged solid bond 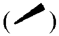 or a wedged dashed bond 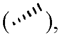 or a wavy line 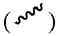 represents a straight solid bond 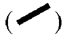 or a straight dashed bond 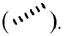 Unless otherwise stated, a single bond or a double bond is represented by -̅ -̅ -̅.

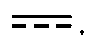

### Specific Pharmaceutical and Medical Terminology

The term "acceptable", as used herein, means that a formulation component or an active ingredient does not unduly adversely affect a general therapeutic target's health.

The terms "treatment," "treatment course," and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

The "active ingredient" refers to the compound of general formula (1), and pharmaceutically acceptable inorganic or organic salts of the compound of general formula (1). The compounds of the present invention may contain one or more asymmetric centers (chiral center or axial chirality) and thus occur in the form of a racemate, racemic mixture, single enantiomer, diastereomeric compound, and single diastereomer. Asymmetric centers that may be present depend on the nature of the various substituents on the molecule. Each of these asymmetric centers will independently produce two optical isomers, and all possible optical isomers, diastereomeric mixtures, and pure or partially pure compounds are included within the scope of the present invention. The present invention is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At the very least, these numerical parameters should be understood as the significant digits indicated or the numerical value obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides use of the compound of general formula (1) or the pharmaceutical composition disclosed herein in inhibiting KIF18A protein, therefore, in treating one or more disorders related to the activity of KIF18A protein. Therefore, in certain embodiments, the present invention provides a method for treating KIF18A protein-mediated disorders, which comprises the step of administering to a patient in need the compound or the pharmaceutically acceptable composition thereof disclosed herein.

In some embodiments, a method for treating a cancer is provided, comprising administering to an individual in need an effective amount of any aforementioned pharmaceutical composition comprising the compound of structural general formula (1). In some embodiments, the cancer is mediated by KIF18A protein. In other embodiments, the cancer is a hematologic cancer or a solid tumor, including, but not limited to, hematologic malignancies (leukemias, lymphomas, and myelomas including multiple myeloma, myelodysplastic syndrome, and myeloproliferative family syndrome), and solid tumors (carcinomas such as prostate, breast, lung, colon, pancreas, kidney, ovary and soft tissue cancers, osteosarcoma, and interstitial tumors), and the like.

### Route of Administration

The compound and the pharmaceutically acceptable salt thereof disclosed herein can be prepared into various formulations comprising a safe and effective amount of the compound or the pharmaceutically acceptable salt thereof disclosed herein, and a pharmaceutically acceptable excipient or carrier, wherein the "safe and effective amount" means that the amount of the compound is sufficient to significantly improve the condition without causing serious adverse effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" means that the components of the composition are capable of intermixing with the compound of the present invention and with each other, without significantly diminishing the pharmaceutical efficacy of the compound. Examples of pharmaceutically acceptable excipients or carriers include cellulose and its derivatives (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol, and sodium lauryl sulfate, or mixtures thereof. In the case of capsules, tablets, and pills, the dosage forms may further comprise buffers.

Solid dosage forms such as tablets, dragees, capsules, pills, and granules can be prepared using coatings and shells such as enteric coatings and other materials well known in the art. They may comprise opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups, and elixirs. In addition to the active compound, the liquid dosage form may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, and sesame oil, or mixtures of these substances.

Besides such inert diluents, the composition may further comprise adjuvants, such as wetting agents, emulsifiers, suspending agents, sweeteners, flavoring agents, and perfuming agents.

Suspensions, in addition to the active compound, may comprise suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may comprise physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present invention include ointments, pulvises, patches, sprays, and inhalants. The active ingredient is mixed under sterile conditions with a physiologically acceptable carrier and any preservatives, buffers, or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the administration dose is a pharmaceutically effective administration dose. For a human of 60 kg, the daily dose of administration is usually 1-2000 mg, preferably 50-1000 mg. In determining a specific dose, such factors as the route of administration, the health condition of the patient, and the like will also be considered, which are well known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise expressly stated, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific embodiments for reference only. After reading the description of the present invention, those skilled in the art can make various changes or modifications to the present invention, and such equivalents also fall within the scope of the present invention defined herein.

In all examples, ¹H-NMR spectra were recorded with a Varian Mercury 400 nuclear magnetic resonance spectrometer, and chemical shifts are expressed in δ (ppm); silica gel for separation was 200-300 mesh silica gel if not specified, and the ratio of the eluents was volume ratio.

The following abbreviations are used in the present invention: (Boc)₂O for di-tert-butyl dicarbonate; BOPCl for bis(2-oxo-3-oxazolidinyl)phosphinic chloride; CDCl₃ for deuterated chloroform; Cs₂CO₃ for cesium carbonate; CuI for cuprous iodide; EtOAc for ethyl acetate; Hexane for n-hexane; HPLC for high-performance liquid chromatography; MeCN for acetonitrile; DCE for 1,2-dichloroethane; DCM for dichloromethane; DIPEA for diisopropylethylamine; 1,4-Dioxane for 1,4-dioxane; DMF for *N*,*N*-dimethylformamide; DMAP for 4-(dimethylamino)pyridine; DMSO for dimethyl sulfoxide; h for hour; HATU for N-[(dimethylamino)-1H-1,2,3-triazolo-[4,5-b]pyridin-1-methylene]-N-methylmethanaminium hexafluorophosphate-N-oxide; IPA for isopropanol; min for minute; K₂CO₃ for potassium carbonate; KOAc for potassium acetate; K₃PO₄ for potassium phosphate; LiBH₄ for lithium borohydride; min for minute; MeOH for methanol; MS for mass spectrometry; NMR for nuclear magnetic resonance; Pd/C for palladium carbon; Pd(PPh₃)₄ for tetrakis(triphenylphosphine)palladium; Pd₂(dba)₃ for tris(dibenzylideneacetone)dipalladium(O); PE for petroleum ether; RuPhos-Pd-G₃ for (2-dicyclohexylphosphino-2',6'-diisopropoxy-1,1'-biphenyl)[2-(2'-amino-1,1'-biphenylyl)]palladium(II) methanesulfonate; Sarcosine for sarcosine; TFA for trifluoroacetic acid; TMSCl for trimethylchlorosilane; T₃P for 1-propanephosphonic anhydride; XantPhos for 4,5-bis(diphenylphosphino)-9,9-dimethylxanthene; X-Phos for 2-dicyclohexylphosphino-2',4',6'-triisopropylbiphenyl; TLC for thin-layer chromatography; XPhos for 2-dicyclohexylphosphino-2',4',6'-triisopropylbiphenyl; and XantPhos for 4,5-bis(diphenylphosphino)-9,9-dimethylxanthene.

### Example 1. Synthesis of Compound 1

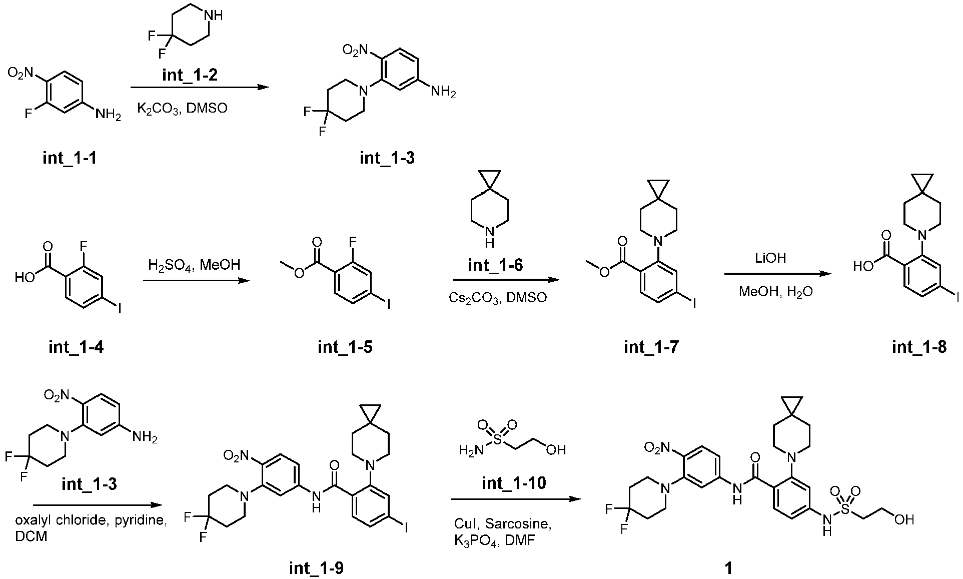

### Step 1: Synthesis of compound int_1-3

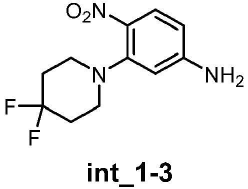

**Int_1-1** (800 mg, 5.124 mmol) was dissolved in DMSO (10 mL), and potassium carbonate (1.41 g, 10.249 mmol) and **int_1-2** (1.24 g, 10.249 mmol) were added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (1.2 g, yield: 91.6%).

ESI-MS m/z: 258 [M+H]⁺.

### Step 2: Synthesis of compound int_1-5

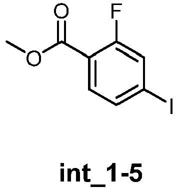

**Int_1-4** (15 g, 56.3 mmol) was dissolved in methanol (150 mL), and concentrated sulfuric acid (2.5 mL) was added. The mixture was heated to 80 °C and incubated for reaction for 4 h, until LC-MS indicated the completion of the reaction. The reaction solution was concentrated at reduced pressure to give a crude product, and the crude product was dissolved in ethyl acetate. The organic phase was washed with saturated sodium bicarbonate solution and then with saturated brine, dried over anhydrous sodium sulfate, filtered, and distilled at reduced pressure to give a white solid (14 g, yield: 89%). The solid was used directly in the next reaction. ESI-MS m/z: 281 [M+H]⁺.

### Step 3: Synthesis of compound int_1-7

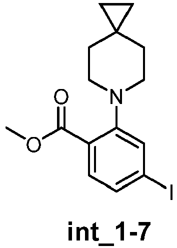

**Int_1-5** (14 g, 49.9 mmol) was dissolved in DMSO (100 mL), and cesium carbonate (23.4 g, 71.7 mmol) and **int_1-6** (6.98 g, 62.8 mmol) were added. The mixture was heated to 90 °C and incubated for reaction for 24 h, until LC-MS indicated the completion of the reaction. The reaction solution was diluted with water (500 mL), and the aqueous phase was extracted with ethyl acetate (100 mL × 3). The organic phase was dried over anhydrous sodium sulfate, filtered, and distilled at reduced pressure to give a crude product, and the crude product was subjected to column chromatography (SiO₂, EtOAc:Hexane = 1:1) to give the target product (16.3 g, yield: 88%).

ESI-MS m/z: 372 [M+H]⁺.

### Step 4: Synthesis of compound int_1-8

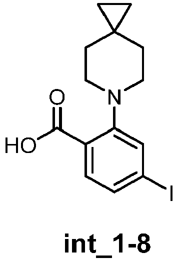

**Int_1-7** (16.3 g, 43.9 mmol) was dissolved in a mixed solvent of methanol (100 mL) and water (10 mL), and lithium hydroxide (2.1 g, 87.8 mmol) was added at room temperature. The mixture was stirred at room temperature for reaction for 6 h, until LC-MS indicated the completion of the reaction. The reaction solution was concentrated at reduced pressure to give a crude product (17 g). The crude product was used directly in the next reaction.

ESI-MS m/z: 358 [M+H]⁺.

### Step 5: Synthesis of compound int_1-9

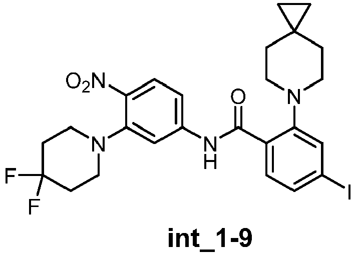

**Int_1-8** (1.1 g, 3.08 mmol) was dissolved in DCM (10 mL), and oxalyl chloride (888.4 mg, 7 mmol) was added. The reaction solution was stirred at room temperature for 2 h and then concentrated at reduced pressure to remove the solvent, thus giving a solid. The solid was dissolved in DCM (10 mL), and **int_1-3** (792 mg, 3.08 mmol) and pyridine (730 mg, 9.24 mmol) were added. The reaction solution was stirred at 40 °C for 10 h, until LC-MS indicated the completion of the reaction. The reaction solution was concentrated at reduced pressure to give a crude product. The crude product was purified by column chromatography (SiO₂, PE:EtOAC = 100:1) to give a solid (1.4 g, yield: 76.1%).

ESI-MS m/z: 597 [M+H]⁺.

### Step 6: Synthesis of compound 1

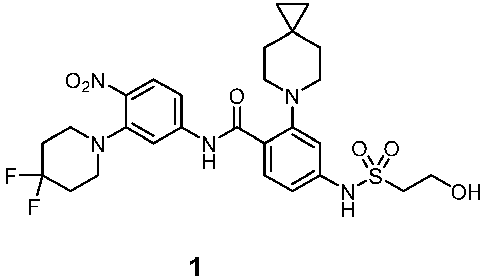

**Int_1-10** (291 mg, 2.374 mmol), sarcosine (209.1 mg, 2.348 mmol), cuprous iodide (227 mg, 1.174 mmol), and potassium phosphate (1.5 g, 7.041 mmol) were dissolved in DMF (20 mL). The mixture was purged with argon three times before **int_1-9** (1.4 g, 2.347 mmol) was added. In argon atmosphere, the reaction solution was heated to 90 °C and incubated for reaction for 3 h, until LC-MS indicated the completion of the reaction. The reaction solution was cooled to room temperature, concentrated by rotary evaporation, and purified by column chromatography (SiO₂, EtOAc:Hexane = 1:1) to give a solid (1 g, yield: 71.8%).

¹H NMR (400 MHz, DMSO-d6) δ 11.92 (s, 1H), 8.03 (d, *J* = 9.0 Hz, 1H), 7.78 (d, *J* = 8.5 Hz, 1H), 7.75 (d, *J* = 2.1 Hz, 1H), 7.49 (dd, *J* = 9.0, 2.1 Hz, 1H), 7.14 (d, *J* = 2.1 Hz, 1H), 7.01 (dd, *J* = 8.5, 2.1 Hz, 1H), 3.74 (t, *J* = 6.5 Hz, 2H), 3.32 (d, *J* = 6.5 Hz, 2H), 3.14 (t, *J* = 5.5 Hz, 4H), 2.95 (t, *J* = 5.2 Hz, 4H), 2.11 (tq, *J* = 14.6, 8.9, 7.2 Hz, 4H), 1.50 (s, 4H), 0.33 (s, 4H).

ESI-MS m/z: 594 [M+H]⁺.

### Example 2. Synthesis of Compound 2

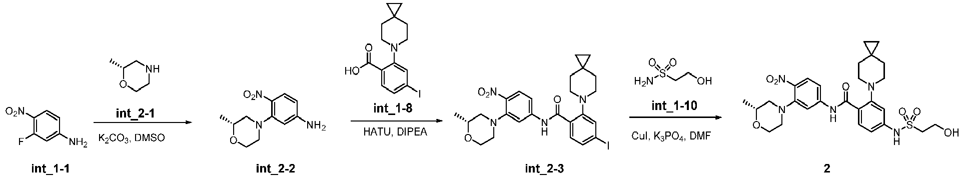

### Step 1: Synthesis of compound int_2-2

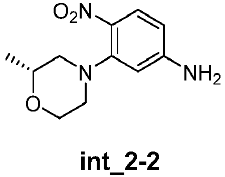

**Int_1-1** (200 mg, 1.281 mmol) was dissolved in DMSO (5 mL), and potassium carbonate (354 mg, 2.562 mmol) and **int_2-1** (259 mg, 2.562 mmol) were added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (300 mg, yield: 99%). ESI-MS m/z: 238 [M+H]⁺.

### Step 2: Synthesis of compound int_2-3

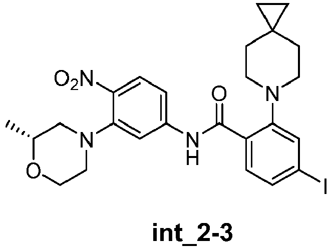

**Int_1-8** (151 mg, 0.422 mmol) was dissolved in DMF (4 mL), and HATU (240 mg, 0.632 mmol), DIPEA (163 mg, 1.264 mmol), and **int_2-2** (100 mg, 0.422 mmol) were added. The reaction solution was stirred at 80 °C for 10 h, until LC-MS indicated the completion of the reaction. The reaction solution was concentrated at reduced pressure to give a crude product. The crude product was purified by column chromatography to give a solid (60 mg, yield: 24.6%).

ESI-MS m/z: 577 [M+H]⁺.

### Step 3: Synthesis of compound 2

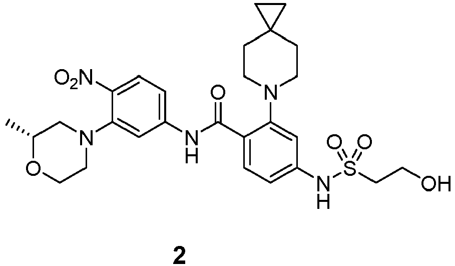

**Int_1-10** (20 mg, 0.15 mmol), (1*S*,2*S*)-*N*,*N*-dimethylcyclohexane (7 mg, 0.05 mmol), cuprous iodide (10 mg, 0.05 mmol), and potassium phosphate (63 mg, 0.3 mmol) were dissolved in DMF (5 mL). The mixture was purged with argon three times before **int_2-3** (60 mg, 0.1 mmol) was added. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (20 mg, yield: 34.9%).

¹H NMR (400 MHz, Chloroform-d) δ 12.95 (s, 1H), 8.21 (d, *J* = 8.1 Hz, 1H), 8.00 (d, *J* = 8.9 Hz, 1H), 7.83 (d, *J* = 2.3 Hz, 1H), 7.35 (s, 1H), 7.22 - 7.16 (m, 1H), 7.08 (d, *J* = 8.0 Hz, 1H), 4.15 (s, 2H), 3.97 - 3.82 (m, 3H), 3.35 (d, *J* = 5.4 Hz, 2H), 3.16 (t, *J* = 10.5 Hz, 2H), 3.07 (q, *J* = 7.6, 6.5 Hz, 4H), 3.04 - 2.96 (m, 1H), 2.70 (t, *J* = 10.9 Hz, 2H), 1.66 (s, 4H), 1.21 (d, *J* = 6.2 Hz, 3H), 0.45 (s, 4H).

ESI-MS m/z: 574 [M+H]⁺.

### Example 3. Synthesis of Compound 3

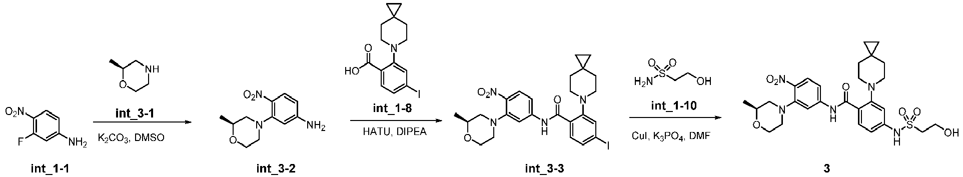

### Step 1: Synthesis of compound int_3-2

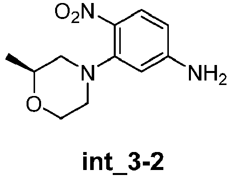

**Int_1-1** (200 mg, 1.281 mmol) was dissolved in DMSO (10 mL), and potassium carbonate (354 mg, 2.562 mmol) and **int_3-1** (259 mg, 2.562 mmol) were added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (280 mg, yield: 92%). ESI-MS m/z: 238 [M+H]⁺.

### Step 2: Synthesis of compound int_3-3

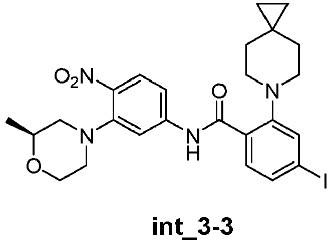

**Int_1-8** (151 mg, 0.422 mmol) was dissolved in DMF (4 mL), and HATU (240 mg, 0.632 mmol), DIPEA (163 mg, 1.264 mmol), and **int_3-2** (100 mg, 0.422 mmol) were added. The reaction solution was stirred at 80 °C for 10 h, until LC-MS indicated the completion of the reaction. The reaction solution was concentrated at reduced pressure to give a crude product. The crude product was purified by column chromatography to give a solid (119 mg, yield: 48.9%).

ESI-MS m/z: 577 [M+H]⁺.

### Step 3: Synthesis of compound 3

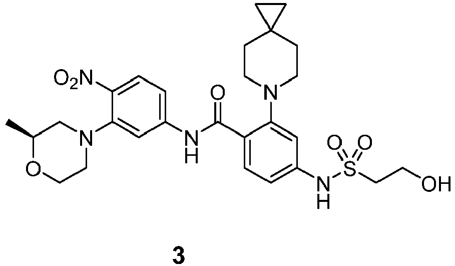

**Int_1-10** (39 mg, 0.310 mmol), (1*S*,2*S*)-*N*,*N*-dimethylcyclohexane (15 mg, 0.103 mmol), cuprous iodide (20 mg, 0.103 mmol), and potassium phosphate (132 mg, 0.620 mmol) were dissolved in DMF (5 mL). The mixture was purged with argon three times before **int_3-3** (119 mg, 0.207 mmol) was added. In argon atmosphere, the reaction solution was heated to 90 °C and incubated for reaction for 3 h, until LC-MS indicated the completion of the reaction. The reaction solution was cooled to room temperature, concentrated by rotary evaporation, and purified by column chromatography to give a solid (50 mg, yield: 42.3%).

¹H NMR (400 MHz, DMSO-d6) δ 12.00 (s, 1H), 7.99 (d, *J* = 9.0 Hz, 1H), 7.80 (d, *J* = 8.5 Hz, 1H), 7.65 (d, *J* = 2.2 Hz, 1H), 7.47 (dd, *J* = 9.0, 2.1 Hz, 1H), 7.14 (d, *J* = 2.1 Hz, 1H), 7.01 (dd, *J* = 8.5, 2.0 Hz, 1H), 3.85 (dd, *J* = 11.4, 2.6 Hz, 1H), 3.79 - 3.60 (m, 4H), 3.07 (dd, *J* = 29.4, 12.0 Hz, 2H), 2.95 (t, *J* = 5.3 Hz, 4H), 2.90 - 2.78 (m, 1H), 2.59 (dd, *J* = 11.9, 9.8 Hz, 1H), 1.52 (d, *J* = 4.7 Hz, 4H), 1.10 (d, *J* = 6.2 Hz, 3H), 0.33 (s, 4H). ESI-MS m/z: 574 [M+H]⁺.

### Example 4. Synthesis of Compound 4

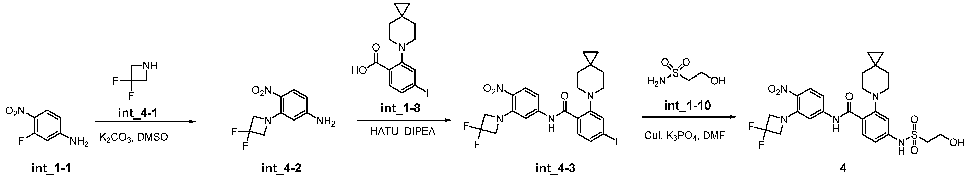

### Step 1: Synthesis of compound int_4-2

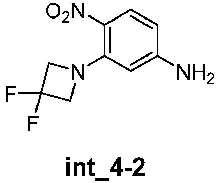

**Int_1-1** (200 mg, 1.281 mmol) was dissolved in DMSO (5 mL), and potassium carbonate (710 mg, 5.124 mmol) and **int_4-1** (hydrochloride, 310 mg, 2.562 mmol) were added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (300 mg, yield: 100%).

ESI-MS m/z: 230 [M+H]⁺.

### Step 2: Synthesis of compound int_4-3

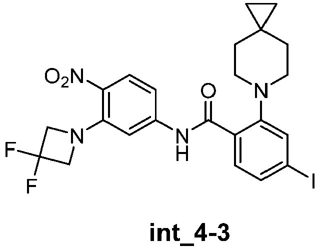

**Int_1-8** (156 mg, 0.436 mmol) was dissolved in DMF (3 mL), and HATU (342 mg, 0.872 mmol), DIPEA (165 mg, 1.308 mmol), and **int_4-2** (100 mg, 0.436 mmol) were added. The reaction solution was stirred at 80 °C for 10 h, until LC-MS indicated the completion of the reaction. The reaction solution was concentrated at reduced pressure to give a crude product. The crude product was purified by column chromatography to give a solid (130 mg, yield: 52.6%).

ESI-MS m/z: 569 [M+H]⁺.

### Step 3: Synthesis of compound 4

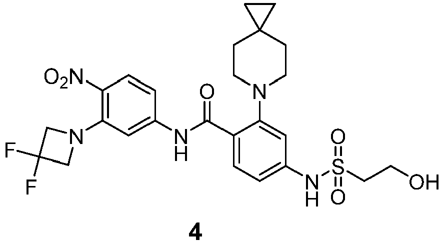

**Int_1-10** (16 mg, 0.123 mmol), (1*S*,2*S*)-*N*,*N*-dimethylcyclohexane (9 mg, 0.062 mmol), cuprous iodide (12 mg, 0.062 mmol), and potassium phosphate (80 mg, 0.369 mmol) were dissolved in DMF (5 mL). The mixture was purged with argon three times before **int_4-3** (70 mg, 0.123 mmol) was added. In argon atmosphere, the reaction solution was heated to 90 °C and incubated for reaction for 3 h, until LC-MS indicated the completion of the reaction. The reaction solution was cooled to room temperature, concentrated by rotary evaporation, and purified by column chromatography to give a solid (54 mg, yield: 77.1%).

¹H NMR (400 MHz, Chloroform-d) δ 12.85 (s, 1H), 8.20 (d, *J* = 8.2 Hz, 1H), 8.02 (d, *J* = 9.0 Hz, 1H), 7.69 (s, 1H), 7.34 (s, 1H), 7.07 (d, *J* = 8.4 Hz, 1H), 6.96 (s, 1H), 6.84 (d, *J* = 9.0 Hz, 1H), 4.39 (t, *J* = 11.9 Hz, 4H), 4.16 (s, 2H), 3.34 (t, *J* = 5.3 Hz, 2H), 3.08 (t, *J* = 5.3 Hz, 4H), 1.63 (s, 4H), 0.45 (s, 4H).

ESI-MS m/z: 566 [M+H]⁺.

### Example 5. Synthesis of Compound 6

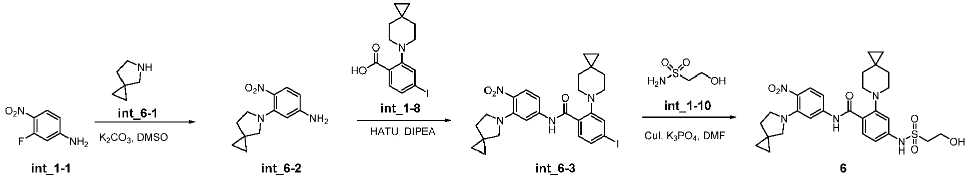

### Step 1: Synthesis of compound int_6-2

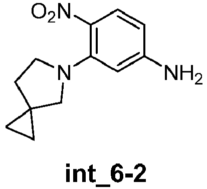

**Int_1-1** (200 mg, 1.281 mmol) was dissolved in DMSO (5 mL), and potassium carbonate (710 mg, 5.124 mmol) and **int_6-1** (hydrochloride, 171 mg, 1.281 mmol) were added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (290 mg, yield: 97.0%).

ESI-MS m/z: 234 [M+H]⁺.

### Step 2: Synthesis of compound int_6-3

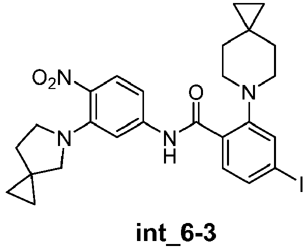

**Int_1-8** (100 mg, 0.28 mmol) was dissolved in DMF (3 mL), and HATU (342 mg, 0.872 mmol), DIPEA (165 mg, 1.308 mmol), and **int_6-2** (65 mg, 0.28 mmol) were added. The reaction solution was stirred at 80 °C for 16 h, until LC-MS indicated the completion of the reaction. The reaction solution was concentrated at reduced pressure to give a crude product. The crude product was purified by column chromatography to give a solid (70 mg, yield: 43.8%).

ESI-MS m/z: 573 [M+H]⁺.

### Step 3: Synthesis of compound 6

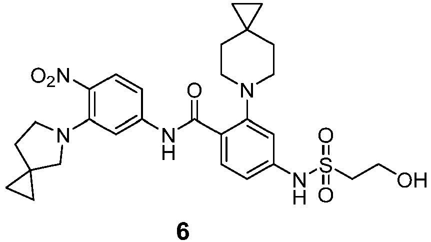

**Int_1-10** (16 mg, 0.123 mmol), (1*S*,2*S*)-*N*,*N*-dimethylcyclohexane (9 mg, 0.062 mmol), cuprous iodide (12 mg, 0.062 mmol), and potassium phosphate (80 mg, 0.369 mmol) were dissolved in DMF (5 mL). The mixture was purged with argon three times before **int_6-3** (70 mg, 0.122 mmol) was added. In argon atmosphere, the reaction solution was heated to 90 °C and incubated for reaction for 3 h, until LC-MS indicated the completion of the reaction. The reaction solution was cooled to room temperature, concentrated by rotary evaporation, and purified by column chromatography to give a solid (45 mg, yield: 64.7%).

¹H NMR (400 MHz, Chloroform-d) δ 12.71 (s, 1H), 8.20 (d, *J* = 8.3 Hz, 1H), 7.90 - 7.76 (m, 2H), 7.33 (s, 1H), 7.06 (d, *J* = 8.3 Hz, 2H), 6.85 - 6.71 (m, 1H), 4.14 (t, *J* = 5.0 Hz, 2H), 3.46 (t, *J* = 6.6 Hz, 2H), 3.34 (t, *J* = 5.1 Hz, 2H), 3.16 (s, 2H), 3.07 (d, *J* = 5.5 Hz, 4H), 1.90 (t, *J* = 6.7 Hz, 2H), 1.65 (s, 4H), 0.62 (d, *J* = 5.3 Hz, 4H), 0.43 (s, 4H).

ESI-MS m/z: 570 [M+H]⁺.

### Example 6. Synthesis of Compound 7

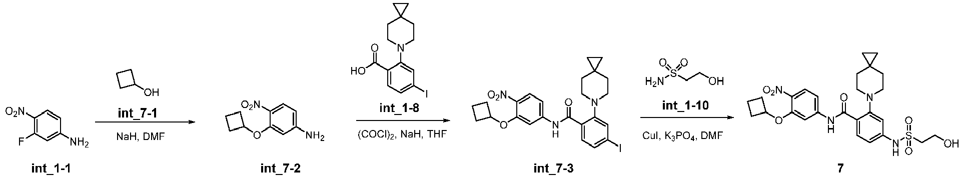

### Step 1: Synthesis of compound int_7-2

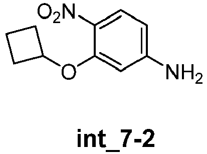

**Int_7-1** (372 mg, 5.17 mmol) was dissolved in DMF (30 mL), and NaH (820 mg, 20.5 mmol, 60% purity) was added at 0 °C in nitrogen atmosphere. The reaction solution was incubated at 0 °C for reaction for 1 h in nitrogen atmosphere before **int_1-1** (400 mg, 2.564 mmol) was added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product, and the crude product was subjected to column chromatography to give the target product (170 mg, yield: 32%).

ESI-MS m/z: 209 [M+H]⁺.

### Step 2: Synthesis of compound int_7-3

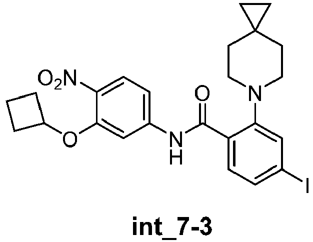

**Int_1-8** (129 mg, 0.361 mmol) was dissolved in DCM (2 mL), and oxalyl chloride (1 mL) was added. The reaction solution was stirred at room temperature for 2 h and then concentrated at reduced pressure to remove the solvent, thus giving an acyl chloride product. **Int_7-2** (50 mg, 0.24 mmol) was dissolved in tetrahydrofuran (5 mL), and sodium hydride (100 mg) was slowly added under an ice bath. The reaction solution was incubated at room temperature for reaction for 1 h, and the prepared acyl chloride product was added to the reaction solution. The reaction solution was incubated at 40 °C for reaction for 5 h, until LC-MS indicated the completion of the reaction. The reaction solution was diluted with water (50 mL), and the aqueous phase was extracted with dichloromethane (50 mL × 3). The organic phase was dried over anhydrous sodium sulfate, filtered, and distilled at reduced pressure to give a crude product. The crude product was purified by column chromatography to give the target product (77 mg, yield: 59%).

ESI-MS m/z: 548 [M+H]⁺.

### Step 3: Synthesis of compound 7

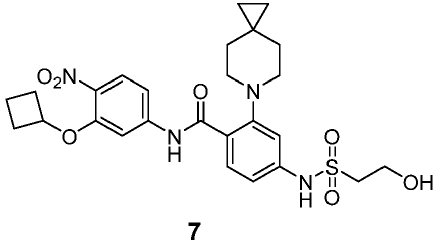

**Int_1-10** (36 mg, 0.29 mmol), (1*S*,2*S*)-*N*,*N*-dimethylcyclohexane (10 mg, 0.021 mmol), cuprous iodide (14 mg, 0.07 mmol), and potassium phosphate (90 mg, 0.42 mmol) were dissolved in DMF (7 mL). The mixture was purged with argon three times before **int_7-3** (77 mg, 0.14 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (44 mg, yield: 57%).

¹H NMR (400 MHz, DMSO-d6) δ 11.80 (s, 1H), 7.99 (d, *J* = 8.9 Hz, 1H), 7.76 (d, *J* = 8.5 Hz, 1H), 7.58 (d, *J* = 2.1 Hz, 1H), 7.47 (dd, *J* = 9.1, 2.1 Hz, 1H), 7.12 (d, *J* = 2.1 Hz, 1H), 7.00 (dd, *J* = 8.5, 2.1 Hz, 1H), 4.79 (t, *J* = 7.2 Hz, 1H), 3.74 (t, *J* = 6.6 Hz, 2H), 2.95 (t, *J* = 5.2 Hz, 4H), 2.22 - 2.04 (m, 2H), 1.91 - 1.61 (m, 2H), 1.49 (t, *J* = 5.0 Hz, 4H), 0.32 (s, 4H).

ESI-MS m/z: 545 [M+H]⁺.

### Example 7. Synthesis of Compound 65

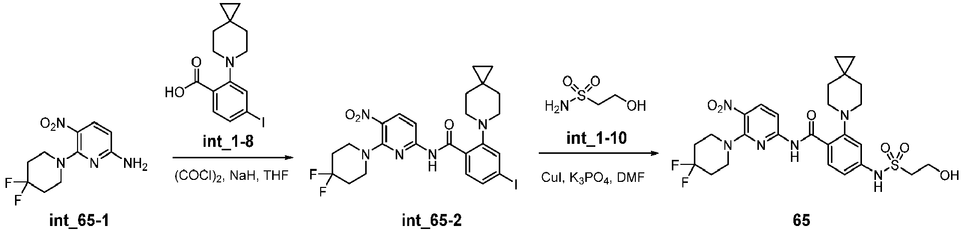

### Step 1: Synthesis of compound int_65-2

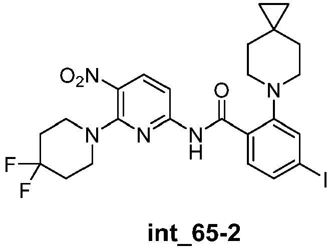

**Int_1-8** (100 mg, 0.279 mmol) was dissolved in DCM (8 mL), and oxalyl chloride (1 mL) was added. The reaction solution was stirred at room temperature for 2 h and then concentrated at reduced pressure to remove the solvent, thus giving an acyl chloride product. **Int_65-1** (72 mg, 0.279 mmol) was dissolved in tetrahydrofuran (5 mL), and sodium hydride (60 mg) was slowly added under an ice bath. The reaction solution was incubated at room temperature for reaction for 1 h, and the prepared acyl chloride product was added to the reaction solution. The reaction solution was incubated at 40 °C for reaction for 12 h, until LC-MS indicated the completion of the reaction. The reaction solution was diluted with water (50 mL), and the aqueous phase was extracted with dichloromethane (50 mL × 3). The organic phase was dried over anhydrous sodium sulfate, filtered, and distilled at reduced pressure to give a crude product. The crude product was purified by column chromatography to give the target product (130 mg, yield: 78%).

ESI-MS m/z: 598 [M+H]⁺.

### Step 2: Synthesis of compound 65

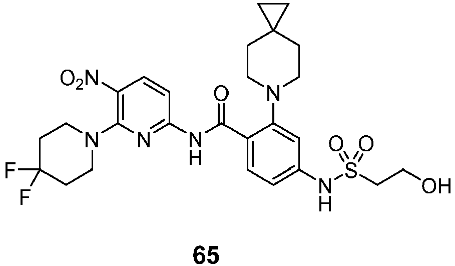

**Int_1-10** (40 mg, 0.325 mmol), (1*S*,2*S*)-*N*,*N*-dimethylcyclohexane (15 mg, 0.108 mmol), cuprous iodide (20 mg, 0.108 mmol), and potassium phosphate (138 mg, 0.651 mmol) were dissolved in DMF (10 mL). The mixture was purged with argon three times before **int_65-2** (130 mg, 0.217 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (10 mg, yield: 7.7%).

¹H NMR (400 MHz, DMSO-d6) δ 13.70 (s, 1H), 8.44 (d, *J* = 8.9 Hz, 1H), 8.06 (d, *J* = 8.7 Hz, 1H), 7.86 (d, *J* = 8.9 Hz, 1H), 7.24 (d, *J* = 2.1 Hz, 1H), 7.10 (dd, *J* = 8.7, 2.1 Hz, 1H), 3.75 (t, *J* = 6.5 Hz, 2H), 3.50 (t, *J* = 5.7 Hz, 4H), 3.33 (s, 2H), 2.99 (t, *J* = 5.2 Hz, 4H), 2.12 (d, *J* = 8.1 Hz, 4H), 1.86 - 1.48 (m, 4H), 0.40 (s, 4H). ESI-MS m/z: 595 [M+H]⁺.

### Example 8. Synthesis of Compound 97

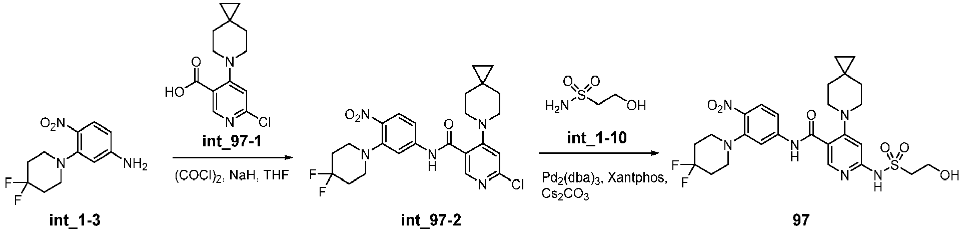

### Step 1: Synthesis of compound int_97-2

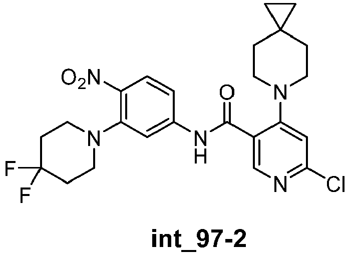

**Int_97-1** (100 mg, 0.375 mmol) was dissolved in DCM (8 mL), and oxalyl chloride (1 mL) was added. The reaction solution was stirred at room temperature for 2 h and then concentrated at reduced pressure to remove the solvent, thus giving an acyl chloride product. **Int_1-3** (96 mg, 0.375 mmol) was dissolved in tetrahydrofuran (5 mL), and sodium hydride (60 mg) was slowly added under an ice bath. The reaction solution was incubated at room temperature for reaction for 1 h, and the prepared acyl chloride product was added to the reaction solution. The reaction solution was incubated at 40 °C for reaction for 12 h, until LC-MS indicated the completion of the reaction. The reaction solution was diluted with water (50 mL), and the aqueous phase was extracted with dichloromethane (50 mL × 3). The organic phase was dried over anhydrous sodium sulfate, filtered, and distilled at reduced pressure to give a crude product. The crude product was purified by column chromatography to give the target product (128 mg, yield: 68.1%).

ESI-MS m/z: 506 [M+H]⁺.

### Step 2: Synthesis of compound 97

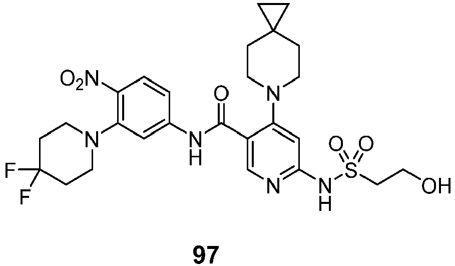

**Int_1-10** (52 mg, 0.414 mmol), cesium carbonate (135 mg, 0.414 mmol), Pd₂(dba)₃ (12 mg, 0.0138 mmol), XantPhos (19 mg, 0.033 mmol), and **int_97-2** (128 mg, 0.253 mmol) were dissolved in dioxane (10 mL), and the mixture was purged with argon three times. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (10 mg, yield: 6.7%).

¹H NMR (400 MHz, DMSO-d6) δ 10.69 (s, 1H), 7.99 (d, *J* = 9.2 Hz, 2H), 7.72 (s, 1H), 7.41 (d, *J* = 8.8 Hz, 1H), 6.53 (d, *J* = 7.8 Hz, 1H), 3.74 (t, *J* = 6.9 Hz, 2H), 3.11 (dd, *J* = 13.5, 6.6 Hz, 8H), 2.09 (d, *J* = 15.1 Hz, 4H), 1.37 (m, 4H), 0.28 (s, 4H).

ESI-MS m/z: 595 [M+H]⁺.

### Example 9. Synthesis of Compound 129

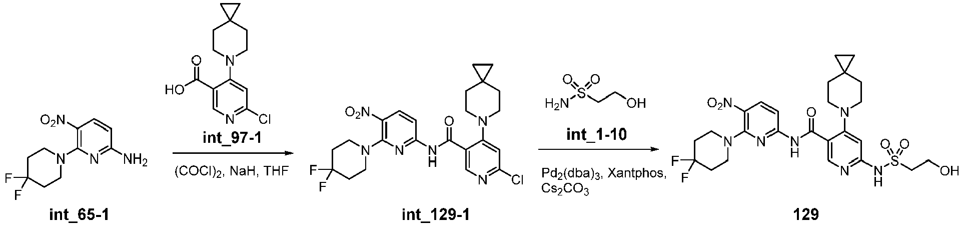

### Step 1: Synthesis of compound int_129-1

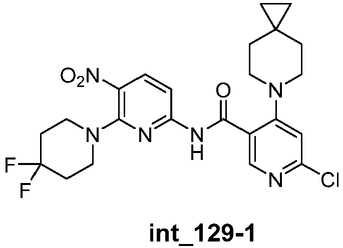

**Int_97-1** (100 mg, 0.375 mmol) was dissolved in DCM (8 mL), and oxalyl chloride (1 mL) was added. The reaction solution was stirred at room temperature for 2 h and then concentrated at reduced pressure to remove the solvent, thus giving an acyl chloride product. **Int_65-1** (96 mg, 0.375 mmol) was dissolved in tetrahydrofuran (5 mL), and sodium hydride (60 mg) was slowly added under an ice bath. The reaction solution was incubated at room temperature for reaction for 1 h, and the prepared acyl chloride product was added to the reaction solution. The reaction solution was incubated at 40 °C for reaction for 12 h, until LC-MS indicated the completion of the reaction. The reaction solution was diluted with water (50 mL), and the aqueous phase was extracted with dichloromethane (50 mL × 3). The organic phase was dried over anhydrous sodium sulfate, filtered, and distilled at reduced pressure to give a crude product. The crude product was purified by column chromatography to give the target product (140 mg, yield: 74.4%).

ESI-MS m/z: 507 [M+H]⁺.

### Step 2: Synthesis of compound 129

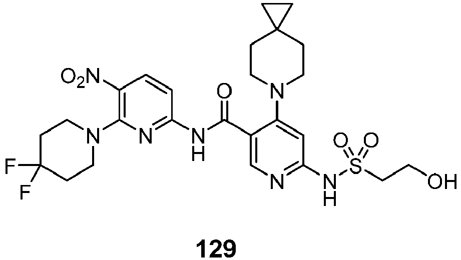

**Int_1-10** (52 mg, 0.414 mmol), cesium carbonate (135 mg, 0.414 mmol), Pd₂(dba)₃ (12 mg, 0.0138 mmol), XantPhos (19 mg, 0.033 mmol), and **int_129-1** (140 mg, 0.276 mmol) were dissolved in dioxane (10 mL), and mixture was purged with argon three times. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (20 mg, yield: 12.2%).

¹H NMR (400 MHz, DMSO-d6) δ 12.69 (s, 1H), 8.57 (s, 1H), 8.39 (d, *J* = 9.0 Hz, 1H), 7.82 (d, *J* = 8.9 Hz, 1H), 6.33 (s, 1H), 4.80 (s, 1H), 3.65 (t, *J* = 7.0 Hz, 2H), 3.48 (t, *J* = 5.7 Hz, 4H), 3.17 - 3.12 (m, 2H), 2.93 (t, *J* = 5.2 Hz, 4H), 2.08 (d, *J* = 14.6 Hz, 4H), 1.65 (s, 4H), 0.35 (s, 4H).

ESI-MS m/z: 596 [M+H]⁺.

### Example 10. Synthesis of Compound 161

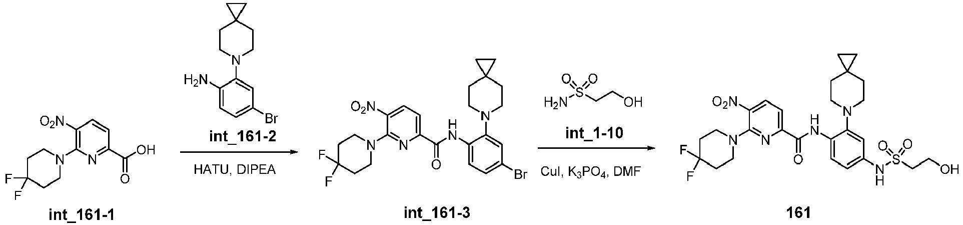

### Step 1: Synthesis of compound int_161-3

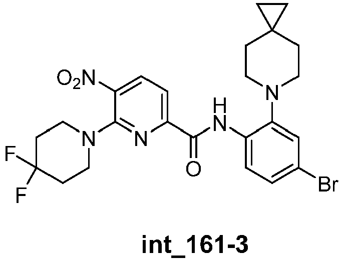

**Int_161-1** (100 mg, 0.348 mmol) was dissolved in DMF (4 mL), and HATU (264 mg, 0.696 mmol), DIPEA (163 mg, 1.264 mmol), and **int_161-2** (98 mg, 0.348 mmol) were added. The reaction solution was stirred at 60 °C for 4 h, until LC-MS indicated the completion of the reaction. The reaction solution was concentrated at reduced pressure to give a crude product. The crude product was purified by column chromatography to give a solid (150 mg, yield: 78%).

ESI-MS m/z: 550 [M+H]⁺.

### Step 2: Synthesis of compound 161

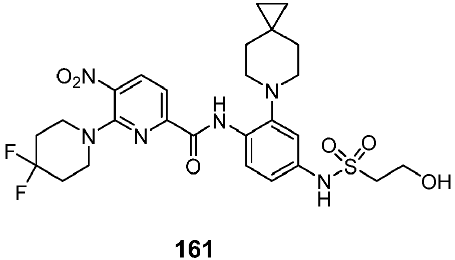

**Int_1-10** (24 mg, 0.191 mmol), sarcosine (6 mg, 0.064 mmol), cuprous iodide (12 mg, 0.062 mmol), and potassium phosphate (80 mg, 0.369 mmol) were dissolved in DMF (5 mL). The mixture was purged with argon three times before **int_161-3** (70 mg, 0.127 mmol) was added. In argon atmosphere, the reaction solution was heated to 130 °C with microwave and incubated for reaction for 3 h, until LC-MS indicated the completion of the reaction. The reaction solution was cooled to room temperature, concentrated by rotary evaporation, and purified by column chromatography to give a solid (22 mg, yield: 19%).

¹H NMR (400 MHz, DMSO-d6) δ 10.37 (s, 1H), 8.54 (d, *J* = 8.2 Hz, 1H), 8.32 (d, *J* = 8.8 Hz, 1H), 7.72 (d, *J* = 8.2 Hz, 1H), 7.19 (d, *J* = 2.4 Hz, 1H), 7.02 (dd, *J* = 8.8, 2.4 Hz, 1H), 3.74 (t, *J* = 6.7 Hz, 2H), 3.64 (t, *J* = 5.8 Hz, 4H), 3.21 (t, *J* = 6.7 Hz, 2H), 2.83 (t, *J* = 5.3 Hz, 4H), 2.25 - 2.13 (m, 4H), 1.53 (s, 4H), 0.37 (s, 4H). ESI-MS m/z: 595 [M+H]⁺.

### Example 11. Synthesis of Compound 257

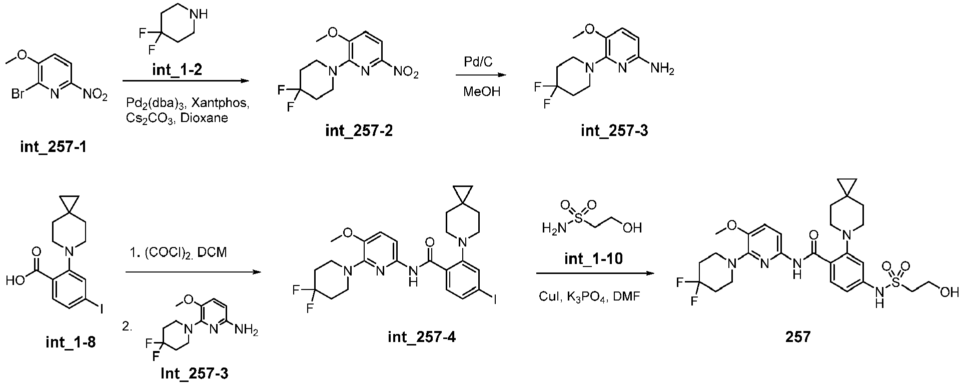

### Step 1: Synthesis of compound int_257-2

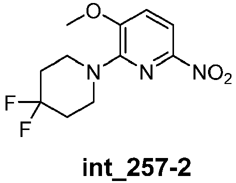

**Int_257-1** (25 g, 107 mmol) was dissolved in dioxane (400 mL), and int_1-2 (20 g, 161 mmol), Pd₂(dba)₃ (5 g, 5.4 mmol), Xantphos (3 g, 5.4 mmol), and Cs₂CO₃ (104 g, 321 mmol) were added. In nitrogen atmosphere, the mixture was heated to 100 °C and incubated for reaction for 16 h, until LC-MS indicated the completion of the reaction. The reaction solution was filtered to give a filtrate, and the filtrate was diluted with water (500 mL). The aqueous phase was extracted with ethyl acetate (500 mL × 3), and the organic phase was dried over anhydrous sodium sulfate, filtered, and distilled at reduced pressure to give a crude product. The crude product was subjected to column chromatography (SiO₂, n-hexane/ethyl acetate = 5:1) to give the target product (5.5 g, yield: 19%).

ESI-MS m/z: 274 [M+H]⁺.

### Step 2: Synthesis of compound int_257-3

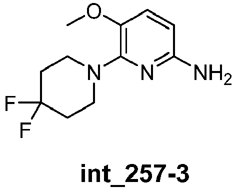

**Int_257-2** (5.5 g, 20 mmol) was dissolved in methanol (100 mL), and Pd/C (2.00 g, 10% purity) was added. The reaction system was purged with hydrogen 3 times. In hydrogen atmosphere, the reaction solution was incubated at 60 °C for reaction for 16 h, until LC-MS indicated the completion of the reaction. The reaction solution was filtered, and the filtrate was concentrated at reduced pressure to give a crude product (4.2 g, yield: 86%). The crude product was used directly in the next reaction.

ESI-MS m/z: 244 [M+H]⁺.

### Step 3: Synthesis of compound int_257-4

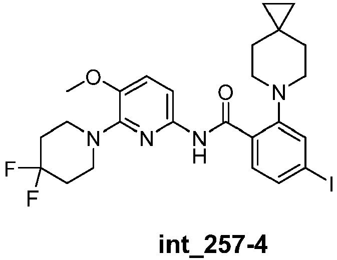

**Int_1-8** (1.2 g, 3.36 mmol) was dissolved in DCM (50 mL), and oxalyl chloride (888.4 mg, 7 mmol) was added. The reaction solution was stirred at room temperature for 2 h and then concentrated at reduced pressure to remove the solvent, thus giving an acyl chloride product.

**Int_257-3** (0.7 g, 3.4 mmol) was dissolved in tetrahydrofuran (40 mL), and in nitrogen atmosphere, NaH (720 mg, 18 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (1.2 g, yield: 71%).

ESI-MS m/z: 583 [M+H]⁺.

### Step 4: Synthesis of compound 257

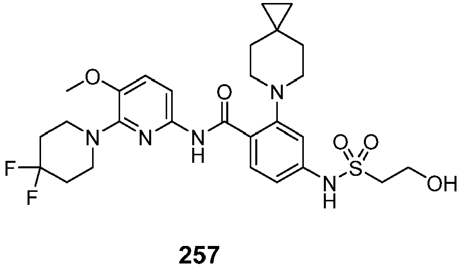

**Int_257-4** (1 g, 1.7 mmol), (1*S*,2*S*)-*N*,*N*'-dimethyl-1,2-cyclohexanediamine (122 mg, 0.85 mmol), cuprous iodide (164 mg, 0.85 mmol), and potassium phosphate (1.1 g, 5.1 mmol) were dissolved in DMF (20 mL). The mixture was purged with argon three times before **int_1-10** (0.43 g, 3.4 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography (SiO₂, n-hexane/ethyl acetate = 1:1) to give a solid (0.77 g, yield: 77%).

¹H NMR (400 MHz, DMSO-d6) δ 12.81 (s, 1H), 10.16 (s, 1H), 8.06 (d, *J* = 8.6 Hz, 1H), 7.81 (d, *J* = 8.5 Hz, 1H), 7.36 (d, *J* = 8.6 Hz, 1H), 7.25 (d, *J* = 2.2 Hz, 1H), 7.11 (dd, *J* = 8.6, 2.1 Hz, 1H), 4.93 (s, 1H), 3.81 (s, 3H), 3.76 (t, *J* = 6.5 Hz, 2H), 3.52 (t, *J* = 5.6 Hz, 4H), 3.35 (t, *J* = 6.5 Hz, 2H), 2.97 (t, *J* = 5.4 Hz, 4H), 2.09 (td, *J* = 14.1, 6.7 Hz, 4H), 1.72 (s, 4H), 0.38 (s, 4H).

ESI-MS m/z: 580 [M+H]⁺.

### Example 12. Synthesis of Compound 258

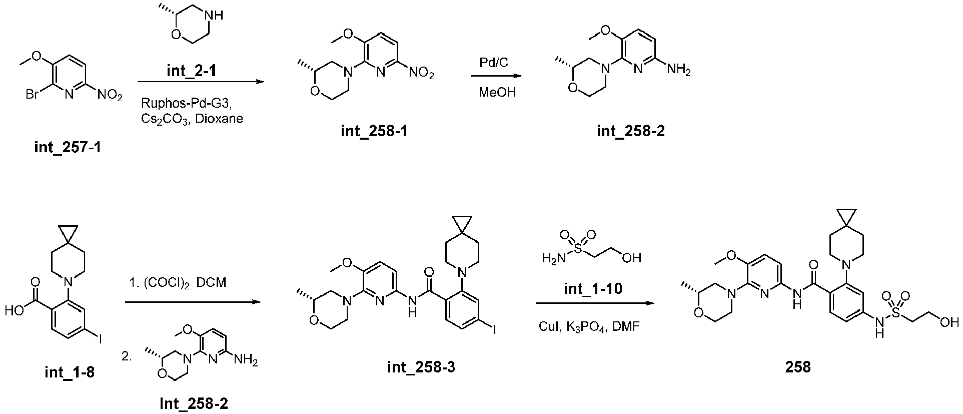

### Step 1: Synthesis of compound int_258-1

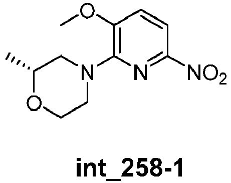

**Int_257-1** (1 g, 4.29 mmol) was dissolved in dioxane (30 mL), and **int_2-1** (480 mg, 4.72 mmol), Ruphos-Pd-G3 (360 mg, 0.429 mmol), and Cs₂CO₃ (2.8 g, 8.58 mmol) were added. In nitrogen atmosphere, the mixture was heated to 100 °C and incubated for reaction for 12 h, until LC-MS indicated the completion of the reaction. The reaction solution was filtered to give a filtrate, and the filtrate was diluted with water (100 mL). The aqueous phase was extracted with ethyl acetate (100 mL × 3), and the organic phase was dried over anhydrous sodium sulfate, filtered, and distilled at reduced pressure to give a crude product. The crude product was subjected to column chromatography (SiO₂, n-hexane/ethyl acetate = 5:1) to give the target product (780 mg, yield: 71.8%).

ESI-MS m/z: 254 [M+H]⁺.

### Step 2: Synthesis of compound int_258-2

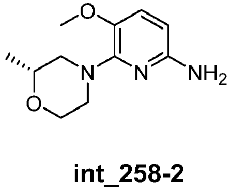

**Int_258-1** (780 mg, 3.08 mmol) was dissolved in methanol (20 mL), and Pd/C (200 mg, 10% purity) was added. The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (550 mg, yield: 80%). The crude product was used directly in the next reaction.

ESI-MS m/z: 224 [M+H]⁺.

### Step 3: Synthesis of compound int_258-3

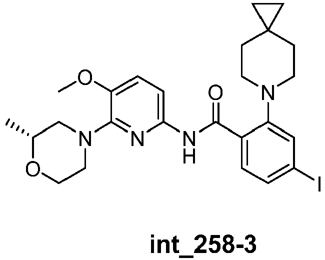

**Int_1-8** (190 mg, 0.532 mmol) was dissolved in DCM (10 mL), and oxalyl chloride (888.4 mg, 7 mmol) was added. The reaction solution was stirred at room temperature for 2 h and then concentrated at reduced pressure to remove the solvent, thus giving an acyl chloride product.

**Int_258-2** (120 mg, 0.532 mmol) was dissolved in tetrahydrofuran (10 mL), and in nitrogen atmosphere, NaH (72 mg, 1.8 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (250 mg, yield: 82.7%).

ESI-MS m/z: 563 [M+H]⁺.

### Step 4: Synthesis of compound 258

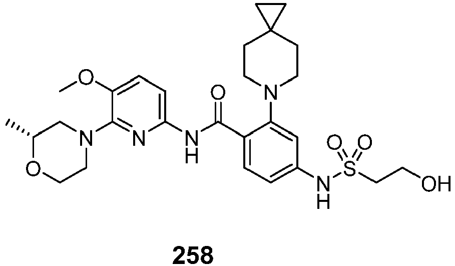

**Int_258-3** (250 mg, 0.444 mmol), (1*S*,2*S*)-*N*,*N*'-dimethyl-1,2-cyclohexanediamine (32 mg, 0.222 mmol), cuprous iodide (42 mg, 0.222 mmol), and potassium phosphate (282 mg, 1.332 mmol) were dissolved in DMF (20 mL). The mixture was purged with argon three times before **int_1-10** (111 mg, 0.888 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (150 mg, yield: 60.4%).

¹H NMR (400 MHz, DMSO-d6) δ 12.77 (s, 1H), 8.05 (d, *J* = 8.6 Hz, 1H), 7.78 (d, *J* = 8.4 Hz, 1H), 7.31 (d, *J* = 8.5 Hz, 1H), 7.24 (d, *J* = 2.1 Hz, 1H), 7.09 (dd, *J* = 8.6, 2.1 Hz, 1H), 3.86 - 3.72 (m, 6H), 3.65 (td, *J* = 11.7, 3.0 Hz, 2H), 2.95 (d, *J* = 5.4 Hz, 4H), 2.76 (td, *J* = 12.3, 3.3 Hz, 1H), 2.51 (d, *J* = 12.9 Hz, 2H), 1.71 (s, 4H), 1.12 (d, *J* = 6.2 Hz, 3H), 0.36 (s, 4H).

ESI-MS m/z: 560 [M+H]⁺.

### Example 13. Synthesis of Compound 259

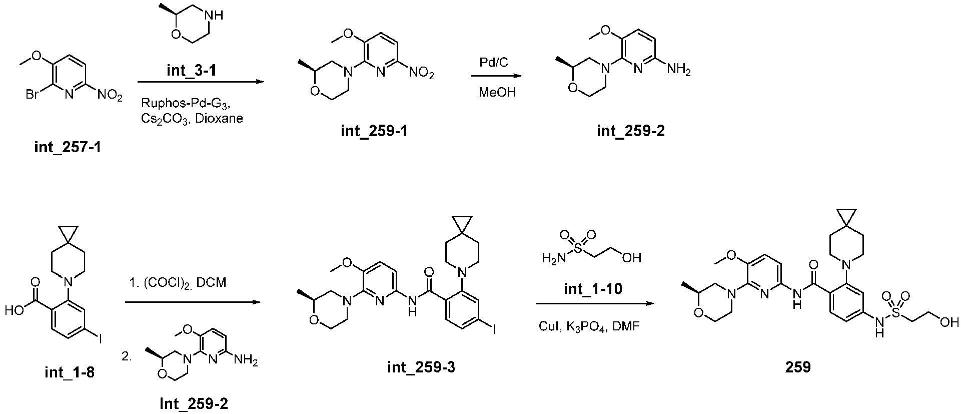

### Step 1: Synthesis of compound int_259-1

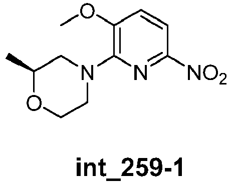

**Int_257-1** (1 g, 4.29 mmol) was dissolved in dioxane (30 mL), and **int_3-1** (480 mg, 4.72 mmol), Ruphos-Pd-G3 (360 mg, 0.429 mmol), and Cs₂CO₃ (2.7 g, 8.38 mmol) were added. In nitrogen atmosphere, the mixture was heated to 100 °C and incubated for reaction for 12 h, until LC-MS indicated the completion of the reaction. The reaction solution was filtered to give a filtrate, and the filtrate was diluted with water (100 mL). The aqueous phase was extracted with ethyl acetate (100 mL × 3), and the organic phase was dried over anhydrous sodium sulfate, filtered, and distilled at reduced pressure to give a crude product. The crude product was subjected to column chromatography (SiO₂, n-hexane/ethyl acetate = 5:1) to give the target product (750 mg, yield: 69.4%).

ESI-MS m/z: 254 [M+H]⁺.

### Step 2: Synthesis of compound int_259-2

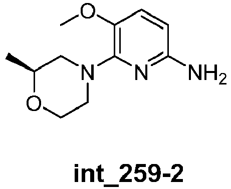

**Int_259-1** (750 mg, 2.964 mmol) was dissolved in methanol (20 mL), and Pd/C (200 mg, 10% purity) was added. The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (560 mg, yield: 84.7%). The crude product was used directly in the next reaction.

ESI-MS m/z: 224 [M+H]⁺.

### Step 3: Synthesis of compound int_259-3

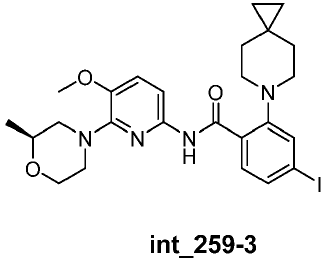

**Int_1-8** (335 mg, 0.938 mmol) was dissolved in DCM (10 mL), and oxalyl chloride (888.4 mg, 7 mmol) was added. The reaction solution was stirred at room temperature for 2 h and then concentrated at reduced pressure to remove the solvent, thus giving an acyl chloride product.

**Int_259-2** (200 mg, 0.893 mmol) was dissolved in tetrahydrofuran (10 mL), and in nitrogen atmosphere, NaH (170 mg, 4.465 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (340 mg, yield: 67.7%).

ESI-MS m/z: 563 [M+H]⁺.

### Step 4: Synthesis of compound 259

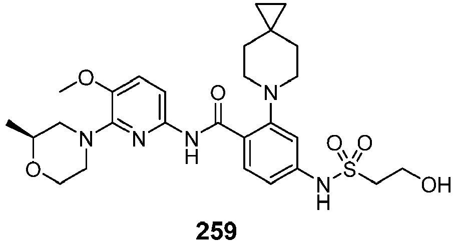

**Int_259-3** (340 mg, 0.604 mmol), (1*S*,2*S*)-*N*,*N*'-dimethyl-1,2-cyclohexanediamine (44 mg, 0.302 mmol), cuprous iodide (58 mg, 0.302 mmol), and potassium phosphate (384 mg, 1.810 mmol) were dissolved in DMF (15 mL). The mixture was purged with argon three times before **int_1-10** (151 mg, 1.210 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (150 mg, yield: 44.4%).

ESI-MS m/z: 560 [M+H]⁺.

### Example 14. Synthesis of Compound 260

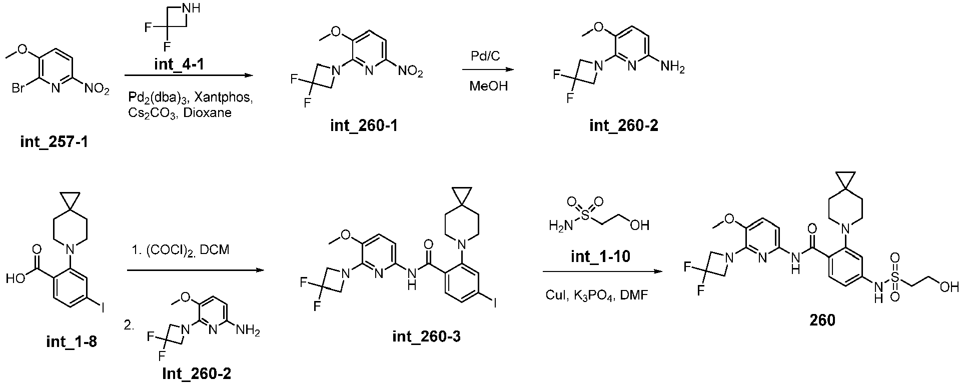

### Step 1: Synthesis of compound int_260-1

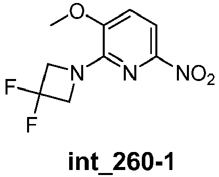

**Int_257-1** (200 mg, 0.858 mmol) was dissolved in dioxane (15 mL), and **int_4-1** (hydrochloride, 167 mg, 1.287 mmol), Pd₂(dba)₃ (78 mg, 0.086 mmol), Xantphos (49 mg, 0.086 mmol), and Cs₂CO₃ (839 mg, 2.575 mmol) were added. In nitrogen atmosphere, the mixture was heated to 100 °C and incubated for reaction for 12 h, until LC-MS indicated the completion of the reaction. The reaction solution was filtered to give a filtrate, and the filtrate was diluted with water (50 mL). The aqueous phase was extracted with ethyl acetate (50 mL × 3), and the organic phase was dried over anhydrous sodium sulfate, filtered, and distilled at reduced pressure to give a crude product, and the crude product was subjected to column chromatography to give the target product (80 mg, yield: 36.7%).

ESI-MS m/z: 246 [M+H]⁺.

### Step 2: Synthesis of compound int_260-2

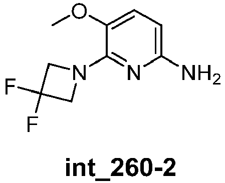

**Int_260-1** (80 mg, 0.858 mmol) was dissolved in methanol (10 mL), and Pd/C (20 mg, 10% purity) was added.

The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (60 mg, yield: 89.5%). The crude product was used directly in the next reaction.

ESI-MS m/z: 216 [M+H]⁺.

### Step 3: Synthesis of compound int_260-3

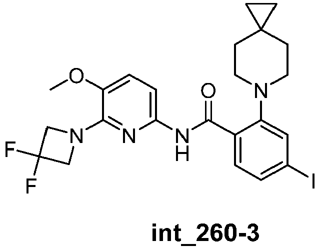

**Int_1-8** (95 mg, 0.266 mmol) was dissolved in DCM (10 mL), and oxalyl chloride (380.7 mg, 3 mmol) was added. The reaction solution was stirred at room temperature for 2 h and then concentrated at reduced pressure to remove the solvent, thus giving an acyl chloride product.

**Int_260-2** (60 mg, 0.279 mmol) was dissolved in tetrahydrofuran (5 mL), and in nitrogen atmosphere, NaH (100 mg, 4.166 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (80 mg, yield: 51.9%).

ESI-MS m/z: 555 [M+H]⁺.

### Step 4: Synthesis of compound 260

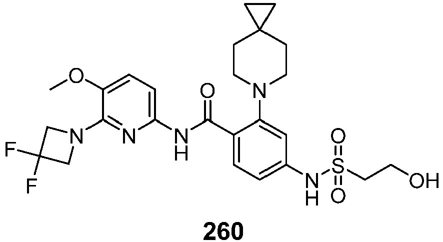

**Int_260-3** (80 mg, 0.144 mmol), (1*S*,2*S*)-*N*,*N*-dimethyl-1,2-cyclohexanediamine (11 mg, 0.072 mmol), cuprous iodide (14 mg, 0.072 mmol), and potassium phosphate (92 mg, 0.433 mmol) were dissolved in DMF (5 mL). The mixture was purged with argon three times before **int_1-10** (36 mg, 0.289 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (40 mg, yield: 50.6%).

¹H NMR (400 MHz, DMSO-d6) δ 13.03 (s, 1H), 8.04 (d, *J* = 8.6 Hz, 1H), 7.67 (d, *J* = 8.4 Hz, 1H), 7.28 (d, *J* = 8.5 Hz, 1H), 7.23 (d, *J* = 2.1 Hz, 1H), 7.09 (dd, *J* = 8.6, 2.1 Hz, 1H), 4.40 (t, *J* = 12.6 Hz, 4H), 3.74 (d, *J* = 3.4 Hz, 5H), 3.33 (m, 2H), 2.94 (t, *J* = 5.5 Hz, 4H), 1.94-1.58 (m, 4H), 0.37 (s, 4H).

ESI-MS m/z: 552 [M+H]⁺.

### Example 15. Synthesis of Compound 261

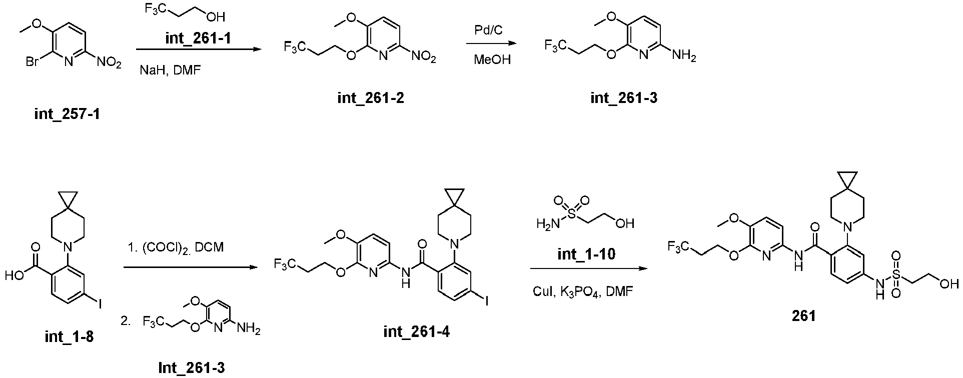

### Step 1: Synthesis of compound int_261-2

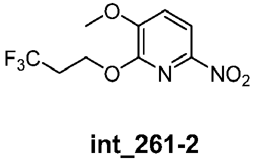

**Int_261-1** (300 mg, 2.618 mmol) was dissolved in DMF (30 mL), and NaH (208 mg, 5.2 mmol, 60% purity) was added at 0 °C in nitrogen atmosphere. The reaction solution was incubated at 0 °C for reaction for 1 h in nitrogen atmosphere before **int_257-1** (610 mg, 2.618 mmol) was added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was subjected to column chromatography to give the target product (500 mg, yield: 71.8%).

ESI-MS m/z: 267 [M+H]⁺.

### Step 2: Synthesis of compound int_261-3

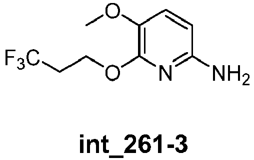

**Int_261-2** (500 mg, 1.880 mmol) was dissolved in methanol (20 mL), and Pd/C (50 mg, 10% purity) was added. The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (410 mg, yield: 92.3%). The crude product was used directly in the next reaction.

ESI-MS m/z: 237 [M+H]⁺.

### Step 3: Synthesis of compound int_261-4

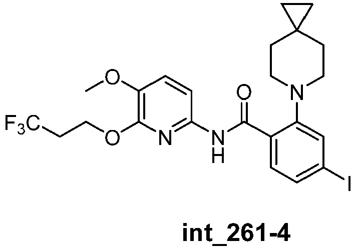

**Int_1-8** (682 mg, 1.910 mmol) was dissolved in DCM (10 mL), and oxalyl chloride (482 mg, 3 mmol) was added. The reaction solution was stirred at room temperature for 2 h and then concentrated at reduced pressure to remove the solvent, thus giving an acyl chloride product.

**Int_261-3** (450 mg, 1.910 mmol) was dissolved in tetrahydrofuran (10 mL), and in nitrogen atmosphere, NaH (366 mg, 9.550 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (500 mg, yield: 45.9%).

ESI-MS m/z: 576 [M+H]⁺.

### Step 4: Synthesis of compound 261

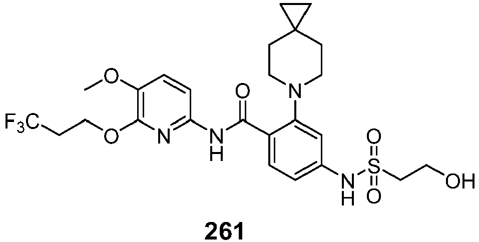

**Int_261-4** (100 mg, 0.174 mmol), (1*S*,2*S*)-*N*,*N*'-dimethyl-1,2-cyclohexanediamine (13 mg, 0.087 mmol), cuprous iodide (17 mg, 0.087 mmol), and potassium phosphate (111 mg, 0.523 mmol) were dissolved in DMF (5 mL). The mixture was purged with argon three times before **int_1-10** (44 mg, 0.348 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (25 mg, yield: 25.3%).

¹H NMR (400 MHz, Methanol-d4) δ 8.11 (d, *J* = 8.6 Hz, 1H), 7.86 (d, *J* = 8.4 Hz, 1H), 7.40-7.27 (m, 2H), 7.14 (dd, *J* = 8.6, 2.2 Hz, 1H), 4.58 (t, *J* = 6.3 Hz, 2H), 3.94 (t, *J* = 6.2 Hz, 2H), 3.84 (s, 3H), 3.36 (t, *J* = 6.2 Hz, 2H), 3.07 (t, *J* = 5.3 Hz, 4H), 2.75 (qt, *J* = 10.9, 6.3 Hz, 2H), 1.79 (s, 4H), 0.42 (s, 4H).

ESI-MS m/z: 573 [M+H]⁺.

### Example 16. Synthesis of Compound 262

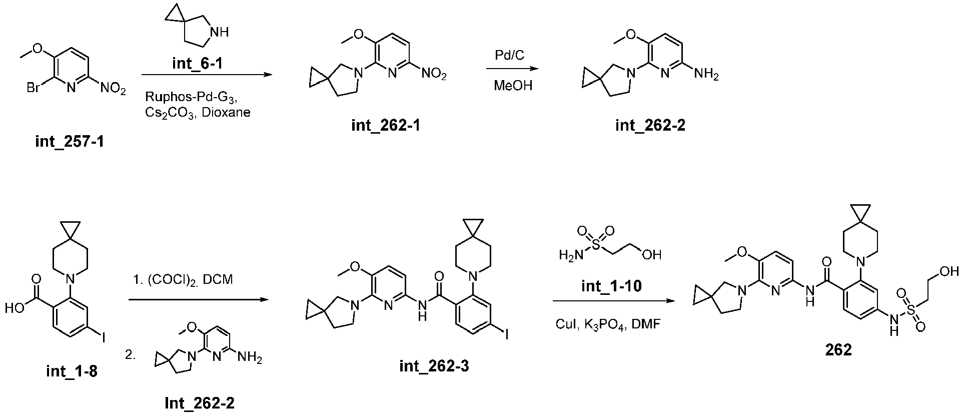

### Step 1: Synthesis of compound int_262-1

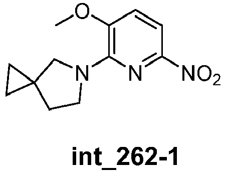

**Int_257-1** (348 mg, 1.5 mmol) was dissolved in dioxane (15 mL), and **int_6-1** (hydrochloride, 200 mg, 1.5 mmol), Ruphos-Pd-G3 (125 mg, 0.15 mmol), and Cs₂CO₃ (977 mg, 3 mmol) were added. In nitrogen atmosphere, the mixture was heated to 100 °C and incubated for reaction for 12 h, until LC-MS indicated the completion of the reaction. The reaction solution was filtered to give a filtrate, and the filtrate was diluted with water (50 mL). The aqueous phase was extracted with ethyl acetate (50 mL × 3), and the organic phase was dried over anhydrous sodium sulfate, filtered, and distilled at reduced pressure to give a crude product. The crude product was subjected to column chromatography to give the target product (320 mg, yield: 68.6%).

ESI-MS m/z: 250 [M+H]⁺.

### Step 2: Synthesis of compound int_262-2

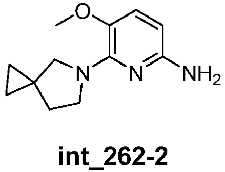

**Int_262-1** (320 mg, 1.28 mmol) was dissolved in methanol (20 mL), and Pd/C (30 mg, 10% purity) was added. The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (165 mg, yield: 57.8%). The crude product was used directly in the next reaction.

ESI-MS m/z: 220 [M+H]⁺.

### Step 3: Synthesis of compound int_262-3

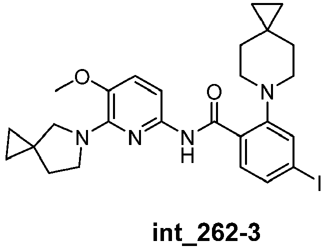

**Int_1-8** (270 mg, 0.752 mmol) was dissolved in DCM (10 mL), and oxalyl chloride (380.7 mg, 3 mmol) was added. The reaction solution was stirred at room temperature for 2 h and then concentrated at reduced pressure to remove the solvent, thus giving an acyl chloride product.

**Int_262-2** (165 mg, 0.752 mmol) was dissolved in tetrahydrofuran (5 mL), and in nitrogen atmosphere, NaH (150 mg, 3.76 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 5:1) to give a solid (170 mg, yield: 40.4%).

ESI-MS m/z: 559 [M+H]⁺.

### Step 4: Synthesis of compound 262

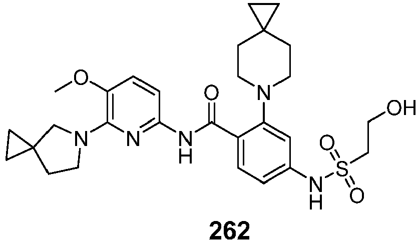

**Int_262-3** (170 mg, 0.304 mmol), (1*S*,2*S*)-*N*,*N*'-dimethyl-1,2-cyclohexanediamine (22 mg, 0.152 mmol), cuprous iodide (29 mg, 0.152 mmol), and potassium phosphate (193 mg, 912 mmol) were dissolved in DMF (10 mL). The mixture was purged with argon three times before **int_1-10** (76 mg, 0.608 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (120 mg, yield: 71%).

¹H NMR (400 MHz, DMSO-d6) δ 12.75 (s, 1H), 8.04 (d, *J* = 8.6 Hz, 1H), 7.48 (d, *J* = 8.3 Hz, 1H), 7.22 (d, *J* = 2.2 Hz, 1H), 7.15 (d, *J* = 8.4 Hz, 1H), 7.08 (dd, *J* = 8.6, 2.1 Hz, 1H), 3.74 (dt, *J* = 7.0, 3.6 Hz, 4H), 3.70 (s, 3H), 3.47 (s, 2H), 2.93 (d, *J* = 5.2 Hz, 4H), 1.79 (s, 6H), 0.57 (s, 4H), 0.33 (s, 4H).

ESI-MS m/z: 556 [M+H]⁺.

### Example 17. Synthesis of Compound 263

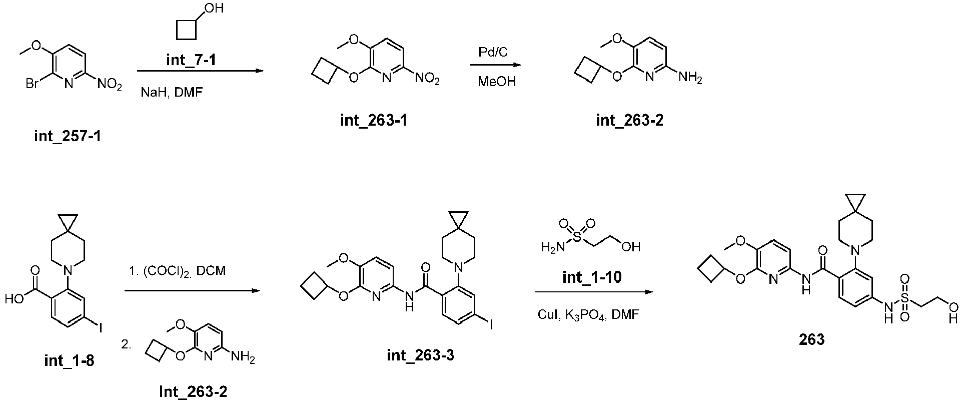

### Step 1: Synthesis of compound int_263-1

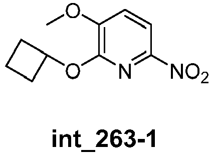

**Int_7-1** (460 mg, 1.974 mmol) was dissolved in DMF (20 mL), and NaH (510 mg, 3.948 mmol, 60% purity) was added at 0 °C in nitrogen atmosphere. The reaction solution was incubated at 0 °C for reaction for 1 h in nitrogen atmosphere before **int_257-1** (1.5 g, 1.974 mmol) was added. The mixture was heated to 80 °C and incubated for reaction for 24 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product The crude product was subjected to column chromatography to give the target product (300 mg, yield: 67.9%).

ESI-MS m/z: 225 [M+H]⁺.

### Step 2: Synthesis of compound int_263-2

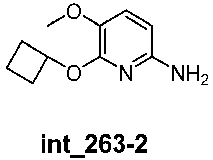

**Int_263-1** (300 mg, 1.339 mmol) was dissolved in methanol (30 mL), and Pd/C (30 mg, 10% purity) was added. The reaction system was purged with hydrogen 3 times. In hydrogen atmosphere, the reaction solution was incubated at room temperature for reaction for 12 h, until LC-MS indicated the completion of the reaction. The reaction solution was filtered, and the filtrate was concentrated at reduced pressure to give a crude product (255 mg, yield: 98%). The crude product was used directly in the next reaction.

ESI-MS m/z: 195 [M+H]⁺.

### Step 3: Synthesis of compound int_263-3

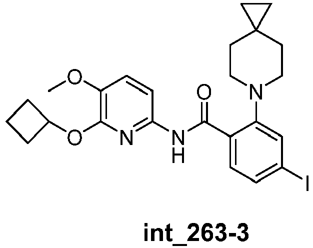

**Int_1-8** (552 mg, 1.546 mmol) was dissolved in DCM (10 mL), and oxalyl chloride (482 mg, 3 mmol) was added. The reaction solution was stirred at room temperature for 2 h and then concentrated at reduced pressure to remove the solvent, thus giving an acyl chloride product.

**Int_263-2** (300 mg, 1.546 mmol) was dissolved in tetrahydrofuran (10 mL), and in nitrogen atmosphere, NaH (180 mg, 4.5 mmol, 60% purity) was added. The mixture was stirred at room temperature for 0.5 h and then the acyl chloride prepared previously was added at room temperature. The reaction solution was warmed to 40 °C and stirred for 2 h, until LC-MS indicated the completion of the reaction. Methanol was added under an ice bath to quench the reaction, and the reaction solution was concentrated at reduced pressure to give a crude product. The crude product was purified by column chromatography to give a solid (510 mg, yield: 63.8%). ESI-MS m/z: 534 [M+H]⁺.

### Step 4: Synthesis of compound 263

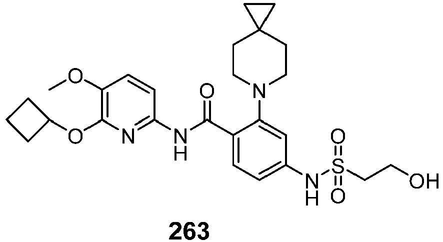

**Int_263-3** (150 mg, 0.281 mmol), (1*S*,2*S*)-*N*,*N*'-dimethyl-1,2-cyclohexanediamine (21 mg, 0.141 mmol), cuprous iodide (27 mg, 0.141 mmol), and potassium phosphate (180 mg, 0.843 mmol) were dissolved in DMF (5 mL). The mixture was purged with argon three times before **int_1-10** (70 mg, 0.562 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (55 mg, yield: 36.9%).

¹H NMR (400 MHz, DMSO-d6) δ 12.63 (s, 1H), 8.04 (d, *J* = 8.6 Hz, 1H), 7.81 (d, *J* = 8.4 Hz, 1H), 7.34 (d, *J* = 8.6 Hz, 1H), 7.24 (d, *J* = 2.1 Hz, 1H), 7.09 (dd, *J* = 8.6, 2.1 Hz, 1H), 5.31-5.19 (m, 1H), 3.74 (d, *J* = 4.5 Hz, 5H), 2.96 (t, *J* = 5.3 Hz, 4H), 2.48-2.39 (m, 2H), 2.08 (dtd, *J* = 12.5, 10.0, 8.0 Hz, 2H), 1.80-1.53 (m, 6H), 0.41 (s, 4H).

ESI-MS m/z: 531 [M+H]⁺.

### Example 18. Synthesis of Compound 273

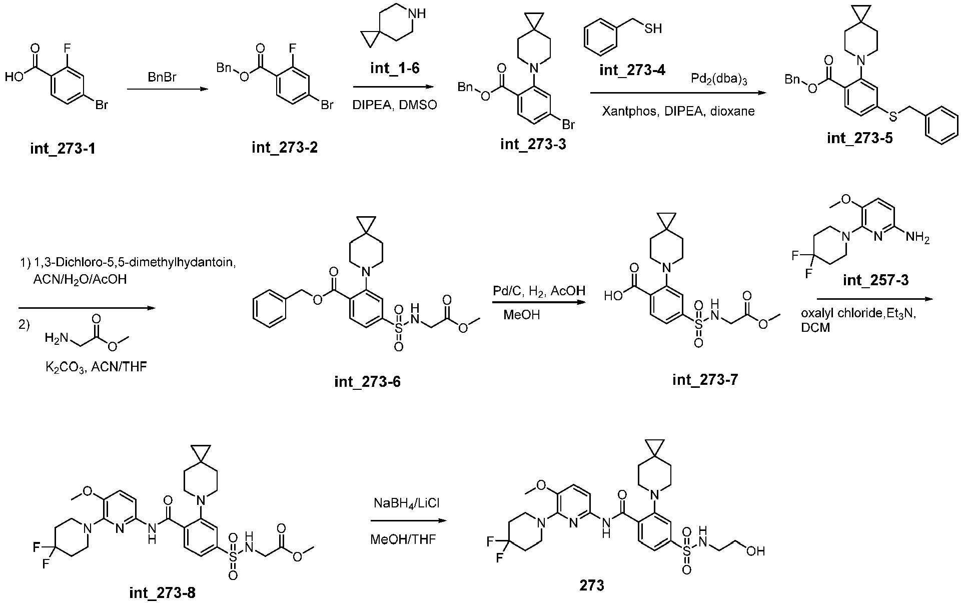

### Step 1: Synthesis of compound int_273-2

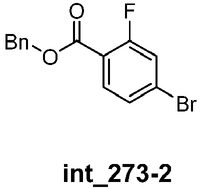

**Int_273-1** (5 g, 22.8 mmol) was dissolved in DMF (50 mL), and sodium carbonate (4.9 g, 46.2 mmol) and benzyl bromide (5.9 g, 34.5 mmol) were added. The mixture was incubated at room temperature for reaction for 24 h, until LC-MS indicated the completion of the reaction. The reaction solution was diluted with water (200 mL), and the aqueous phase was extracted with ethyl acetate (200 mL × 3). The organic phase was dried over anhydrous sodium sulfate, filtered, and distilled at reduced pressure to give a crude product. The crude product was purified by column chromatography (SiO₂, n-hexane/ethyl acetate = 30:1) to give the target product (6.9 g, yield: 99%).

ESI-MS m/z: 309 [M+H]⁺.

### Step 2: Synthesis of compound int_273-3

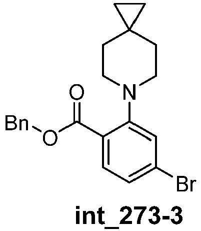

**Int_273-2** (5 g, 16.2 mmol) was dissolved in DMSO (20 mL), and DIPEA (6.3 g, 48.8 mmol) and **int_1-6** (hydrochloride, 4.8 g, 32.5 mmol) were added. The mixture was heated to 100 °C and incubated for reaction for 16 h, until LC-MS indicated the completion of the reaction. The reaction solution was diluted with water (100 mL), and the aqueous phase was extracted with ethyl acetate (100 mL × 3). The organic phase was dried over anhydrous sodium sulfate, filtered, and distilled at reduced pressure to give a crude product. The crude product was purified by column chromatography (SiO₂, n-hexane/ethyl acetate = 30:1) to give the target product (6.1 g, yield: 92%).

ESI-MS m/z: 400 [M+H]⁺.

### Step 3: Synthesis of compound int_273-5

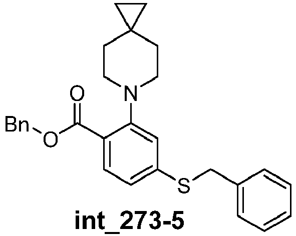

**Int_273-3** (6.1 g, 15.3 mmol) was dissolved in dioxane (40 mL), and benzylmercaptan (5.7 g, 45.9 mmol), Pd₂(dba)₃ (2 g, 2.2 mmol), Xantphos (2 g, 3.6 mmol), and DIPEA (7.9 g, 61.2 mmol) were added. In nitrogen atmosphere, the mixture was heated to 100 °C and incubated for reaction for 16 h, until LC-MS indicated the completion of the reaction. The reaction solution was diluted with water (100 mL), and the aqueous phase was extracted with ethyl acetate (100 mL × 3). The organic phase was dried over anhydrous sodium sulfate, filtered, and distilled at reduced pressure to give a crude product. The crude product was purified by column chromatography (SiO₂, n-hexane/ethyl acetate = 10:1) to give the target product (6.7 g, yield: 97%).

ESI-MS m/z: 444 [M+H]⁺.

### Step 4: Synthesis of compound int_273-6

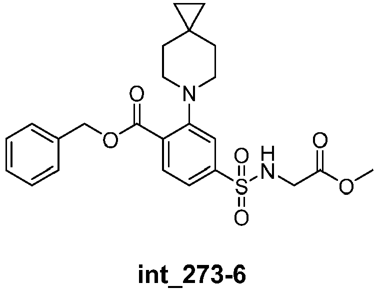

**Int_273-5** (16.3 g, 43.9 mmol) was dissolved in a mixed solvent of acetonitrile/water/acetic acid (40 mL/1 mL/0.5 mL), and dichlorohydantoin (3.4 g, 17.3 mmol) was added under an ice bath. In nitrogen atmosphere, the mixture was stirred at 0 °C for reaction for 0.5 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was dissolved in a mixed solution of acetonitrile and tetrahydrofuran (30 mL/10 mL), and glycine methyl ester hydrochloride (5.4 g, 43 mmol) and potassium carbonate (12 g, 87 mmol) were added. The mixture was stirred at room temperature for 1 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was purified by column chromatography (SiO₂, n-hexane/ethyl acetate = 3:1) to give the target product (3.2 g, yield: 80%).

ESI-MS m/z: 473 [M+H]⁺.

### Step 5: Synthesis of compound int_273-7

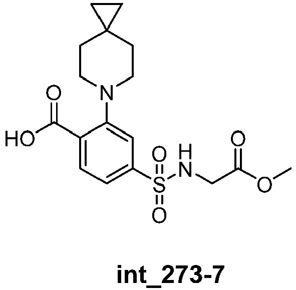

**Int_273-6** (3.2 g, 6.8 mmol) was dissolved in methanol (30 mL), and Pd/C (1.00 g, 10% purity) and 5 drops of acetic acid were added. The reaction system was purged with hydrogen 3 times. In hydrogen atmosphere, the reaction solution was incubated at 50 °C for reaction for 16 h, until LC-MS indicated the completion of the reaction. The reaction solution was filtered, and the filtrate was concentrated at reduced pressure to give a crude product (2.5 g, yield: 96%). The crude product was used directly in the next reaction.

ESI-MS m/z: 383 [M+H]⁺.

### Step 6: Synthesis of compound int_273-8

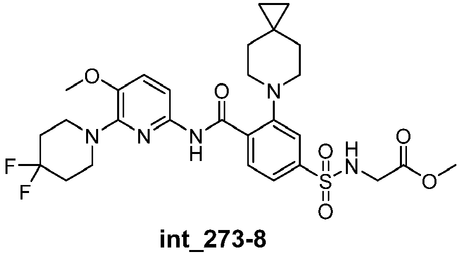

**Int_273-7** (0.5 g, 1.3 mmol) was dissolved in DCM (15 mL), and oxalyl chloride (888 mg, 7 mmol) was added. The reaction solution was stirred at room temperature for 2 h and then concentrated at reduced pressure to remove the solvent, thus giving an acyl chloride product. The acyl chloride was dissolved in tetrahydrofuran (20 mL), and **int_257-3** (318 mg, 1.3 mmol) and triethylamine (1.3 g, 13 mmol) were slowly added under an ice bath. The mixture was incubated at room temperature for reaction for 1 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was subjected to column chromatography to give the target product (250 mg, yield: 31%).

¹H NMR (400 MHz, DMSO-d6) δ 12.77 (s, 1H), 8.36 (s, 1H), 8.23 (d, *J =* 8.2 Hz, 1H), 7.82-7.75 (m, 2H), 7.66 (dd, *J* = 8.2, 1.7 Hz, 1H), 7.37 (d, *J* = 8.6 Hz, 1H), 3.80 (s, 3H), 3.76 (s, 2H), 3.52 (d, *J* = 6.3 Hz, 4H), 3.49 (s, 3H), 3.39 (d, *J* = 6.7 Hz, 2H), 3.03 (t, *J* = 5.4 Hz, 4H), 2.06 (t, *J* = 5.1 Hz, 2H), 1.70 (s, 4H), 0.36 (s, 4H).

ESI-MS m/z: 608 [M+H]⁺.

### Step 7: Synthesis of compound 273

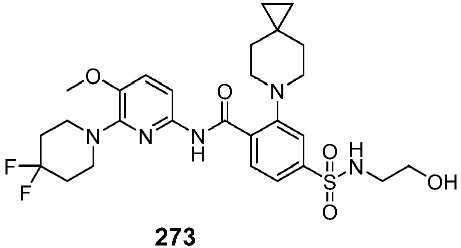

**Int_273-8** (230 mg, 0.38 mmol) was dissolved in a mixed solvent of methanol and tetrahydrofuran (5 mL/5 mL), and sodium borohydride (43 mg, 1.1 mmol) and lithium chloride (48 mg, 1.1 mmol) were slowly added under an ice bath. The reaction solution was incubated at room temperature for reaction for 3 h, until LC-MS indicated the completion of the reaction. The reaction solution was diluted with water (20 mL), and the aqueous phase was extracted with ethyl acetate (20 mL × 3). The organic phase was dried over anhydrous sodium sulfate, filtered, and distilled at reduced pressure to give a crude product. The crude product was subjected to column chromatography to give the target product (210 mg, yield: 95%).

¹H NMR (400 MHz, DMSO-d6) δ 12.75 (s, 1H), 8.23 (d, *J* = 8.2 Hz, 1H), 7.79 (dd, *J* = 5.1, 3.4 Hz, 2H), 7.67 (dd, *J* = 8.2, 1.7 Hz, 1H), 7.37 (d, *J* = 8.6 Hz, 1H), 4.73 (s, 1H), 3.80 (s, 3H), 3.51 (t, *J* = 5.7 Hz, 4H), 3.42-3.36 (m, 2H), 3.03 (t, *J* = 5.3 Hz, 4H), 2.81 (t, *J* = 6.2 Hz, 2H), 2.05 (q, *J* = 11.2, 8.5 Hz, 4H), 1.70 (m, 4H), 0.36 (s, 4H).

ESI-MS m/z: 580 [M+H]⁺.

### Example 19. Synthesis of Compound 321

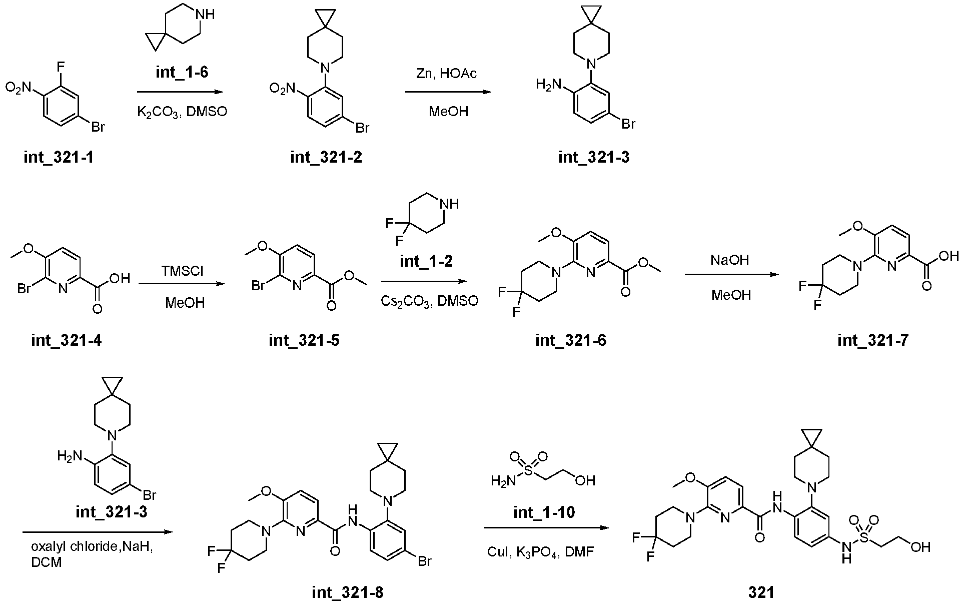

### Step 1: Synthesis of compound int_321-2

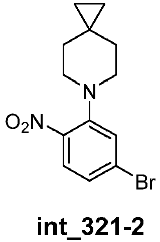

**Int_321-1** (1 g, 4.55 mmol) was dissolved in DMF (20 mL), and **int_1-6** (670 mg, 4.55 mmol) and potassium carbonate (1.8 g, 13.64 mmol) were added. In argon atmosphere, the mixture was incubated at 100 °C for reaction for 3 h, until LC-MS indicated the completion of the reaction. The reaction solution was concentrated at reduced pressure, and 100 mL of water was added. The aqueous phase was extracted with ethyl acetate (200 mL × 3), and the organic phase was dried over anhydrous sodium sulfate, filtered, and distilled at reduced pressure to give a crude product (1.5 g, yield: 100%).

ESI-MS m/z: 311 [M+H]⁺.

### Step 2: Synthesis of compound int_321-3

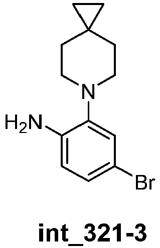

**Int_321-2** (1.5 g, 4.82 mmol) was dissolved in methanol (50 mL), and 20 mL of acetic acid was added. The mixture was stirred for 10 min under an ice bath, and zinc powder (1.5 g, 24.10 mmol) was added in small batches. The reaction solution was warmed to room temperature and incubated for reaction for 1 h, until LC-MS indicated the completion of the reaction. The reaction solution was filtered to give a filtrate, and the filtrate was concentrated at reduced pressure to give a crude product. 100 mL of water was added to the crude product, and the aqueous phase was extracted with dichloromethane (200 mL × 3). The organic phase was dried over anhydrous sodium sulfate, filtered, and distilled at reduced pressure to give a crude product (1 g, yield: 77%). ESI-MS m/z: 281 [M+H]⁺.

### Step 3: Synthesis of compound int_321-5

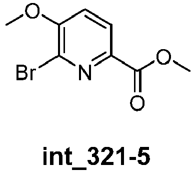

**Int_321-4** (5 g, 21.55 mmol) was suspended in methanol (100 mL), and trimethylchlorosilane (7 g, 64.65 mmol) was added. The reaction solution was incubated at room temperature for reaction for 4 h and was gradually clarified, until LC-MS indicated the completion of the reaction. The reaction solution was concentrated at reduced pressure to give a crude product (5.2 g, yield: 98%).

ESI-MS m/z: 246 [M+H]⁺.

### Step 4: Synthesis of compound int_321-6

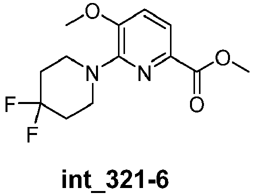

**Int_321-5** (5 g, 16.2 mmol) was dissolved in 1,4-dioxane (100 mL), and cesium carbonate (20 g, 63.4 mmol), Pd₂(dba)₃ (1.9 g, 2.11 mmol), and Xantphos (1.2 g, 2.11 mmol) were added. In argon atmosphere, the mixture was heated to 100 °C and incubated for reaction for 18 h, until LC-MS indicated the completion of the reaction. The reaction solution was diluted with water (300 mL), and the aqueous phase was extracted with dichloromethane (300 mL × 3). The organic phase was dried over anhydrous sodium sulfate, filtered, and distilled at reduced pressure to give a crude product. The crude product was purified by column chromatography (SiO₂, n-hexane/ethyl acetate = 10:1) to give the target product (4.3 g, yield: 71%).

ESI-MS m/z: 287 [M+H]⁺.

### Step 5: Synthesis of compound int_321-7

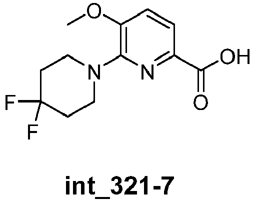

**Int_273-6** (4.3 g, 15.02 mmol) was dissolved in methanol (50 mL), and NaOH (2 M, 20 mL) was added with stirring at room temperature. The reaction solution was incubated at room temperature for reaction for 4 h, until LC-MS indicated the completion of the reaction. The reaction solution was filtered to give a filtrate, and the filtrate was concentrated at reduced pressure to give a solid. Water (100 mL) was added to the solid. The aqueous phase was extracted with dichloromethane (50 mL × 2) and then distilled at reduced pressure to give a crude product. The crude product was purified by column chromatography to give the target product (2 g, yield: 58%).

ESI-MS m/z: 273 [M+H]⁺.

### Step 6: Synthesis of compound int_321-8

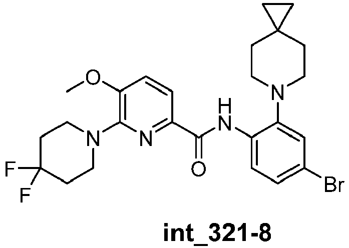

**Int_321-7** (2 g, 7.37 mmol) was dissolved in DCM (100 mL), and oxalyl chloride (1.4 g, 11 mmol) was added. The reaction solution was stirred at room temperature for 2 h and then concentrated at reduced pressure to remove the solvent, thus giving an acyl chloride product. **Int_321-3** (2.1 g, 7.37 mmol) was dissolved in tetrahydrofuran (50 mL), and sodium hydride (2.9 g, 73.7 mmol, 60% purity) was slowly added under an ice bath. The reaction solution was incubated at room temperature for reaction for 1 h, and the prepared acyl chloride product was added to the reaction solution. The reaction solution was incubated at 40 °C for reaction for 5 h, until LC-MS indicated the completion of the reaction. The reaction solution was diluted with water (100 mL), and the aqueous phase was extracted with dichloromethane (100 mL × 3). The organic phase was dried over anhydrous sodium sulfate, filtered, and distilled at reduced pressure to give a crude product. The crude product was purified by column chromatography (SiO₂, *n*-hexane/ethyl acetate = 5:1) to give the target product (3.7 g, yield: 95%).

ESI-MS m/z: 535 [M+H]⁺.

### Step 7: Synthesis of compound 321

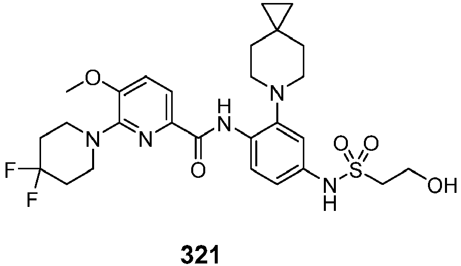

**Int_321-8** (500 mg, 0.94 mmol), *N*,*N*-dimethylglycine (66 mg, 0.47 mmol), cuprous iodide (89 mg, 0.47 mmol), and potassium phosphate (596 mg, 2.8 mmol) were dissolved in DMF (20 mL). The mixture was purged with argon three times before **int_1-10** (266 mg, 1.87 mmol) was added. In argon atmosphere, the reaction solution was heated to 130 °C with microwave and incubated for reaction for 3.5 h, until LC-MS indicated the completion of the reaction. The reaction solution was cooled to room temperature, concentrated by rotary evaporation, and purified by column chromatography to give a solid (260 mg, yield: 48%).

¹H NMR (400 MHz, DMSO-d6) δ 10.37 (s, 1H), 8.39 (s, 1H), 8.30 (d, *J* = 8.8 Hz, 1H), 7.76 (d, *J* = 8.2 Hz, 1H), 7.47 (d, *J* = 8.3 Hz, 1H), 7.14 (d, *J* = 2.4 Hz, 1H), 6.97 (dd, *J* = 8.7, 2.4 Hz, 1H), 3.90 (s, 3H), 3.71 (t, *J* = 6.7 Hz, 2H), 3.56 (t, *J* = 5.5 Hz, 4H), 3.18 (t, *J* = 6.7 Hz, 2H), 2.78 (t, *J* = 5.3 Hz, 4H), 2.13 (tt, *J* = 13.7, 5.6 Hz, 4H), 1.52 (s, 4H), 0.33 (s, 4H).

ESI-MS m/z: 580 [M+H]⁺.

### Example 20. Synthesis of Compound 337

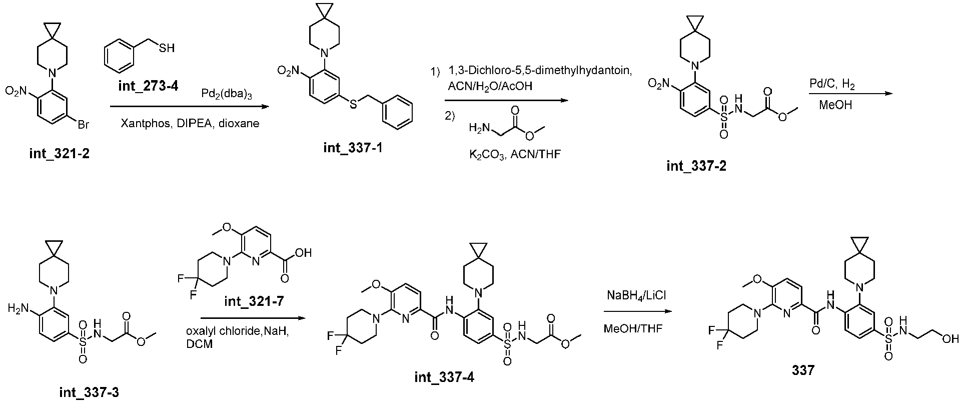

### Step 1: Synthesis of compound int_337-1

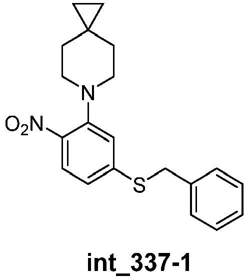

Int_321-2 (2 g, 6.43 mmol) was dissolved in dioxane (30 mL), and benzylmercaptan (2.4 g, 19.28 mmol), Pd₂(dba)₃ (300 mg, 0.33 mmol), Xantphos (300 mg, 0.54 mmol), and DIPEA (3.3 g, 25.72 mmol) were added. In nitrogen atmosphere, the mixture was heated to 100 °C and incubated for reaction for 16 h, until LC-MS indicated the completion of the reaction. The reaction solution was diluted with water (100 mL), and the aqueous phase was extracted with ethyl acetate (100 mL × 3). The organic phase was dried over anhydrous sodium sulfate, filtered, and distilled at reduced pressure to give a crude product. The crude product was subjected to column chromatography to give the target product (1.7 g, yield: 74.9%).

ESI-MS m/z: 355 [M+H]⁺.

### Step 2: Synthesis of compound int_337-2

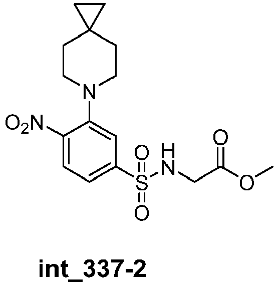

**Int_337-1** (360 mg, 1.02 mmol) was dissolved in a mixed solvent of acetonitrile/water/acetic acid (30 mL/1 mL/1 mL), and dichlorohydantoin (402 mg, 2.04 mmol) was added under an ice bath. In nitrogen atmosphere, the mixture was stirred at 0 °C for reaction for 0.5 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was dissolved in a mixed solution of acetonitrile and tetrahydrofuran (30 mL/10 mL), and glycine methyl ester hydrochloride (1 g, 7.96 mmol) and potassium carbonate (3.3 g, 24 mmol) were added. The mixture was stirred at room temperature for 1 h, until LC-MS indicated the completion of the reaction. The reaction solution was diluted with water (50 mL), and the aqueous phase was extracted with ethyl acetate (50 mL × 3). The organic phase was dried over anhydrous sodium sulfate, filtered, and distilled at reduced pressure to give a crude product. The crude product was subjected to column chromatography to give the target product (100 mg, yield: 25.6%).

ESI-MS m/z: 384 [M+H]⁺.

### Step 3: Synthesis of compound int_337-3

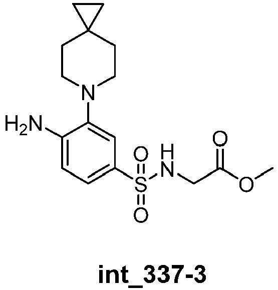

**Int_337-2** (100 mg, 0.261 mmol) was dissolved in methanol (10 mL), and Pd/C (30 mg, 10% purity) and 5 drops of acetic acid were added. The reaction system was purged with hydrogen 3 times. In hydrogen atmosphere, the reaction solution was incubated at room temperature for reaction for 16 h, until LC-MS indicated the completion of the reaction. The reaction solution was filtered, and the filtrate was concentrated at reduced pressure to give a crude product (90 mg, yield: 97.8%). The crude product was used directly in the next reaction.

ESI-MS m/z: 354 [M+H]⁺.

### Step 4: Synthesis of compound int_337-4

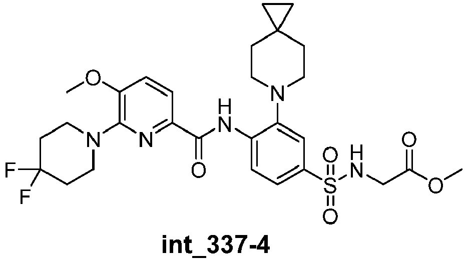

**Int_321-7** (220 mg, 0.809 mmol) was dissolved in DCM (10 mL), and oxalyl chloride (1 g, 8 mmol) was added. The reaction solution was stirred at room temperature for 2 h and then concentrated at reduced pressure to remove the solvent, thus giving an acyl chloride product. **Int_337-3** (140 mg, 0.396 mmol) was dissolved in tetrahydrofuran (10 mL), and sodium hydride (56 mg, 1.4 mmol, 60% purity) was slowly added under an ice bath. The reaction solution was incubated at room temperature for reaction for 1 h, and the prepared acyl chloride product was added to the reaction solution. The reaction solution was incubated at 40 °C for reaction for 5 h, until LC-MS indicated the completion of the reaction. The reaction solution was diluted with water (30 mL), and the aqueous phase was extracted with dichloromethane (30 mL × 3). The organic phase was dried over anhydrous sodium sulfate, filtered, and distilled at reduced pressure to give a crude product. The crude product was purified by column chromatography to give the target product (100 mg, yield: 41.7%).

ESI-MS m/z: 608 [M+H]⁺.

### Step 5: Synthesis of compound 337

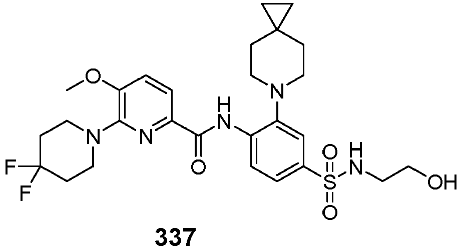

**Int_337-4** (100 mg, 0.165 mmol) was dissolved in a mixed solvent of methanol and tetrahydrofuran (5 mL/5 mL), and sodium borohydride (38 mg, 1 mmol) and lithium chloride (42 mg, 1 mmol) were slowly added under an ice bath. The reaction solution was incubated at room temperature for reaction for 3 h, until LC-MS indicated the completion of the reaction. The reaction solution was diluted with water (20 mL), and the aqueous phase was extracted with ethyl acetate (20 mL × 3). The organic phase was dried over anhydrous sodium sulfate, filtered, and distilled at reduced pressure to give a crude product. The crude product was subjected to column chromatography to give the target product (82 mg, yield: 85.8%).

¹H NMR (400 MHz, DMSO-d6) δ 10.66 (s, 1H), 8.58 (d, *J* = 8.6 Hz, 1H), 7.81 (d, *J* = 8.2 Hz, 1H), 7.68 (s, 1H), 7.58 (d, *J* = 8.7 Hz, 1H), 7.49 (d, *J* = 8.8 Hz, 1H), 4.69 (d, *J* = 6.5 Hz, 1H), 3.91 (s, 3H), 3.58 (d, *J* = 6.5 Hz, 4H), 2.81 (dt, *J* = 37.8, 5.6 Hz, 6H), 2.15 (d, *J* = 7.6 Hz,4H), 1.55 (s, 4H), 0.35 (s, 4H).

ESI-MS m/z: 580 [M+H]⁺.

### Example 21. Synthesis of Compound 353

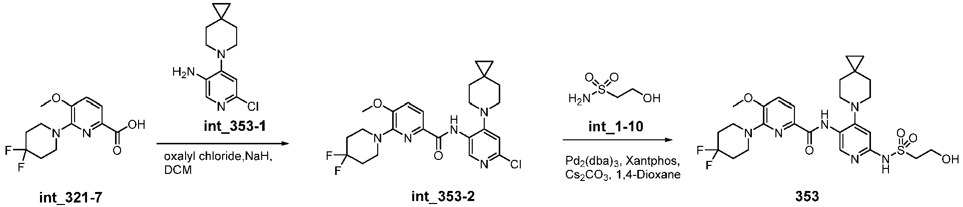

### Step 1: Synthesis of compound int_353-2

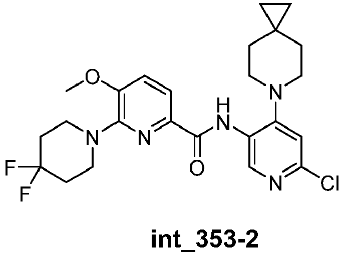

**Int_321-7** (50 mg, 0.184 mmol) was dissolved in DCM (5 mL), and oxalyl chloride (12 mg, 1 mmol) was added. The reaction solution was stirred at room temperature for 2 h and then concentrated at reduced pressure to remove the solvent, thus giving an acyl chloride product. **Int_353-1** (44 mg, 0.185 mmol) was dissolved in tetrahydrofuran (5 mL), and sodium hydride (50 mg, 1.25 mmol, 60% purity) was slowly added under an ice bath. The reaction solution was incubated at room temperature for reaction for 1 h, and the prepared acyl chloride product was added to the reaction solution. The reaction solution was incubated at room temperature for reaction for 5 h, until LC-MS indicated the completion of the reaction. The reaction solution was diluted with water (10 mL), and the aqueous phase was extracted with dichloromethane (10 mL × 3). The organic phase was dried over anhydrous sodium sulfate, filtered, and distilled at reduced pressure to give a crude product. The crude product was purified by column chromatography to give the target product (50 mg, yield: 55%).

ESI-MS m/z: 492 [M+H]⁺.

### Step 2: Synthesis of compound 353

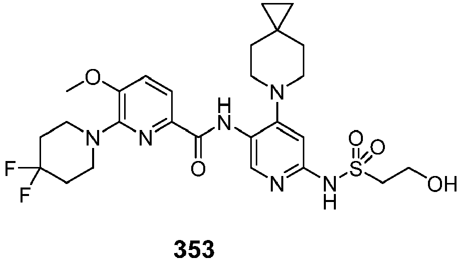

**Int_353-2** (190 mg, 0.39 mmol), cesium carbonate (129.8 mg, 1.16 mmol), Pd₂(dba)₃ (95 mg, 0.218 mmol), and Xantphos (95 mg, 0.346 mmol) were dissolved in 1,4-dioxane (10 mL). The mixture was purged with argon three times before **int_1-10** (97.2 mg, 0.78 mmol) was added. In argon atmosphere, the reaction solution was heated to 110 °C and incubated for reaction for 12 h, until LC-MS indicated the completion of the reaction. The reaction solution was cooled to room temperature, concentrated by rotary evaporation, and purified by column chromatography to give a solid (56 mg, yield: 27%).

¹H NMR (400 MHz, DMSO-d6) δ 9.75 (s, 1H), 8.66 (s, 1H), 7.72 (d, *J* = 8.2 Hz, 1H), 7.45 (d, *J* = 8.3 Hz, 1H), 6.35 (s, 1H), 3.89 (s, 3H), 3.64 (t, *J* = 6.6 Hz, 2H), 3.54 (d, *J* = 3.1 Hz, 4H), 3.09 (t, *J* = 6.6 Hz, 2H), 2.78 (d, *J=* 5.5 Hz, 4H), 2.19-2.03 (m, 4H), 1.47 (s, 4H), 0.30 (s, 4H).

ESI-MS m/z: 581 [M+H]⁺.

### Example 22. Synthesis of Compound 369

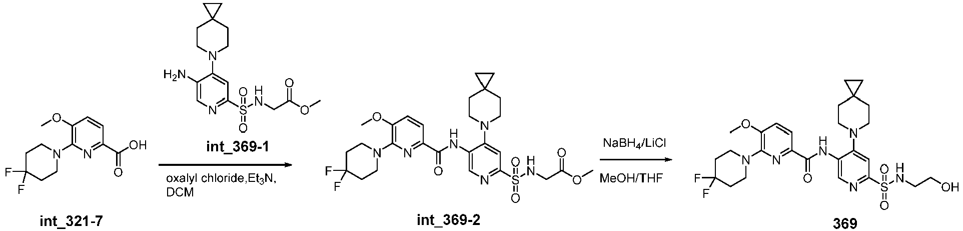

### Step 1: Synthesis of compound int_369-2

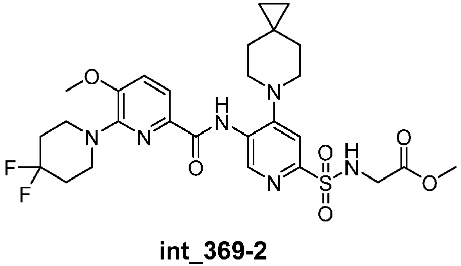

**Int_321-7** (27 mg, 0.1 mmol) was dissolved in DCM (5 mL), and oxalyl chloride (12 mg, 1 mmol) was added. The reaction solution was stirred at room temperature for 2 h and then concentrated at reduced pressure to remove the solvent, thus giving an acyl chloride product. **Int_369-1** (37 mg, 0.1 mmol) was dissolved in tetrahydrofuran (5 mL), and triethylamine (202 mg, 2 mmol) and the prepared acyl chloride product were slowly added under an ice bath. The reaction solution was incubated at 40 °C for reaction for 12 h, until LC-MS indicated the completion of the reaction. The reaction solution was diluted with water (10 mL), and the aqueous phase was extracted with dichloromethane (10 mL × 3). The organic phase was dried over anhydrous sodium sulfate, filtered, and distilled at reduced pressure to give a crude product. The crude product was purified by column chromatography to give the target product (2 mg, yield: 3.3%).

¹H NMR (400 MHz, DMSO-d6) δ 10.16 (s, 1H), 9.38 (s, 1H), 8.29 (t, *J* = 6.1 Hz, 1H), 7.80 (d, *J* = 8.2 Hz, 1H), 7.63 (s, 1H), 7.50 (d, *J* = 8.3 Hz, 1H), 3.92 (s, 3H), 3.85 (d, *J* = 5.8 Hz, 2H), 3.56 (d, *J* = 11.2 Hz, 6H), 3.31 (s, 2H), 2.99 (t, *J* = 5.2 Hz, 3H), 2.21-1.95 (m, 4H), 1.53 (t, *J* = 5.3 Hz, 4H), 0.34 (s, 4H).

ESI-MS m/z: 609 [M+H]⁺.

### Step 2: Synthesis of compound 369

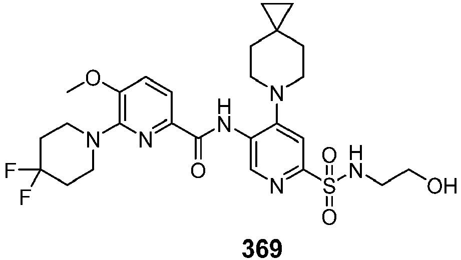

**Int_369-2** (70 mg, 0.115 mmol) was dissolved in a mixed solvent of methanol and tetrahydrofuran (5 mL/5 mL), and sodium borohydride (38 mg, 1 mmol) and lithium chloride (42 mg, 1 mmol) were slowly added under an ice bath. The reaction solution was incubated at room temperature for reaction for 3 h, until LC-MS indicated the completion of the reaction. The reaction solution was diluted with water (20 mL), and the aqueous phase was extracted with ethyl acetate (20 mL × 3). The organic phase was dried over anhydrous sodium sulfate, filtered, and distilled at reduced pressure to give a crude product. The crude product was subjected to column chromatography to give the target product (9 mg, yield: 12.9%).

¹H NMR (400 MHz, DMSO-d6) δ 10.15 (s, 1H), 9.40 (s, 1H), 7.80 (d, *J* = 8.2 Hz, 1H), 7.67 (d, *J* = 92 Hz, 2H), 7.50 (d, *J* = 8.3 Hz, 1H), 4.63 (t, *J* = 5.6 Hz,1H), 3.92 (s, 3H), 3.57 (t, *J* = 5.6 Hz, 4H), 3.44-3.34 (m, 2H), 2.97 (dt, *J* = 26.4, 6.1 Hz, 6H), 2.27-2.04 (m, 4H), 1.53 (t, *J* = 5.2 Hz, 4H), 0.34 (s, 4H).

ESI-MS m/z: 581 [M+H]⁺.

### Example 23. Synthesis of Compound 385

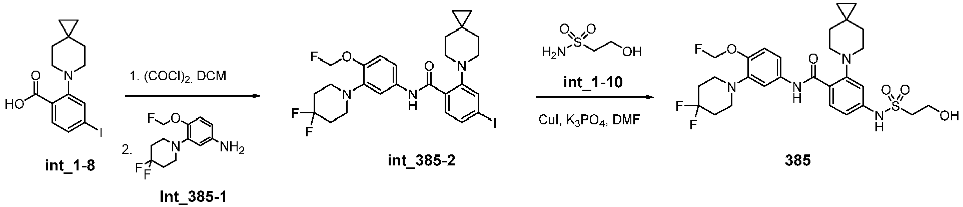

### Step 1: Synthesis of compound int_385-2

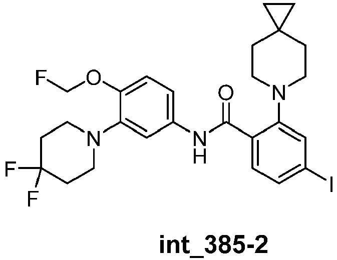

**Int_1-8** (150 mg, 0.42 mmol) was dissolved in DCM (50 mL), and oxalyl chloride (507 mg, 4 mmol) was added. The reaction solution was stirred at room temperature for 2 h and then concentrated at reduced pressure to remove the solvent, thus giving an acyl chloride product.

**Int_385-1** (70 mg, 0.28 mmol) was dissolved in tetrahydrofuran (40 mL), and in nitrogen atmosphere, NaH (67 mg, 1.68 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 8:1) to give a solid (140 mg, yield: 87%).

ESI-MS m/z: 600 [M+H]⁺.

### Step 2: Synthesis of compound 385

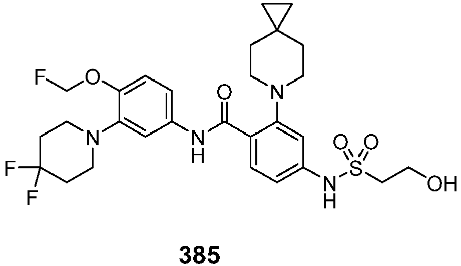

**Int_385-2** (140 mg, 0.23 mmol), (1*S*,2*S*)-*N*,*N*'-dimethyl-1,2-cyclohexanediamine (16 mg, 0.115 mmol), cuprous iodide (22 mg, 0.115 mmol), and potassium phosphate (146 mg, 0.69 mmol) were dissolved in DMF (5 mL). The mixture was purged with argon three times before **int_1-10** (58 mg, 0.46 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (77 mg, yield: 56.2%).

¹H NMR (400 MHz, Chloroform-d) δ 12.45 (s, 1H), 8.20 (d, *J* = 8.3 Hz, 1H), 7.75-7.70 (m, 1H), 7.33 (s, 1H), 7.20-7.16 (m, 1H), 7.12 (d, *J* = 8.6 Hz, 1H), 7.02 (t, *J* = 9.3 Hz, 1H), 5.80 (s, 1H), 5.66 (s, 1H), 4.13 (d, *J* = 5.7 Hz, 2H), 3.37-3.29 (m, 2H), 3.23 (t, *J* = 5.6 Hz, 4H), 3.07 (s, 4H), 2.14 (tt, *J* = 13.1, 5.4 Hz, 4H), 1.62 (s, 4H), 0.43 (s, 4H).

ESI-MS m/z: 597 [M+H]⁺.

### Example 24. Synthesis of Compound 577

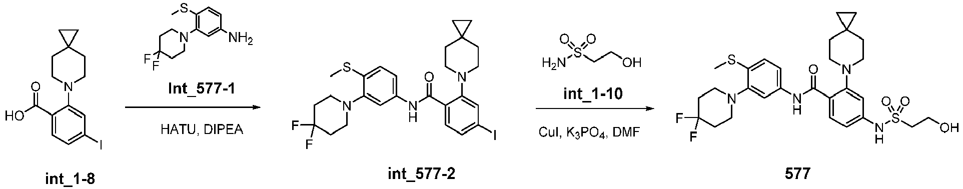

### Step 1: Synthesis of compound int_577-2

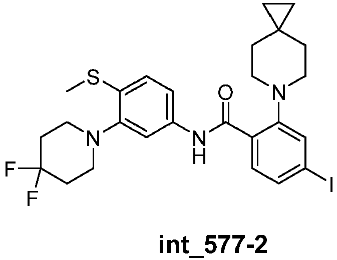

**Int_1-8** (138 mg, 0.387 mmol) was dissolved in DCM (50 mL), and int_577-1 (100 mg, 0.387 mmol), HATU (294 mg, 0.774 mmol), and DIPEA (193.8 mg, 1.5 mmol) in DMF (10 mL) were added. In nitrogen atmosphere, the reaction solution was warmed to 60 °C and stirred for 2 h, until LC-MS indicated the completion of the reaction. The reaction solution was concentrated at reduced pressure to give a crude product. The crude product was purified by column chromatography to give a solid (130 mg, yield: 56%).

ESI-MS m/z: 598 [M+H]⁺.

### Step 2: Synthesis of compound 577

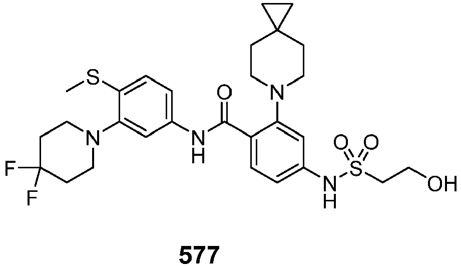

**Int_577-2** (130 mg, 0.217 mmol), (1*S*,2*S*)-*N*,*N*'-dimethyl-1,2-cyclohexanediamine (9 mg, 0.065 mmol), cuprous iodide (12 mg, 0.065 mmol), and potassium phosphate (138 mg, 0.653 mmol) were dissolved in DMF (10 mL). The mixture was purged with argon three times before **int_1-10** (54 mg, 0.435 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (80 mg, yield: 62%).

¹H NMR (400 MHz, DMSO-d6) δ 11.76 (s, 1H), 7.80 (d, *J* = 8.5 Hz, 1H), 7.63 (d, *J* = 2.2 Hz, 1H), 7.48 (dd, *J* = 8.5, 2.1 Hz, 1H), 7.17 (d, *J* = 8.6 Hz, 1H), 7.12 (d, *J* = 2.1 Hz, 1H), 6.99 (dd, *J* = 8.5, 2.0 Hz, 1H), 3.74 (t, *J* = 6.5 Hz, 2H), 3.27 (d, *J* = 6.6 Hz, 2H), 3.01 (t, *J* = 5.6 Hz, 4H), 2.94 (t, *J* = 5.3 Hz, 4H), 2.39 (s, 3H), 2.10 (dt, *J* = 14.3, 7.9 Hz, 4H), 1.53 (s, 4H), 0.34 (s, 4H).

ESI-MS m/z: 595 [M+H]⁺.

### Example 25. Synthesis of Compound 641

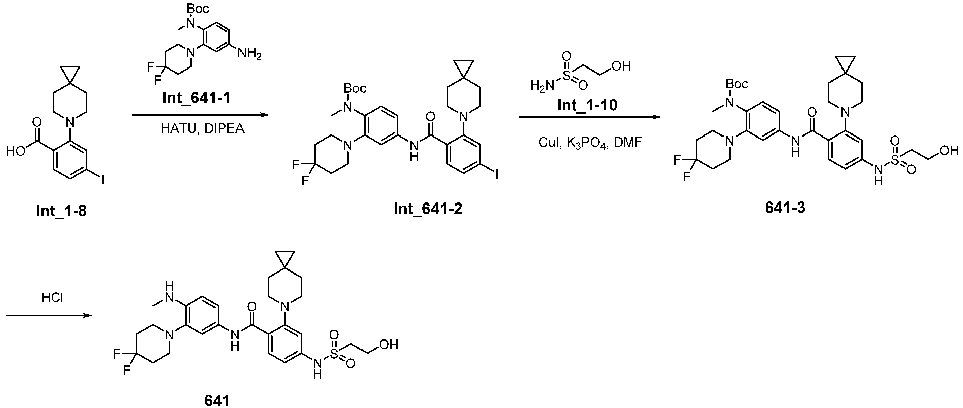

### Step 1: Synthesis of compound int_641-2

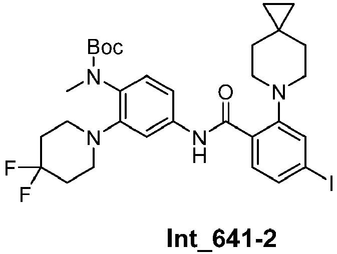

**Int_1-8** (100 mg, 0.29 mmol) was dissolved in DCM (50 mL), and **int_641-1** (100 mg, 0.29 mmol), HATU (220 mg, 0.585 mmol), and DIPEA (193.8 mg, 1.5 mmol) in DMF (8 mL) were added. In nitrogen atmosphere, the reaction solution was stirred at room temperature for 12 h, until LC-MS indicated the completion of the reaction. The reaction solution was concentrated at reduced pressure to give a crude product. The crude product was purified by column chromatography to give a solid (110 mg, yield: 55.2%).

ESI-MS m/z: 681 [M+H]⁺.

### Step 2: Synthesis of compound 641-3

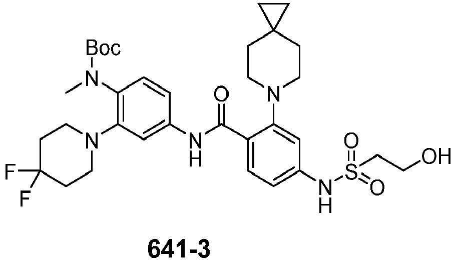

**Int_641-2** (160 mg, 0.235 mmol), (1*S*,2*S*)-(+)-*N*,*N*'-dimethyl-1,2-cyclohexanediamine (17 mg, 0.117 mmol), cuprous iodide (22 mg, 0.117 mmol), and potassium phosphate (150 mg, 0.705 mmol) were dissolved in DMF (10 mL). The mixture was purged with argon three times before **int_1-10** (60 mg, 0.47 mmol) was added. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (110 mg, yield: 69.1%).

ESI-MS m/z: 678 [M+H]⁺.

### Step 3: Synthesis of compound 641

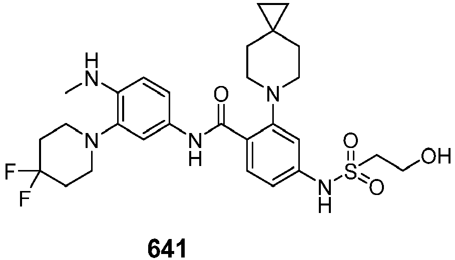

**Int_641-3** (110 mg, 0.162 mmol) was dissolved in methanol/hydrochloric acid (4 N, 15 mL), and the reaction solution was incubated at room temperature for reaction for 12 h, until LC-MS indicated the completion of the reaction. The reaction solution was concentrated by rotary evaporation and purified by column chromatography to give a solid (60 mg, yield: 64.5%).

¹H NMR (400 MHz, Chloroform-d) δ 12.26 (s, 1H), 8.11 (d, *J* = 8.4 Hz, 1H), 7.63 (d, *J* = 2.3 Hz, 1H), 7.34 (s, 1H), 7.25 (d, *J* = 3.0 Hz, 1H), 6.99 (d, *J* = 8.3 Hz, 1H), 6.63 (d, *J* = 8.6 Hz, 1H), 4.09 (t, *J* = 5.1 Hz, 2H), 3.30 (t, *J* = 5.1 Hz, 2H), 3.07-2.98 (m, 8H), 2.87 (s, 3H), 2.12 (ddt, *J* = 16.7, 11.5, 5.6 Hz, 4H), 1.62 (s, 4H), 0.40 (s, 4H).

ESI-MS m/z: 578 [M+H]⁺.

### Example 26. Synthesis of Compound 643

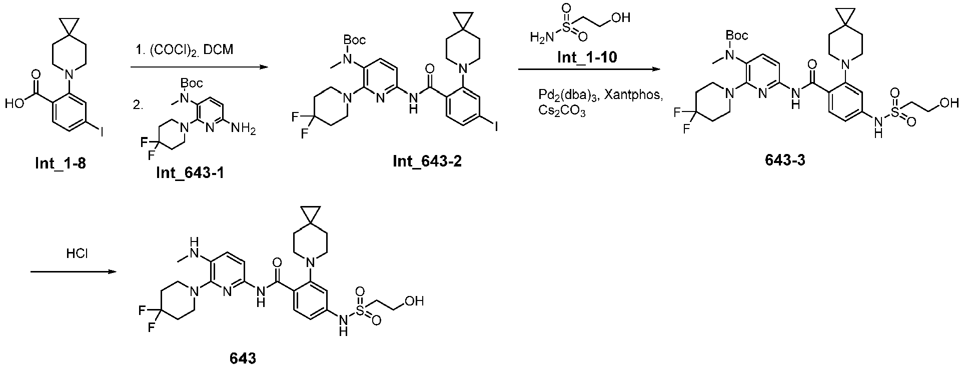

### Step 1: Synthesis of compound int_643-2

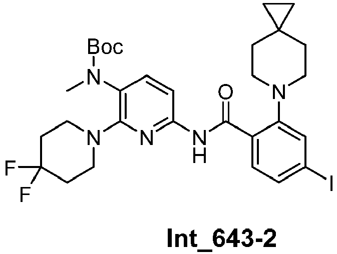

**Int_1-8** (1.17 g, 3.3 mmol) was dissolved in DCM (50 mL), and oxalyl chloride (1.9 g, 15 mmol) was added. The reaction solution was stirred at room temperature for 2 h and then concentrated at reduced pressure to remove the solvent, thus giving an acyl chloride product.

**Int_643-1** (800 mg, 3.3 mmol) was dissolved in tetrahydrofuran (50 mL), and in nitrogen atmosphere, triethylamine (666 mg, 6.6 mmol) was added. The mixture was stirred at room temperature for 0.5 h and then the acyl chloride prepared previously was added at room temperature. The reaction solution was stirred at room temperature for 6 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 7:1) to give a solid (1.1 g, yield: 57.8%).

ESI-MS m/z: 682 [M+H]⁺.

### Step 2: Synthesis of compound 643-3

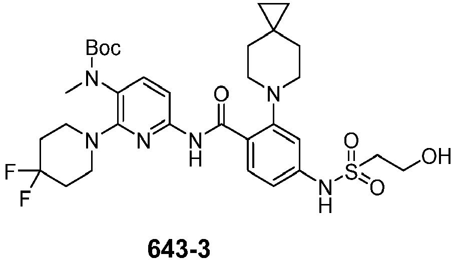

**Int_643-2** (1.1 g, 1.89 mmol), cesium carbonate (921 mg, 2.83 mmol), Pd₂(dba)₃ (35 mg, 0.037 mmol), and X-Phos (27 mg, 0.056 mmol) were dissolved in 1,4-dioxane (40 mL), and **int_1-10** (473 mg, 3.78 mmol) was added. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (550 mg, yield: 50.45%).

ESI-MS m/z: 679 [M+H]⁺.

### Step 3: Synthesis of compound 643

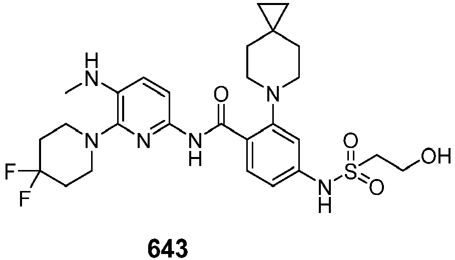

**Int_643-3** (100 mg, 0.147 mmol) was dissolved in methanol/hydrochloric acid (4 N, 15 mL), and the reaction solution was incubated at room temperature for reaction for 12 h, until LC-MS indicated the completion of the reaction. The reaction solution was concentrated by rotary evaporation and purified by column chromatography to give a solid (57 mg, yield: 67%).

¹H NMR (400 MHz, DMSO-d6) δ 12.87 (s, 1H), 8.02 (d, *J* = 8.6 Hz, 1H), 7.86 (d, *J* = 8.4 Hz, 1H), 7.21 (d, *J* = 2.1 Hz, 1H), 7.08 (dd, *J* = 8.6, 2.1 Hz, 1H), 6.91 (d, *J* = 8.6 Hz, 1H), 4.98 (q, *J* = 5.2 Hz, 1H), 3.73 (t, *J* = 6.5 Hz, 2H), 3.12 (t, *J* = 5.7 Hz, 4H), 2.94 (d, *J* = 5.2 Hz, 4H), 2.71 (d, *J* = 5.1 Hz, 3H), 2.16 (tt, *J* = 11.8, 5.2 Hz, 4H), 1.73 (s, 4H), 0.35 (s, 4H).

ESI-MS m/z: 579 [M+H]⁺.

### Example 27. Synthesis of Compound 645

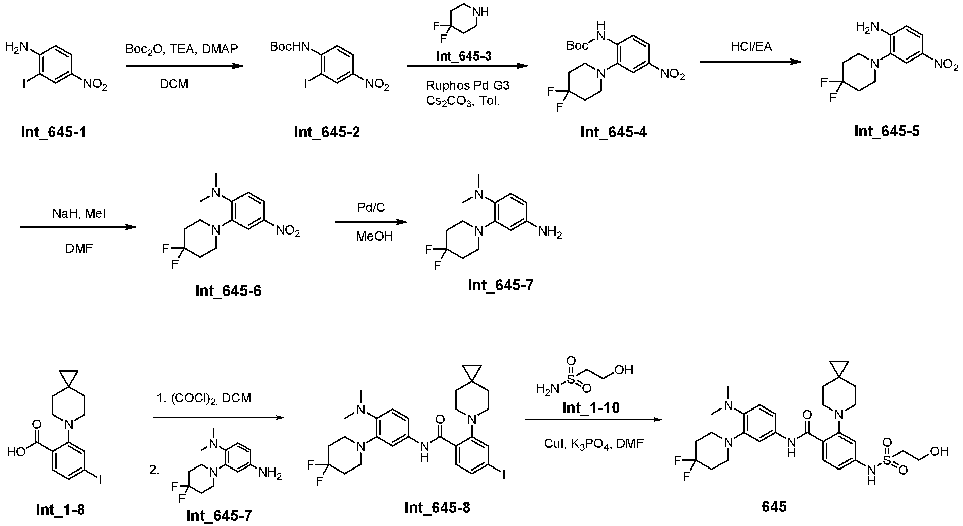

### Step 1: Synthesis of compound int_645-2

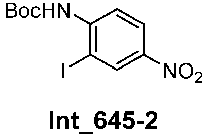

**Int_645-1** (10.0 g, 37.8 mmol) was dissolved in DCM (20 mL), and Boc₂O (8.27 g, 37.8 mmol, 8.70 mL), TEA (4.98 g, 49.2 mmol, 6.85 mL), and DMAP (231 mg, 1.89 mmol) were added. The reaction solution was incubated at 25 °C for reaction for 16 h, until LC-MS indicated the completion of the reaction. The reaction solution was filtered to give a filtrate, and the filtrate was concentrated at reduced pressure to give a crude product. The crude product was purified by column chromatography (SiO₂, n-hexane/ethyl acetate = 5:1) to give the target product (10 g, yield: 68.6%).

¹H NMR (400 MHz, DMSO-d6) δ = 8.62 (s, 1H), 8.58 (d, *J* = 2.8 Hz, 1H), 8.22 (dd, *J* = 2.8, 9.0 Hz, 1H), 7.82 (d, *J=* 9.0 Hz, 1H), 1.54-1.46 (m, 9H).

### Step 2: Synthesis of compound int_645-4

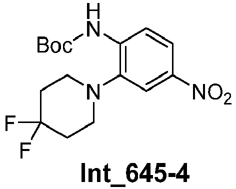

**Int_645-2** (10.00 g, 27.4 mmol), **int_645-3** (6.65 g, 54.9 mmol), RuPhos Pd G3 (2.30 g, 2.75 mmol), and Cs₂CO₃ (26.8 g, 82.4 mmol) were dissolved in toluene (50 mL). The mixture was purged with nitrogen three times and heated to 100 °C. In nitrogen atmosphere, the mixture was incubated for reaction for 2 h, until LC-MS indicated the completion of the reaction. The reaction solution was filtered, and the filtrate was concentrated at reduced pressure to give a crude product. Water (300 mL) was added to the crude product, and the aqueous phase was extracted with ethyl acetate (100 mL × 3). The organic phase was dried over anhydrous sodium sulfate, filtered, and distilled at reduced pressure to give a crude product. The crude product was purified by column chromatography (SiO₂, n-hexane/ethyl acetate = 3:1) to give the target product (6 g, yield: 44.6%).

¹H NMR (400 MHz, DMSO-d6) δ = 8.49 (s, 1H), 8.14-8.07 (m, 1H), 8.06-8.00 (m, 1H), 7.98 (d, *J* = 2.5 Hz, 1H), 2.97 (br t, *J* = 5.4 Hz, 4H), 2.31-2.13 (m, 4H), 1.53-1.50 (m, 9H).

### Step 3: Synthesis of compound int_645-5

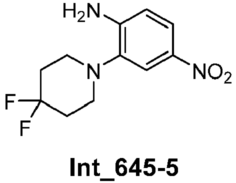

**Int_645-4** (3.60 g, 10.1 mmol) was dissolved in DCM (20 mL) and an HCl/EtOAc solution (4 M, 2.52 mL). The reaction solution was incubated at 25 °C for reaction for 16 h, until LC-MS indicated the completion of the reaction. The reaction solution was filtered to give a filtrate, and the filtrate was concentrated at reduced pressure to give a crude product (2.4 g, yield: 81.1%). The crude product was used directly in the next reaction. ¹H NMR (400 MHz, DMSO-d6) δ = 7.82 (dd, *J* = 2.6, 8.9 Hz, 1H), 7.74 (d, *J* = 2.5 Hz, 1H), 6.75 (d, *J* = 8.9 Hz, 1H), 2.92 (br s, 4H), 2.28-2.11 (m, 4H).

### Step 4: Synthesis of compound int_645-6

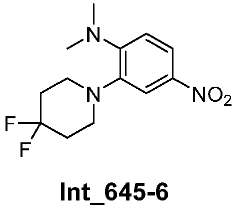

**Int_645-5** (2.40 g, 8.17 mmol) was dissolved in DMF (20 mL), and in nitrogen atmosphere, NaH (1.63 g, 40.86 mmol, 60% purity, 5.00 *eq*) and MeI (5.80 g, 40.9 mmol, 2.54 mL, 5.00 *eq*) were added to the reaction solution at 0 °C. After the addition, the reaction solution was warmed to room temperature and incubated for reaction for another 16 h, until LC-MS indicated the completion of the reaction. 30 mL of ice water was added to the reaction solution, and the mixture was stirred for another 0.5 h. Water (300 mL) was then added, and the aqueous phase was extracted with ethyl acetate (100 mL × 3). The organic phases were combined, dried over anhydrous sodium sulfate, filtered, and distilled at reduced pressure to give a crude product. The crude product was purified by column chromatography (SiO₂, n-hexane/ethyl acetate = 3:1) to give the target product (2 g, yield: 83.7%).

¹H NMR (400 MHz, DMSO-d6) δ = 7.86 (dd, *J* = 2.7, 9.0 Hz, 1H), 7.70 (d, *J* = 2.6 Hz, 1H), 6.98 (d, *J* = 9.1 Hz, 1H), 3.10 (br d, *J* = 7.2 Hz, 4H), 2.99 (s, 6H), 2.26-2.09 (m, 4H).

### Step 5: Synthesis of compound int_645-7

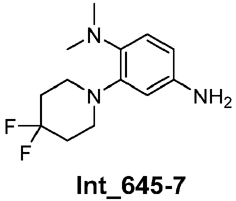

**Int_645-6** (2.00 g, 7.01 mmol) was dissolved in methanol (20 mL), and Pd/C (1.00 g, 7.01 mmol, 10% purity) was added. The reaction system was purged with hydrogen 3 times. In hydrogen atmosphere, the reaction solution was incubated at 25 °C for reaction for 16 h, until LC-MS indicated the completion of the reaction. The reaction solution was filtered, and the filtrate was concentrated at reduced pressure to give a crude product. The crude product was purified by column chromatography (SiO₂, dichloromethane/methanol = 10: 1) to give a solid (0.85 g, yield: 46.6%).

¹H NMR (400 MHz, DMSO-d6) δ = 6.65 (d, *J=* 8.3 Hz, 1H), 6.23 (d, *J=* 2.3 Hz, 1H), 6.18 (dd, *J =* 2.4, 8.3 Hz, 1H), 4.60 (s, 2H), 3.12 (br s, 4H), 2.63 (s, 6H), 2.19-1.99 (m, 4H).

### Step 6: Synthesis of compound int_645-8

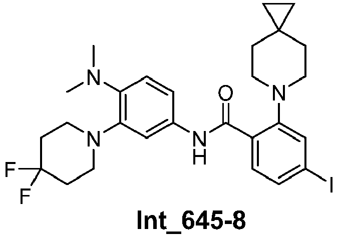

**Int_1-8** (1.2 g, 3.36 mmol) was dissolved in DCM (50 mL), and oxalyl chloride (888.4 mg, 7 mmol) was added. The reaction solution was stirred at room temperature for 2 h and then concentrated at reduced pressure to remove the solvent, thus giving an acyl chloride product.

**Int_645-7** (760 mg, 3 mmol) was dissolved in tetrahydrofuran (40 mL), and in nitrogen atmosphere, NaH (720 mg, 18 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 15:1) to give a solid (1.75 g, yield: 98.3%).

ESI-MS m/z: 595 [M+H]⁺.

### Step 7: Synthesis of compound 645

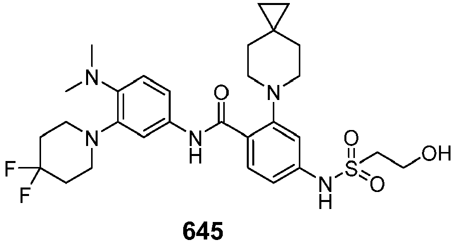

**Int_645-8** (1.75 g, 2.95 mmol), (1*S*,2*S*)-(+)-*N*,*N*'-dimethyl-1,2-cyclohexanediamine (210 mg, 1.475 mmol), cuprous iodide (281 mg, 1.475 mmol), and potassium phosphate (1.879 g, 8.85 mmol) were dissolved in DMF (50 mL). The mixture was purged with argon three times before **int_1-10** (553 mg, 4.42 mmol) was added. In argon atmosphere, the reaction solution was heated to 90 °C and incubated for reaction for 3 h, until LC-MS indicated the completion of the reaction. The reaction solution was cooled to room temperature, concentrated by rotary evaporation, and purified by column chromatography (SiO₂, dichloromethane/methanol = 100:1) to give a solid (580 mg, yield: 33.2%).

¹H NMR (400 MHz, Chloroform-d) δ 12.36 (s, 1H), 8.18 (d, *J =* 8.3 Hz, 1H), 7.52 (s, 1H), 7.33 (s, 1H), 7.13 (s, 1H), 7.02 (d, *J* = 8.2 Hz, 1H), 6.93 (d, *J* = 8.4 Hz, 1H), 4.12 (s, 2H), 3.30 (dt, *J* = 12.2, 5.1 Hz, 6H), 3.06 (t, *J* = 5.4 Hz, 4H), 2.82 (s, 6H), 2.12 (d, *J* = 15.2 Hz, 4H), 1.65 (s, 4H), 0.41 (s, 4H).

ESI-MS m/z: 592 [M+H]⁺.

### Example 28. Synthesis of Compound 653

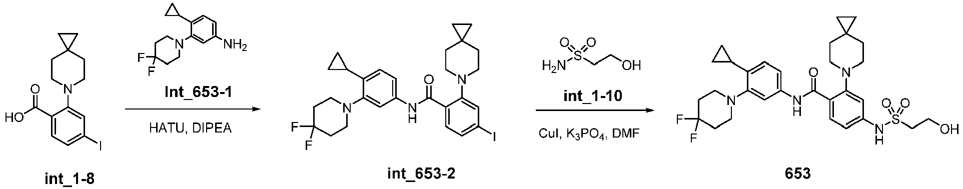

### Step 1: Synthesis of compound int_653-2

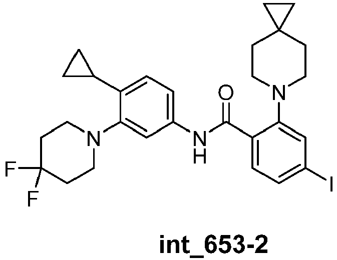

**Int_1-8** (140 mg, 0.396 mmol) was dissolved in DCM (50 mL), and **int_653-1** (100 mg, 0.396 mmol), HATU (300 mg, 0.792 mmol), and DIPEA (206.8 mg, 1.6 mmol) in DMF (8 mL) were added. In nitrogen atmosphere, the reaction solution was stirred at room temperature for 12 h, until LC-MS indicated the completion of the reaction. The reaction solution was concentrated at reduced pressure to give a crude product. The crude product was purified by column chromatography to give a solid (90 mg, yield: 38.4%).

ESI-MS m/z: 592 [M+H]⁺.

### Step 2: Synthesis of compound 653

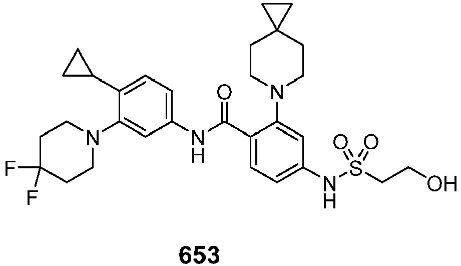

**Int_653-2** (90 mg, 0.152 mmol), (1*S*,2*S*)-*N*,*N*'-dimethyl-1,2-cyclohexanediamine (11 mg, 0.076 mmol), cuprous iodide (14 mg, 0.076 mmol), and potassium phosphate (96 mg, 0.456 mmol) were dissolved in DMF (8 mL). The mixture was purged with argon three times before **int_1-10** (38 mg, 0.304 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (25 mg, yield: 55%).

¹H NMR (400 MHz, Chloroform-d) δ 12.40 (s, 1H), 8.19 (d, *J* = 8.3 Hz, 1H), 7.67 (s, 1H), 7.32 (s, 1H), 7.24 (s, 1H), 7.02 (d, *J* = 9.7 Hz, 1H), 6.80 (d, *J* = 8.4 Hz, 1H), 4.13 (s, 2H), 3.32 (d, *J* = 5.7 Hz, 2H), 3.19 (d, *J* = 5.8 Hz, 4H), 3.06 (d, *J* = 5.9 Hz, 4H), 2.70 (s, 1H), 2.16 (dd, *J* = 18.3, 10.7 Hz, 4H), 1.62 (s, 4H), 1.01-0.95 (m, 2H), 0.71 (dd, *J* = 5.6, 1.8 Hz, 2H), 0.41 (s, 4H).

ESI-MS m/z: 589 [M+H]⁺.

### Example 29. Synthesis of Compound 655

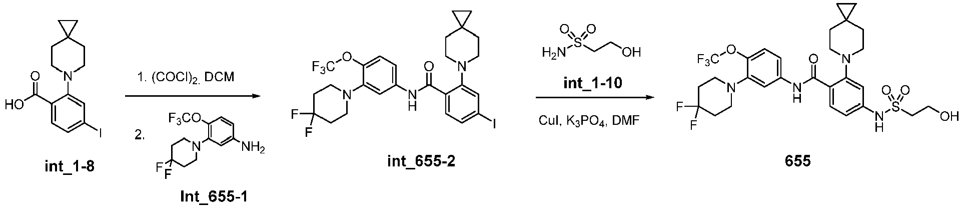

### Step 1: Synthesis of compound int_655-2

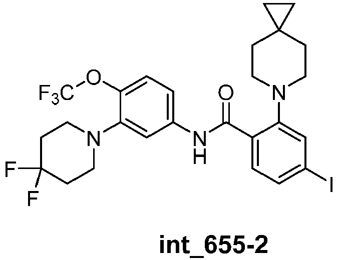

**Int_1-8** (216.8 mg, 0.607 mmol) was dissolved in DCM (50 mL), and oxalyl chloride (761.4 mg, 6 mmol) was added. The reaction solution was stirred at room temperature for 2 h and then concentrated at reduced pressure to remove the solvent, thus giving an acyl chloride product.

**Int_655-1** (150 mg, 0.507 mmol) was dissolved in tetrahydrofuran (5 mL), and in nitrogen atmosphere, NaH (300 mg, 7.5 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography to give a solid (320 mg, yield: 99%).

ESI-MS m/z: 636 [M+H]⁺.

### Step 2: Synthesis of compound 655

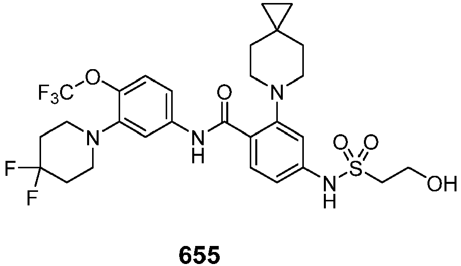

**Int_655-2** (350 mg, 0.55 mmol), (1*S*,2*S*)-*N*,*N*'-dimethyl-1,2-cyclohexanediamine (39 mg, 0.27 mmol), cuprous iodide (53 mg, 0.28 mmol), and potassium phosphate (351 mg, 1.66 mmol) were dissolved in DMF (7 mL). The mixture was purged with argon three times before **int_1-10** (138 mg, 1.1 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (250 mg, yield: 72%).

¹H NMR (400 MHz, DMSO-d6) δ 11.78 (s, 1H), 7.79 (d, *J* = 8.5 Hz, 1H), 7.65 (d, *J* = 2.5 Hz, 1H), 7.43 (dd, *J* = 8.8, 2.4 Hz, 1H), 7.30 (dd, *J* = 8.7, 1.4 Hz, 1H), 7.13 (d, *J* = 2.1 Hz, 1H), 7.00 (dd, *J* = 8.5, 2.1 Hz, 1H), 3.74 (t, *J* = 6.6 Hz, 2H), 3.29 (m, 2H), 3.13 (t, *J* = 5.6 Hz, 4H), 2.95 (t, *J* = 5.3 Hz, 4H), 2.09 (p, *J* = 8.1 Hz, 4H), 1.52 (s, 4H), 0.33 (s, 4H).

ESI-MS m/z: 633 [M+H]⁺.

### Example 30. Synthesis of Compound 661

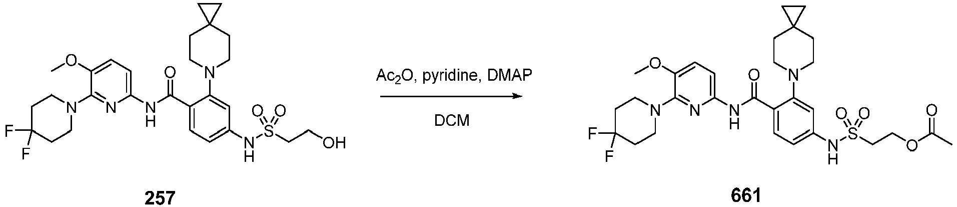

### Step 1: Synthesis of compound 661

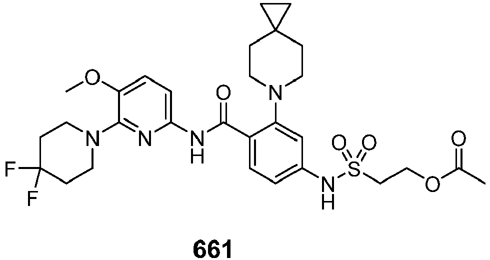

257 (1 g, 1.7 mmol) was dissolved in dichloromethane (20 mL), and acetic anhydride (176 mg, 1.7 mmol), pyridine (273 mg, 3.5 mmol), and DMAP (11 mg, 0.09 mmol) were added. The mixture was incubated at room temperature for reaction for 16 h, until LC-MS indicated the completion of the reaction. The reaction solution was filtered to give a filtrate, and the filtrate was concentrated at reduced pressure to give a crude product. The crude product was subjected to column chromatography to give the target product (0.7 g, yield: 70%).

¹H NMR (400 MHz, DMSO-d6) δ 12.81 (s, 1H), 8.03 (d, *J* = 8.6 Hz, 1H), 7.79 (d, *J* = 8.5 Hz, 1H), 7.34 (d, *J* = 8.6 Hz, 1H), 7.20 (d, *J* = 2.2 Hz, 1H), 7.06 (dd, *J* = 8.7, 2.1 Hz, 1H), 4.27 (t, *J* = 5.7 Hz, 2H), 3.78 (s, 3H), 3.56 (t, *J* = 5.7 Hz, 2H), 3.49 (t, *J* = 5.5 Hz, 4H), 2.94 (t, *J* = 5.2 Hz, 4H), 2.05 (dt, *J* = 16.4, 6.8 Hz, 4H), 1.87 (s, 3H), 1.71 (m, 4H), 0.35 (s, 4H).

ESI-MS m/z: 622 [M+H]⁺.

### Example 31. Synthesis of Compound 663

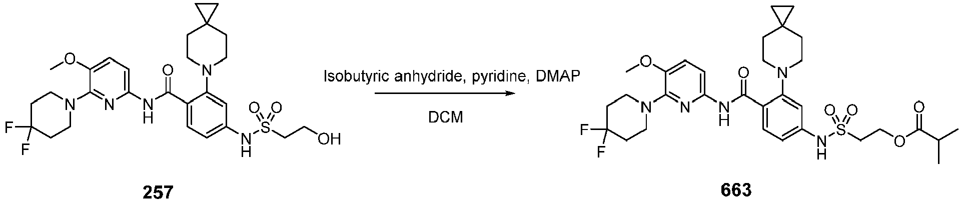

### Step 1: Synthesis of compound 663

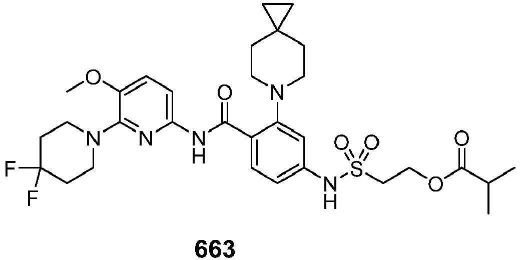

**257** (1 g, 1.7 mmol) was dissolved in dichloromethane (20 mL), and isobutyric anhydride (273 mg, 1.72 mmol), pyridine (273 mg, 3.5 mmol), and DMAP (11 mg, 0.09 mmol) were added. The mixture was incubated at room temperature for reaction for 16 h, until LC-MS indicated the completion of the reaction. The reaction solution was filtered to give a filtrate, and the filtrate was concentrated at reduced pressure to give a crude product. The crude product was subjected to column chromatography to give the target product (1.05 g, yield: 93.7%).

¹H NMR (400 MHz, DMSO-d6) δ 12.79 (s, 1H), 10.43 (s, 1H), 8.04 (d, *J =* 8.6 Hz, 1H), 7.79 (d, *J* = 8.5 Hz, 1H), 7.34 (d, *J=* 8.6 Hz, 1H), 7.19 (d, *J=* 2.2 Hz, 1H), 7.08 (dd, *J=* 8.7, 2.1 Hz, 1H), 4.31 (t, *J* = 5.5 Hz, 2H), 3.79 (s, 3H), 3.59 (t, *J* = 5.6 Hz, 2H), 3.50 (t, *J* = 5.7 Hz, 4H), 2.94 (t, *J* = 5.3 Hz, 4H), 2.38 (p, *J* = 7.0 Hz, 1H), 2.07 (q, *J* = 9.6, 6.0 Hz, 4H), 1.73 (m, 4H), 0.98 (d, *J =* 7.0 Hz, 6H), 0.35 (s, 4H).

ESI-MS m/z: 650 [M+H]⁺.

### Example 32. Synthesis of Compound 669

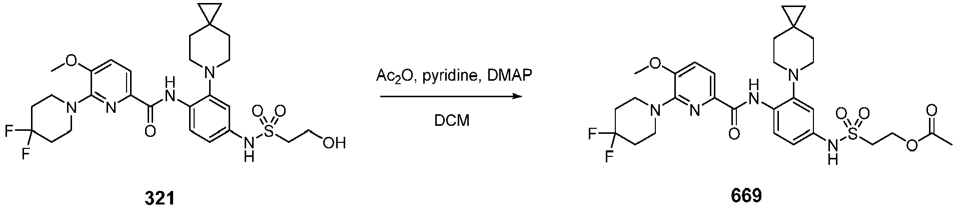

### Step 1: Synthesis of compound 669

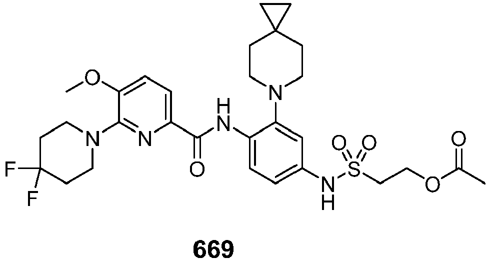

**321** (455 mg, 0.784 mmol) was dissolved in dichloromethane (14 mL), and acetic anhydride (80 mg, 0.784 mmol), pyridine (125 mg, 1.58 mmol), and DMAP (5.2 mg, 0.04 mmol) were added. The mixture was incubated at room temperature for reaction for 16 h, until LC-MS indicated the completion of the reaction.

The reaction solution was filtered to give a filtrate, and the filtrate was concentrated at reduced pressure to give a crude product. The crude product was subjected to column chromatography to give the target product (233 mg, yield: 48%).

¹H NMR (400 MHz, DMSO-d6) δ 10.37 (s, 1H), 9.73 (s, 1H), 8.31 (d, *J* = 8.8 Hz, 1H), 7.76 (d, *J* = 8.2 Hz, 1H), 7.48 (d, *J* = 8.3 Hz, 1H), 7.13 (d, *J* = 2.4 Hz, 1H), 6.97 (dd, *J* = 8.8, 2.4 Hz, 1H), 4.27 (t, *J* = 6.0 Hz, 2H), 3.90 (s, 3H), 3.56 (s, 4H), 3.41 (t, *J* = 6.0 Hz, 2H), 2.79 (d, *J* = 5.3 Hz, 4H), 2.12 (d, *J* = 15.3 Hz, 4H), 1.95 (s, 3H), 1.53 (s, 4H), 0.33 (s, 4H).

ESI-MS m/z: 622 [M+H]⁺.

### Example 33. Synthesis of Compound 714

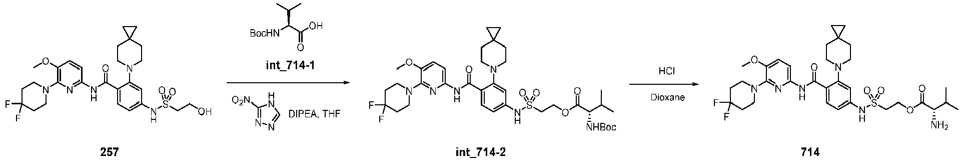

### Step 1: Synthesis of compound int_714-2

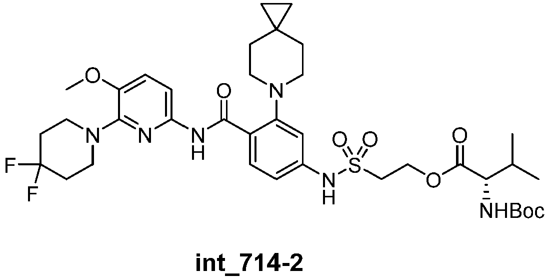

**257** (1 g, 1.73 mmol) was dissolved in tetrahydrofuran (20 mL), and **int_714-1** (1.87 g, 8.63 mmol), BOPCl (1.10 g, 4.31 mmol), 3-nitro-4H-1,2,4-triazole (491.94 mg, 4.31 mmol), and DIPEA (1.11 g, 8.63 mmol, 1.50 mL) were added. The mixture was incubated at room temperature for reaction for 16 h, until LC-MS indicated the completion of the reaction. The reaction solution was filtered to give a filtrate, and the filtrate was concentrated at reduced pressure to give a crude product. The crude product was purified by column chromatography (SiO₂, ethyl acetate/methanol = 1:1) to give the target product (1.16 g, yield: 86.3%).

¹H NMR (400 MHz, DMSO-d6) δ 12.79 (s, 1H), 8.03 (d, *J* = 8.6 Hz, 1H), 7.79 (d, *J* = 8.5 Hz, 1H), 7.34 (d, *J* = 8.7 Hz, 1H), 7.20 (s, 1H), 7.08 (t, *J* = 8.6 Hz, 2H), 4.35 (s, 2H), 3.79 (m, 4H), 3.54 (s, 2H), 3.49 (d, *J* = 5.9 Hz, 4H), 2.95 (s, 4H), 2.20-1.94 (m, 4H), 1.89-1.82 (m, 5H), 1.33 (s, 9H), 0.74 (dd, *J* = 6.8, 4.7 Hz, 5H), 0.35 (s, 4H).

ESI-MS m/z: 779 [M+H]⁺.

### Step 2: Synthesis of compound 714

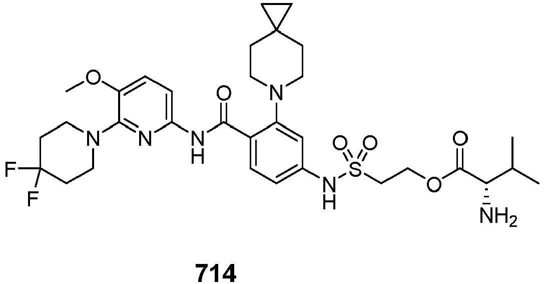

**Int_714-2** (1.16 g, 1.49 mmol) was dissolved in an HCl/dioxane solution (4 M, 11.6 mL), and the mixture was incubated at room temperature for reaction for 1 h, until LC-MS indicated the completion of the reaction. The reaction solution was concentrated at reduced pressure to give a crude product, and a saturated NaHCO₃ solution (15 mL) was added to the crude product to adjust the pH to 7-8. The aqueous phase was extracted with dichloromethane (20 mL × 2), and the organic phases were combined, dried over anhydrous sodium sulfate, filtered, and distilled at reduced pressure to give the target product (300 mg, yield: 29.6%).

¹H NMR: (400 MHz, DMSO-d6) δ 12.81 (s, 1H), δ 8.05 (d, *J=* 8.6 Hz, 1H), 7.82 (d, *J=* 8.5 Hz, 1H), 7.37 (d, *J =* 8.6 Hz, 1H), 7.21 (d, *J=* 2.2 Hz, 1H), 7.09 (dd, *J =* 8.6, 2.1 Hz, 1H), 4.37 (t, *J =* 5.7 Hz, 2H), 3.81 (s, 3H), 3.64-3.55 (m, 2H), 3.54-3.41 (m, 4H), 3.05 (d, *J=* 5.3 Hz, 1H), 2.97 (br s, 4H), 2.15-2.02 (m, 4H), 1.93-1.52 (m, 5H), δ 0.81 (d, *J =* 6.8 Hz, 3H), 0.75 (d, *J =* 6.8 Hz, 3H), 0.37 (s, 4H).

ESI-MS m/z: 679 [M+H]⁺.

### Example 34. Synthesis of Compound 727

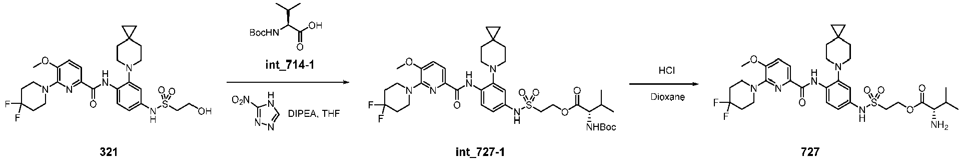

### Step 1: Synthesis of compound int_727-1

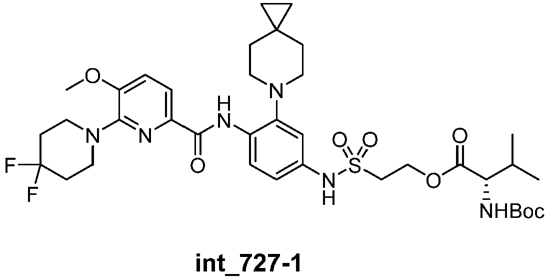

**321** (1.3 g, 2.25 mmol) was dissolved in tetrahydrofuran (100 mL), and **int_714-1** (2.4 g, 11.22 mmol), BOPCl (1.4 g, 5.61 mmol), 3-nitro-4H-1,2,4-triazole (640 mg, 5.61 mmol), and DIPEA (646.25 mg, 5 mmol) were added. The mixture was incubated at room temperature for reaction for 4 h, until LC-MS indicated the completion of the reaction. The reaction solution was filtered to give a filtrate, and the filtrate was concentrated at reduced pressure to give a crude product. The crude product was purified by column chromatography (SiO₂, n-hexane/ethyl acetate = 5:1 to 1:1) to give the target product (1.1 g, yield: 62.9%).

ESI-MS m/z: 779 [M+H]⁺.

### Step 2: Synthesis of compound 727

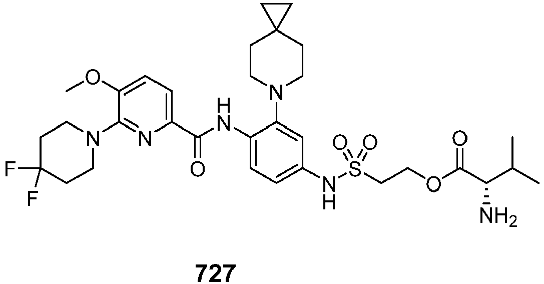

**Int_727-1** (550 mg, 0.71 mmol) was dissolved in an HCl/dioxane solution (4 M, 11 mL), and the mixture was incubated at room temperature for reaction for 1 h, until LC-MS indicated the completion of the reaction. The reaction solution was concentrated at reduced pressure to give a crude product, and a saturated NaHCO₃ solution (6 mL) was added to the crude product to adjust the pH to 7-8. The aqueous phase was extracted with dichloromethane (6 mL × 3), and the organic phases were combined, dried over anhydrous sodium sulfate, filtered, and distilled at reduced pressure to give a product. The product was further purified by prep-HPLC (column: Phenomenex C18 250 × 50 mm × 10 µm; mobile phase: [water (ammonia hydroxide v/v)-ACN]; B%: 43%-73%, 8 min) to give the target product (291 mg, yield: 58.8%).

¹H NMR: (400 MHz, DMSO-d6) δ 10.44-10.34 (m, 1H), 8.34 (d, *J* = 8.8 Hz, 1H), 7.79 (d, *J* = 8.1 Hz, 1H), 7.50 (d, *J* = 8.4 Hz, 1H), 7.15 (d, *J* = 2.4 Hz, 1H), 7.06-6.96 (m, 1H), 4.35 (t, *J* = 6.0 Hz, 2H), 3.93 (s, 3H), 3.65-3.54 (m, 4H), 3.07 (br d, *J* = 5.3 Hz, 1H), 2.82 (br t, *J* = 4.9 Hz, 4H), 2.22-2.10 (m, 4H), 1.85-1.71 (m, 1H), 1.67-1.42 (m, 4H), 0.88-0.73 (m, 6H), 0.35 (s, 4H).

ESI-MS m/z: 679 [M+H]⁺.

### Example 35. Synthesis of Compound 740

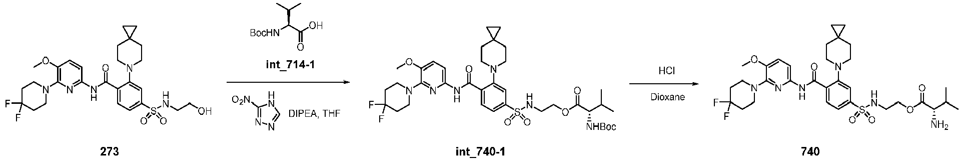

### Step 1: Synthesis of compound int_740-1

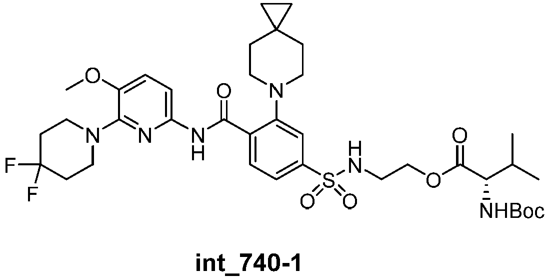

**273** (1.3 g, 2.25 mmol) was dissolved in tetrahydrofuran (100 mL), and **int_714-1** (2.4 g, 11.22 mmol), BOPCl (1.4 g, 5.61 mmol), 3-nitro-4H-1,2,4-triazole (640 mg, 5.61 mmol), and DIPEA (646.25 mg, 5 mmol) were added. The mixture was incubated at room temperature for reaction for 4 h, until LC-MS indicated the completion of the reaction. The reaction solution was filtered to give a filtrate, and the filtrate was concentrated at reduced pressure to give a crude product. The crude product was purified by column chromatography to give the target product (1 g, yield: 65.8%).

ESI-MS m/z: 779 [M+H]⁺.

### Step 2: Synthesis of compound 740

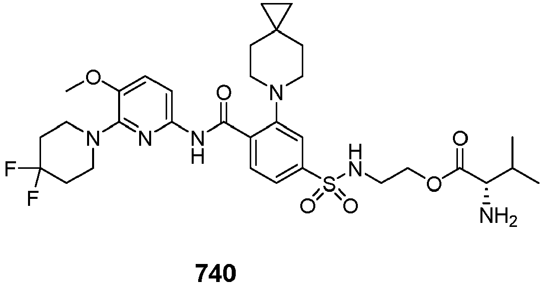

**Int_740-1** (800 mg, 1.03 mmol) was dissolved in an HCl/dioxane solution (4 M, 11 mL), and the mixture was incubated at room temperature for reaction for 1 h, until LC-MS indicated the completion of the reaction. The reaction solution was concentrated at reduced pressure to give a crude product, and a saturated NaHCO₃ solution (8 mL) was added to the crude product to adjust the pH to 7-8. The aqueous phase was extracted with dichloromethane (8 mL × 3), and the organic phases were combined, dried over anhydrous sodium sulfate, filtered, and distilled at reduced pressure to give the target product (310 mg, yield: 44.3%).

¹H NMR: (400 MHz, Chloroform-d) δ 12.53 (s, 1H), 8.36-8.34 (m, 1H), 7.85-7.80 (m, 1H), 7.73 (s, 1H), 7.66-7.64 (m, 1H), 7.11-7.04 (m, 1H), 4.21-4.10 (m, 2H), 4.13 (s, 3H), 3.52-3.35 (m, 4H), 3.25-3.20 (m, 1H), 3.20-3.10 (m, 2H), 3.05 (m, 4H), 2.12-2.10 (m, 4H), 1.90-1.71 (m, 1H), 1.75-1.42 (m, 4H), 0.75-0.85 (m, 6H), 0.34 (s, 4H).

ESI-MS m/z: 679 [M+H]⁺.

### Example 36. Synthesis of Compound 825

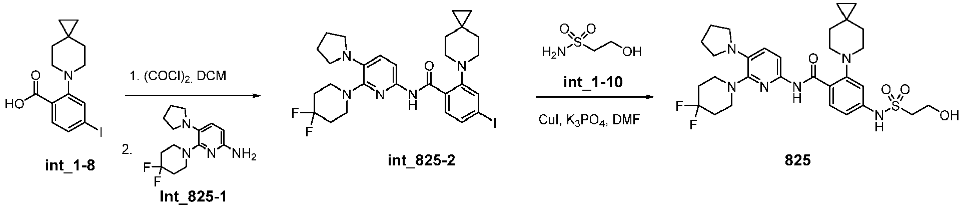

### Step 1: Synthesis of compound int_825-2

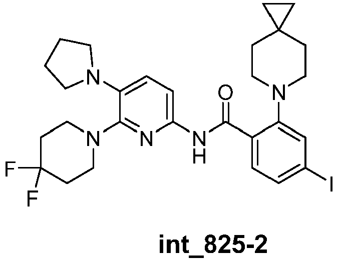

**Int_1-8** (95 mg, 0.266 mmol) was dissolved in DCM (50 mL), and oxalyl chloride (380 mg, 3 mmol) was added. The reaction solution was stirred at room temperature for 2 h and then concentrated at reduced pressure to remove the solvent, thus giving an acyl chloride product.

**Int_825-1** (75 mg, 0.266 mmol) was dissolved in tetrahydrofuran (6 mL), and in nitrogen atmosphere, NaH (100 mg, 2.5 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 10:1) to give a solid (59 mg, yield: 35.7%).

ESI-MS m/z: 622 [M+H]⁺.

### Step 2: Synthesis of compound 825

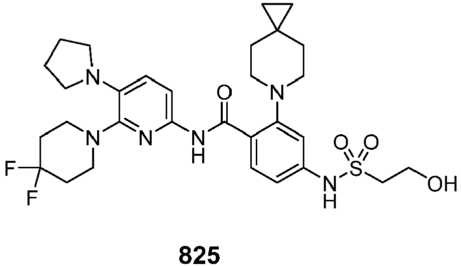

**Int_825-2** (59 mg, 0.095 mmol), (1*S*,2*S*)-*N*,*N*'-dimethyl-1,2-cyclohexanediamine (7 mg, 0.047 mmol), cuprous iodide (10 mg, 0.047 mmol), and potassium phosphate (60 mg, 0.285 mmol) were dissolved in DMF (5 mL). The mixture was purged with argon three times before **int_1-10** (24 mg, 0.189 mmol) was added. In argon atmosphere, the reaction solution was heated to 90 °C and incubated for reaction for 16 h, until LC-MS indicated the completion of the reaction. The reaction solution was cooled to room temperature, concentrated by rotary evaporation, and purified by column chromatography to give a solid (7 mg, yield: 12.1%).

¹H NMR (400 MHz, DMSO-d6) δ 12.84 (s, 1H), 8.03 (d, *J* = 8.6 Hz, 1H), 7.79 (d, *J* = 8.4 Hz, 1H), 7.31-7.14 (m, 2H), 7.07 (dd, *J* = 8.6, 2.1 Hz, 1H), 3.73 (t, *J* = 6.5 Hz, 2H), 3.38 (t, *J* = 5.6 Hz, 4H), 3.31 (t, *J* = 6.5 Hz, 2H), 3.06 (d, *J* = 5.9 Hz, 4H), 2.95 (d, *J* = 5.4 Hz, 4H), 2.10 (tt, *J* = 13.1, 5.5 Hz, 4H), 1.93-1.50 (m, 8H), 0.36 (s, 4H).

ESI-MS m/z: 619 [M+H]⁺.

### Example 37. Synthesis of Compound 826

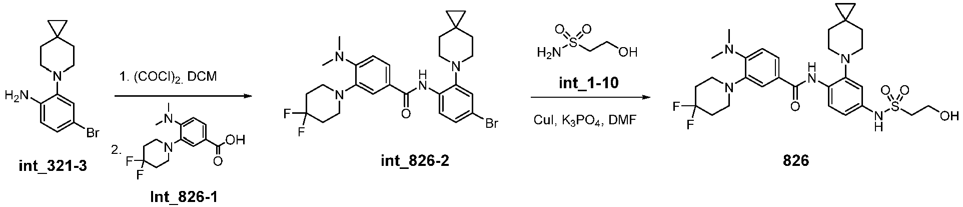

### Step 1: Synthesis of compound int_826-2

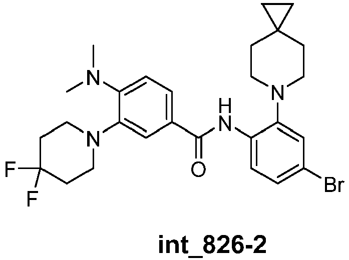

**Int_321-3** (187.6 mg, 0.605 mmol) was dissolved in DCM (10 mL), and oxalyl chloride (760 mg, 6 mmol) was added. The reaction solution was stirred at room temperature for 2 h and then concentrated at reduced pressure to remove the solvent, thus giving an acyl chloride product.

**Int_826-1** (170 mg, 0.605 mmol) was dissolved in tetrahydrofuran (10 mL), and in nitrogen atmosphere, NaH (170 mg, 4.25 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 10:1) to give a solid (190 mg, yield: 58.1%).

ESI-MS m/z: 547 [M+H]⁺.

### Step 2: Synthesis of compound 826

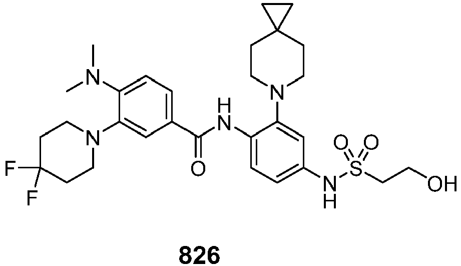

**Int_826-2** (190 mg, 0.345 mmol), *N*,*N*-dimethylglycine (25 mg, 0.173 mmol), cuprous iodide (33 mg, 0.173 mmol), and potassium phosphate (219 mg, 1.035 mmol) were dissolved in DMF (4 mL). The mixture was purged with argon three times before **int_1-10** (65 mg, 0.518 mmol) was added. In argon atmosphere, the reaction solution was heated to 130 °C and incubated for reaction for 3 h, until LC-MS indicated the completion of the reaction. The reaction solution was cooled to room temperature, concentrated by rotary evaporation, and purified by column chromatography to give a solid (51 mg, yield: 25%).

¹H NMR (400 MHz, DMSO-d6) δ 9.36 (s, 1H), 8.06 (d, *J=* 8.7 Hz, 1H), 7.55 (dd, *J=* 8.4, 2.1 Hz, 1H), 7.48 (d, *J* = 2.2 Hz, 1H), 7.11 (d, *J* = 2.4 Hz, 1H), 7.01 (d, *J* = 8.4 Hz, 1H), 6.95 (dd, *J* = 8.7, 2.4 Hz, 1H), 3.72 (t, *J* = 6.8 Hz, 2H), 3.18 (q, *J* = 7.0 Hz, 6H), 2.88 (s, 6H), 2.82 (t, *J* = 5.3 Hz, 4H), 2.21-2.10 (m, 4H), 1.51 (s, 4H), 0.34 (s, 4H).

ESI-MS m/z: 592 [M+H]⁺.

### Example 38. Synthesis of Compound 828

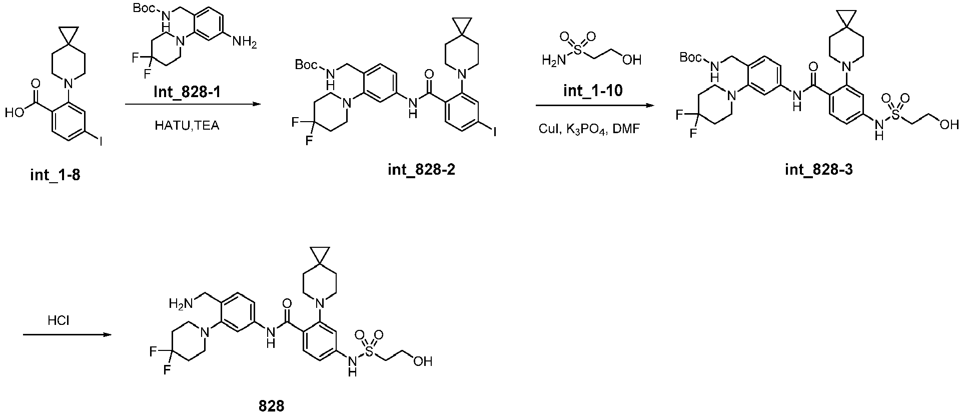

### Step 1: Synthesis of compound int_828-2

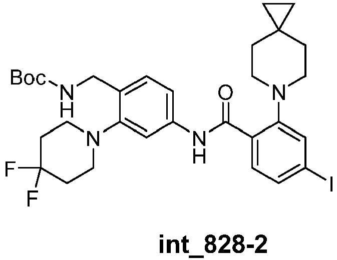

**Int_1-8** (356 mg, 1 mmol) was dissolved in DCM (50 mL), and **int_828-1** (340 mg, 1 mmol), HATU (760 mg, 2 mmol), and TEA (304 mg, 3 mmol) in DMF (8 mL) were added. In nitrogen atmosphere, the reaction solution was stirred at room temperature for 12 h, until LC-MS indicated the completion of the reaction. The reaction solution was concentrated at reduced pressure to give a crude product. The crude product was purified by column chromatography to give a solid (500 mg, yield: 83.6%).

ESI-MS m/z: 681 [M+H]⁺.

### Step 2: Synthesis of compound int_828-3

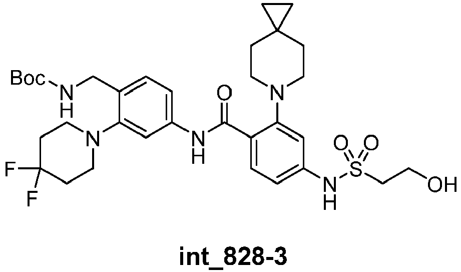

**Int_828-2** (200 mg, 0.29 mmol), (1*S*,2*S*)-(+)-*N*,*N*'-dimethyl-1,2-cyclohexanediamine (20 mg, 0.145 mmol), cuprous iodide (30 mg, 0.145 mmol), and potassium phosphate (180 mg, 0.87 mmol) were dissolved in DMF (8 mL). The mixture was purged with argon three times before **int_1-10** (60 mg, 0.47 mmol) was added. In argon atmosphere, the reaction solution was heated to 90 °C and incubated for reaction for 12 h, until LC-MS indicated the completion of the reaction. The reaction solution was cooled to room temperature, concentrated by rotary evaporation, and purified by column chromatography to give a solid (140 mg, yield: 70.3%).

ESI-MS m/z: 678 [M+H]⁺.

### Step 3: Synthesis of compound 828

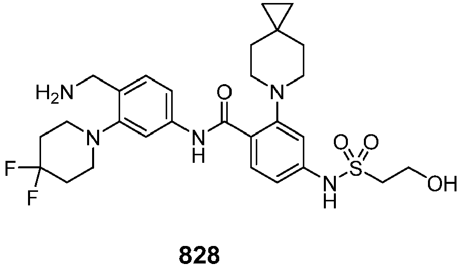

**Int_828-3** (80 mg, 0.118 mmol) was dissolved in methanol/hydrochloric acid (4 N, 10 mL), and the reaction solution was incubated at room temperature for reaction for 12 h, until LC-MS indicated the completion of the reaction. The reaction solution was concentrated by rotary evaporation and purified by column chromatography to give a solid (50 mg, yield: 73.5%).

¹H NMR (400 MHz, DMSO-d6) δ 12.10 (s, 1H), 7.76 (d, *J* = 8.6 Hz, 1H), 7.60 (d, *J* = 2.0 Hz, 1H), 7.48 (dd, *J =* 8.3, 2.0 Hz, 1H), 7.41 (s, 1H), 7.00 (d, *J* = 2.1 Hz, 1H), 6.86 (dd, *J* = 8.6, 2.0 Hz, 1H), 3.84 (s, 2H), 3.71 (t, *J* = 6.6 Hz, 2H), 3.18-3.10 (m, 2H), 2.94 (dt, *J* = 11.8, 5.5 Hz, 8H), 2.19-2.07 (m, 4H), 1.54 (s, 4H), 0.34 (s, 4H).

ESI-MS m/z: 578 [M+H]⁺.

### Example 39. Synthesis of Compound 829

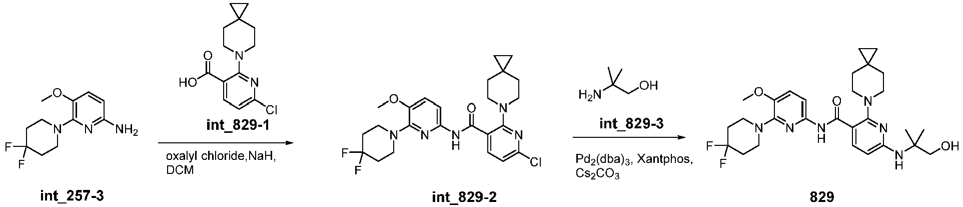

### Step 1: Synthesis of compound int_829-2

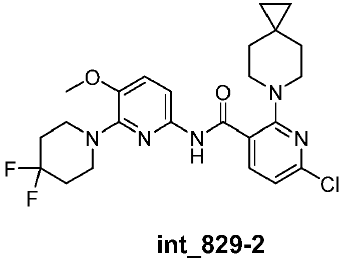

**Int_257-3** (100 mg, 0.374 mmol) was dissolved in DCM (10 mL), and oxalyl chloride (380 mg, 3 mmol) was added. The reaction solution was stirred at room temperature for 2 h and then concentrated at reduced pressure to remove the solvent, thus giving an acyl chloride product.

**Int_829-1** (100 mg, 0.411 mmol) was dissolved in tetrahydrofuran (10 mL), and in nitrogen atmosphere, NaH (80 mg, 2 mmol, 60% purity) was added. The mixture was stirred at room temperature for 0.5 h and then the acyl chloride prepared previously was added at room temperature. The reaction solution was warmed to 40 °C and stirred for 10 h, until LC-MS indicated the completion of the reaction. Methanol was added under an ice bath to quench the reaction, and the reaction solution was concentrated at reduced pressure to give a crude product. The crude product was purified by column chromatography (SiO₂, n-hexane/ethyl acetate = 6:1) to give a solid (90 mg, yield: 44.5%).

ESI-MS m/z: 492 [M+H]⁺.

### Step 2: Synthesis of compound 829

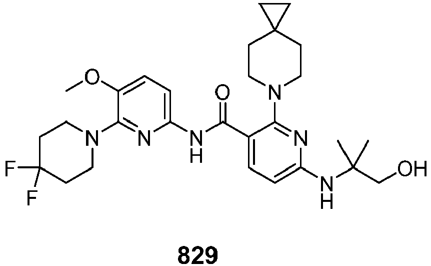

**Int_829-2** (90 mg, 0.183 mmol), **int_829-3** (33 mg, 0.366 mmol), cesium carbonate (90 mg, 0.274 mmol), Pd₂(dba)₃ (17 mg, 0.0183 mmol), and XantPhos (10 mg, 0.0183 mmol) were dissolved in 1,4-dioxane (8 mL), and the mixture was purged with argon three times. In argon atmosphere, the reaction solution was heated to 95 °C and incubated for reaction for 16 h, until LC-MS indicated the completion of the reaction. The reaction solution was cooled to room temperature, concentrated by rotary evaporation, and purified by column chromatography to give a solid (50 mg, yield: 50.5%).

¹H NMR (400 MHz, DMSO-d6) δ 11.82 (s, 1H), 8.24 (d, *J* = 8.4 Hz, 1H), 7.73 (d, *J* = 8.4 Hz, 1H), 7.34 (d, *J* = 8.6 Hz, 1H), 6.68 (d, *J* = 8.4 Hz, 1H), 4.18 (s, 2H), 3.78 (s, 3H), 3.51 (d, *J* = 5.8 Hz, 4H), 3.12 (t, *J* = 5.4 Hz, 4H), 2.05 (tt, *J* = 13.7, 5.4 Hz, 4H), 1.64 (d, *J* = 5.7 Hz, 4H), 1.22 (s, 6H), 0.33 (s, 4H).

ESI-MS m/z: 545 [M+H]⁺.

Target **compounds 5, 8-64, 66-96, 98-128, 130-160, 162-256, 264-272, 274-320, 322-336, 338-352, 354-368, 370-384, 386-576, 578-640, 642, 644, 646-652, 654, 656-660, 662, 664-668, 670-713, 715-726, 728-739, 741-824, 827**, **and 830-837** in **Table 1** were obtained by using the above synthesis methods with different starting materials.

**Table 1**

| **Compound** | **Compound structure** | **MS (M+H)⁺** | **Compound** | **Compound structure** | **MS (M+H)⁺** |
|---|---|---|---|---|---|
| **2** | 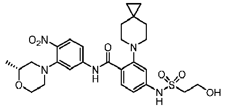 | 574 | **3** | 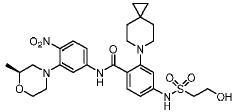 | 574 |
| **4** | 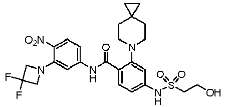 | 566 | **5** | 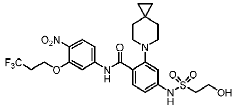 | 587 |
| **6** | 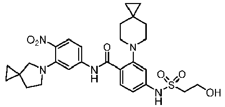 | 570 | **7** | 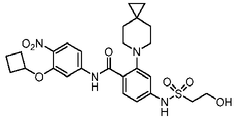 | 545 |
| **8** | 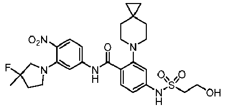 | 576 | **9** | 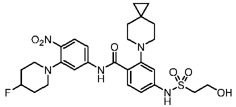 | 576 |
| **10** | 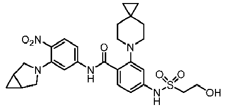 | 556 | **11** | 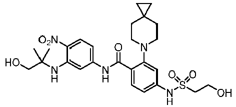 | 562 |
| **12** | 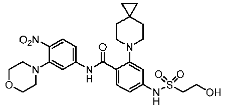 | 560 | **13** | 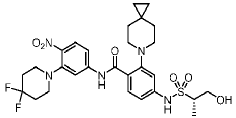 | 608 |
| **14** | 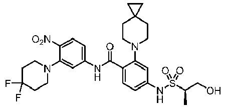 | 608 | **15** | 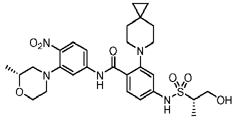 | 588 |
| **16** | 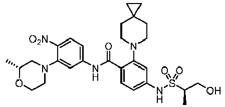 | 588 | **17** | 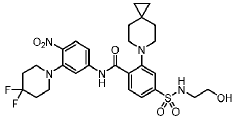 | 594 |
| **18** | 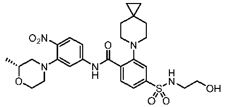 | 574 | **19** | 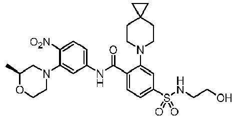 | 574 |
| **20** | 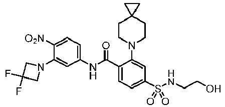 | 566 | **21** | 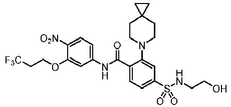 | 587 |
| **22** | 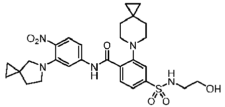 | 570 | **23** | 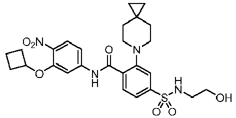 | 545 |
| **24** | 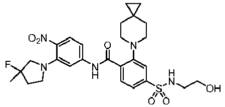 | 576 | **25** | 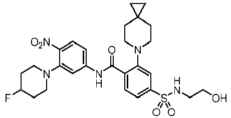 | 576 |
| **26** | 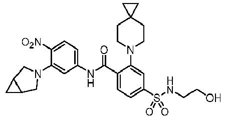 | 556 | **27** | 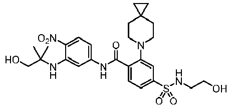 | 562 |
| **28** | 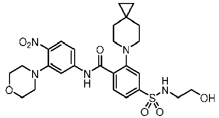 | 560 | **29** | 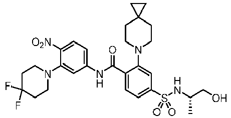 | 608 |
| **30** | 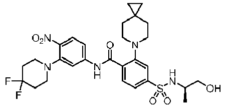 | 608 | **31** | 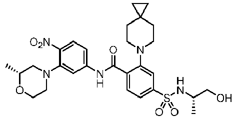 | 588 |
| **32** | 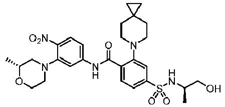 | 588 | **33** | 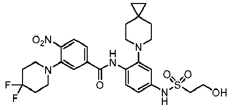 | 594 |
| **34** | 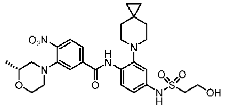 | 574 | **35** | 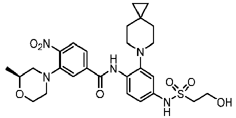 | 574 |
| **36** | 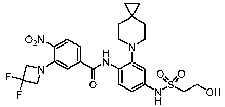 | 566 | **37** | 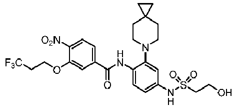 | 587 |
| **38** | 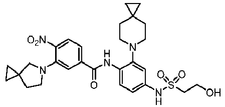 | 570 | **39** | 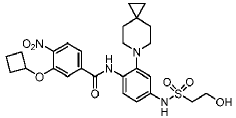 | 545 |
| **40** | 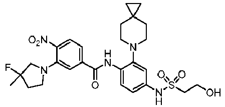 | 576 | **41** | 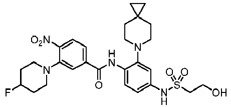 | 576 |
| **42** | 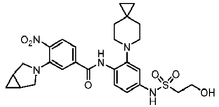 | 556 | **43** | 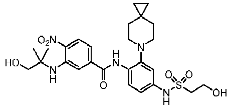 | 562 |
| **44** | 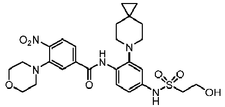 | 560 | **45** | 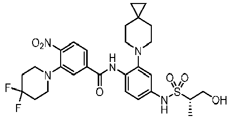 | 608 |
| **46** | 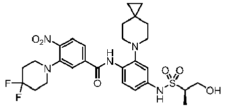 | 608 | **47** | 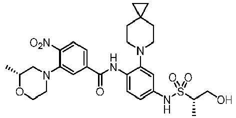 | 588 |
| **48** | 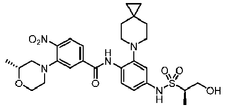 | 588 | **49** | 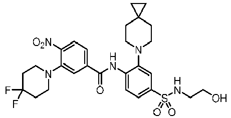 | 594 |
| **50** | 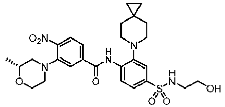 | 574 | **51** | 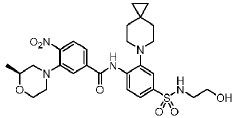 | 574 |
| **52** | 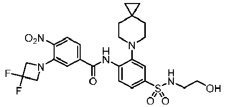 | 566 | **53** | 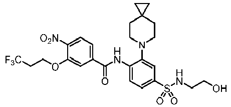 | 587 |
| **54** | 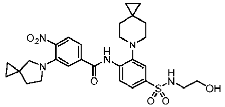 | 570 | **55** | 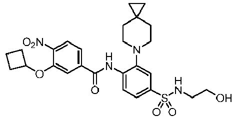 | 545 |
| **56** | 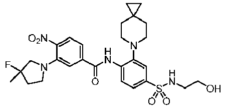 | 576 | **57** | 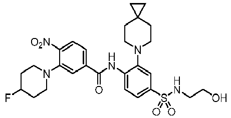 | 576 |
| **58** | 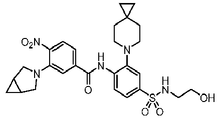 | 556 | **59** | 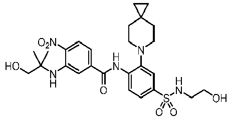 | 562 |
| **60** | 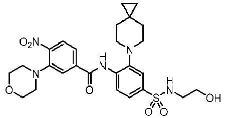 | 560 | **61** | 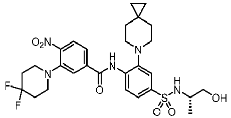 | 608 |
| **62** | 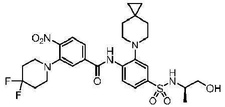 | 608 | **63** | 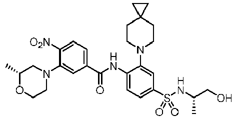 | 588 |
| **64** | 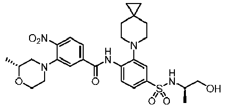 | 588 | **65** | 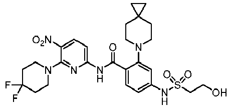 | 595 |
| **66** | 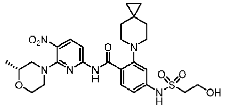 | 575 | **67** | 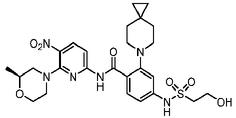 | 575 |
| **68** | 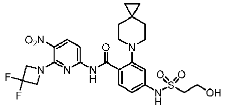 | 567 | **69** | 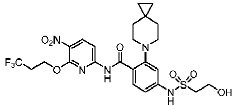 | 588 |
| **70** | 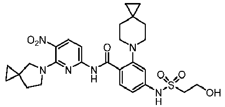 | 571 | **71** | 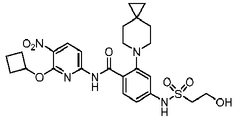 | 546 |
| **72** | 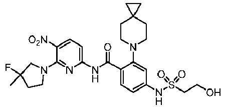 | 577 | **73** | 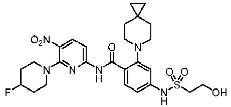 | 577 |
| **74** | 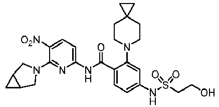 | 557 | **75** | 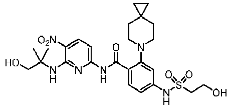 | 563 |
| **76** | 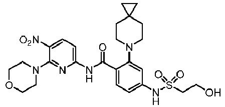 | 561 | 77 | 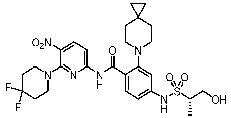 | 609 |
| **78** | 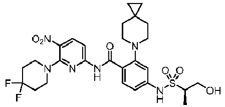 | 609 | **79** | 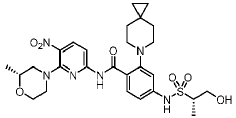 | 589 |
| **80** | 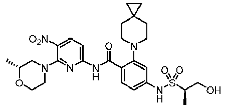 | 589 | **81** | 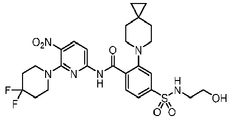 | 595 |
| **82** | 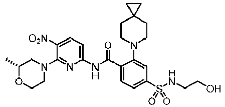 | 575 | **83** | 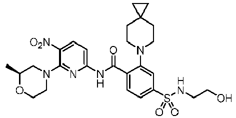 | 575 |
| **84** | 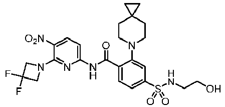 | 567 | **85** | 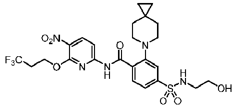 | 588 |
| **86** | 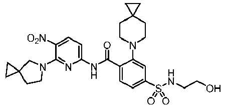 | 571 | **87** | 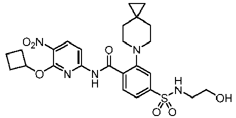 | 546 |
| **88** | 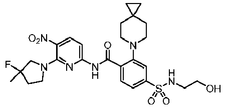 | 577 | **89** | 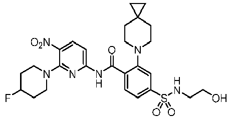 | 577 |
| **90** | 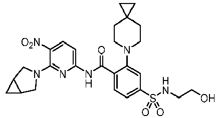 | 557 | **91** | 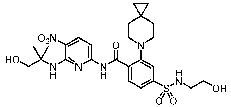 | 563 |
| **92** | 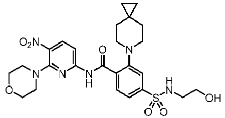 | 561 | **93** | 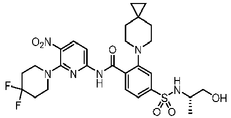 | 609 |
| **94** | 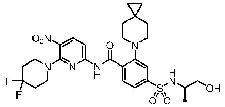 | 609 | **95** | 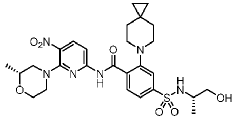 | 589 |
| **96** | 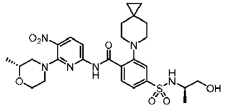 | 589 | **97** | 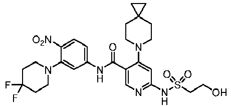 | 595 |
| **98** | 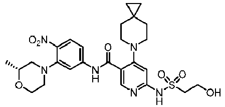 | 575 | **99** | 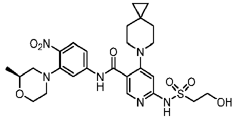 | 575 |
| **100** | 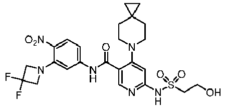 | 567 | **101** | 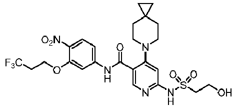 | 588 |
| **102** | 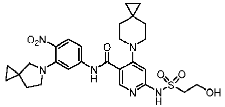 | 571 | **103** | 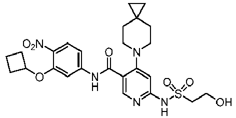 | 546 |
| **104** | 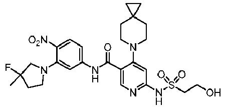 | 577 | **105** | 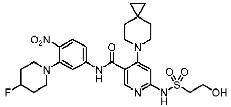 | 577 |
| **106** | 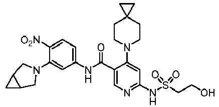 | 557 | **107** | 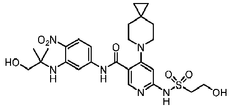 | 563 |
| **108** | 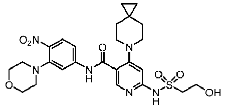 | 561 | **109** | 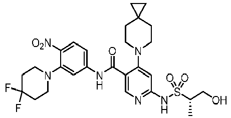 | 609 |
| **110** | 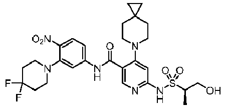 | 609 | **111** | 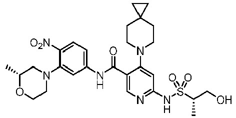 | 589 |
| **112** | 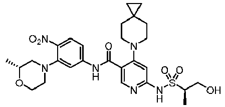 | 589 | **113** | 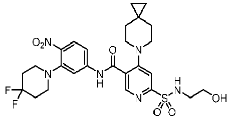 | 595 |
| **114** | 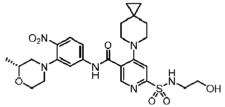 | 575 | **115** | 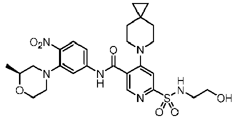 | 575 |
| **116** | 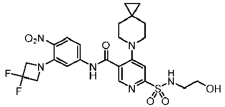 | 567 | **117** | 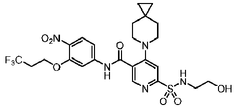 | 588 |
| **118** | 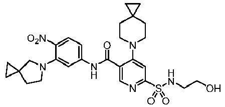 | 571 | **119** | 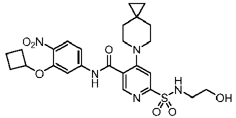 | 546 |
| **120** | 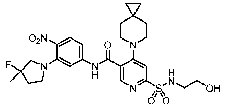 | 577 | **121** | 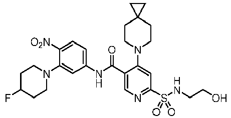 | 577 |
| **122** | 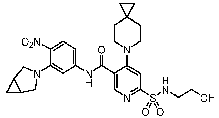 | 557 | **123** | 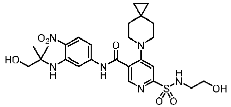 | 563 |
| **124** | 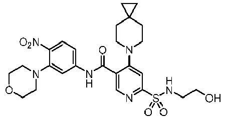 | 561 | **125** | 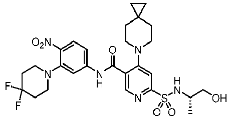 | 609 |
| **126** | 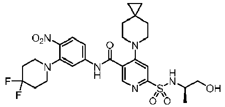 | 609 | **127** | 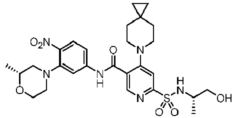 | 589 |
| **128** | 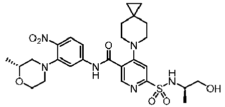 | 589 | **129** | 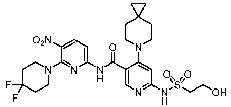 | 596 |
| **130** | 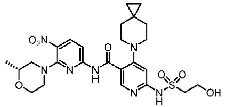 | 576 | **131** | 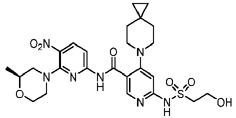 | 576 |
| **132** | 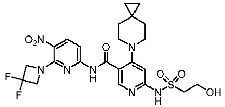 | 568 | **133** | 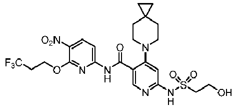 | 589 |
| **134** | 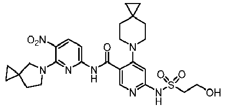 | 572 | **135** | 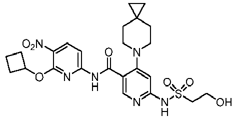 | 547 |
| **136** | 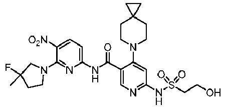 | 578 | **137** | 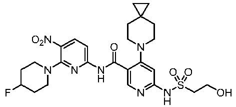 | 578 |
| **138** | 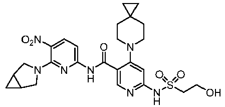 | 558 | **139** | 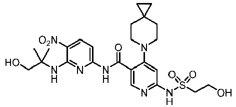 | 564 |
| **140** | 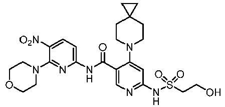 | 562 | **141** | 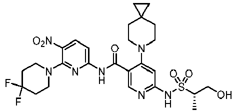 | 610 |
| **142** | 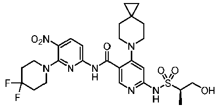 | 610 | **143** | 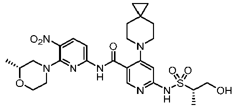 | 590 |
| **144** | 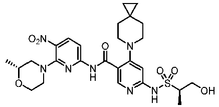 | 590 | **145** | 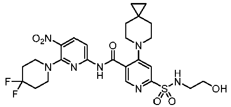 | 596 |
| **146** | 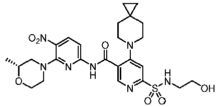 | 576 | **147** | 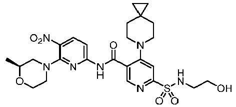 | 576 |
| **148** | 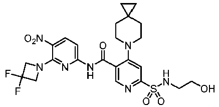 | 568 | **149** | 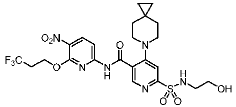 | 589 |
| **150** | 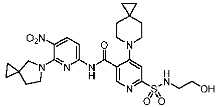 | 572 | **151** | 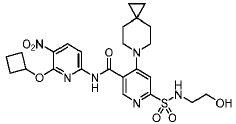 | 547 |
| **152** | 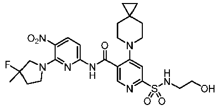 | 578 | **153** | 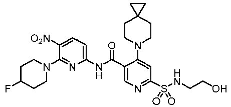 | 578 |
| **154** | 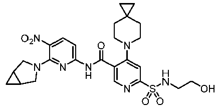 | 558 | **155** | 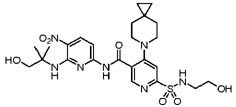 | 564 |
| **156** | 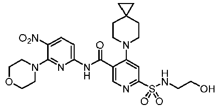 | 562 | **157** | 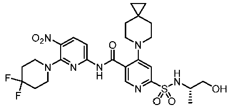 | 610 |
| **158** | 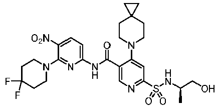 | 610 | **159** | 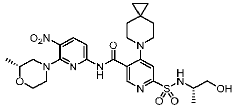 | 590 |
| **160** | 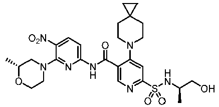 | 590 | **161** | 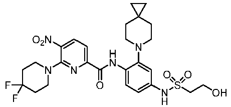 | 595 |
| **162** | 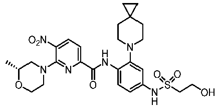 | 575 | **163** | 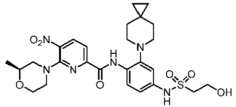 | 575 |
| **164** | 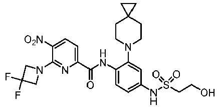 | 567 | **165** | 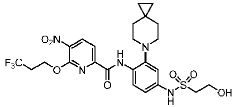 | 588 |
| **166** | 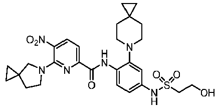 | 571 | **167** | 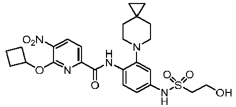 | 546 |
| **168** | 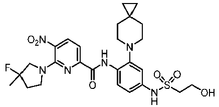 | 577 | **169** | 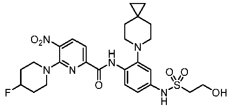 | 577 |
| **170** | 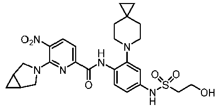 | 557 | **171** | 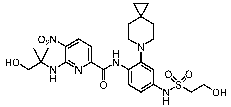 | 563 |
| **172** | 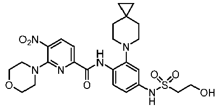 | 561 | **173** | 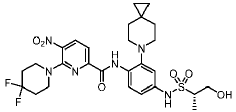 | 609 |
| **174** | 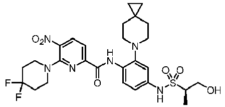 | 609 | **175** | 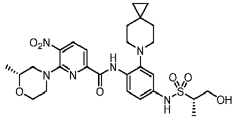 | 589 |
| **176** | 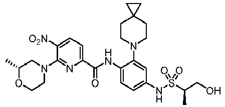 | 589 | **177** | 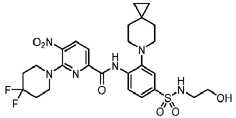 | 595 |
| **178** | 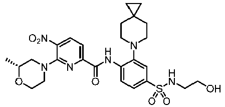 | 575 | **179** | 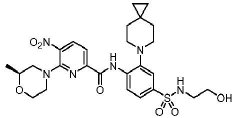 | 575 |
| **180** | 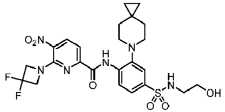 | 567 | **181** | 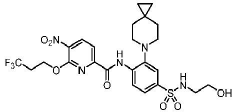 | 588 |
| **182** | 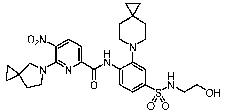 | 571 | **183** | 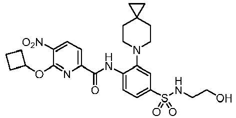 | 546 |
| **184** | 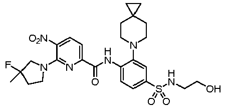 | 577 | **185** | 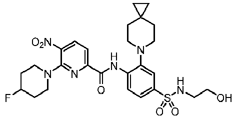 | 577 |
| **186** | 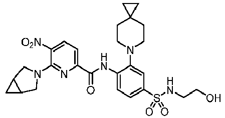 | 557 | **187** | 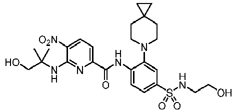 | 563 |
| **188** | 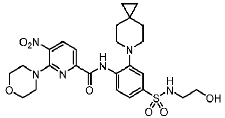 | 561 | **189** | 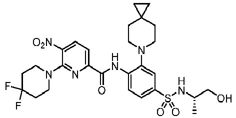 | 609 |
| **190** | 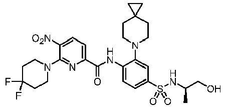 | 609 | **191** | 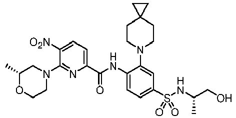 | 589 |
| **192** | 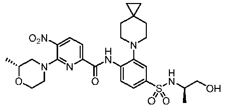 | 589 | **193** | 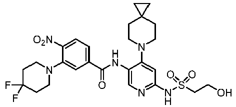 | 595 |
| **194** | 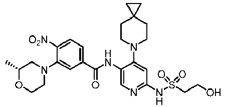 | 575 | **195** | 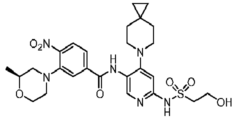 | 575 |
| **196** | 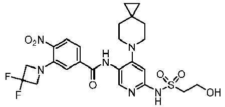 | 567 | **197** | 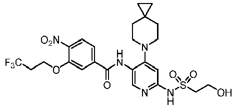 | 588 |
| **198** | 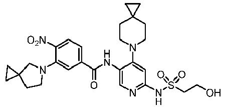 | 571 | **199** | 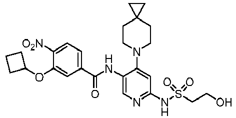 | 546 |
| **200** | 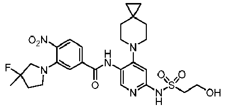 | 577 | **201** | 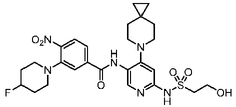 | 577 |
| **202** | 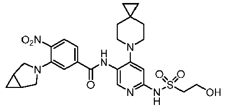 | 557 | **203** | 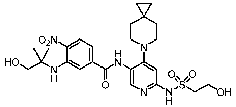 | 563 |
| **204** | 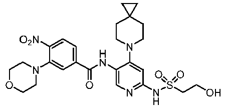 | 561 | **205** | 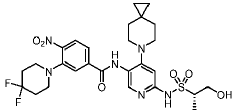 | 609 |
| **206** | 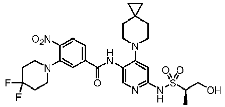 | 609 | **207** | 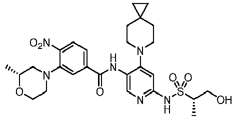 | 589 |
| **208** | 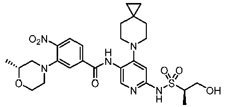 | 589 | **209** | 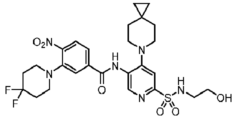 | 595 |
| **210** | 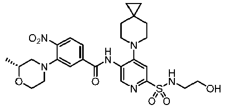 | 575 | **211** | 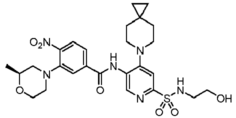 | 575 |
| **212** | 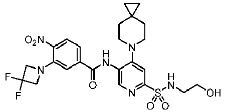 | 567 | **213** | 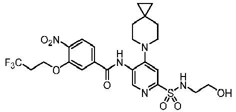 | 588 |
| **214** | 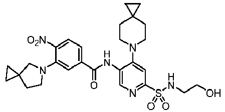 | 571 | **215** | 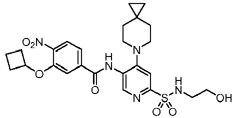 | 546 |
| **216** | 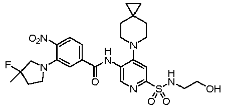 | 577 | **217** | 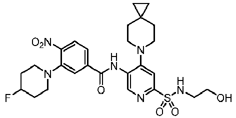 | 577 |
| **218** | 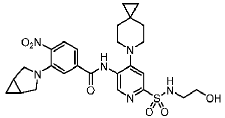 | 557 | **219** | 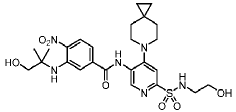 | 563 |
| **220** | 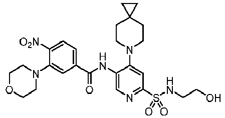 | 561 | **221** | 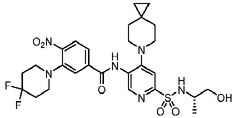 | 609 |
| **222** | 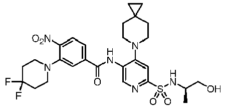 | 609 | **223** | 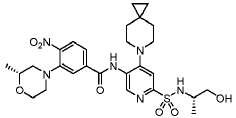 | 589 |
| **224** | 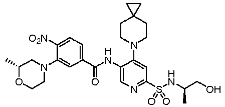 | 589 | **225** | 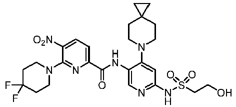 | 596 |
| **226** | 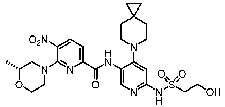 | 576 | **227** | 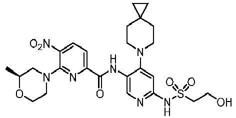 | 576 |
| **228** | 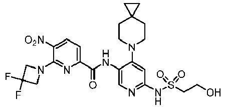 | 568 | **229** | 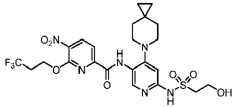 | 589 |
| **230** | 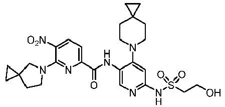 | 572 | **231** | 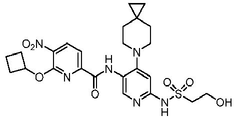 | 547 |
| **232** | 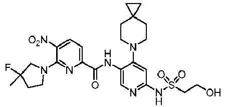 | 578 | **233** | 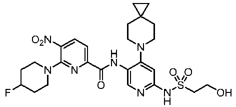 | 578 |
| **234** | 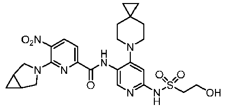 | 558 | **235** | 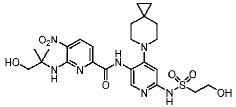 | 564 |
| **236** | 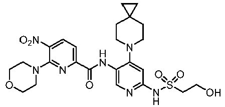 | 562 | **237** | 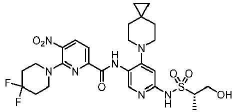 | 610 |
| **238** | 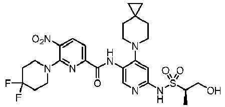 | 610 | **239** | 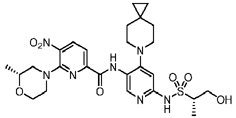 | 590 |
| **240** | 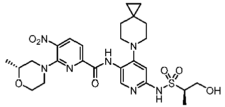 | 590 | **241** | 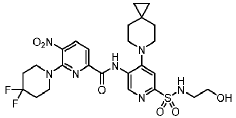 | 596 |
| **242** | 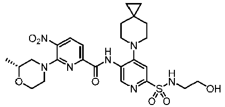 | 576 | **243** | 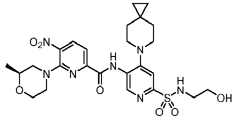 | 576 |
| **244** | 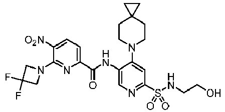 | 568 | **245** | 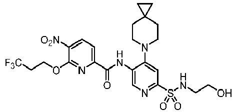 | 589 |
| **246** | 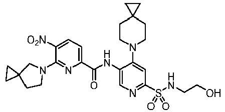 | 572 | **247** | 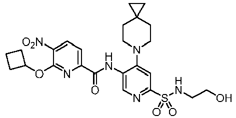 | 547 |
| **248** | 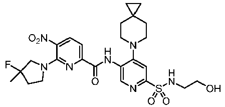 | 578 | **249** | 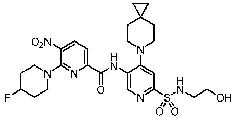 | 578 |
| **250** | 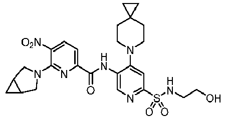 | 558 | **251** | 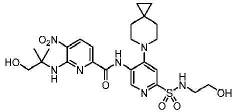 | 564 |
| **252** | 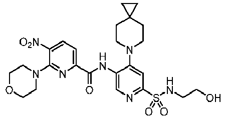 | 562 | **253** | 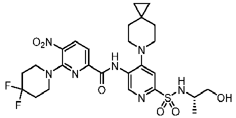 | 610 |
| **254** | 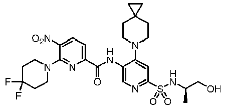 | 610 | **255** | 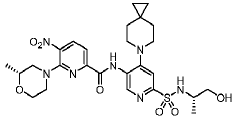 | 590 |
| **256** | 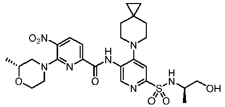 | 590 | **257** | 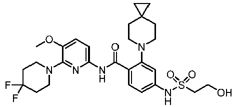 | 580 |
| **258** | 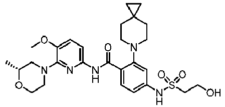 | 560 | **259** | 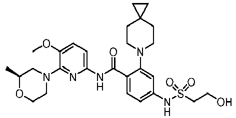 | 560 |
| **260** | 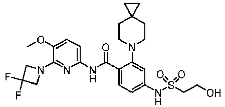 | 552 | **261** | 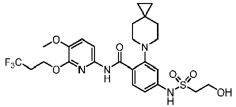 | 573 |
| **262** | 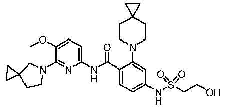 | 556 | **263** | 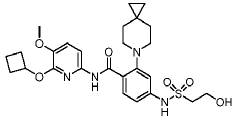 | 531 |
| **264** | 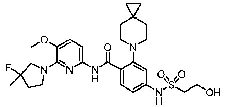 | 562 | **265** | 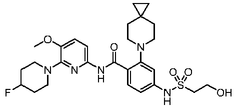 | 562 |
| **266** | 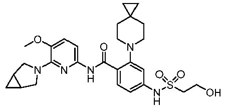 | 542 | **267** | 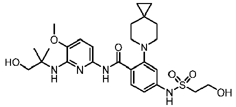 | 548 |
| **268** | 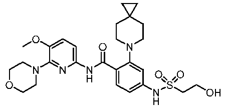 | 546 | **269** | 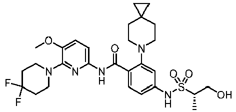 | 594 |
| **270** | 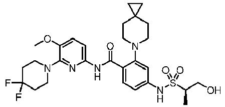 | 594 | **271** | 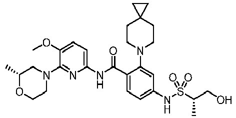 | 574 |
| **272** | 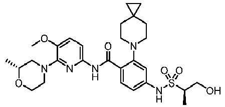 | 574 | **273** | 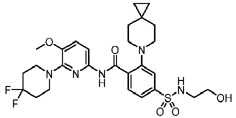 | 580 |
| **274** | 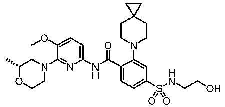 | 560 | **275** | 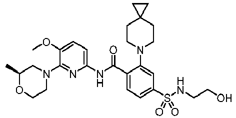 | 560 |
| **276** | 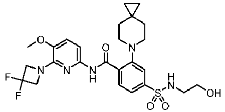 | 552 | **277** | 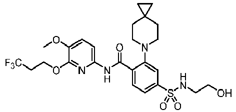 | 572 |
| **278** | 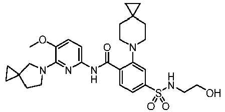 | 556 | **279** | 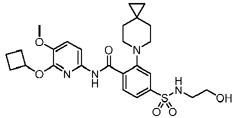 | 531 |
| **280** | 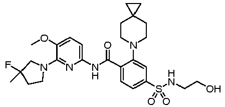 | 562 | **281** | 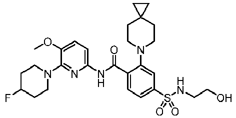 | 562 |
| **282** | 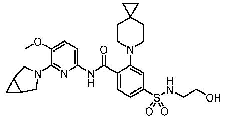 | 542 | **283** | 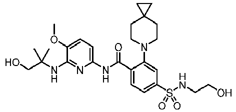 | 548 |
| **284** | 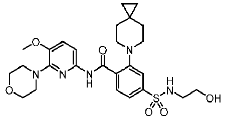 | 546 | **285** | 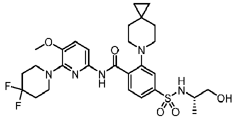 | 594 |
| **286** | 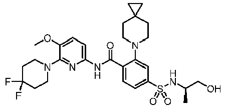 | 594 | **287** | 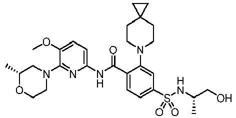 | 574 |
| **288** | 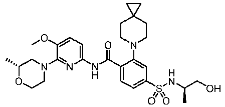 | 574 | **289** | 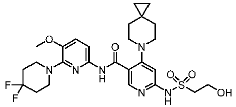 | 581 |
| **290** | 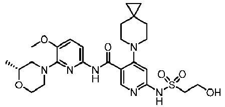 | 561 | **291** | 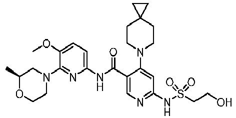 | 561 |
| **292** | 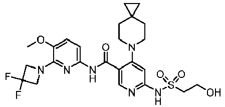 | 553 | **293** | 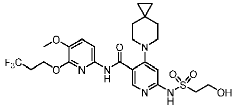 | 574 |
| **294** | 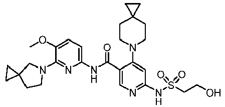 | 557 | **295** | 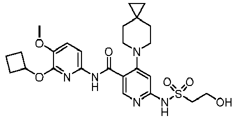 | 532 |
| **296** | 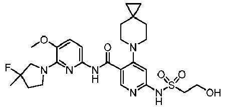 | 563 | **297** | 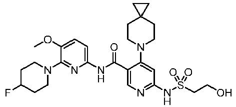 | 563 |
| **298** | 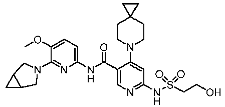 | 543 | **299** | 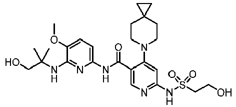 | 549 |
| **300** | 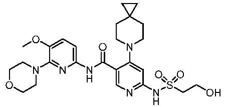 | 547 | **301** | 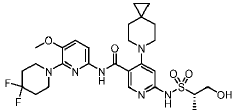 | 595 |
| **302** | 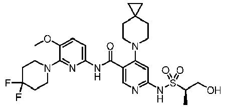 | 595 | **303** | 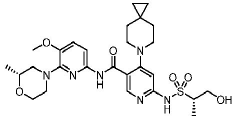 | 575 |
| **304** | 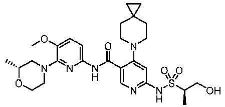 | 575 | **305** | 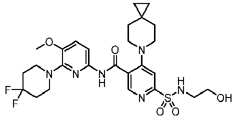 | 581 |
| **306** | 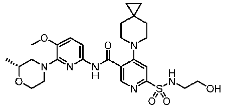 | 561 | **307** | 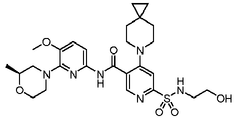 | 561 |
| **308** | 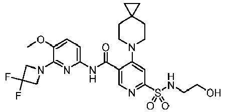 | 553 | **309** | 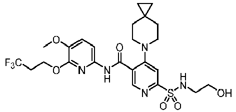 | 574 |
| **310** | 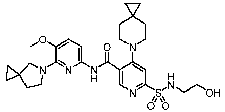 | 557 | **311** | 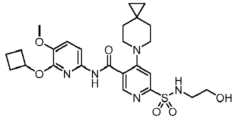 | 532 |
| **312** | 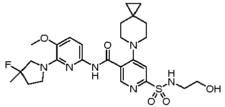 | 563 | **313** | 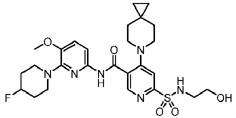 | 563 |
| **314** | 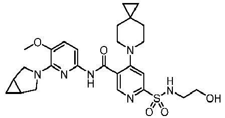 | 543 | **315** | 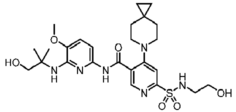 | 549 |
| **316** | 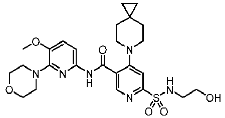 | 547 | **317** | 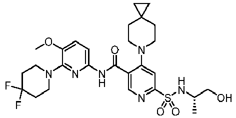 | 595 |
| **318** | 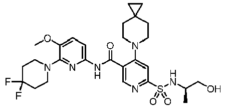 | 595 | **319** | 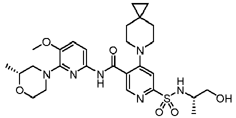 | 575 |
| **320** | 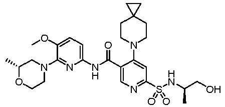 | 575 | **321** | 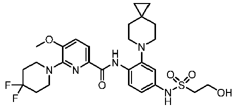 | 580 |
| **322** | 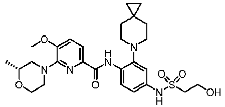 | 560 | **323** | 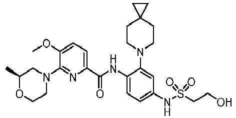 | 560 |
| **324** | 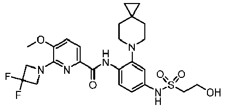 | 552 | **325** | 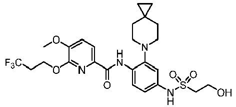 | 573 |
| **326** | 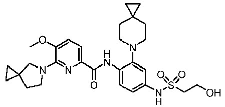 | 556 | **327** | 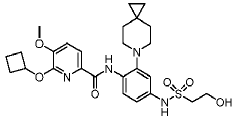 | 531 |
| **328** | 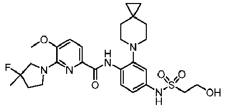 | 562 | **329** | 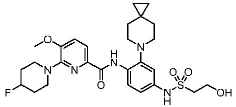 | 562 |
| **330** | 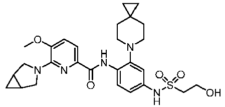 | 542 | **331** | 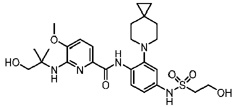 | 548 |
| **332** | 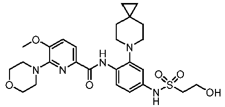 | 546 | **333** | 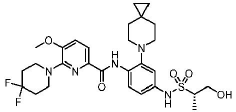 | 594 |
| **334** | 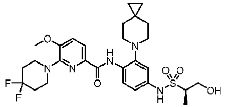 | 594 | **335** | 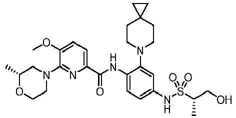 | 574 |
| **336** | 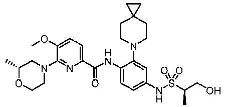 | 574 | **337** | 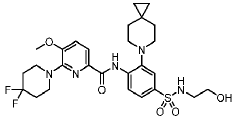 | 580 |
| **338** | 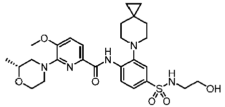 | 560 | **339** | 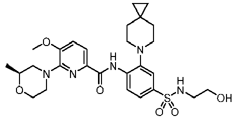 | 560 |
| **340** | 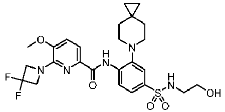 | 552 | **341** | 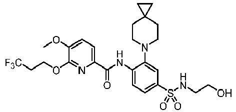 | 573 |
| **342** | 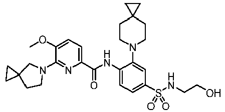 | 556 | **343** | 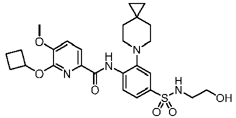 | 531 |
| **344** | 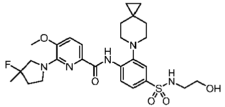 | 562 | **345** | 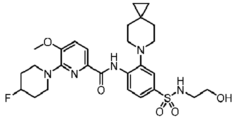 | 562 |
| **346** | 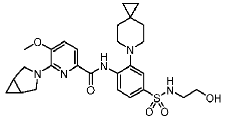 | 542 | **347** | 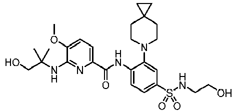 | 548 |
| **348** | 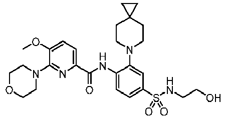 | 546 | **349** | 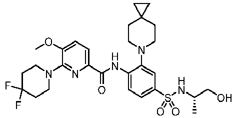 | 594 |
| **350** | 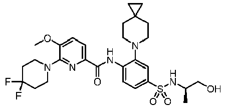 | 594 | **351** | 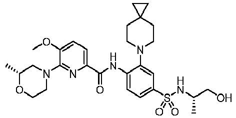 | 574 |
| **352** | 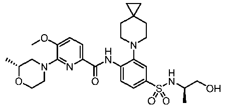 | 574 | **353** | 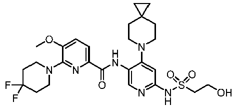 | 581 |
| **354** | 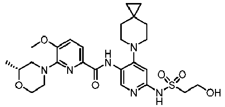 | 561 | **355** | 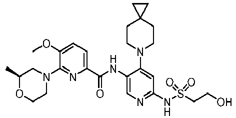 | 561 |
| **356** | 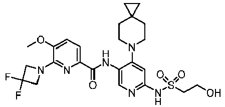 | 553 | **357** | 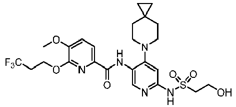 | 574 |
| **358** | 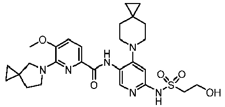 | 557 | **359** | 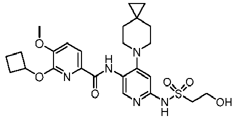 | 532 |
| **360** | 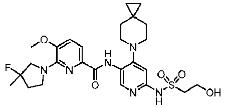 | 563 | **361** | 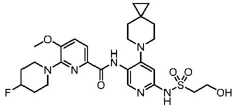 | 563 |
| **362** | 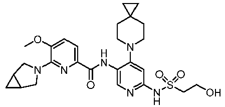 | 543 | **363** | 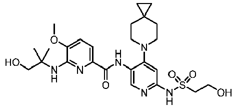 | 549 |
| **364** | 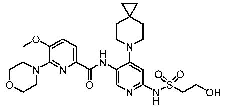 | 547 | **365** | 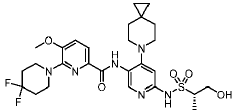 | 595 |
| **366** | 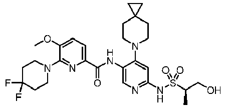 | 595 | **367** | 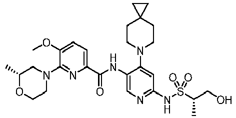 | 575 |
| **368** | 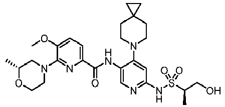 | 575 | **369** | 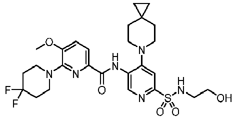 | 581 |
| **370** | 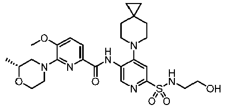 | 561 | **371** | 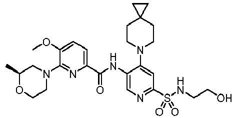 | 561 |
| **372** | 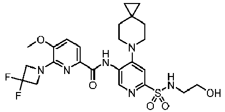 | 553 | **373** | 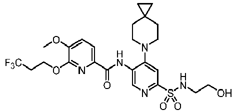 | 574 |
| **374** | 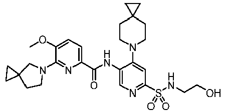 | 557 | **375** | 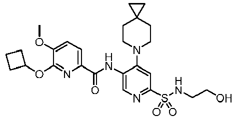 | 532 |
| **376** | 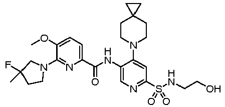 | 563 | **377** | 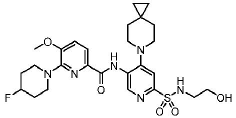 | 563 |
| **378** | 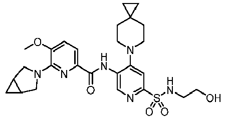 | 543 | **379** | 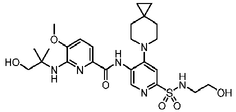 | 549 |
| **380** | 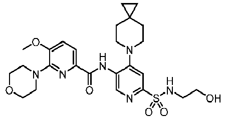 | 547 | **381** | 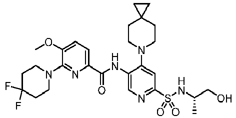 | 595 |
| **382** | 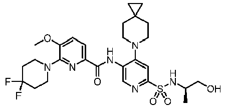 | 595 | **383** | 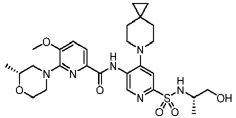 | 575 |
| **384** | 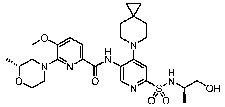 | 575 | **385** | 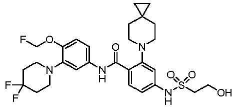 | 597 |
| **386** | 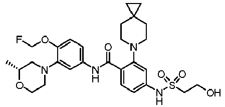 | 577 | **387** | 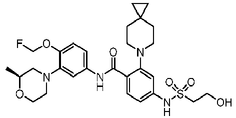 | 577 |
| **388** | 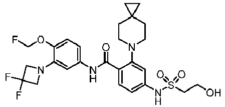 | 569 | **389** | 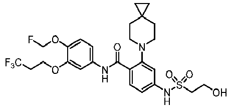 | 590 |
| **390** | 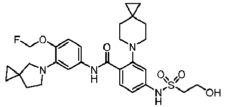 | 573 | **391** | 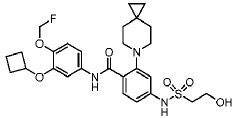 | 548 |
| **392** | 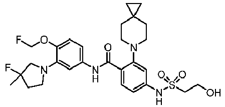 | 579 | **393** | 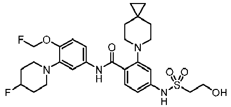 | 579 |
| **394** | 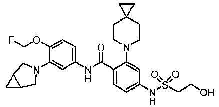 | 559 | **395** | 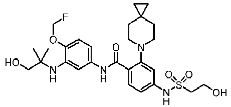 | 565 |
| **396** | 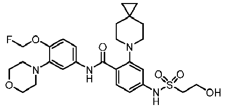 | 563 | **397** | 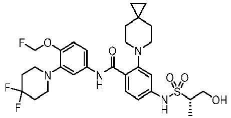 | 611 |
| **398** | 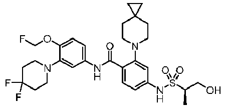 | 611 | **399** | 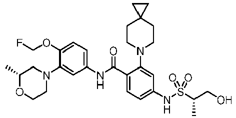 | 591 |
| **400** | 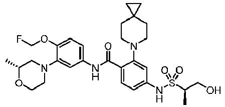 | 591 | **401** | 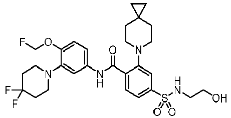 | 597 |
| **402** | 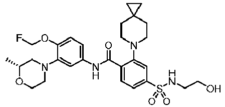 | 577 | **403** | 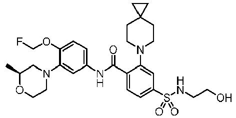 | 577 |
| **404** | 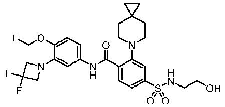 | 569 | **405** | 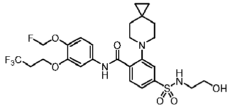 | 590 |
| **406** | 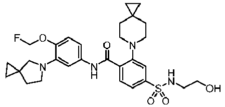 | 573 | **407** | 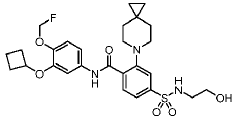 | 548 |
| **408** | 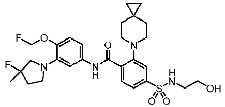 | 579 | **409** | 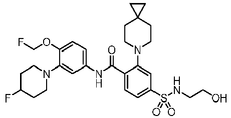 | 579 |
| **410** | 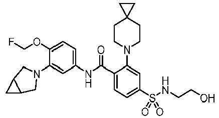 | 559 | **411** | 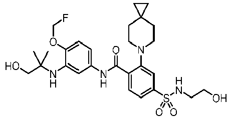 | 565 |
| **412** | 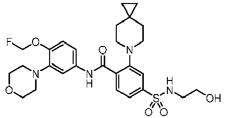 | 563 | **413** | 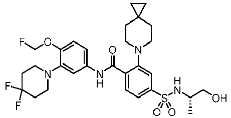 | 611 |
| **414** | 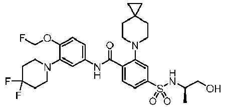 | 611 | **415** | 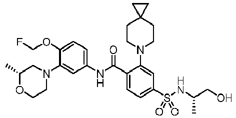 | 591 |
| **416** | 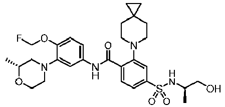 | 591 | **417** | 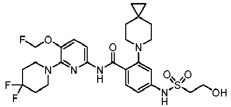 | 598 |
| **418** | 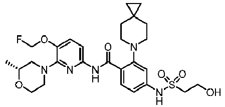 | 578 | **419** | 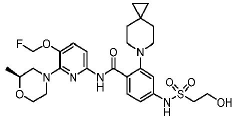 | 578 |
| **420** | 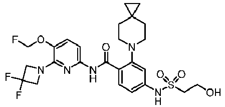 | 570 | **421** | 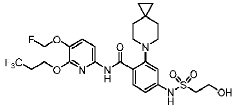 | 591 |
| **422** | 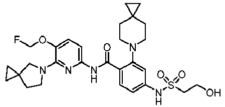 | 574 | **423** | 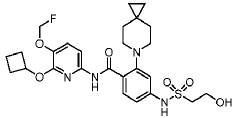 | 549 |
| **424** | 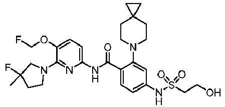 | 580 | **425** | 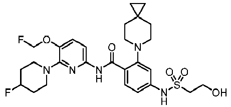 | 580 |
| **426** | 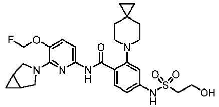 | 560 | **427** | 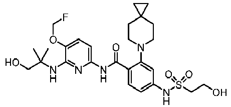 | 566 |
| **428** | 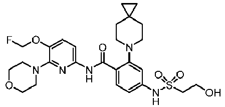 | 564 | **429** | 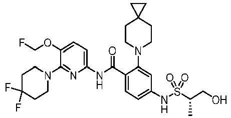 | 612 |
| **430** | 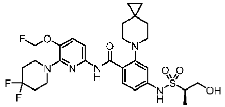 | 612 | **431** | 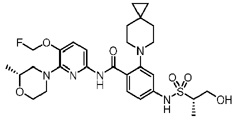 | 592 |
| **432** | 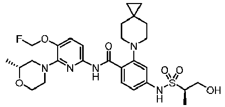 | 592 | **433** | 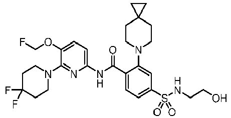 | 598 |
| **434** | 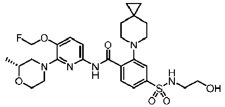 | 578 | **435** | 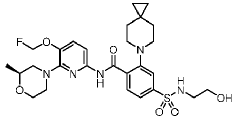 | 578 |
| **436** | 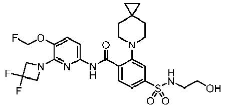 | 570 | **437** | 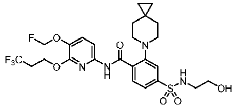 | 591 |
| **438** | 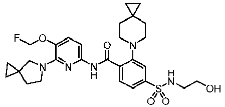 | 574 | **439** | 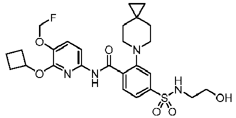 | 549 |
| **440** | 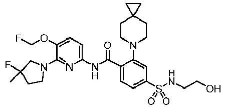 | 580 | **441** | 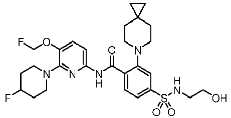 | 580 |
| **442** | 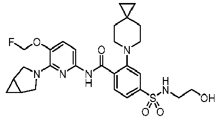 | 560 | **443** | 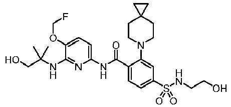 | 566 |
| **444** | 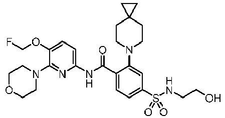 | 564 | **445** | 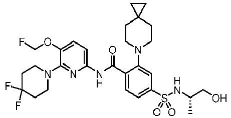 | 612 |
| **446** | 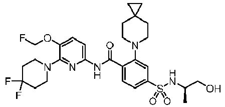 | 612 | **447** | 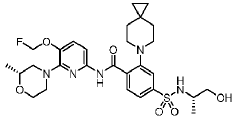 | 592 |
| **448** | 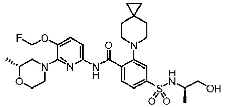 | 592 | **449** | 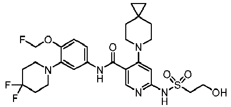 | 598 |
| **450** | 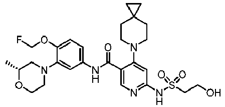 | 578 | **451** | 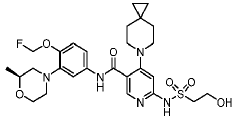 | 578 |
| **452** | 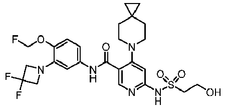 | 570 | **453** | 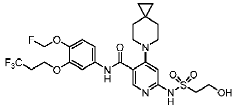 | 591 |
| **454** | 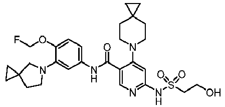 | 574 | **455** | 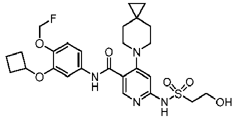 | 549 |
| **456** | 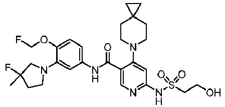 | 580 | **457** | 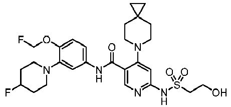 | 580 |
| **458** | 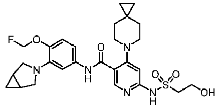 | 560 | **459** | 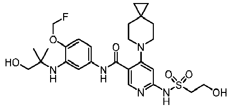 | 566 |
| **460** | 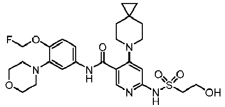 | 564 | **461** | 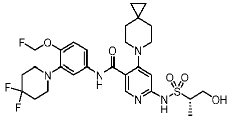 | 612 |
| **462** | 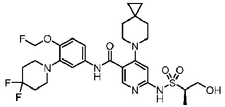 | 612 | **463** | 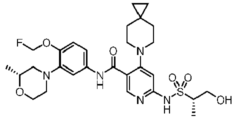 | 592 |
| **464** | 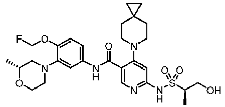 | 592 | **465** | 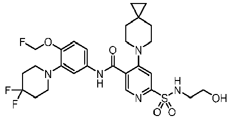 | 598 |
| **466** | 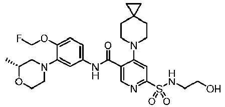 | 578 | **467** | 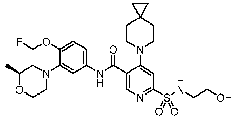 | 578 |
| **468** | 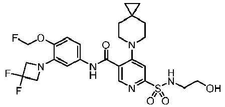 | 570 | **469** | 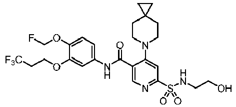 | 591 |
| **470** | 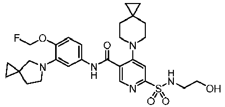 | 574 | **471** | 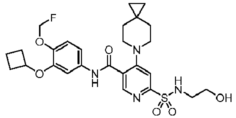 | 549 |
| **472** | 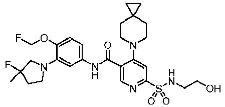 | 580 | **473** | 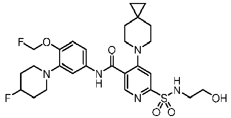 | 580 |
| **474** | 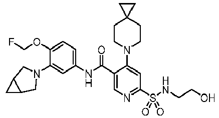 | 560 | **475** | 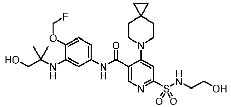 | 566 |
| **476** | 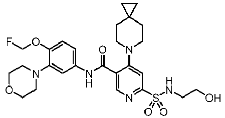 | 564 | **477** | 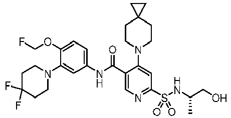 | 612 |
| **478** | 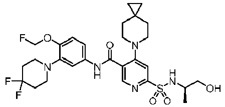 | 612 | **479** | 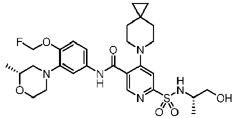 | 592 |
| **480** | 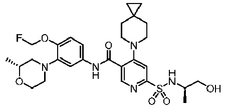 | 592 | **481** | 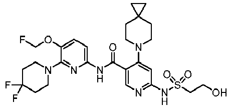 | 599 |
| **482** | 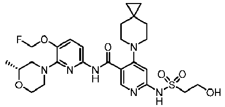 | 579 | **483** | 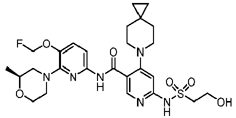 | 579 |
| **484** | 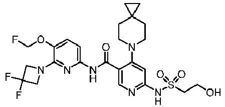 | 571 | **485** | 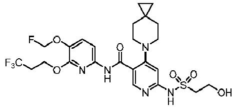 | 592 |
| **486** | 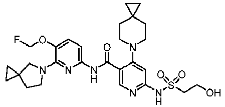 | 575 | **487** | 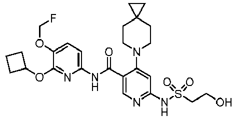 | 550 |
| **488** | 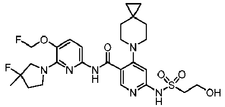 | 581 | **489** | 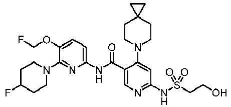 | 581 |
| **490** | 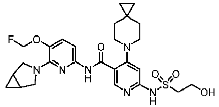 | 561 | **491** | 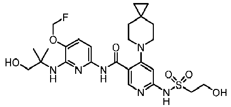 | 567 |
| **492** | 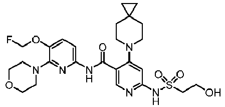 | 565 | **493** | 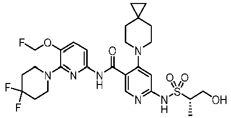 | 613 |
| **494** | 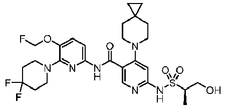 | 613 | **495** | 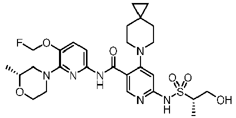 | 593 |
| **496** | 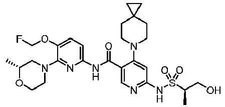 | 593 | **497** | 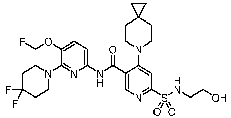 | 599 |
| **498** | 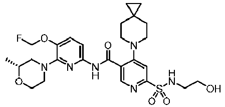 | 579 | **499** | 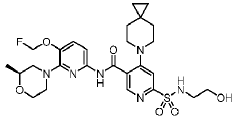 | 579 |
| **500** | 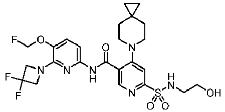 | 571 | **501** | 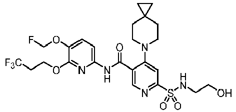 | 592 |
| **502** | 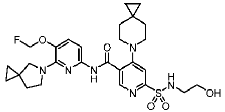 | 575 | **503** | 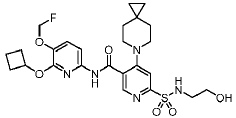 | 550 |
| **504** | 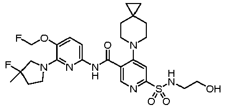 | 581 | **505** | 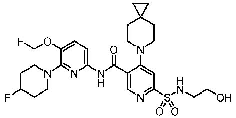 | 581 |
| **506** | 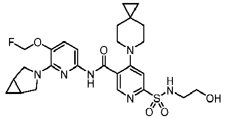 | 561 | **507** | 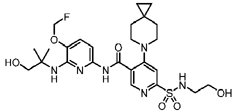 | 567 |
| **508** | 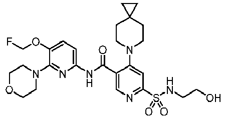 | 565 | **509** | 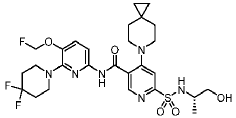 | 613 |
| **510** | 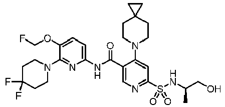 | 613 | **511** | 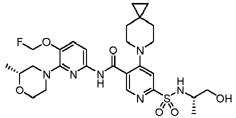 | 593 |
| **512** | 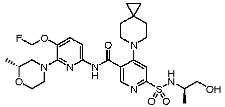 | 593 | **513** | 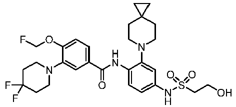 | 597 |
| **514** | 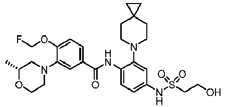 | 577 | **515** | 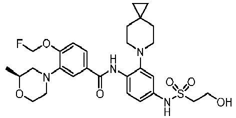 | 577 |
| **516** | 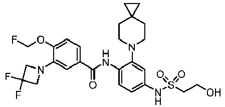 | 569 | **517** | 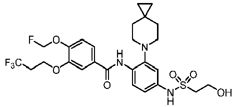 | 590 |
| **518** | 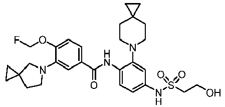 | 573 | **519** | 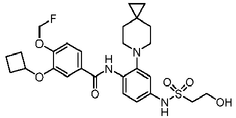 | 548 |
| **520** | 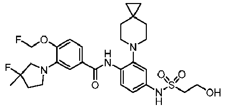 | 579 | **521** | 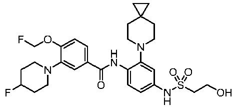 | 579 |
| **522** | 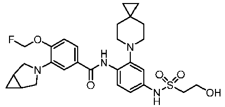 | 559 | **523** | 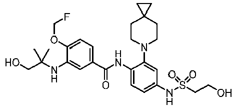 | 565 |
| **524** | 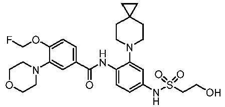 | 563 | **525** | 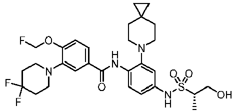 | 611 |
| **526** | 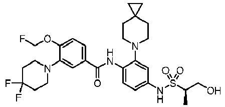 | 611 | **527** | 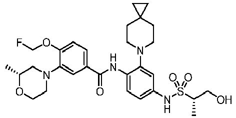 | 591 |
| **528** | 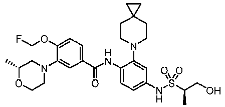 | 591 | **529** | 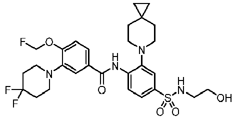 | 597 |
| **530** | 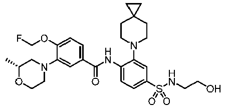 | 577 | **531** | 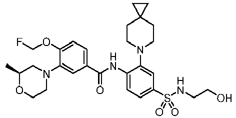 | 577 |
| **532** | 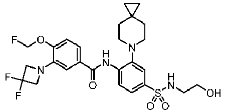 | 569 | **533** | 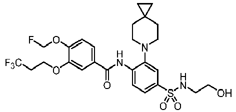 | 590 |
| **534** | 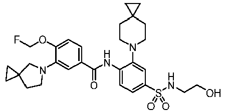 | 573 | **535** | 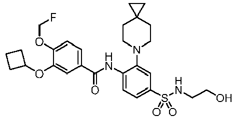 | 548 |
| **536** | 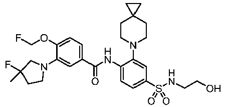 | 579 | **537** | 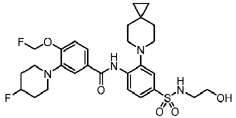 | 579 |
| **538** | 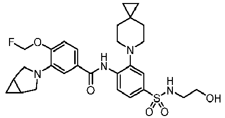 | 559 | **539** | 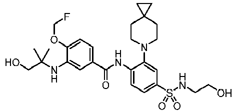 | 565 |
| **540** | 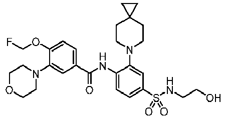 | 563 | **541** | 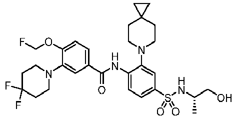 | 611 |
| **542** | 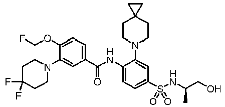 | 611 | **543** | 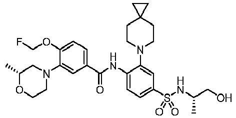 | 591 |
| **544** | 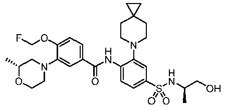 | 591 | **545** | 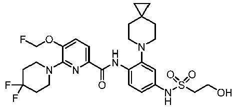 | 598 |
| **546** | 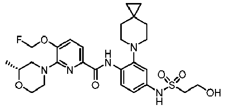 | 578 | **547** | 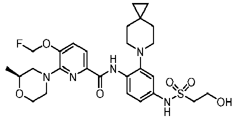 | 578 |
| **548** | 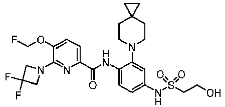 | 570 | **549** | 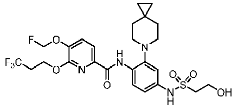 | 591 |
| **550** | 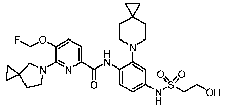 | 574 | **551** | 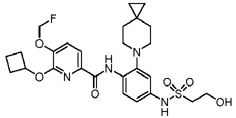 | 549 |
| **552** | 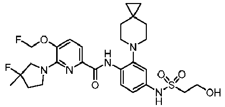 | 580 | **553** | 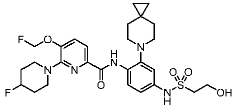 | 580 |
| **554** | 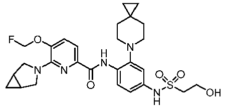 | 560 | **555** | 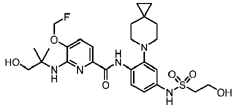 | 566 |
| **556** | 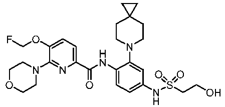 | 564 | **557** | 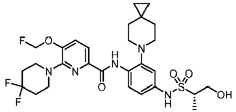 | 612 |
| **558** | 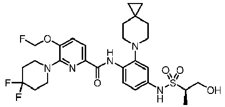 | 612 | **559** | 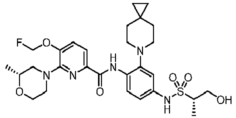 | 592 |
| **560** | 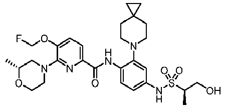 | 592 | **561** | 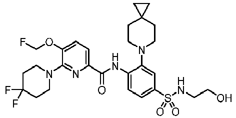 | 598 |
| **562** | 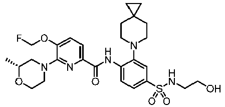 | 578 | **563** | 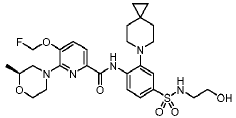 | 578 |
| **564** | 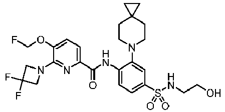 | 570 | **565** | 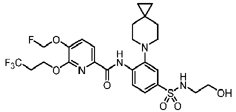 | 591 |
| **566** | 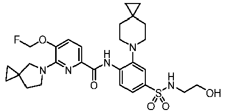 | 574 | **567** | 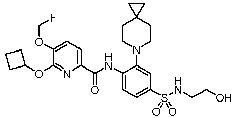 | 549 |
| **568** | 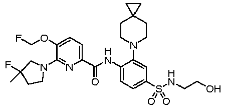 | 580 | **569** | 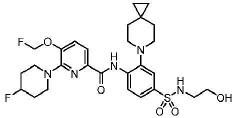 | 580 |
| **570** | 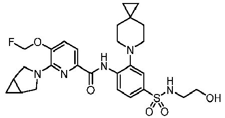 | 560 | **571** | 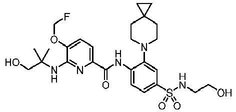 | 566 |
| **572** | 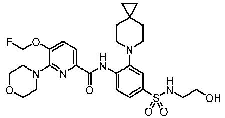 | 564 | **573** | 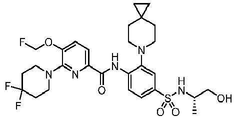 | 612 |
| **574** | 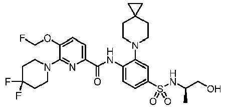 | 612 | **575** | 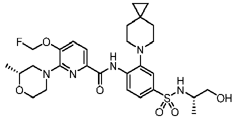 | 592 |
| **576** | 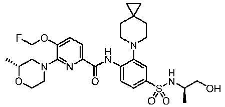 | 592 | **577** | 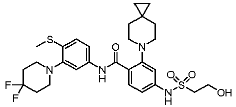 | 595 |
| **578** | 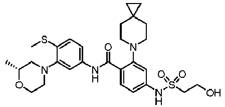 | 575 | **579** | 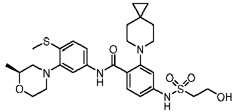 | 575 |
| **580** | 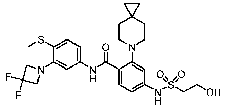 | 567 | **581** | 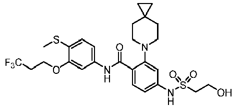 | 588 |
| **582** | 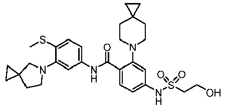 | 571 | **583** | 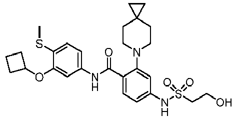 | 546 |
| **584** | 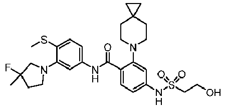 | 577 | **585** | 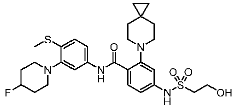 | 577 |
| **586** | 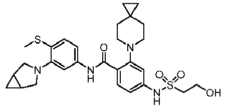 | 557 | **587** | 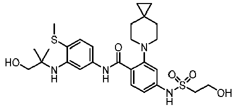 | 563 |
| **588** | 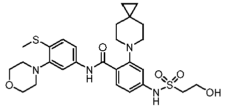 | 561 | **589** | 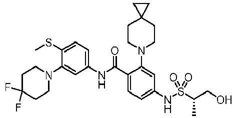 | 609 |
| **590** | 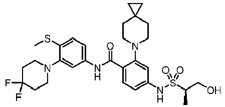 | 609 | **591** | 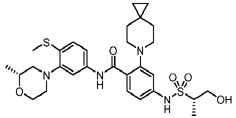 | 589 |
| **592** | 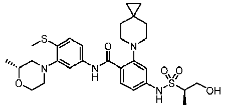 | 589 | **593** | 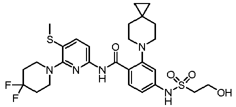 | 596 |
| **594** | 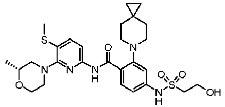 | 576 | **595** | 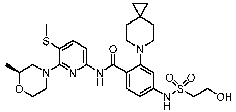 | 576 |
| **596** | 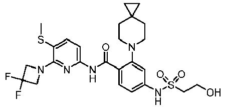 | 568 | **597** | 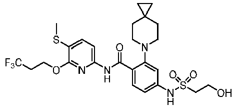 | 589 |
| **598** | 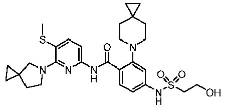 | 572 | **599** | 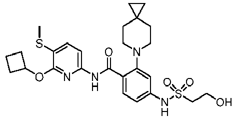 | 547 |
| **600** | 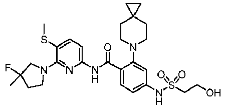 | 578 | **601** | 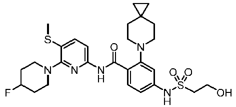 | 578 |
| **602** | 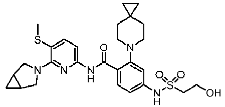 | 558 | **603** | 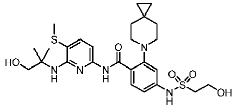 | 564 |
| **604** | 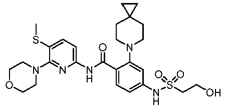 | 562 | **605** | 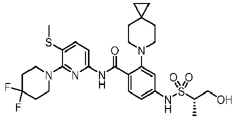 | 610 |
| **606** | 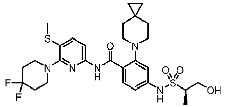 | 610 | **607** | 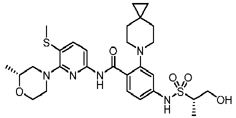 | 590 |
| **608** | 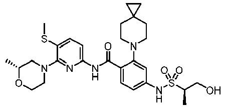 | 590 | **609** | 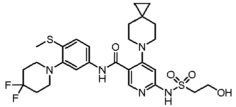 | 596 |
| **610** | 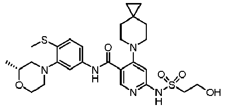 | 576 | **611** | 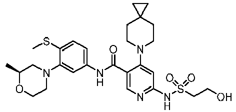 | 576 |
| **612** | 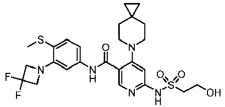 | 568 | **613** | 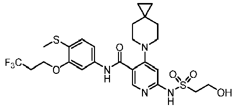 | 589 |
| **614** | 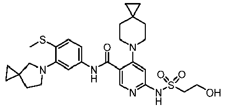 | 572 | **615** | 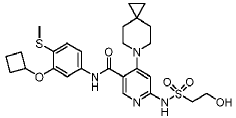 | 547 |
| **616** | 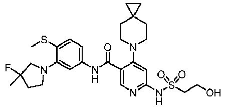 | 578 | **617** | 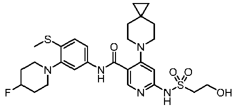 | 578 |
| **618** | 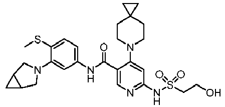 | 558 | **619** | 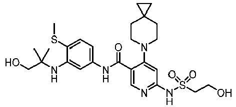 | 564 |
| **620** | 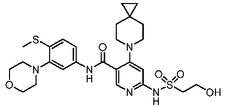 | 562 | **621** | 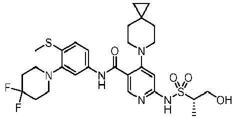 | 610 |
| **622** | 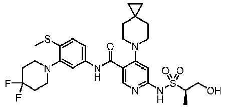 | 610 | **623** | 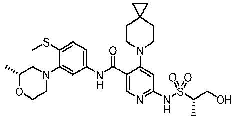 | 590 |
| **624** | 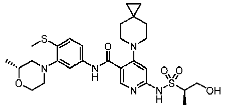 | 590 | **625** | 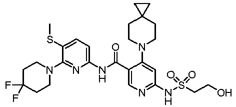 | 597 |
| **626** | 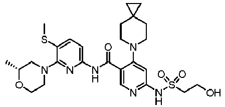 | 577 | **627** | 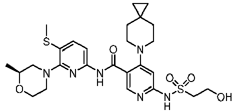 | 577 |
| **628** | 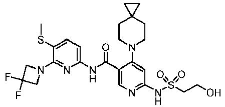 | 569 | **629** | 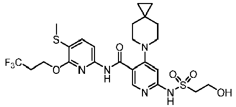 | 590 |
| **630** | 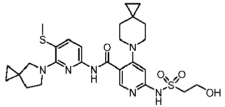 | 573 | **631** | 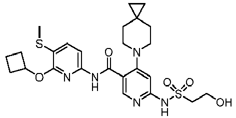 | 548 |
| **632** | 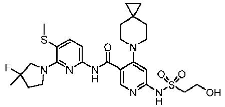 | 579 | **633** | 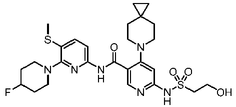 | 579 |
| **634** | 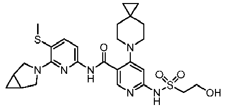 | 559 | **635** | 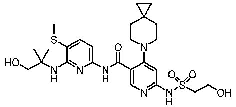 | 565 |
| **636** | 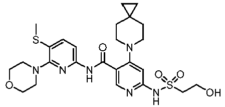 | 563 | **637** | 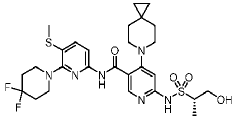 | 611 |
| **638** | 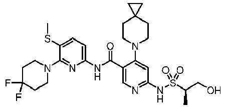 | 611 | **639** | 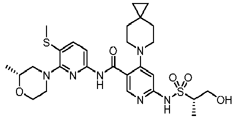 | 591 |
| **640** | 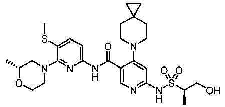 | 591 | **641** | 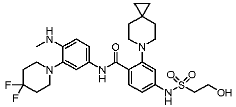 | 578 |
| **642** | 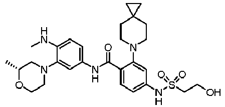 | 558 | **643** | 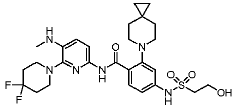 | 579 |
| **644** | 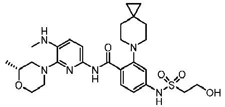 | 559 | **645** | 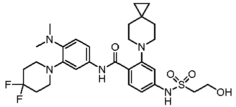 | 592 |
| **646** | 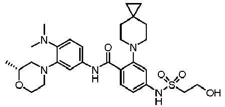 | 572 | **647** | 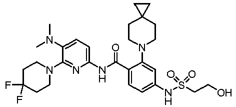 | 593 |
| **648** | 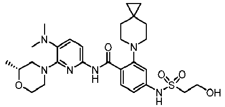 | 573 | **649** | 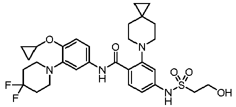 | 605 |
| **650** | 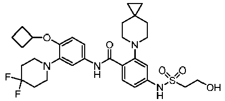 | 619 | **651** | 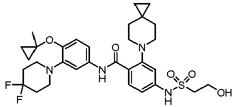 | 619 |
| **652** | 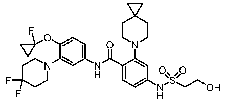 | 623 | **653** | 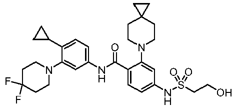 | 589 |
| **654** | 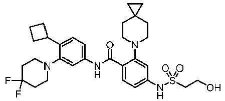 | 603 | **655** | 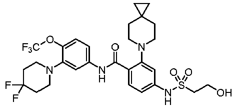 | 633 |
| **656** | 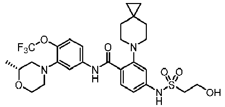 | 613 | **657** | 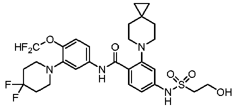 | 615 |
| **658** | 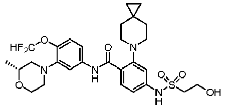 | 595 | **659** | 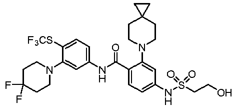 | 649 |
| **660** | 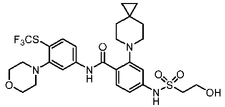 | 615 | **661** | 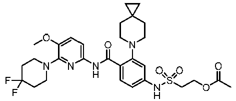 | 622 |
| **662** | 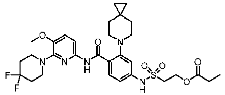 | 636 | **663** | 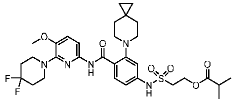 | 650 |
| **664** | 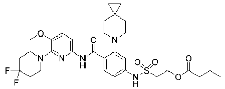 | 650 | **665** | 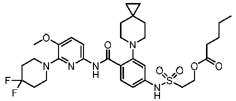 | 664 |
| **666** | 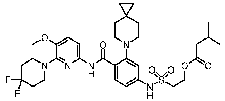 | 664 | **667** | 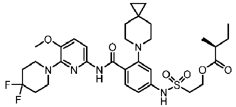 | 664 |
| **668** | 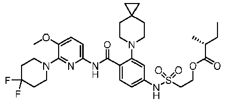 | 664 | **669** | 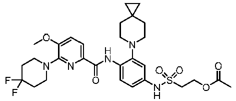 | 622 |
| **670** | 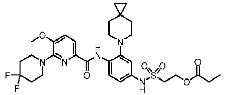 | 636 | **671** | 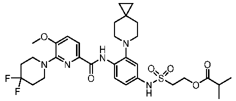 | 650 |
| **672** | 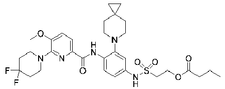 | 650 | **673** | 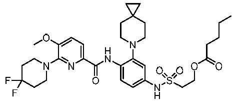 | 664 |
| **674** | 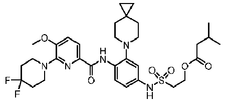 | 664 | **675** | 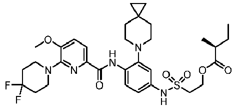 | 664 |
| **676** | 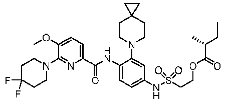 | 664 | **677** | 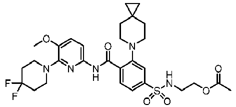 | 622 |
| **678** | 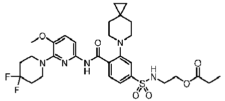 | 636 | **679** | 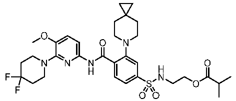 | 650 |
| **680** | 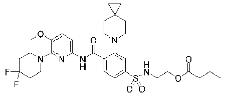 | 650 | **681** | 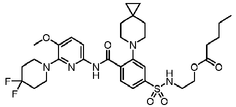 | 664 |
| **682** | 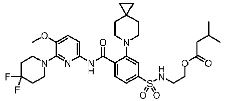 | 664 | **683** | 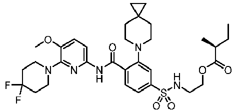 | 664 |
| **684** | 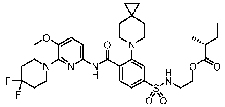 | 664 | **685** | 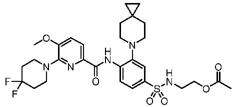 | 622 |
| **686** | 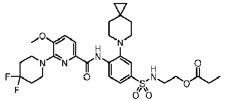 | 636 | **687** | 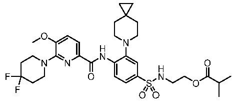 | 650 |
| **688** | 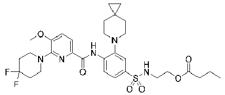 | 650 | **689** | 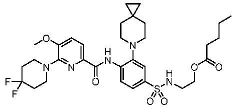 | 664 |
| **690** | 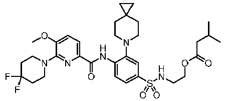 | 664 | **691** | 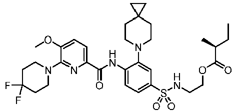 | 664 |
| **692** | 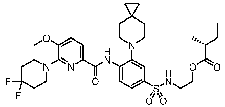 | 664 | **693** | 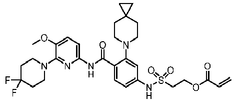 | 634 |
| **694** | 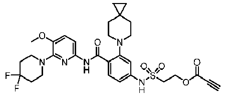 | 632 | **695** | 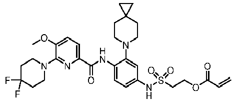 | 633 |
| **696** | 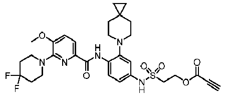 | 632 | **697** | 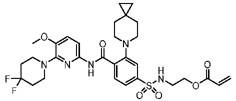 | 634 |
| **698** | 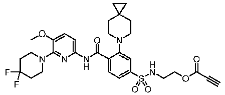 | 632 | **699** | 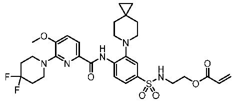 | 634 |
| **700** | 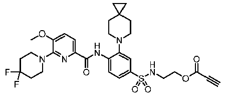 | 632 | **701** | 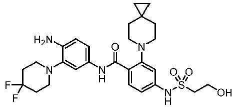 | 564 |
| **702** | 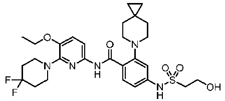 | 594 | **703** | 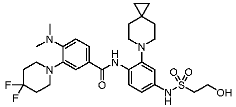 | 592 |
| **704** | 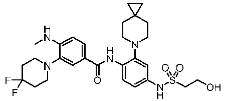 | 578 | **705** | 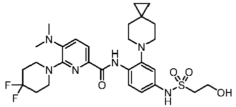 | 593 |
| **706** | 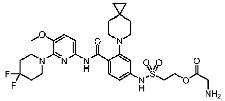 | 637 | **707** | 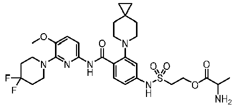 | 651 |
| **708** | 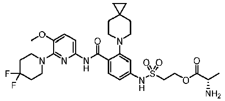 | 651 | **709** | 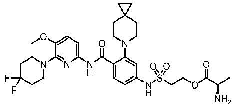 | 651 |
| **710** | 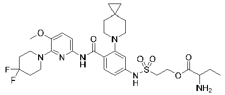 | 665 | **711** | 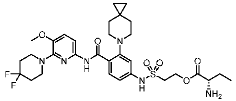 | 665 |
| **712** | 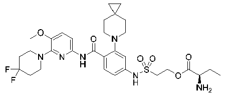 | 665 | **713** | 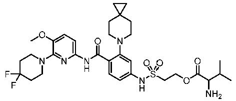 | 679 |
| **715** | 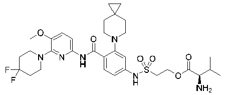 | 679 | **716** | 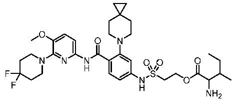 | 693 |
| **717** | 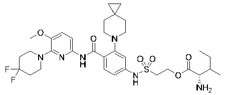 | 693 | **718** | 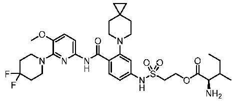 | 693 |
| **719** | 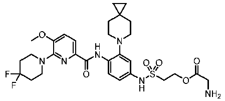 | 637 | **720** | 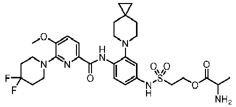 | 651 |
| **721** | 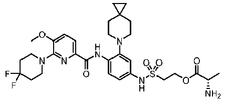 | 651 | **722** | 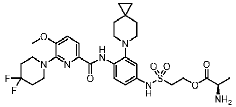 | 651 |
| **723** | 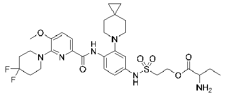 | 665 | **724** | 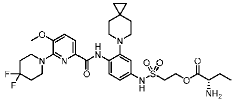 | 665 |
| **725** | 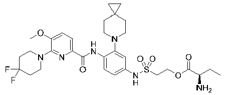 | 665 | **726** | 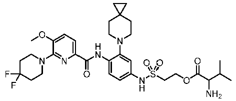 | 679 |
| **728** | 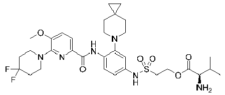 | 679 | **729** | 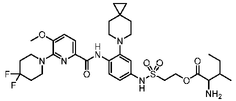 | 693 |
| **730** | 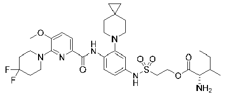 | 693 | **731** | 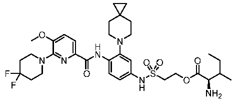 | 693 |
| **732** | 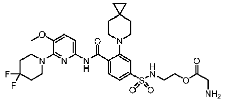 | 637 | **733** | 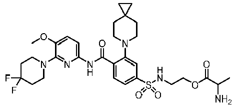 | 651 |
| **734** | 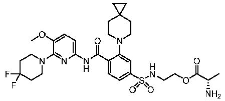 | 651 | **735** | 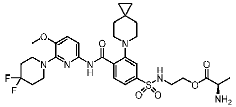 | 651 |
| **736** | 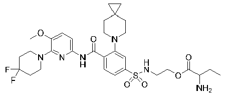 | 665 | **737** | 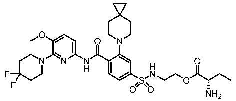 | 665 |
| **738** | 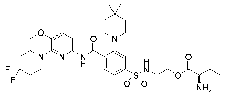 | 665 | **739** | 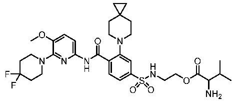 | 679 |
| **741** | 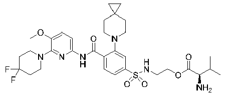 | 679 | **742** | 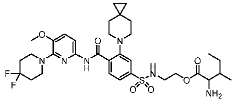 | 693 |
| **743** | 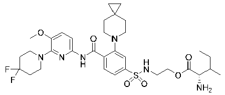 | 693 | **744** | 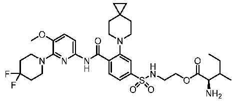 | 693 |
| **745** | 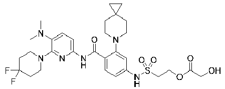 | 651 | **746** | 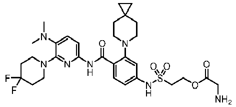 | 650 |
| **747** | 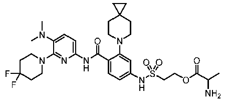 | 664 | **748** | 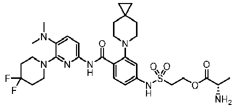 | 664 |
| **749** | 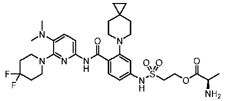 | 664 | **750** | 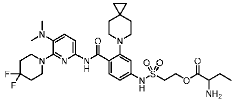 | 678 |
| **751** | 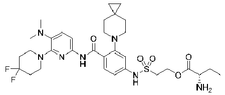 | 678 | **752** | 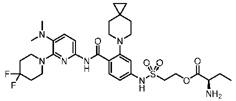 | 678 |
| **753** | 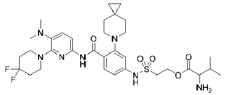 | 692 | **754** | 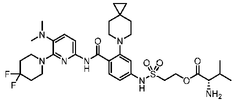 | 692 |
| **755** | 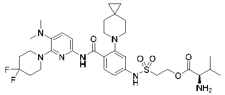 | 692 | **756** | 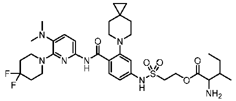 | 706 |
| **757** | 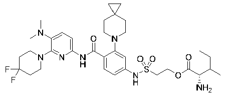 | 706 | **758** | 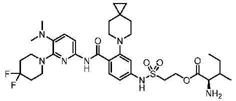 | 706 |
| **759** | 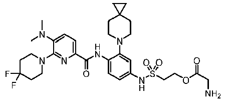 | 650 | **760** | 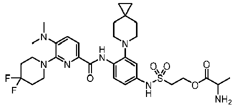 | 664 |
| **761** | 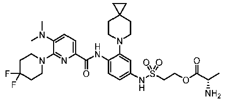 | 664 | **762** | 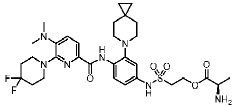 | 664 |
| **763** | 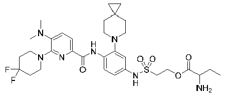 | 678 | **764** | 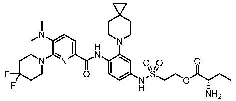 | 678 |
| **765** | 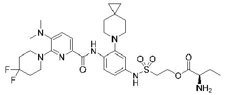 | 678 | **766** | 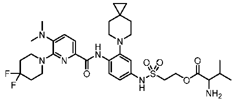 | 692 |
| **767** | 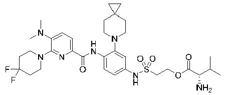 | 692 | **768** | 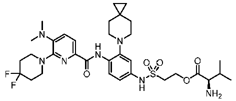 | 692 |
| **769** | 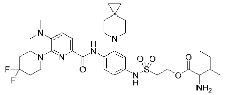 | 706 | **770** | 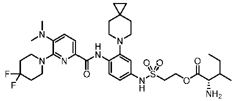 | 706 |
| **771** | 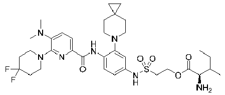 | 706 | **772** | 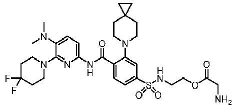 | 650 |
| **773** | 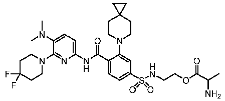 | 664 | **774** | 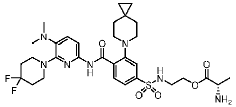 | 664 |
| **775** | 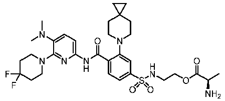 | 664 | **776** | 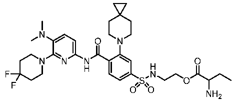 | 678 |
| **777** | 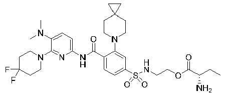 | 678 | **778** | 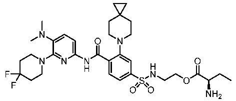 | 678 |
| **779** | 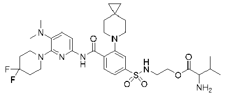 | 692 | **780** | 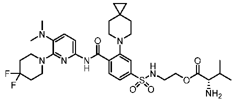 | 692 |
| **781** | 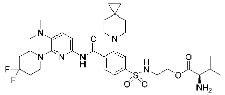 | 692 | **782** | 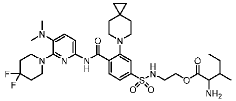 | 706 |
| **783** | 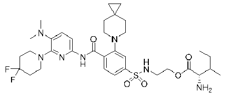 | 706 | **784** | 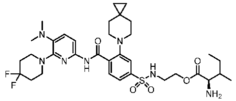 | 706 |
| **785** | 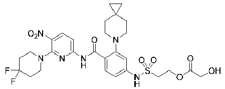 | 653 | **786** | 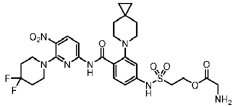 | 652 |
| **787** | 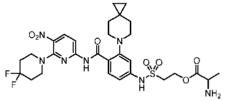 | 666 | **788** | 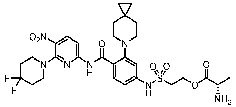 | 666 |
| **789** | 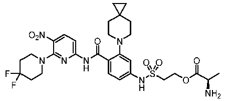 | 666 | **790** | 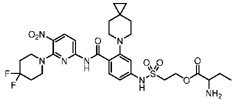 | 680 |
| **791** | 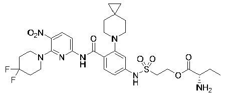 | 680 | **792** | 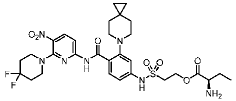 | 680 |
| **793** | 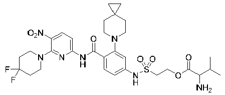 | 694 | **794** | 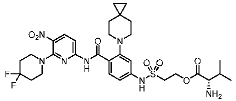 | 694 |
| **795** | 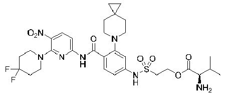 | 694 | **796** | 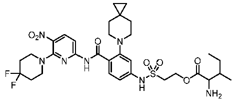 | 708 |
| **797** | 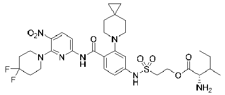 | 708 | **798** | 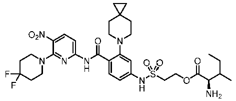 | 708 |
| **799** | 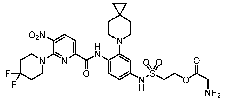 | 652 | **800** | 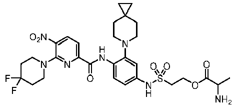 | 666 |
| **801** | 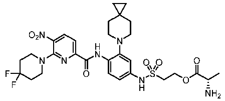 | 666 | **802** | 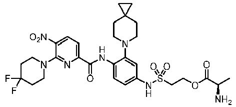 | 666 |
| **803** | 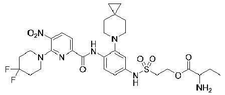 | 680 | **804** | 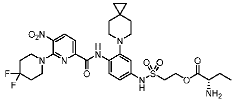 | 680 |
| **805** | 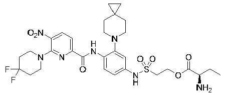 | 680 | **806** | 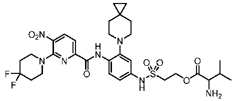 | 694 |
| **807** | 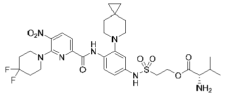 | 694 | **808** | 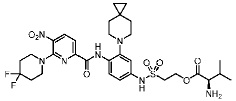 | 694 |
| **809** | 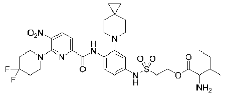 | 708 | **810** | 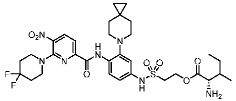 | 708 |
| **811** | 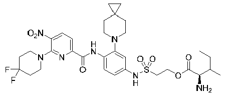 | 708 | **812** | 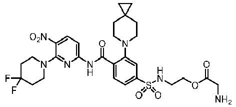 | 652 |
| **813** | 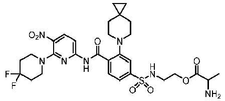 | 666 | **814** | 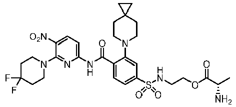 | 666 |
| **815** | 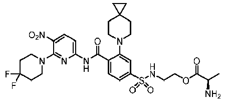 | 666 | **816** | 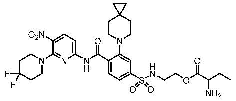 | 680 |
| **817** | 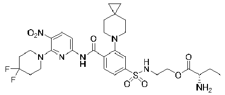 | 680 | **818** | 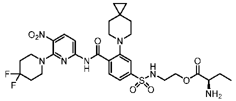 | 680 |
| **819** | 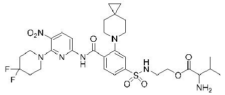 | 694 | **820** | 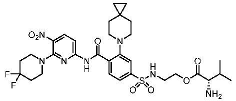 | 694 |
| **821** | 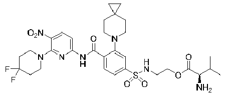 | 694 | **822** | 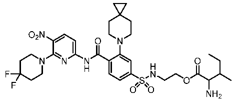 | 708 |
| **823** | 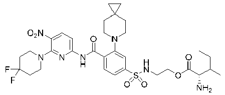 | 708 | **824** | 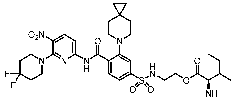 | 708 |
| **825** | 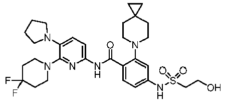 | 619 | **826** | 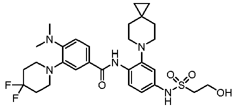 | 592 |
| **827** | 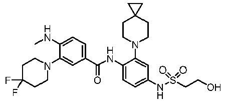 | 578 | **828** | 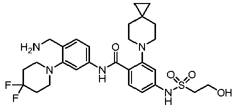 | 578 |
| **829** | 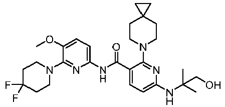 | 545 | **830** | 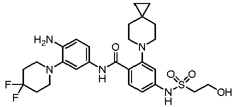 | 564 |
| **831** | 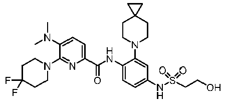 | 593 | **832** | 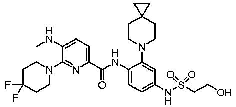 | 579 |
| **833** | 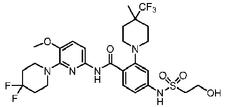 | 636 | **834** | 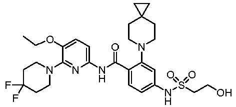 | 594 |
| **835** | 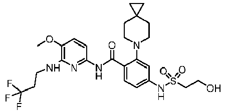 | 572 | **836** | 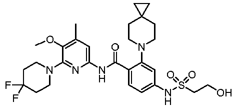 | 594 |
| **837** | 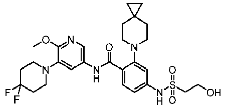 | 580 | | | |

**Table 2. NMR data of some of the compounds in Table 1**

| **Compound** | **NMR** |
|---|---|
| **827** | ¹H NMR (400 MHz, DMSO-d6) δ 9.26 (s, 1H), 8.10 (d, *J* = 8.7 Hz, 1H), 7.62 (d, *J* = 8.5 Hz, 1H), 7.53 (s, 1H), 7.10 (s, 1H), 6.94 (d, *J* = 8.3 Hz, 1H), 6.63 (d, *J* = 8.5 Hz, 1H), 5.86 (d, *J* = 5.8 Hz, 1H), 3.72 (t, *J* = 6.8 Hz, 2H), 3.18 (t, *J* = 6.6 Hz, 2H), 2.92 (s, 4H), 2.81 (d, *J* = 5.3 Hz, 7H), 2.18 (s, 4H), 1.53 (s, 4H), 0.34 (s, 4H). |
| **830** | ¹H NMR (400 MHz, Chloroform-d) δ 12.21 (s, 1H), 8.22 - 8.04 (m, 1H), 7.69 (s, 1H), 7.44 - 7.29 (m, 1H), 7.21 - 6.95 (m, 2H), 6.75 (d, *J* = 8.3 Hz, 1H), 4.11 (s, 2H), 3.31 (s, 2H), 3.06 (s, 8H), 2.14 (t, *J* = 14.6 Hz, 4H), 1.61 (s, 4H), 0.43 (s, 4H). |
| **831** | ¹H NMR (400 MHz, DMSO-d6) δ 10.35 (s, 1H), 9.57 (s, 1H), 8.33 (d, *J* = 8.8 Hz, 1H), 7.73 (d, *J* = 8.1 Hz, 1H), 7.35 (d, *J* = 8.2 Hz, 1H), 7.15 (d, *J* = 2.4 Hz, 1H), 6.98 (dd, *J* = 8.8, 2.4 Hz, 1H), 4.91 (s, 1H), 3.71 (t, *J* = 6.8 Hz, 2H), 3.53 (t, *J* = 5.6 Hz, 4H), 3.19 (t, *J* = 6.7 Hz, 2H), 2.85 (s, 6H), 2.78 (d, *J* = 5.5 Hz, 4H), 2.26 - 2.12 (m, 4H), 1.53 (s, 4H), 0.34 (s, 4H). |
| **832** | ¹H NMR (400 MHz, DMSO-d6) δ 10.33 (s, 1H), 8.33 (d, *J* = 8.8 Hz, 1H), 7.79 (d, *J* = 8.2 Hz, 1H), 7.12 (d, *J* = 2.4 Hz, 1H), 6.96 (d, *J* = 8.9 Hz, 2H), 5.94 (d, *J* = 5.4 Hz, 1H), 3.71 (t, *J* = 6.7 Hz, 2H), 3.18 (dd, *J* = 12.3, 6.2 Hz, 6H), 2.80 (d, *J* = 5.4 Hz, 7H), 2.23 (s, 4H), 1.57 (s, 4H), 0.33 (s, 4H). |
| **833** | ¹H NMR (400 MHz, Chloroform-d) δ 11.25 (s, 1H), 8.16 (d, *J* = 8.5 Hz, 1H), 7.92 (d, *J* = 8.6 Hz, 1H), 7.52 (s, 1H), 7.14 (d, *J* = 8.6 Hz, 1H), 7.01 (d, *J* = 10.2 Hz, 1H), 6.68 (s, 1H), 4.15 (s, 2H), 3.87 (s, 3H), 3.49 (d, *J* = 5.6 Hz, 4H), 3.33 - 3.30 (m, 2H), 3.21 (d, *J* = 12.3 Hz, 2H), 2.99 (t, *J* = 11.9 Hz, 2H), 1.69 (d, *J* = 14.0 Hz, 4H), 1.43 - 1.38 (m, 4H), 1.27 (s, 3H). |
| **834** | ¹H NMR (400 MHz, DMSO-d6) δ 12.89 (s, 1H), 7.95 (d, *J* = 8.7 Hz, 1H), 7.77 (d, *J* = 8.5 Hz, 1H), 7.31 (d, *J* = 8.5 Hz, 1H), 7.10 (d, *J* = 2.2 Hz, 1H), 6.95 (dd, *J* = 8.8, 2.1 Hz, 1H), 4.02 (q, *J* = 6.9 Hz, 2H), 3.72 (t, *J* = 6.5 Hz, 2H), 3.53 (t, *J* = 5.7 Hz, 4H), 3.18 (d, *J* = 6.5 Hz, 2H), 2.93 (s, 4H), 2.10 - 2.02 (m, 4H), 1.70 (s, 4H), 1.33 (t, *J* = 6.9 Hz, 3H), 0.36 (s, 4H) |
| **835** | ¹H NMR (400 MHz, DMSO-d6) δ 12.35 (s, 1H), 7.96 (d, *J* = 8.6 Hz, 1H), 7.46 (d, *J* = 8.3 Hz, 1H), 7.15 (d, *J* = 2.1 Hz, 1H), 7.05 (d, *J* = 8.4 Hz, 1H), 7.01 (dd, *J* = 8.6, 2.1 Hz, 1H), 6.25 (t, *J* = 5.8 Hz, 1H), 3.76 (s, 3H), 3.72 (t, *J* = 6.5 Hz, 2H), 3.62 - 3.56 (m, 2H), 2.94 (t, *J* = 5.2 Hz, 4H), 2.58 (qt, *J* = 11.7, 7.2 Hz, 2H), 1.88 - 1.37 (s, 4H), 0.33 (s, 4H). |
| **836** | ¹H NMR (400 MHz, DMSO-d6) δ 12.87 (s, 1H), 8.04 (d, *J* = 8.6 Hz, 1H), 7.71 (s, 1H), 7.23 (d, *J* = 2.2 Hz, 1H), 7.09 (dd, *J* = 8.7, 2.1 Hz, 1H), 3.74 (t, *J* = 6.5 Hz, 2H), 3.65 (s, 3H), 3.54 (t, *J* = 5.5 Hz, 4H), 2.94 (t, *J* = 5.2 Hz, 4H), 2.20 (s, 3H), 2.08 (dq, *J* = 13.4, 6.6, 5.3 Hz, 4H), 1.71 (s, 4H), 0.36 (s, 4H). |
| **837** | ¹H NMR (400 MHz, DMSO-d6) δ 11.13 (s, 1H), 8.49 (s, 1H), 7.91 (d, *J* = 8.5 Hz, 1H), 7.17 (d, *J* = 2.1 Hz, 1H), 7.03 (dd, *J* = 8.5, 2.0 Hz, 1H), 6.48 (s, 1H), 3.80 (s, 3H), 3.74 (t, *J* = 6.5 Hz, 2H), 3.34 (d, *J* = 6.6 Hz, 2H), 3.12 (d, *J* = 5.8 Hz, 4H), 2.95 (t, *J* = 5.1 Hz, 4H), 1.98 (d, *J* = 12.5 Hz, 4H), 1.43 (s, 4H), 0.31 (s, 4H). |

### Biological Example 1. In Vitro Assay of the Compounds of the Present Invention for Inhibiting Enzymatic Activity of KIF18A

KIF18A enzyme assay: The enzymatic activity of KIF18A after treatment with the compounds was measured by an assay of microtubule-stimulated ATPase activity. ADP generated from the ATPase reaction was measured in this assay. The compounds were serially diluted 2-fold in DMSO over a range of 22 concentration points. Recombinant human KIF18A (1-467His-tagged) protein was expressed using a baculovirus system.

Concentrations of KIF18A protein, microtubules, and ATP in the reaction were optimized for a standardized homogenous enzyme assay using an ADP-Glo kinase/ATPase assay kit. A reaction buffer [(15 mM Tris, pH 7.5), 10 mM MgCl₂, 0.01% PluronicF-68, 1 µM paclitaxel, and 30 µg/mL pig microtubules] was prepared. The compound and KIF18A protein (30 nM) were added to the prepared reaction buffer, and the reaction mixture was incubated at room temperature for 15 min, followed by addition of ATP (Km, 75 µM). The resulting reaction mixture was incubated at room temperature for another 15 min. 5 µL of ADP-Glo reagent and 2.5 µL of the reaction mixture were mixed and the resulting mixture was incubated at room temperature for 40 min. 10 µL of ADP-Glo detection reagent was added and the mixture was incubated at room temperature for 40 min. Luminescence was read using a microplate reader and compared with that of the DMSO group, and then the inhibition percentages and IC₅₀ values of the compounds were calculated. The results are shown in Table 3 below.

**Table 3. Inhibitory activity of the compounds of the present invention against KIF18A (IC₅₀, nM)**

| **Compound** | **IC₅₀** | **Compound** | **IC₅₀** | **Compound** | **IC₅₀** | **Compound** | **IC₅₀** | **Compound** | **IC₅₀** |
|---|---|---|---|---|---|---|---|---|---|
| **1** | +++ | **2** | +++ | **3** | +++ | **4** | +++ | **5** | +++ |
| **6** | +++ | **7** | +++ | **8** | +++ | **9** | +++ | **10** | +++ |
| **11** | +++ | **12** | +++ | **13** | +++ | **14** | +++ | **15** | +++ |
| **16** | +++ | **17** | +++ | **18** | +++ | **19** | +++ | **20** | +++ |
| **21** | +++ | **22** | +++ | **23** | +++ | **24** | +++ | **25** | +++ |
| **26** | +++ | **27** | +++ | **28** | +++ | **29** | +++ | **30** | +++ |
| **31** | +++ | **32** | +++ | **33** | +++ | **34** | +++ | **35** | ++ |
| **36** | +++ | **37** | +++ | **38** | +++ | **39** | +++ | **40** | +++ |
| **41** | +++ | **42** | +++ | **43** | +++ | **44** | +++ | **45** | ++ |
| **46** | ++ | **47** | ++ | **48** | ++ | **49** | ++ | **50** | ++ |
| **51** | ++ | **52** | ++ | **53** | ++ | **54** | ++ | **55** | ++ |
| **56** | ++ | **57** | ++ | **58** | ++ | **59** | ++ | **60** | ++ |
| **61** | ++ | **62** | ++ | **63** | ++ | **64** | ++ | **65** | +++ |
| **66** | +++ | **67** | +++ | **68** | +++ | **69** | +++ | **70** | +++ |
| **71** | +++ | **72** | +++ | **73** | +++ | **74** | +++ | **75** | +++ |
| **76** | +++ | **77** | +++ | **78** | +++ | **79** | +++ | **80** | +++ |
| **81** | +++ | **82** | +++ | **83** | +++ | **84** | +++ | **85** | +++ |
| **86** | +++ | **87** | +++ | **88** | +++ | **89** | +++ | **90** | +++ |
| **91** | +++ | **92** | +++ | **93** | +++ | **94** | +++ | **95** | +++ |
| **96** | +++ | **97** | +++ | **98** | +++ | **99** | +++ | **100** | +++ |
| **101** | +++ | **102** | +++ | **103** | +++ | **104** | +++ | **105** | +++ |
| **106** | +++ | **107** | +++ | **108** | +++ | **109** | +++ | **110** | +++ |
| **111** | +++ | **112** | +++ | **113** | +++ | **114** | +++ | **115** | +++ |
| **116** | +++ | **117** | +++ | **118** | +++ | **119** | +++ | **120** | +++ |
| **121** | +++ | **122** | +++ | **123** | +++ | **124** | +++ | **125** | +++ |
| **126** | +++ | **127** | +++ | **128** | +++ | **129** | +++ | **130** | +++ |
| **131** | +++ | **132** | +++ | **133** | +++ | **134** | +++ | **135** | +++ |
| **136** | +++ | **137** | +++ | **138** | +++ | **139** | +++ | **140** | +++ |
| **141** | +++ | **142** | +++ | **143** | +++ | **144** | +++ | **145** | +++ |
| **146** | +++ | **147** | +++ | **148** | +++ | **149** | +++ | **150** | +++ |
| **151** | +++ | **152** | +++ | **153** | +++ | **154** | +++ | **155** | +++ |
| **156** | +++ | **157** | +++ | **158** | +++ | **159** | +++ | **160** | +++ |
| **161** | +++ | **162** | +++ | **163** | +++ | **164** | +++ | **165** | +++ |
| **166** | +++ | **167** | +++ | **168** | +++ | **169** | +++ | **170** | +++ |
| **171** | +++ | **172** | +++ | **173** | +++ | **174** | +++ | **175** | +++ |
| **176** | +++ | **177** | ++ | **178** | ++ | **179** | ++ | **180** | ++ |
| **181** | ++ | **182** | ++ | **183** | ++ | **184** | ++ | **185** | ++ |
| **186** | ++ | **187** | ++ | **188** | ++ | **189** | ++ | **190** | ++ |
| **191** | ++ | **192** | ++ | **193** | +++ | **194** | +++ | **195** | +++ |
| **196** | +++ | **197** | +++ | **198** | +++ | **199** | +++ | **200** | +++ |
| **201** | +++ | **202** | +++ | **203** | +++ | **204** | +++ | **205** | +++ |
| **206** | +++ | **207** | +++ | **208** | +++ | **209** | ++ | **210** | ++ |
| **211** | ++ | **212** | ++ | **213** | ++ | **214** | ++ | **215** | ++ |
| **216** | ++ | **217** | ++ | **218** | ++ | **219** | ++ | **220** | ++ |
| **221** | ++ | **222** | ++ | **223** | ++ | **224** | ++ | **225** | +++ |
| **226** | +++ | **227** | +++ | **228** | +++ | **229** | +++ | **230** | +++ |
| **231** | +++ | **232** | +++ | **233** | +++ | **234** | +++ | **235** | +++ |
| **236** | +++ | **237** | +++ | **238** | +++ | **239** | +++ | **240** | +++ |
| **241** | ++ | **242** | ++ | **243** | ++ | **244** | ++ | **245** | ++ |
| **246** | ++ | **247** | ++ | **248** | ++ | **249** | ++ | **250** | ++ |
| **251** | ++ | **252** | ++ | **253** | ++ | **254** | ++ | **255** | ++ |
| **256** | ++ | **257** | +++ | **258** | +++ | **259** | +++ | **260** | +++ |
| **261** | +++ | **262** | +++ | **263** | +++ | **264** | +++ | **265** | +++ |
| **266** | +++ | **267** | +++ | **268** | +++ | **269** | +++ | **270** | +++ |
| **271** | +++ | **272** | +++ | **273** | +++ | **274** | +++ | **275** | +++ |
| **276** | +++ | **277** | +++ | **278** | +++ | **279** | +++ | **280** | +++ |
| **281** | +++ | **282** | +++ | **283** | +++ | **284** | +++ | **285** | +++ |
| **286** | +++ | **287** | +++ | **288** | +++ | **289** | +++ | **290** | +++ |
| **291** | +++ | **292** | +++ | **293** | +++ | **294** | +++ | **295** | +++ |
| **296** | +++ | **297** | +++ | **298** | +++ | **299** | +++ | **300** | +++ |
| **301** | +++ | **302** | +++ | **303** | +++ | **304** | +++ | **305** | +++ |
| **306** | +++ | **307** | +++ | **308** | +++ | **309** | +++ | **310** | +++ |
| **311** | +++ | **312** | +++ | **313** | +++ | **314** | +++ | **315** | +++ |
| **316** | +++ | **317** | +++ | **318** | +++ | **319** | +++ | **320** | +++ |
| **321** | +++ | **322** | +++ | **323** | +++ | **324** | +++ | **325** | +++ |
| **326** | +++ | **327** | +++ | **328** | +++ | **329** | +++ | **330** | +++ |
| **331** | +++ | **332** | +++ | **333** | +++ | **334** | +++ | **335** | +++ |
| **336** | +++ | **337** | +++ | **338** | +++ | **339** | +++ | **340** | +++ |
| **341** | +++ | **342** | +++ | **343** | +++ | **344** | +++ | **345** | +++ |
| **346** | +++ | **347** | +++ | **348** | +++ | **349** | +++ | **350** | +++ |
| **351** | +++ | **352** | +++ | **353** | +++ | **354** | +++ | **355** | +++ |
| **356** | +++ | **357** | +++ | **358** | +++ | **359** | +++ | **360** | +++ |
| **361** | +++ | **362** | +++ | **363** | +++ | **364** | +++ | **365** | +++ |
| **366** | +++ | **367** | +++ | **368** | +++ | **369** | +++ | **370** | +++ |
| **371** | +++ | **372** | +++ | **373** | +++ | **374** | +++ | **375** | +++ |
| **376** | +++ | **377** | +++ | **378** | +++ | **379** | +++ | **380** | +++ |
| **381** | +++ | **382** | +++ | **383** | +++ | **384** | +++ | **385** | +++ |
| **386** | +++ | **387** | +++ | **388** | +++ | **389** | +++ | **390** | +++ |
| **391** | +++ | **392** | +++ | **393** | +++ | **394** | +++ | **395** | +++ |
| **396** | +++ | **397** | +++ | **398** | +++ | **399** | +++ | **400** | +++ |
| **401** | +++ | **402** | +++ | **403** | +++ | **404** | +++ | **405** | +++ |
| **406** | +++ | **407** | +++ | **408** | +++ | **409** | +++ | **410** | +++ |
| **411** | +++ | **412** | +++ | **413** | +++ | **414** | +++ | **415** | +++ |
| **416** | +++ | **417** | +++ | **418** | +++ | **419** | +++ | **420** | +++ |
| **421** | +++ | **422** | +++ | **423** | +++ | **424** | +++ | **425** | +++ |
| **426** | +++ | **427** | +++ | **428** | +++ | **429** | +++ | **430** | +++ |
| **431** | +++ | **432** | +++ | **433** | +++ | **434** | +++ | **435** | +++ |
| **436** | +++ | **437** | +++ | **438** | +++ | **439** | +++ | **440** | +++ |
| **441** | +++ | **442** | +++ | **443** | +++ | **444** | +++ | **445** | +++ |
| **446** | +++ | **447** | +++ | **448** | +++ | **449** | +++ | **450** | +++ |
| **451** | +++ | **452** | +++ | **453** | +++ | **454** | +++ | **455** | +++ |
| **456** | +++ | **457** | +++ | **458** | +++ | **459** | +++ | **460** | +++ |
| **461** | +++ | **462** | +++ | **463** | +++ | **464** | +++ | **465** | +++ |
| **466** | +++ | **467** | +++ | **468** | +++ | **469** | +++ | **470** | +++ |
| **471** | +++ | **472** | +++ | **473** | +++ | **474** | +++ | **475** | +++ |
| **476** | +++ | **477** | +++ | **478** | +++ | **479** | +++ | **480** | +++ |
| **481** | +++ | **482** | +++ | **483** | +++ | **484** | +++ | **485** | +++ |
| **486** | +++ | **487** | +++ | **488** | +++ | **489** | +++ | **490** | +++ |
| **491** | +++ | **492** | +++ | **493** | +++ | **494** | +++ | **495** | +++ |
| **496** | +++ | **497** | +++ | **498** | +++ | **499** | +++ | **500** | +++ |
| **501** | +++ | **502** | +++ | **503** | +++ | **504** | +++ | **505** | +++ |
| **506** | +++ | **507** | +++ | **508** | +++ | **509** | +++ | **510** | +++ |
| **511** | +++ | **512** | +++ | **513** | +++ | **514** | +++ | **515** | +++ |
| **516** | +++ | **517** | +++ | **518** | +++ | **519** | +++ | **520** | +++ |
| **521** | +++ | **522** | +++ | **523** | +++ | **524** | +++ | **525** | +++ |
| **526** | +++ | **527** | +++ | **528** | +++ | **529** | ++ | **530** | ++ |
| **531** | ++ | **532** | ++ | **533** | ++ | **534** | ++ | **535** | ++ |
| **536** | ++ | **537** | ++ | **538** | ++ | **539** | ++ | **540** | ++ |
| **541** | ++ | **542** | ++ | **543** | ++ | **544** | ++ | **545** | +++ |
| **546** | +++ | **547** | +++ | **548** | +++ | **549** | +++ | **550** | +++ |
| **551** | +++ | **552** | +++ | **553** | +++ | **554** | +++ | **555** | +++ |
| **556** | +++ | **557** | +++ | **558** | +++ | **559** | +++ | **560** | +++ |
| **561** | ++ | **562** | ++ | **563** | ++ | **564** | ++ | **565** | ++ |
| **566** | ++ | **567** | ++ | **568** | ++ | **569** | ++ | **570** | ++ |
| **571** | ++ | **572** | ++ | **573** | ++ | **574** | ++ | **575** | ++ |
| **576** | ++ | **577** | +++ | **578** | +++ | **579** | +++ | **580** | +++ |
| **581** | +++ | **582** | +++ | **583** | +++ | **584** | +++ | **585** | +++ |
| **586** | +++ | **587** | +++ | **588** | +++ | **589** | +++ | **590** | +++ |
| **591** | +++ | **592** | +++ | **593** | +++ | **594** | +++ | **595** | +++ |
| **596** | +++ | **597** | +++ | **598** | +++ | **599** | +++ | **600** | +++ |
| **601** | +++ | **602** | +++ | **603** | +++ | **604** | +++ | **605** | +++ |
| **606** | +++ | **607** | +++ | **608** | +++ | **609** | +++ | **610** | +++ |
| **611** | +++ | **612** | +++ | **613** | +++ | **614** | +++ | **615** | +++ |
| **616** | +++ | **617** | +++ | **618** | +++ | **619** | +++ | **620** | +++ |
| **621** | +++ | **622** | +++ | **623** | +++ | **624** | +++ | **625** | +++ |
| **626** | +++ | **627** | +++ | **628** | +++ | **629** | +++ | **630** | +++ |
| **631** | +++ | **632** | +++ | **633** | +++ | **634** | +++ | **635** | +++ |
| **636** | +++ | **637** | +++ | **638** | +++ | **639** | +++ | **640** | +++ |

| | | | | | | | | | |
|---|---|---|---|---|---|---|---|---|---|
| +++ means that IC₅₀ is less than or equal to 100 nM. ++ means that IC ₅₀ is 100 nM to 500 nM. + means that IC ₅₀ is greater than 500 nM. | | | | | | | | | |

As can be seen from the data in Table 3, the compounds of the present invention have good inhibitory activities against the enzymatic activity of KIF18A.

### Biological Example 2. In Vitro Anti-Proliferative Activity of the Compounds of the Present Invention against HT-29 Cells

HT-29 cells were seeded on a 384-well plate at 3000 cells/well. After overnight adherence culture, DMSO or the compounds serially 1:5-diluted from 5 µM were added. The viability of the cells was assessed 72 h after dosing by measuring the intracellular ATP content. The inhibition percentages of viable cells by the compounds were calculated by comparing with the DMSO group, and the IC ₅₀ values were also calculated. The results are shown in Table 4 below.

**Table 4. Anti-proliferative activity of the compounds of the present invention against HT-29 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | 75.31 | **3** | 1600.00 | **4** | 191.20 | **6** | 825.90 |
| **7** | 339.10 | **65** | 55.34 | **97** | >2000 | **129** | 134.50 |
| **161** | 66.11 | **257** | 11.10 | **258** | 190.00 | **259** | 281.90 |
| **260** | 55.21 | **261** | 75.72 | **262** | 242.40 | **263** | 85.65 |
| **289** | 27.71 | **305** | 40.36 | **321** | 59.30 | **337** | 48.78 |
| **353** | 74.56 | **385** | 145.60 | **593** | 97.88 | **641** | 75.82 |
| **643** | 34.17 | **645** | 71.30 | **647** | 334.20 | **655** | 1171.00 |
| **661** | 25.76 | **663** | 34.99 | **669** | 25.78 | **701** | 76.69 |
| **702** | 71.18 | **703** | 183.80 | **704** | 64.19 | **705** | 146.00 |
| **833** | 52.94 | **835** | 70.70 | **836** | 253.20 | **AMG650** | 109 |

The reference compound AMG650 is compound 4 in WO2020132648A1.

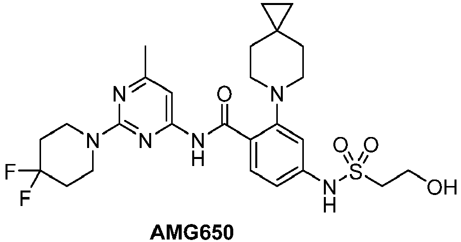

As can be seen from the data in Table 4, some of the compounds of the present invention have stronger anti-proliferative activities against HT-29 cells compared with AMG650.

### Biological Example 3. In Vitro Anti-Proliferative Activity of the Compounds of the Present Invention against HCT116 Cells

HCT116 cells were seeded on a 384-well plate at 3000 cells/well. After overnight adherence culture, DMSO or the compounds serially 1:5-diluted from 5 µM were added. The viability of the cells was assessed 72 h after dosing by measuring the intracellular ATP content. The inhibition percentages of viable cells by the compounds were calculated by comparing with the DMSO group, and the IC ₅₀ values were also calculated. The results are shown in Table 5 below.

**Table 5. Anti-proliferative activity of the compounds of the present invention against HCT116 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | >10000 | **3** | >10000 | **4** | >10000 | **6** | >10000 |
| **7** | >10000 | **65** | >10000 | **97** | >10000 | **129** | >10000 |
| **161** | >10000 | **257** | >10000 | **258** | >10000 | **259** | >10000 |
| **260** | >10000 | **261** | >10000 | **262** | >10000 | **263** | >10000 |
| **289** | >10000 | **305** | >10000 | **321** | >10000 | **337** | >10000 |
| **353** | >10000 | **385** | >10000 | **593** | >10000 | **641** | >10000 |
| **643** | >10000 | **645** | >10000 | **647** | >10000 | **655** | >10000 |
| **661** | >10000 | **663** | >10000 | **669** | >10000 | **701** | >10000 |
| **702** | >10000 | **703** | >10000 | **704** | >10000 | **705** | >10000 |
| **833** | >10000 | **835** | >10000 | **836** | >10000 | **AMG650** | >10000 |

As can be seen from the data in Table 5, neither the compounds of the present invention nor AMG650 have anti-proliferative activity against HCT116 cells.

### Biological Example 4. In Vivo Pharmacodynamic Study - Mouse HT29 Subcutaneous Xenograft Tumor Model

BALB/c nude mice were inoculated subcutaneously with 5 × 10⁶ HT29 cells on the left dorsal side. After the tumors grew to 100-150 mm³, the mice were randomly divided into the following groups for intragastric administration once daily: group 1: vehicle control group; group 2: compound 661 (80 mg/kg); group 3: compound 669 (80 mg/kg); group 4: compound 677 (80 mg/kg); group 5: compound 714 (80 mg/kg); group 6: compound 727 (80 mg/kg); group 7: compound 740 (80 mg/kg); and group 8: AMG650 (80 mg/kg). The tumor volume was measured twice weekly and at the end of treatment. Tumor growth inhibition rate of the compound was calculated according to the following equation: tumor growth inhibition (TGI) = 1 - (tumor volume on day 28 in treatment group - tumor volume on day 1 in treatment group) / (tumor volume on day 28 in vehicle control group - tumor volume on day 1 in treatment group). The results are shown in Table 6.

**Table 6. Growth inhibition in mouse HT29 subcutaneous xenoeraft tumor**

| **Group** | **Compound** | **Dose** | **Tumor volume on day 1 of treatment (mm³)** | **Tumor volume on day 28 of treatment (mm³)** | **TGI** |
|---|---|---|---|---|---|
| **1** | **Control** | Not applicable | 121 | 1606 | Not applicable |
| **2** | **661** | 80 mg/kg | 121 | 704 | 61% |
| **3** | **669** | 80 mg/kg | 121 | 1158 | 30% |
| **4** | **677** | 80 mg/kg | 121 | 797 | 55% |
| **5** | **714** | 80 mg/kg | 121 | 424 | 80% |
| **6** | **727** | 80 mg/kg | 121 | 519 | 73% |
| **7** | **740** | 80 mg/kg | 121 | 605 | 67% |
| **8** | **AMG650** | 80 mg/kg | 121 | 649 | 64% |

As can be seen from Table 6, the compounds of the present invention were able to inhibit tumor growth at a dose of 80 mg/kg in the subcutaneous xenograft tumor model of HT29-bearing mice, and compound 714, compound 727, and compound 740 showed stronger inhibitory effects on the HT29 mouse subcutaneous xenograft tumor compared with AMG650.

### Biological Example 5. Phosphorylation Assay of Histone H3 SerlO Sites in HT29 Cells (Immunofluorescence Assay)

HT29 cells were seeded in a 96-well plate (Fisher 160376) at 8000 cells/well. The next day, the serially diluted compounds were added. 6 h after the addition of the compounds, the cells were washed once with 1× PBS, immobilized with 4% PFA for 15 min, further washed three times with 1× PBS, and permeabilized with 0.02% Triton-X100 for 10 min. A blocking buffer was then added for blocking for 15-30 min, and 1:3000-diluted primary antibodies (Phospho-Histone H3 (Ser10) (D7N8E) XP^{®} Rabbit mAb #53348) were added. The plate was placed at 4 °C overnight. The next day, the cells were washed three times with 1 × PBS, and 1:1000-diluted secondary antibodies (Fluorescein (FITC)-conjugated Affinipure Goat Anti-Rabbit IgG (H+L)) were added. The cells were incubated in the dark for 1-2 h and washed three times with 1 × PBS. The nuclei were stained with DAPI, and after the staining was completed, photographs were taken using an MD ImageXpress Pico personal high-content imaging analysis system. The ratio of phosphorylation (FITC/DAPI) at H3 Ser10 sites in HT29 cells was quantified. To evaluate the effects of the compounds on the mitosis phase of HT29 cells, the EC₅₀ values of the compounds were calculated. The results are shown in Table 7 below.

**Table 7. Phosphorylation at H3 Ser10 sites in HT29 cells induced by the compounds of the present invention (EC₅₀, nM)**

| **Compound** | **EC₅₀ (nM)** | **Compound** | **EC₅₀ (nM)** | **Compound** | **EC₅₀ (nM)** |
|---|---|---|---|---|---|
| **65** | 45.5 | **161** | 26.4 | **257** | 8.4 |
| **273** | 8.5 | **321** | 15.8 | **353** | 42.2 |
| **641** | 21.7 | **645** | 69.6 | **661** | 23.7 |
| **663** | 28.3 | **714** | 39.2 | **AMG650** | 52.3 |

As can be seen from Table 7, the compounds of the present invention have relatively strong activities in inducing the phosphorylation at H3 Ser10 sites in HT-29 cells, and compared with AMG650, the compounds of the present invention have stronger activities in inducing the phosphorylation at H3 Ser10 sites.

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

## Claims

1. A compound of general formula (1), or an isomer, a crystalline form, a pharmaceutically acceptable salt, a hydrate or a solvate thereof: 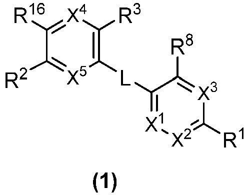 wherein, in general formula (1):
X¹ is -CR⁵= or N;
X² is -CR⁶= or N;
X³ is -CR⁷= or N;
X⁴ is -CR⁴= or N;
X⁵ is -CR¹⁵= or N;
when X⁵ is -CR¹⁵= and X⁴ is -CR⁴=, R¹⁶ is -C₃₋₈ cycloalkyl, -OR¹⁷, -SR¹⁸, -NR¹⁸R¹⁹, or -NO₂;
when X⁵ is -CR¹⁵= and X⁴ is N, R¹⁶ is -O-C₁₋₈ hydrocarbyl, -C₃₋₈ cycloalkyl, -OR¹⁷, -SR¹⁸, -NR²⁰R²¹, or -NO₂;
when X⁵ is N, R¹⁶ is -O-C₁₋₈ hydrocarbyl, -C₃₋₈ cycloalkyl, -OR¹⁷, -SR¹⁸, -NR²⁰R²¹, or -NO₂;
L is -(C=O)-NR⁹-* or -NR⁹-(C=O)-*; and no more than four of X¹, X², X³, X⁴, and X⁵ are N;
* represents a position linking to 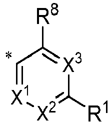 terminal;
R¹⁷ is H, -C₁₋₈ halohydrocarbyl, -C₃₋₈ cycloalkyl, or -C₃₋₈ halocycloalkyl, wherein the -C₁₋₈ halohydrocarbyl, - C₃₋₈ cycloalkyl, or -C₃₋₈ halocycloalkyl may be optionally substituted with 0, 1, 2, or 3 of the following groups:
H, halogen, and -C₁₋₄ hydrocarbyl;
R¹⁸ and R¹⁹ are each independently H, -C₁₋₈ hydrocarbyl, -C₁₋₈ halohydrocarbyl, -C₃₋₈ cycloalkyl, or -C₃₋₈ halocycloalkyl, wherein the -C₁₋₈ hydrocarbyl, -C₁₋₈ halohydrocarbyl, -C₃₋₈ cycloalkyl, or -C₃₋₈ halocycloalkyl may be optionally substituted with 0, 1, 2, or 3 of the following groups: H, halogen, and -C₁₋₄ hydrocarbyl;
R²⁰ and R²¹ are each independently H, -C₁₋₈ hydrocarbyl, -C₁₋₈ halohydrocarbyl, -C₃₋₈ cycloalkyl, or -C₃₋₈ halocycloalkyl, wherein the -C₁₋₈ hydrocarbyl, -C₁₋₈ halohydrocarbyl, -C₃₋₈ cycloalkyl, or -C₃₋₈ halocycloalkyl may be optionally substituted with 0, 1, 2, or 3 of the following groups: H, halogen, and -C₁₋₄ hydrocarbyl; or
R²⁰ and R²¹ may be combined with the nitrogen atom to which they are each connected to form a saturated or partially saturated 3-, 4-, 5-, or 6-membered monocyclic ring or 4-, 5-, 6-, 7-, 8-, 9-, 10-, 11-, or 12-membered bicyclic ring containing 0, 1, 2, or 3 N atoms and 0, 1, or 2 atoms selected from O and S;
R¹ is -CN or -Z-R¹⁰, wherein Z is a chemical bond, -C₀₋₄ hydrocarbyl-, -NR¹¹-, -NR¹¹SO₂-, -SO₂NR¹¹-, -NR¹¹-S(=O)(=NH)-, -S(=O)(=NH)-, -S-, -S(=O)-, -SO₂-, -C₀₋₄ hydrocarbyl-O-, -(C=O)-, -(C=O)NR¹¹-, -C(=N-OH)-, or -NR¹¹(C=O)-; or the group -Z-R¹⁰ is -N=S(=O)-(R¹⁰)₂, wherein two R¹⁰ may be combined with the sulfur atom to which they are each connected to form a saturated or partially saturated 3-, 4-, 5-, or 6-membered monocyclic ring containing 0, 1, 2, or 3 N atoms and 0, 1, or 2 atoms selected from O and S;
R² is halogen or a group -Y-R¹², wherein Y is a chemical bond, -C₀₋₄ hydrocarbyl-, -N(C₀₋₁ hydrocarbyl)-C₀₋₄ hydrocarbyl-, -C(=O)NR^{a}R^{a} (C₁₋₄ hydrocarbyl)-, -O-C₀₋₄ hydrocarbyl-, -S-, -S(=O)-, -SO₂-, -SO₂NR¹²-, or - S(=O)(=NH)-;
R³ is H, halogen, C₁₋₈ hydrocarbyl, or C₁₋₄ halohydrocarbyl;
R⁴ is H, halogen, R^{4a}, or R^{4b};
R⁵ is H, halogen, C₁₋₈ alkyl, or C₁₋₄ haloalkyl;
R⁶ is H, halogen, C₁₋₈ alkyl, C₁₋₄ haloalkyl, -OH, -O-R^{6a}, or -O-R⁶¹⁵;
R⁷ is H, halogen, C₁₋₈ hydrocarbyl, or C₁₋₄ halohydrocarbyl;
R⁸ is selected from the group consisting of: 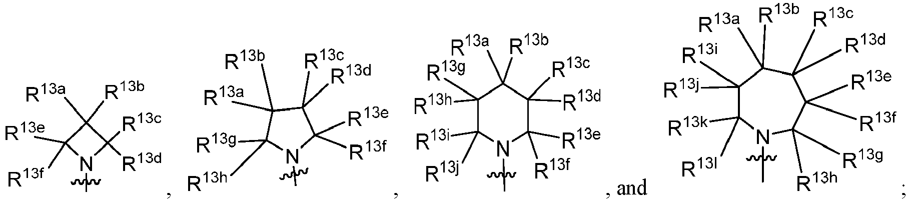
R^{13a}, R^{13b}, R^{13c}, R^{13d}, R^{13e}, R^{13f}, R^{13g}, R^{13h}, R¹³ⁱ, R^{13j}, R^{13k}, and R^{13l} are each independently H, halogen, R^{13m}, or R¹³ⁿ; or each of the pairs of R^{13a}/R^{13b}, R^{13c}/R^{13d}, R^{13e}/R^{13f}, R^{13g}/R^{13h}, R¹³ⁱ/R^{13j}, and R^{13k}/R^{13l} may independently form, with the carbon atom to which they are each connected, a saturated or partially saturated 3-, 4-, 5-, or 6-membered monocyclic ring spiro-linked to R⁸ ring, wherein the 3-, 4-, 5-, or 6-membered monocyclic ring contains 0, 1, 2, or 3 N atoms and 0, 1, or 2 atoms selected from O and S, and further, the 3-, 4-, 5-, or 6-membered monocyclic ring is substituted with 0, 1, 2, or 3 groups selected from: F, Cl, Br, C₁₋₆ hydrocarbyl, C₁₋₄ halohydrocarbyl, -OR^{a}, -OC₁₋ₐ halohydrocarbyl, CN, -NR^{a}R^{a}, and oxo;
R⁹ is H or C₁₋₆ hydrocarbyl;
R¹⁰ is H, R^{10a}, R^{10b}, or R^{10c};
R¹¹ is H, R^{11a}, or R^{11b};
R¹² is R^{12a} or R^{12b};
R¹⁵ is H, halogen, C₁₋₈ hydrocarbyl, C₁₋₄ halohydrocarbyl, -O-C₁₋₈ hydrocarbyl, or -O-R^{15a}, wherein R^{15a} is a saturated or partially saturated 3-, 4-, 5-, or 6-membered monocyclic ring containing 0, 1, 2, or 3 N atoms and 0, 1, or 2 atoms selected from O and S;
R^{4a}, R^{6a}, R^{10a}, R^{11a}, R^{12a}, or R^{13m}, in each case, is independently selected from: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0, 1 or 2 atoms selected from O and S, wherein the monocyclic ring and bicyclic ring may be each independently and optionally substituted with 0, 1, 2, or 3 of the following groups: F, Cl, Br, C₁₋₆ hydrocarbyl, C₁₋₄ halohydrocarbyl, -OR^{a}, -OC₁₋₄ halohydrocarbyl, CN, -C(=O)R^{b}, - C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, R¹⁴, and oxo;
R^{4b}, R^{6b}, R^{10b}, R^{11b}, R^{12b}, or R¹³ⁿ, in each case, is independently selected from: C₁₋₆ hydrocarbyl, wherein the hydrocarbyl may be optionally substituted with 0, 1, 2, 3, 4, or 5 of the following groups: F, Cl, Br, -R^{a}, -OR^{a}, -OC₁₋₄ halohydrocarbyl, and CN;
R^{10c}, in each case, is independently selected from: C₁₋₆ hydrocarbyl, wherein the hydrocarbyl may be optionally substituted with 0, 1, 2, 3, 4, or 5 of the following groups: F, Cl, Br, -R^{a}, -R^{c}, -OR^{a}, -OC₁₋₄ halohydrocarbyl, and CN;
R¹⁴, in each case, is independently selected from: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 of the following groups: F, Cl, Br, C₁₋₆ hydrocarbyl, C₁₋₄ halohydrocarbyl, -OR^{a}, -OC₁₋₄ halohydrocarbyl, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, - S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, - N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, and oxo;
R^{a}, in each case, is independently H or R^{b};
R^{b}, in each case, is independently C₁₋₆ hydrocarbyl, phenyl, or benzyl, wherein the hydrocarbyl may be optionally substituted with 0, 1, 2, or 3 of the following groups: halogen, -OH, -OC₁₋₄ hydrocarbyl, -NH₂, - NHC₁₋₄ hydrocarbyl, -OC(=O)C₁₋₄ hydrocarbyl, and -N(C₁₋₄ hydrocarbyl) C₁₋₄ hydrocarbyl; and the phenyl and benzyl may be each independently and optionally substituted with 0, 1, 2, or 3 of the following groups: halogen, C₁₋₄ hydrocarbyl, C₁₋₃ halohydrocarbyl, -OH, -OC₁₋₄ hydrocarbyl, -NH₂, -NHC₁₋₄ hydrocarbyl, - OC(=O)C₁₋₄ hydrocarbyl, and -N(C₁₋₄ hydrocarbyl) C₁₋₄ hydrocarbyl; and
R^{c}, in each case, is independently -OC(=O)C₁₋₅ hydrocarbyl, wherein the hydrocarbyl may be optionally substituted with 1, 2, or 3 of the following groups: -OH and -NH₂.

2. The compound, or the isomer, the crystalline form, the pharmaceutically acceptable salt, the hydrate or the solvate thereof according to claim 1, wherein general formula (1) has the following structure: 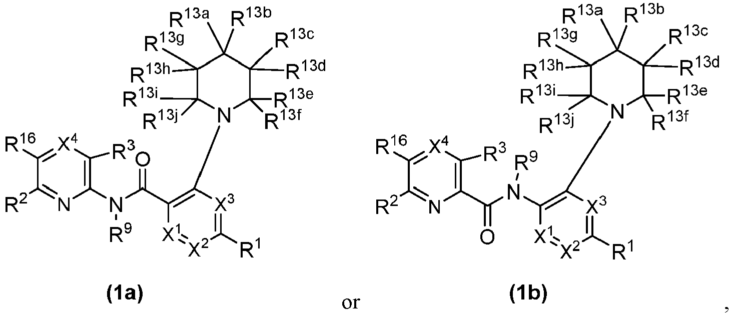 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂.

3. The compound, or the isomer, the crystalline form, the pharmaceutically acceptable salt, the hydrate or the solvate thereof according to claim 1, wherein general formula (1) has the following structure: 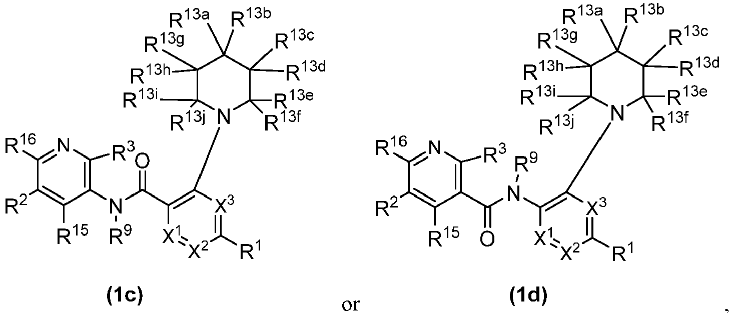 wherein R¹⁶ is -C₃₋₆ cycloalkyl, -OH, -O-C₁₋₄ hydrocarbyl, -O-C₁₋₄ halohydrocarbyl, -O-C₃₋₆ cycloalkyl, -O-C₃₋₆ halocycloalkyl, -SH, -S-C₁₋₆ hydrocarbyl, -S-C₁₋₄ halohydrocarbyl, -S-C₃₋₆ cycloalkyl, -S-C₃₋₆ halocycloalkyl, -NR²⁰R²¹, or -NO₂.

4. The compound, or the isomer, the crystalline form, the pharmaceutically acceptable salt, the hydrate or the solvate thereof according to claim 1, wherein general formula (1) has the following structure: 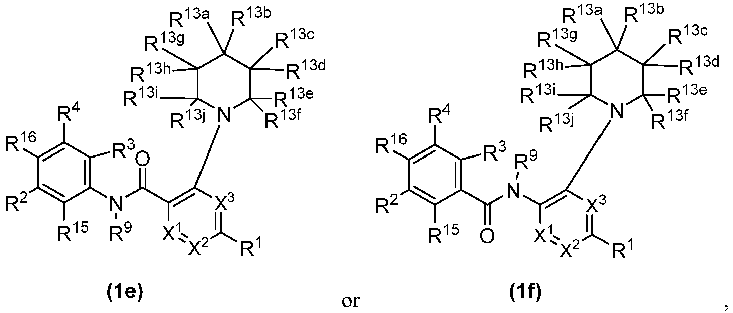 wherein R¹⁶ is -C₃₋₆ cycloalkyl, -OH, -O-C₁₋₄ halohydrocarbyl, -O-C₃₋₆ cycloalkyl, -O-C₃₋₆ halocycloalkyl, - SH, -S-C₁₋₆ hydrocarbyl, -S-C₁₋₄ halohydrocarbyl, -S-C₃₋₆ cycloalkyl, -S-C₃₋₆ halocycloalkyl, -NR¹⁸R¹⁹, or - NO₂.

5. The compound, or the isomer, the crystalline form, the pharmaceutically acceptable salt, the hydrate or the solvate thereof according to any one of claims 1-4, wherein, in general formula (1), R¹⁶ is -OH, -OCF₃, - OCH₂F, -OCHF₂, -OCH₂CF₃, -OCF₂CF₃, -OCF₂Cl, -OCFCl₂, 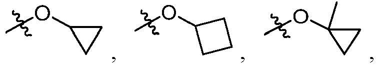 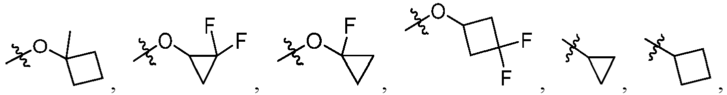 -SH, -SCH₃, -SCH₂CH₃, 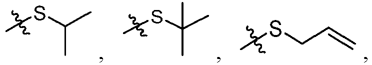 -SCF₃, -SCH₂CF₃, -SCF₂CF₃, -SCF₂Cl, -SCFCl₂, 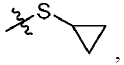 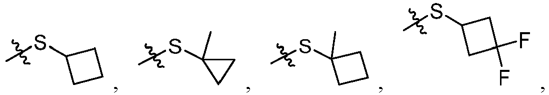 -NH₂, 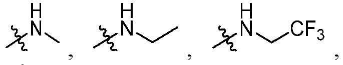 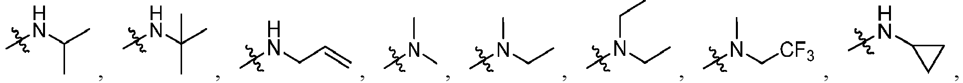 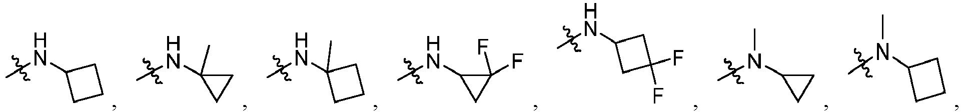 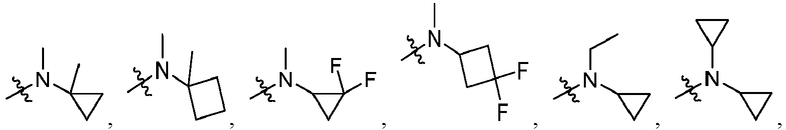 or -NO₂, preferably -OCF₃, -OCH₂F, -OCHF₂, 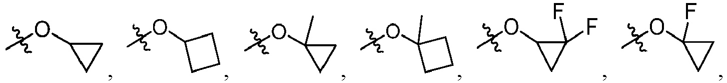 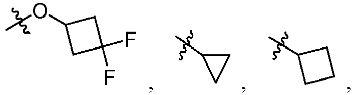 -SCH₃, -SCF₃, -SCF₂Cl, -SCFCl₂, 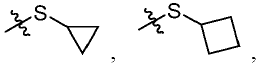 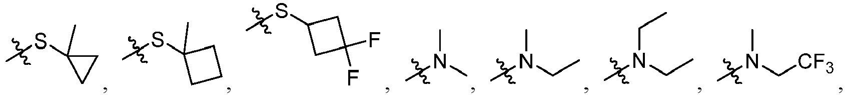 or -NO₂, and more preferably -OCF₃, -OCH₂F, -OCHF₂, 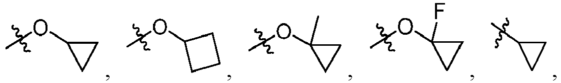 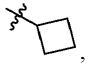 -SCH₃, -SCF₃, 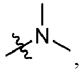 or -NO₂.

6. The compound, or the isomer, the crystalline form, the pharmaceutically acceptable salt, the hydrate or the solvate thereof according to any one of claims 1-3, wherein, in general formula (1), R¹⁶ is -OCH₃, -OCH₂CH₃, -OCH₂CH₂CH₃, 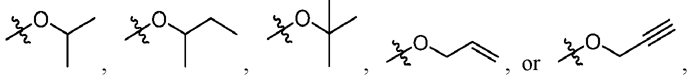 preferably - OCH₃.

7. The compound, or the isomer, the crystalline form, the pharmaceutically acceptable salt, the hydrate or the solvate thereof according to any one of claims 1-6, wherein, in general formula (1), R⁹ is H, methyl, or ethyl, preferably H.

8. The compound, or the isomer, the crystalline form, the pharmaceutically acceptable salt, the hydrate or the solvate thereof according to claim 1, wherein, in general formula (1), R^{13c}, R^{13d}, R^{13e}, R^{13f}, R^{13g}, R^{13h}, R¹³ⁱ, R^{13j}, R^{13k}, and R^{13l} are each independently H, halogen, C₁₋₆ hydrocarbyl, or C₁₋₄ halohydrocarbyl; and R^{13a} and R^{13b} in the pair of R^{13a}/R^{13b} may be combined with the carbon atom to which they are each connected to form a saturated 3-, 4-, or 5-membered monocyclic ring spiro-linked to R⁸ ring, wherein the monocyclic ring contains 0, 1, 2, or 3 N atoms and 0, 1, or 2 atoms selected from O and S; preferably, R^{13c}, R^{13d}, R^{13e}, R^{13f}, R^{13g}, R^{13h}, R^{13l}, R^{13j}, R^{13k}, and R^{13l} are each independently H, methyl, or ethyl; and R^{13a} and R^{13b} in the pair of R^{13a}/R^{13b} may be combined with the carbon atom to which they are each connected to form a cyclopropyl, cyclobutyl, or cyclopentyl ring spiro-linked to R⁸ ring.

9. The compound, or the isomer, the crystalline form, the pharmaceutically acceptable salt, the hydrate or the solvate thereof according to any one of claims 1-8, wherein, in general formula (1), structural unit 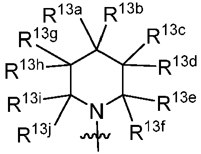 is: 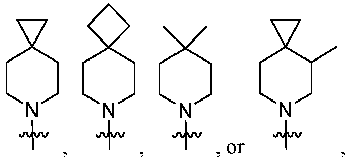 preferably

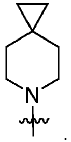

10. The compound, or the isomer, the crystalline form, the pharmaceutically acceptable salt, the hydrate or the solvate thereof according to any one of claims 1-9, wherein, in general formula (1), Z is a chemical bond, - NH-, -NHSO₂-, -SO₂NH-, -S(=O)(=NH)-, -S-, -S(=O)-, -SO₂-, -(C=O)-, -(C=O)NH-, or -NH(C=O)-.

11. The compound, or the isomer, the crystalline form, the pharmaceutically acceptable salt, the hydrate or the solvate thereof according to any one of claims 1-10, wherein, in general formula (1), R¹⁰ is selected from: (a) H; (b) C₁₋₆ hydrocarbyl, wherein the hydrocarbyl may be optionally substituted with 0, 1, 2, or 3 of the following groups: F, Cl, Br, -OH, and -OCH₃; (c) a group such that when the group -Z-R¹⁰ is -N=S(=O)-(R¹⁰)₂, two R¹⁰ may be combined with the sulfur atom to which they are each connected to form a saturated, partially saturated, or unsaturated 3-, 4-, 5-, 6-, or 7-membered monocyclic ring containing 0, 1, 2, or 3 N atoms and 0 or 1 atom selected from O and S, wherein the monocyclic ring is substituted with 0, 1, 2, or 3 groups selected from: F, Cl, Br, C₁₋₆ hydrocarbyl, C₁₋₄ halohydrocarbyl, -C₁₋₆ hydrocarbyl OH, -OH, -OCH₃, -NH₂, and oxo; and (d) C₁₋₆ hydrocarbyl, wherein the C₁₋₆ hydrocarbyl may be optionally substituted with 1, 2, or 3 of the following group: -OC(=O)C₁₋₅ hydrocarbyl, wherein the C₁₋₅ hydrocarbyl may be optionally substituted with 1 or 2 of the following groups: -OH and -NH₂; and the C₁₋₆ hydrocarbyl may be optionally substituted with 0, 1, 2, or 3 of the following groups: F, Cl, Br, -OH, and -OCH₃.

12. The compound, or the isomer, the crystalline form, the pharmaceutically acceptable salt, the hydrate or the solvate thereof according to any one of claims 1-11, wherein, in general formula (1), R¹ is -CN or a group -Z-R¹⁰, wherein Z is a chemical bond, -NH-, -NHSO₂-, -SO₂NH-, -S(=O)(=NH)-, -S-, -S(=O)-, -SO₂-, -(C=O)-, - (C=O)NH-, or -NH(C=O)-; and R¹⁰ is selected from:
(a) H;
(b) cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 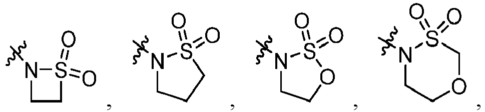 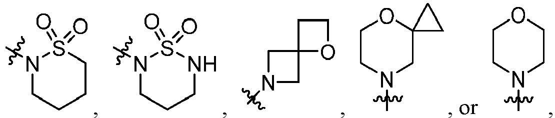 wherein the rings may be each independently and optionally substituted with 0, 1, 2, or 3 of the following groups: OH, F, methyl, -CH₂OH, -C(=O)OCH₃, - C(=O)OC(CH₃)₃, NH₂, CN, and oxo; and oxetanyl and cyclopropyl are preferred;
(c) C₁₋₆ hydrocarbyl substituted with 0, 1, 2, or 3 OH, F, -C(=O)OCH₃, -NH₂, -NH(CH₃), or -N(CH₃)₂, preferably C₁₋₆ hydrocarbyl substituted with 0, 1, 2, or 3 OH groups, and more preferably C₁₋₆ hydrocarbyl substituted with 1 OH group; and
(d) C₁₋₆ hydrocarbyl, wherein the C₁₋₆ hydrocarbyl may be optionally substituted with 1, 2, or 3 of the following groups: 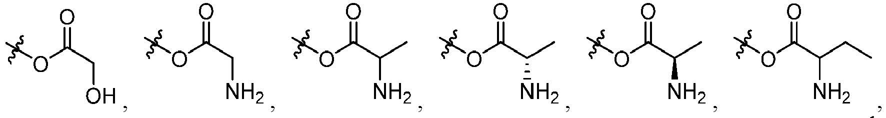 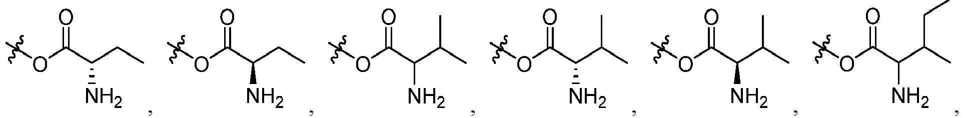 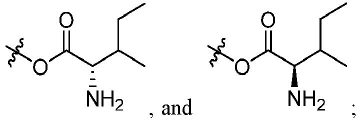 and the C₁₋₆ hydrocarbyl may be optionally substituted with 0, 1, 2, or 3 of the following groups: F, Cl, Br, -OH, and -OCH₃.

13. The compound, or the isomer, the crystalline form, the pharmaceutically acceptable salt, the hydrate or the solvate thereof according to any one of claims 1-12, wherein, in general formula (1), the group -Z-R¹⁰ is - N=S(=O)-(R¹⁰)₂, wherein two R¹⁰ may be combined with the sulfur atom to which they are each connected to form a saturated or partially saturated 3-, 4-, 5-, or 6-membered monocyclic ring containing 0, 1, 2, or 3 N atoms and 0, 1 or 2 atoms selected from O and S; preferably, the group -Z-R¹⁰ is selected from:

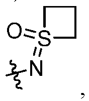

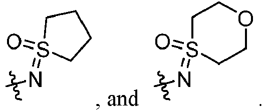

14. The compound, or the isomer, the crystalline form, the pharmaceutically acceptable salt, the hydrate or the solvate thereof according to any one of claims 1-12, wherein, in general formula (1), R¹ is a group -Z-R¹⁰, wherein Z is -NHSO₂- or -SO₂NH-; and R¹⁰ is oxetanyl or cyclopropyl, or R¹⁰ is C₁₋₆ hydrocarbyl substituted with 0, 1, 2, or 3 OH groups; or R¹⁰ is C₁₋₆ hydrocarbyl, wherein the C₁₋₆ hydrocarbyl may be optionally substituted with 1, 2, or 3 of the following groups:

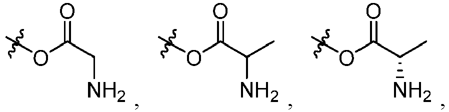

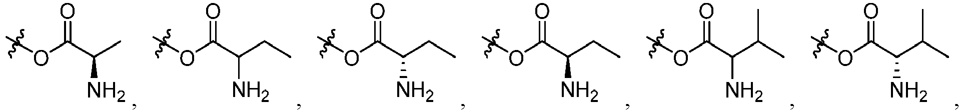

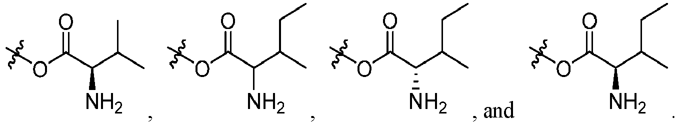

15. The compound, or the isomer, the crystalline form, the pharmaceutically acceptable salt, the hydrate or the solvate thereof according to any one of claims 1-10, wherein, in general formula (1), R¹⁰ is selected from C₁₋₆ hydrocarbyl, wherein the hydrocarbyl may be optionally substituted with 0, 1, 2, or 3 of the following groups: 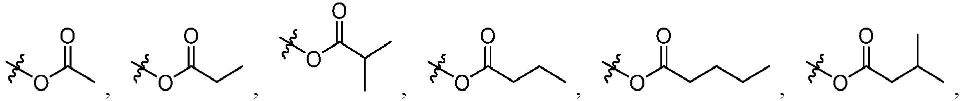 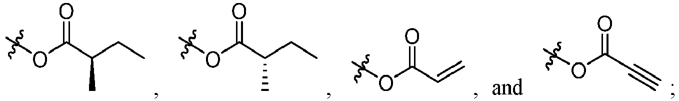 the hydrocarbyl is preferably substituted with 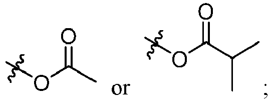 Z is -NHSO₂- or -SO₂NH-; Z is preferably -NHSO₂-.

16. The compound, or the isomer, the crystalline form, the pharmaceutically acceptable salt, the hydrate or the solvate thereof according to any one of claims 1-10, wherein, in general formula (1), R¹⁰ is selected from C₁₋₆ hydrocarbyl, wherein the hydrocarbyl may be optionally substituted with 1, 2, or 3 of the following groups: 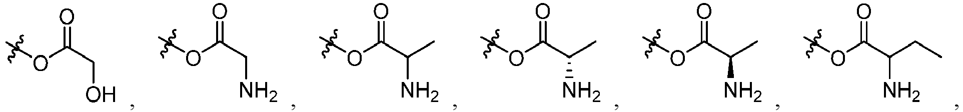 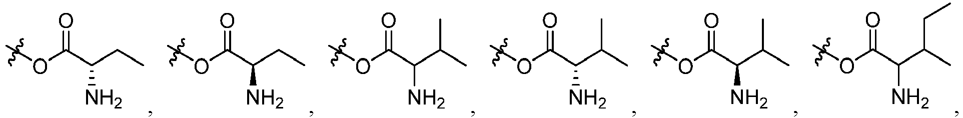 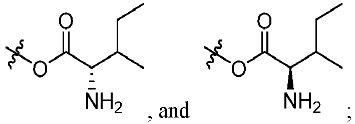 Z is -NHSO₂- or -SO₂NH-.

17. The compound, or the isomer, the crystalline form, the pharmaceutically acceptable salt, the hydrate or the solvate thereof according to any one of claims 1-16, wherein, in general formula (1), R² is halogen or a group -Y-R¹², wherein Y is a chemical bond, -NH-, -NH-(CH₂)₀₋₄-, or -O-(CH₂)₀₋₄-; and R¹² is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 of the following groups: F, Cl, Br, C₁₋₆ hydrocarbyl, C₁₋₄ halohydrocarbyl, -OH, -OC₁₋₄ halohydrocarbyl, CN, R¹⁴, and oxo; or R¹² is C₁₋₆ hydrocarbyl, wherein the hydrocarbyl may be optionally substituted with 0, 1, 2, 3, 4, or 5 of the following groups: F, Cl, Br, -OH, -OC₁₋₄ halohydrocarbyl, and CN.

18. The compound, or the isomer, the crystalline form, the pharmaceutically acceptable salt, the hydrate or the solvate thereof according to any one of claims 1-17, wherein, in general formula (1), R² is a saturated 5- or 6-membered monocyclic ring, wherein the ring contains 0, 1 or 2 N atoms and 0 or 1 O atom, and the ring is substituted with 0, 1, 2, or 3 groups selected from: F, Cl, Br, C₁₋₆ hydrocarbyl, C₁₋₄ halohydrocarbyl, -OH, - OC₁₋₄ halohydrocarbyl, CN, R¹⁴, and oxo.

19. The compound, or the isomer, the crystalline form, the pharmaceutically acceptable salt, the hydrate or the solvate thereof according to any one of claims 1-18, wherein, in general formula (1), R² is: (a) halogen; (b) a group -Y-R¹², wherein Y is a chemical bond; and R¹² is morpholinyl, piperidinyl, azetidinyl, pyrrolidinyl, cyclopropyl, cyclobutyl, cyclopentyl, cyclohexyl, piperazinyl, tetrahydrofuranyl, 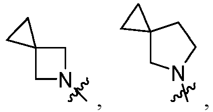 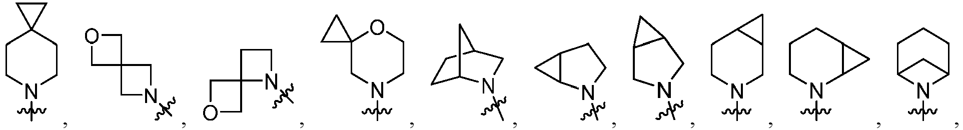 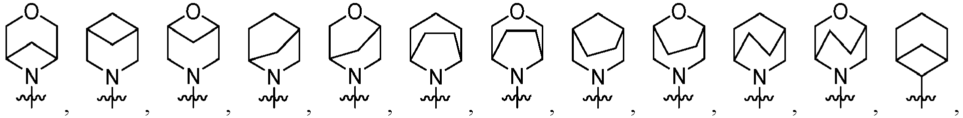 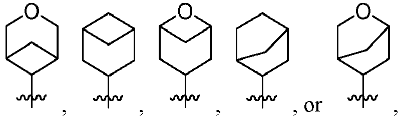 wherein each of the rings is substituted with 0, 1, 2, or 3 groups selected from: F, Cl, Br, methyl, CF₃, -OH, -OCHF₂, CN, and oxo; or (c) a group -Y-R¹², wherein Y is -NH-, -O-, -O-(CH₂)-, -O-(CH₂)-(CH₂)-, or -O-(CH₂)-(CH₂)-(CH₂)-, and R¹² is 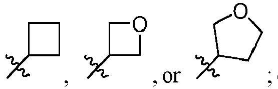 or R¹² is C₁₋₆ hydrocarbyl, wherein the hydrocarbyl may be optionally substituted with 0, 1, 2, 3, 4, or 5 of the following groups: F, Cl, Br, methyl, CF₃, -OH, and CN.

20. The compound, or the isomer, the crystalline form, the pharmaceutically acceptable salt, the hydrate or the solvate thereof according to any one of claims 1-19, wherein, in general formula (1), R² is morpholinyl or piperidinyl, wherein the morpholinyl and piperidinyl may be optionally substituted with 0, 1, 2, or 3 of the following groups: F, Cl, Br, methyl, CF₃, -OH, -OCHF₂, and CN.

21. The compound, or the isomer, the crystalline form, the pharmaceutically acceptable salt, the hydrate or the solvate thereof according to any one of claims 1-20, wherein, in general formula (1), R² is piperidinyl substituted with 1, 2, or 3 fluorine groups.

22. The compound, or the isomer, the crystalline form, the pharmaceutically acceptable salt, the hydrate or the solvate thereof according to any one of claims 1-19, wherein, in general formula (1), R² is:

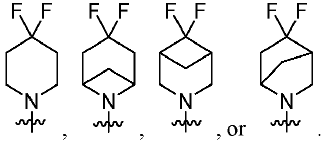

23. The compound, or the isomer, the crystalline form, the pharmaceutically acceptable salt, the hydrate or the solvate thereof according to any one of claims 1-20, wherein, in general formula (1), R² is morpholinyl substituted with 1, 2, or 3 methyl groups.

24. The compound, or the isomer, the crystalline form, the pharmaceutically acceptable salt, the hydrate or the solvate thereof according to any one of claims 1-19, wherein, in general formula (1), R² is 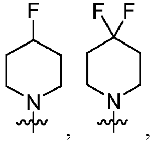 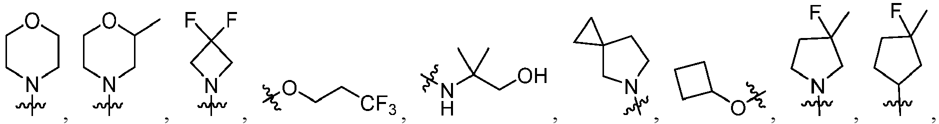 or

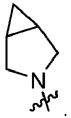

25. The compound, or the isomer, the crystalline form, the pharmaceutically acceptable salt, the hydrate or the solvate thereof according to any one of claims 1-11, wherein, in general formula (1), R¹⁰ is selected from cyclopropyl, cyclobutyl, cyclopentyl, oxetanyl, azetidinyl, tetrahydrofuranyl, and 1,3,4-oxathiazinanyl.

26. The compound, or the isomer, the crystalline form, the pharmaceutically acceptable salt, the hydrate or the solvate thereof according to any one of claims 1-25, wherein, in general formula (1), R³ is H.

27. The compound, or the isomer, the crystalline form, the pharmaceutically acceptable salt, the hydrate or the solvate thereof according to any one of claims 1-26, wherein, in general formula (1), R⁴ is selected from: (a) H; (b) C₁₋₆ hydrocarbyl substituted with 0, 1, 2, or 3 OH groups; (c) cyclopropyl; and (d) F; R⁴ is preferably H, F, or methyl; R⁴ is more preferably H.

28. The compound, or the isomer, the crystalline form, the pharmaceutically acceptable salt, the hydrate or the solvate thereof according to any one of claims 1-27, wherein, in general formula (1), R⁵ is H or F, preferably H.

29. The compound, or the isomer, the crystalline form, the pharmaceutically acceptable salt, the hydrate or the solvate thereof according to any one of claims 1-28, wherein, in general formula (1), R⁶ is H or F, preferably H.

30. The compound, or the isomer, the crystalline form, the pharmaceutically acceptable salt, the hydrate or the solvate thereof according to any one of claims 1-29, wherein, in general formula (1), R⁷ is H.

31. The compound, or the isomer, the crystalline form, the pharmaceutically acceptable salt, the hydrate or the solvate thereof according to any one of claims 1-30, wherein, in general formula (1), R¹⁵ is H or F, preferably H.

32. The compound, or the isomer, the crystalline form, the pharmaceutically acceptable salt, the hydrate or the solvate thereof according to any one of claims 1-31, wherein the compound has one of the following structures:

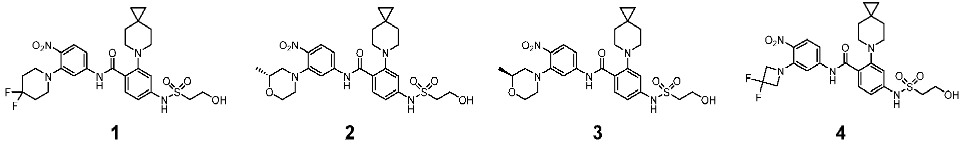

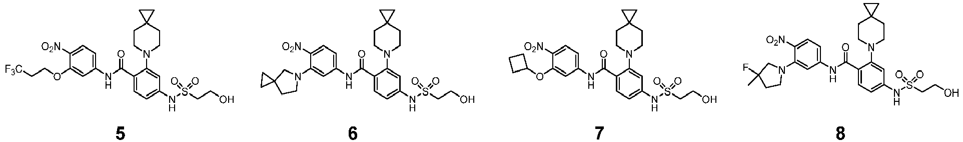

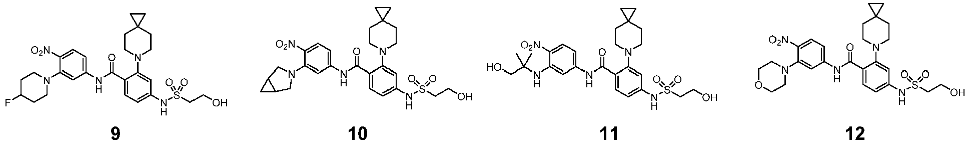

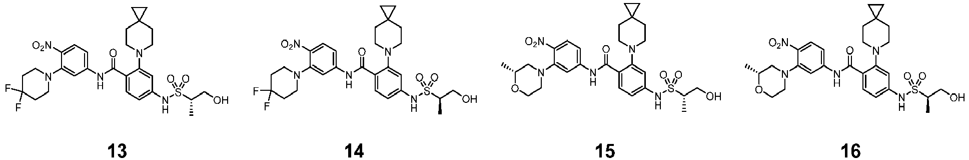

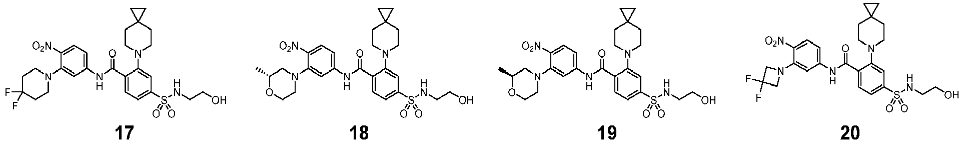

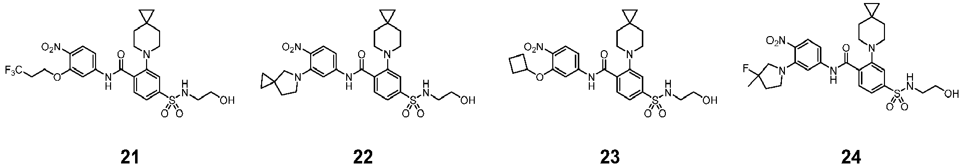

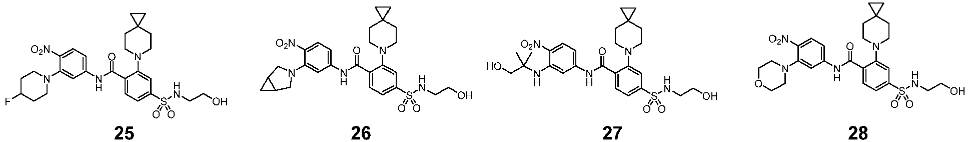

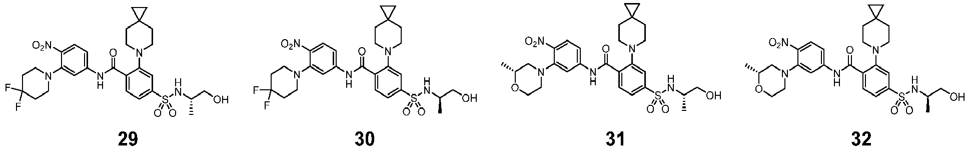

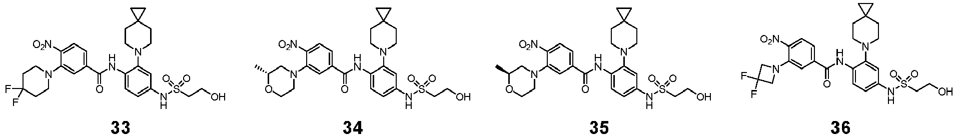

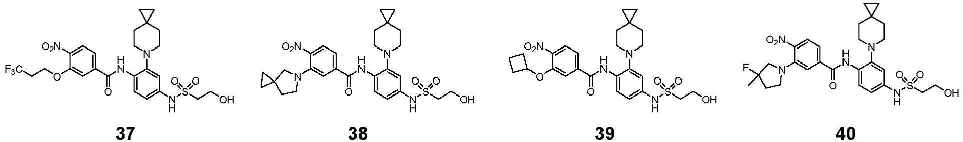

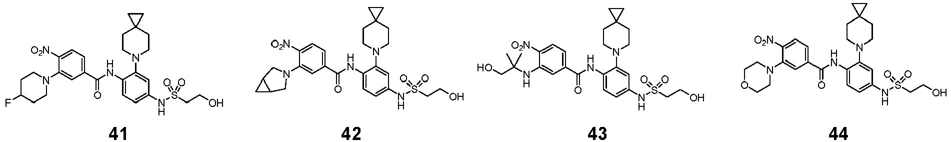

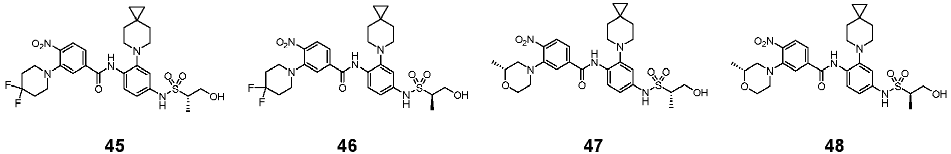

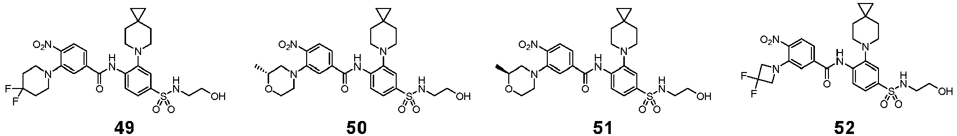

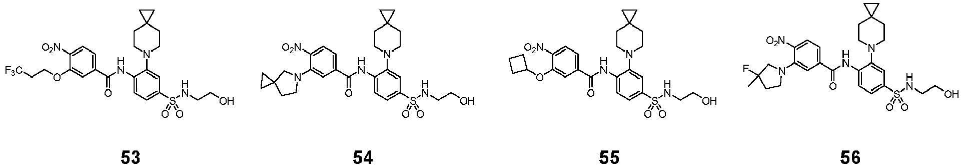

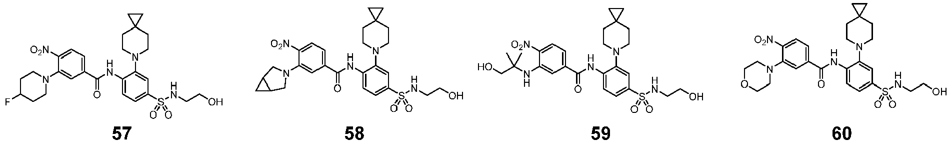

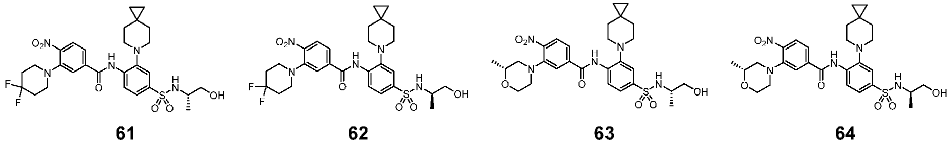

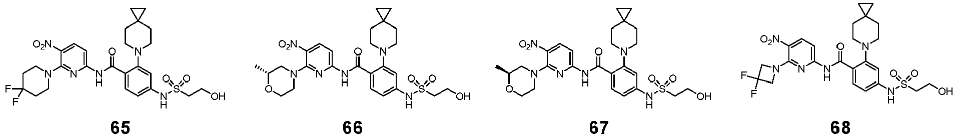

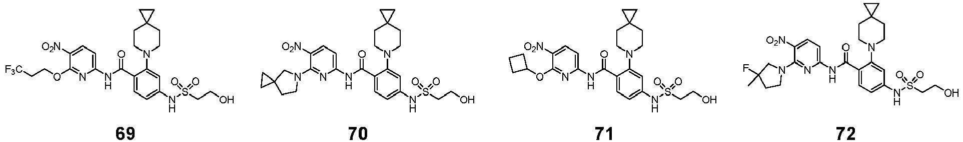

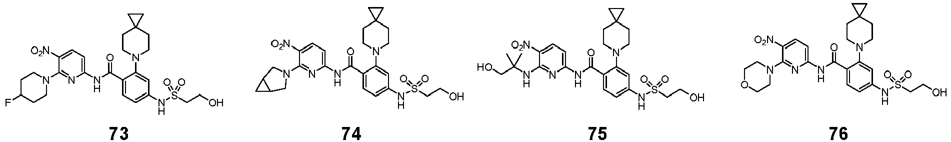

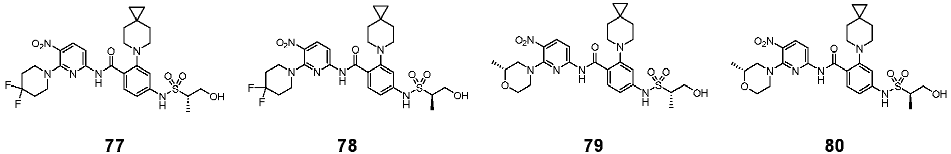

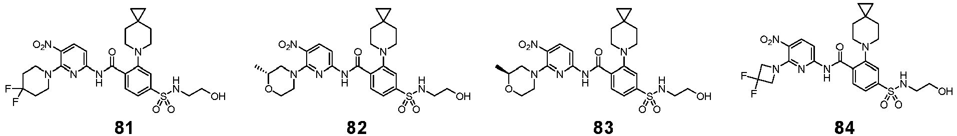

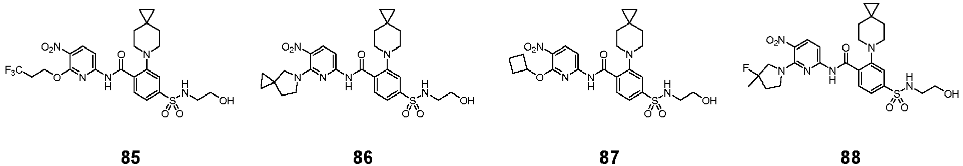

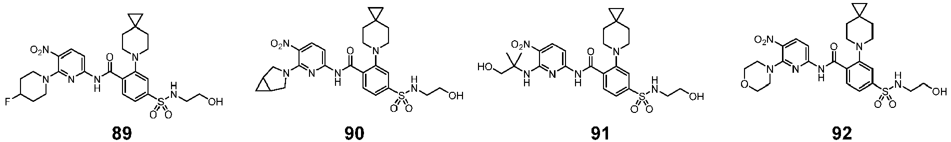

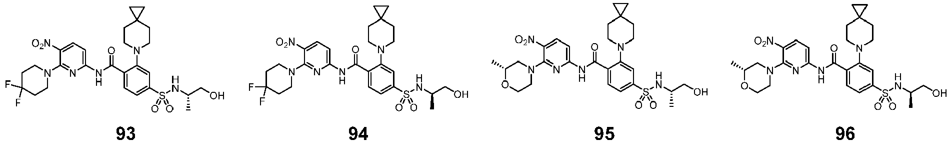

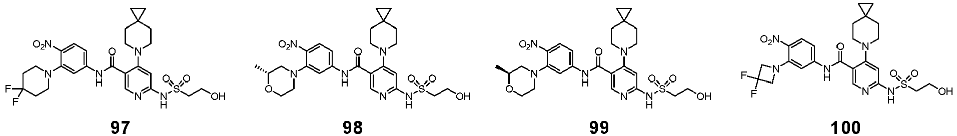

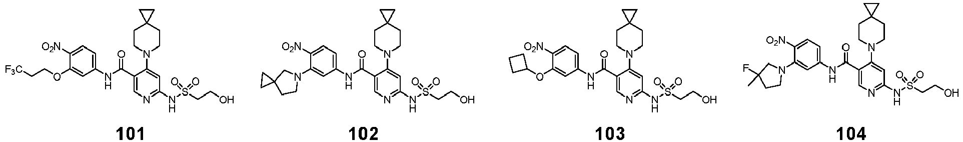

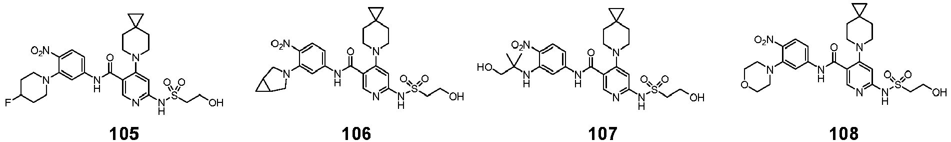

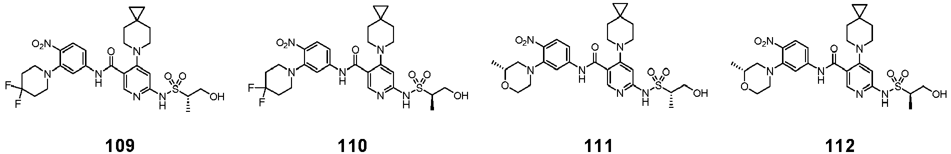

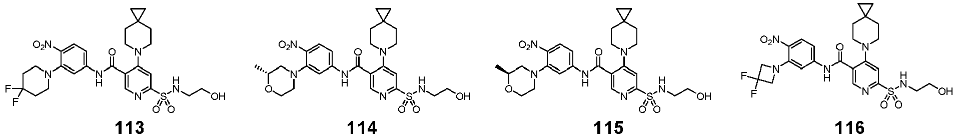

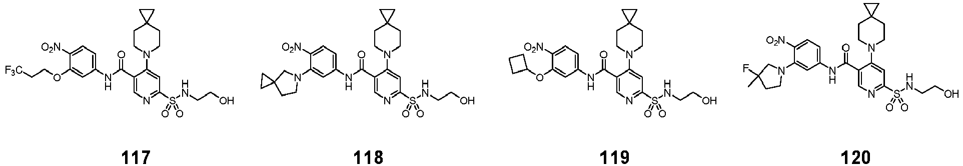

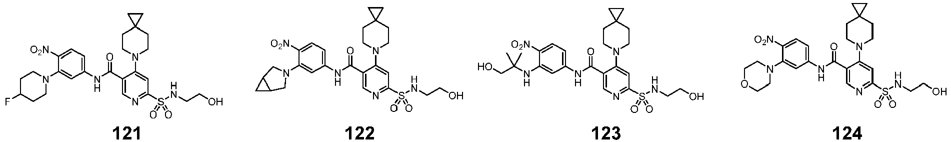

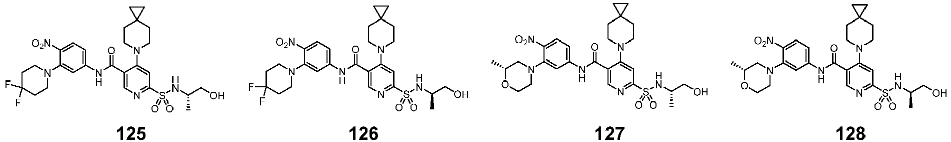

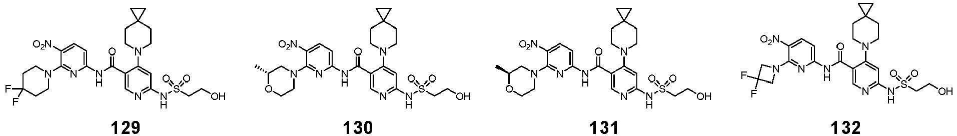

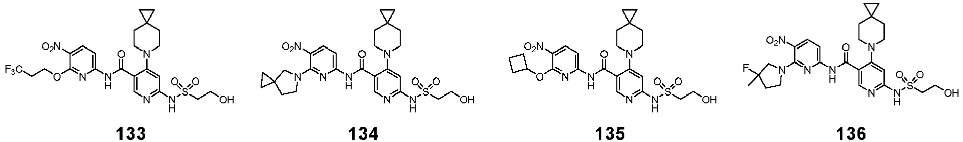

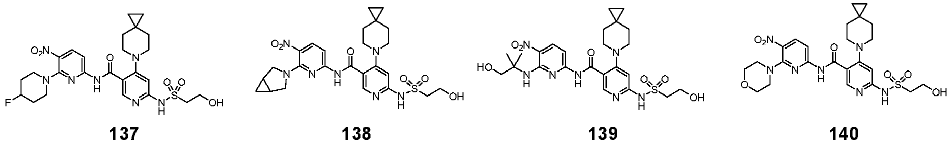

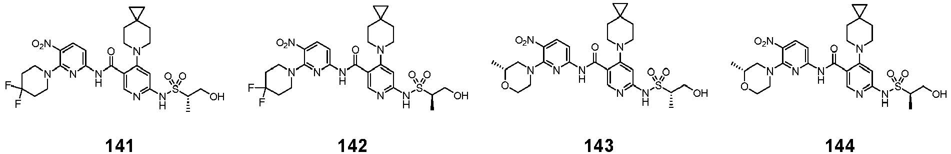

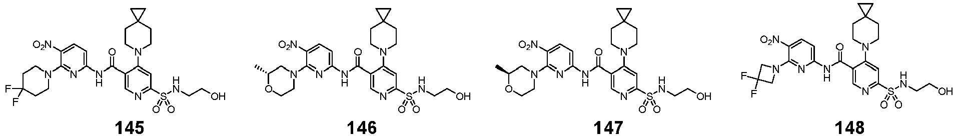

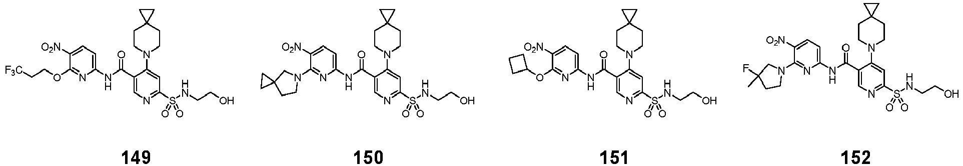

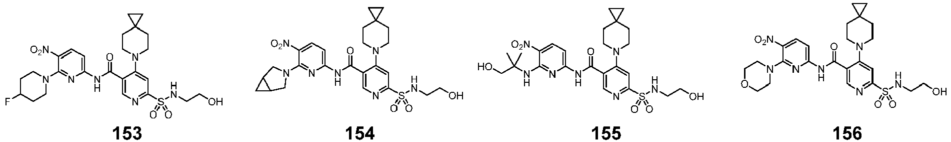

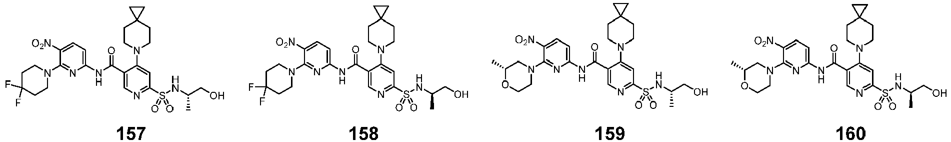

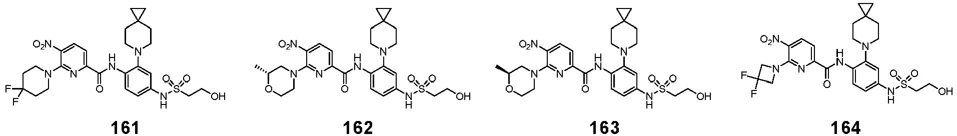

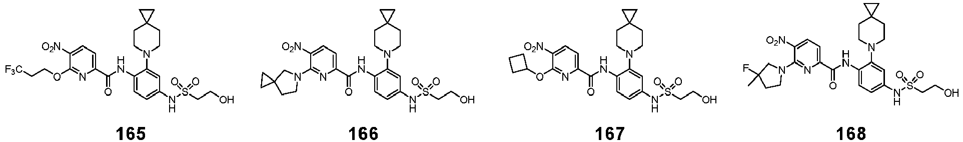

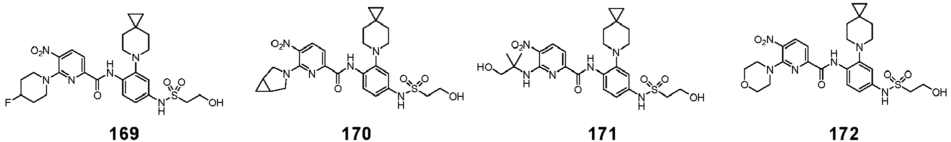

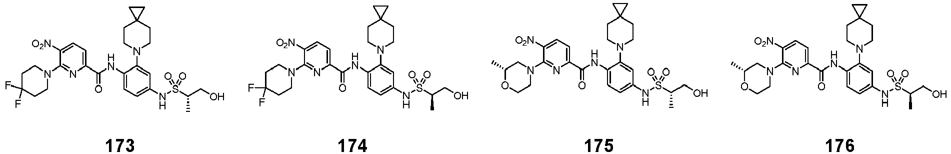

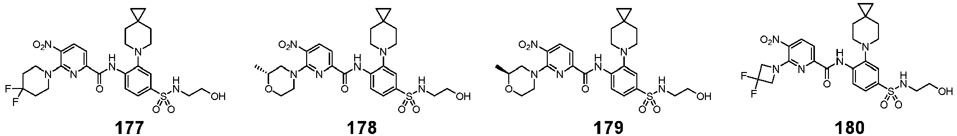

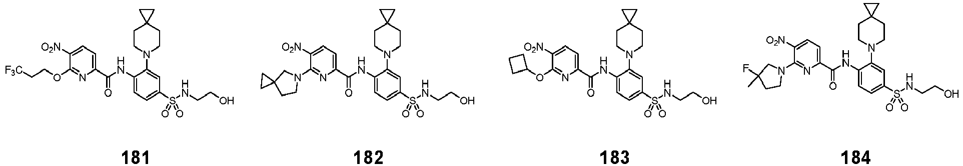

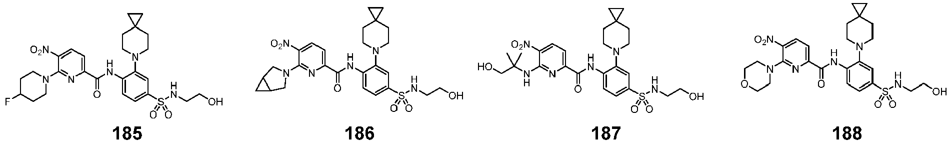

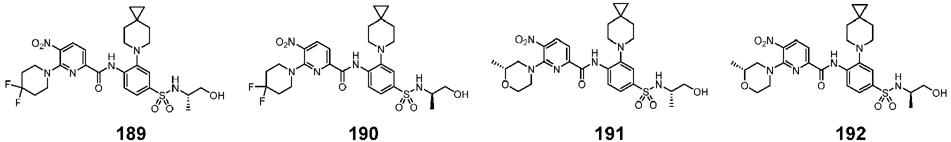

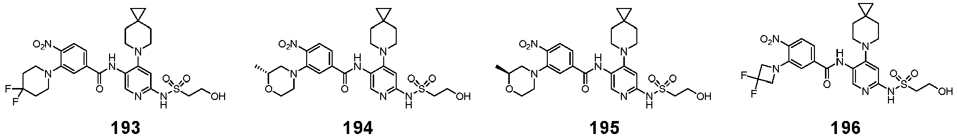

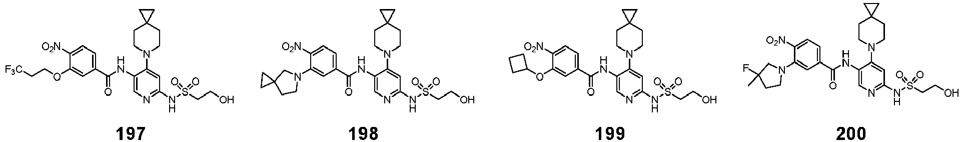

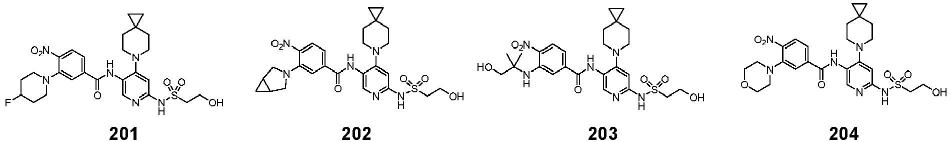

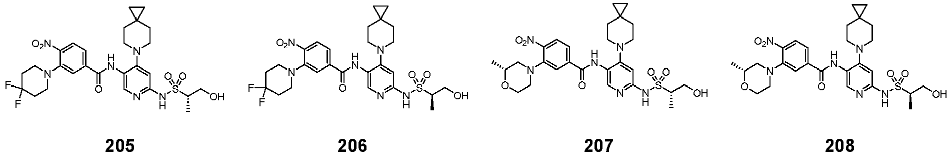

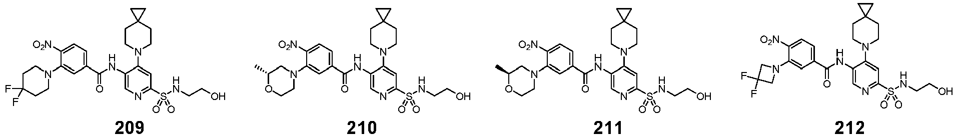

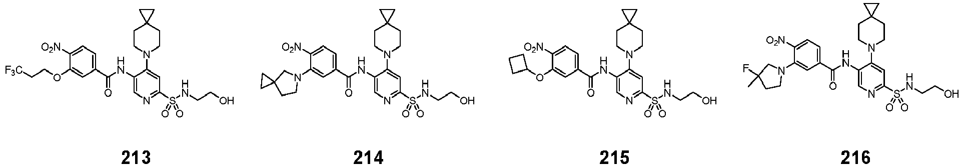

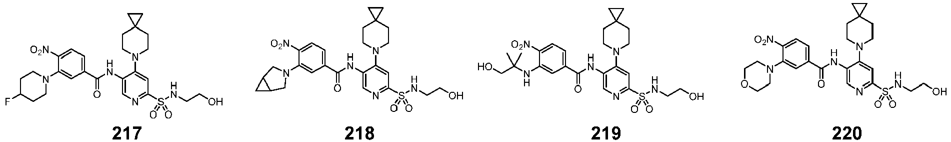

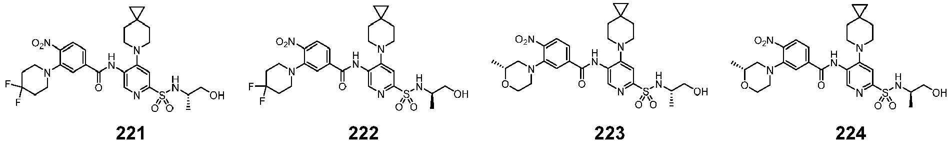

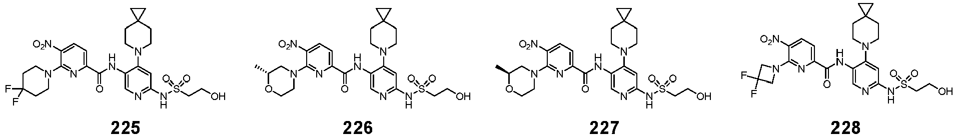

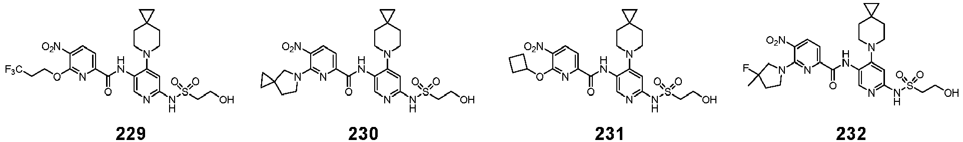

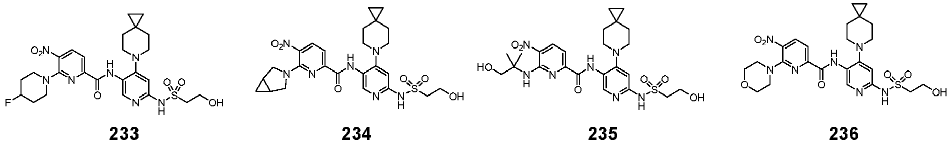

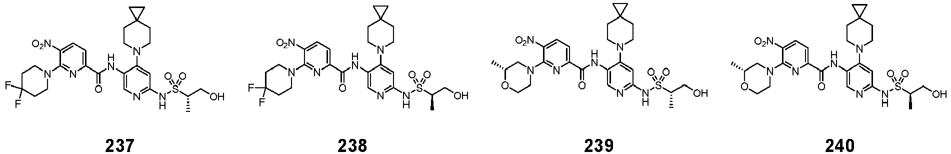

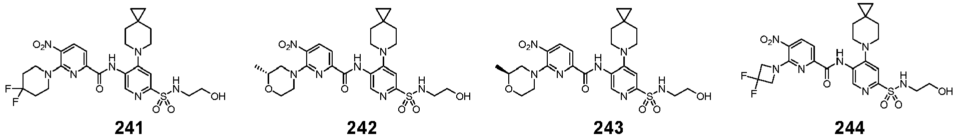

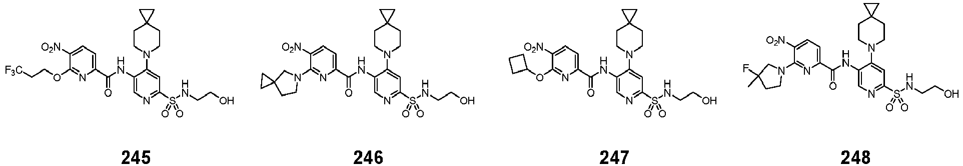

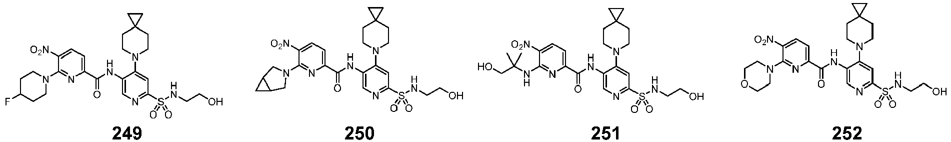

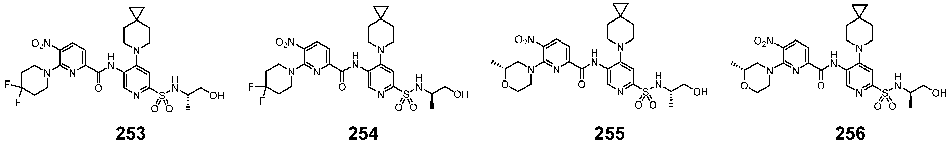

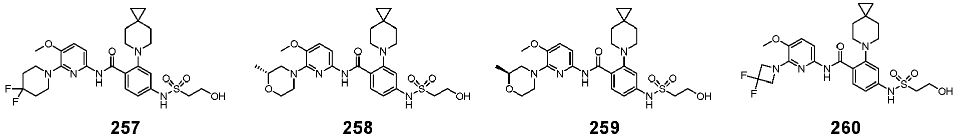

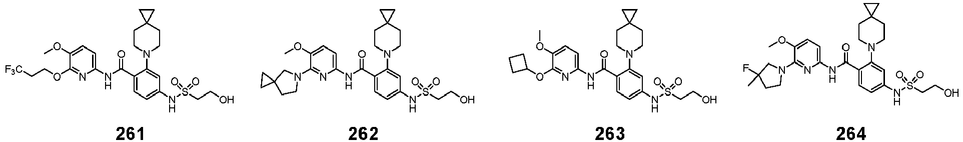

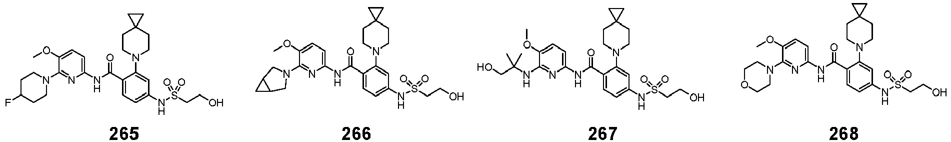

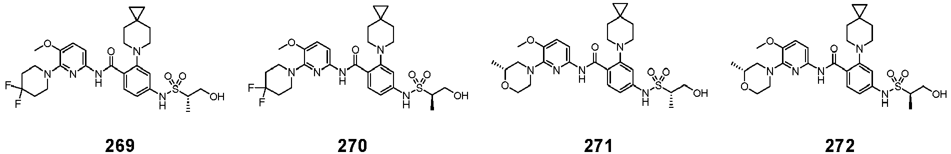

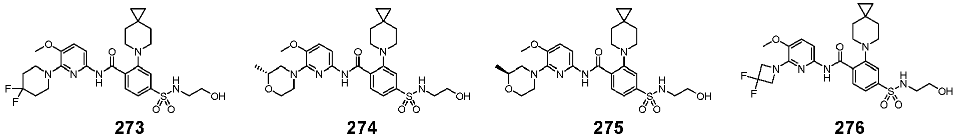

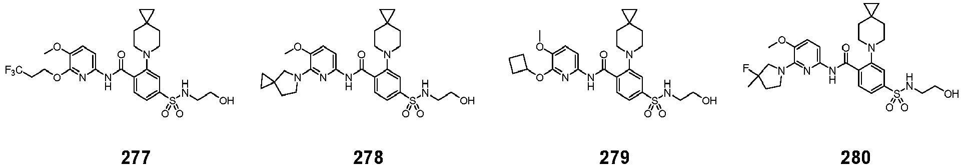

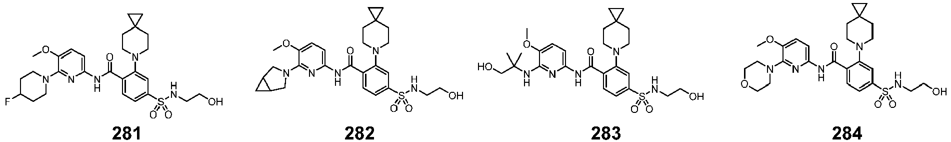

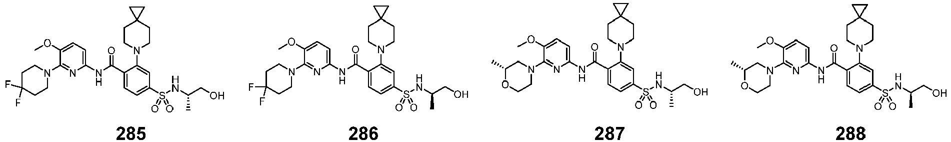

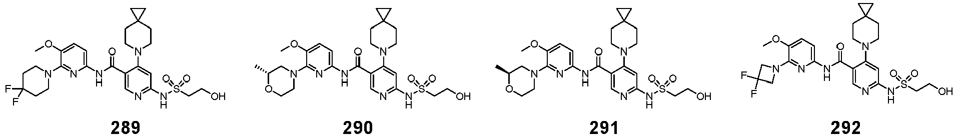

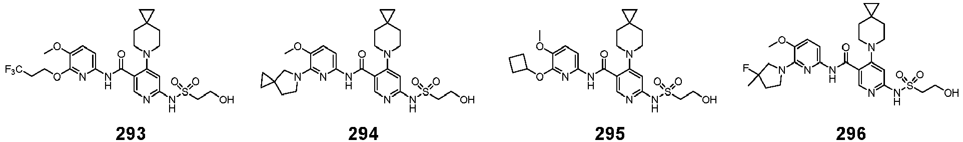

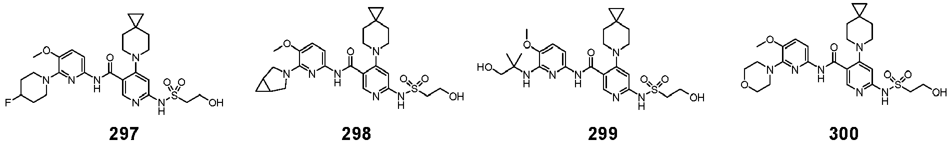

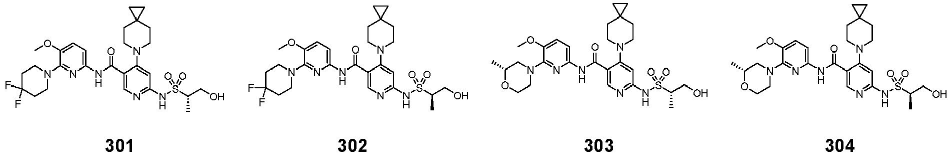

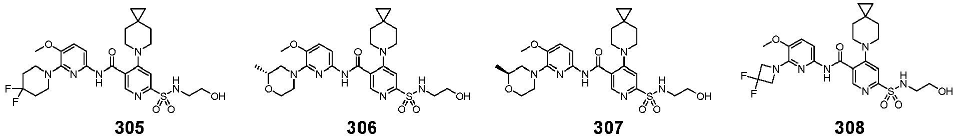

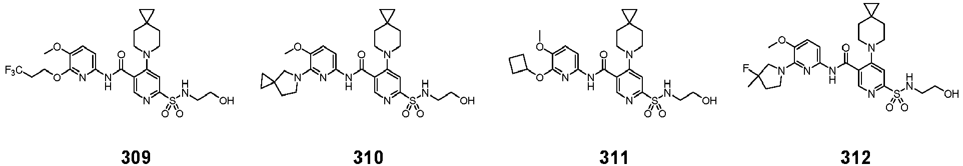

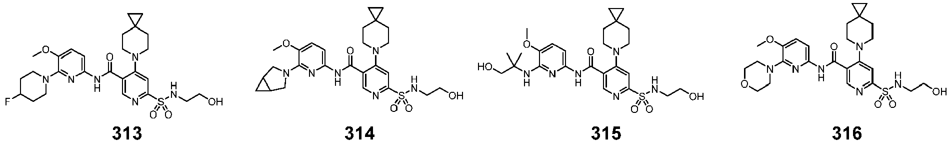

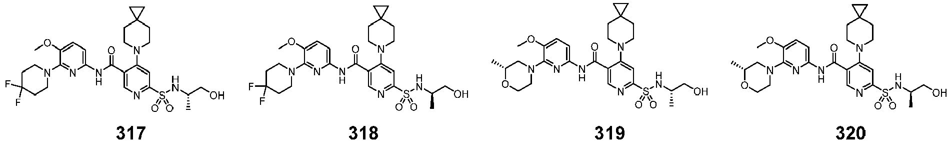

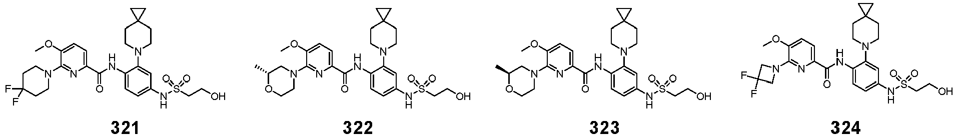

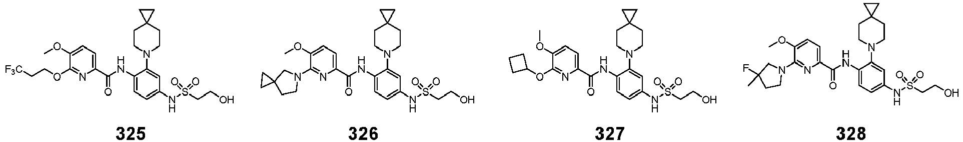

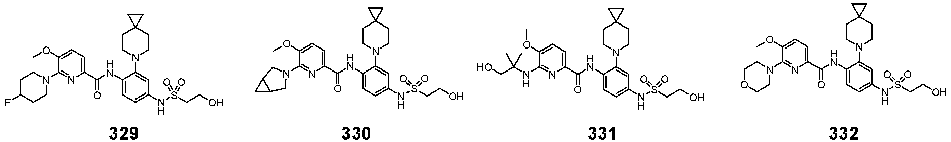

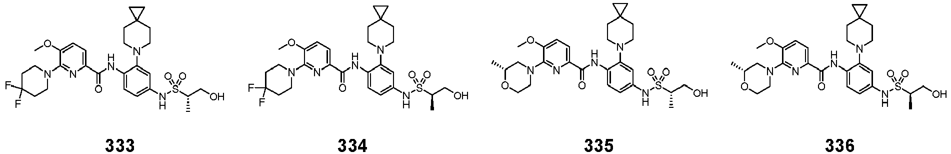

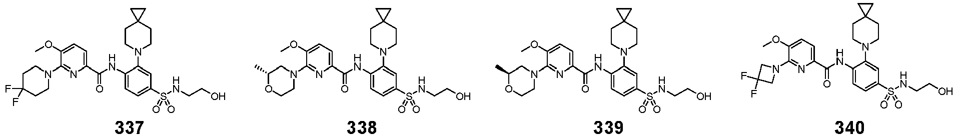

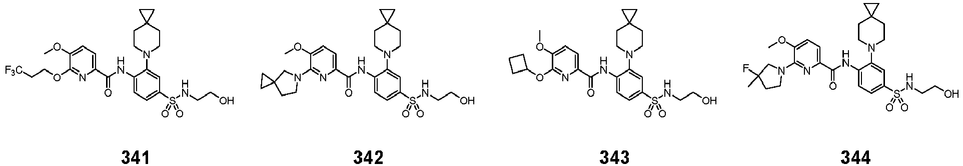

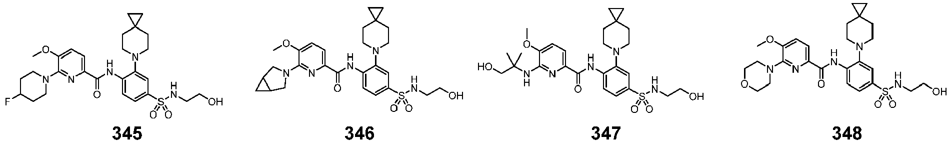

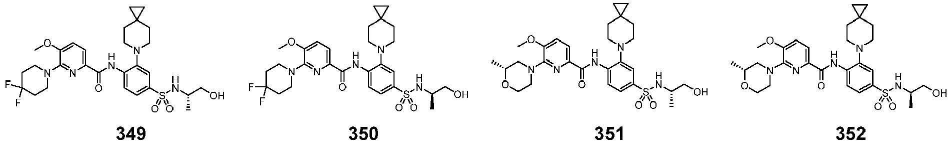

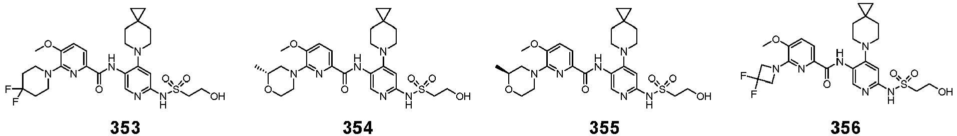

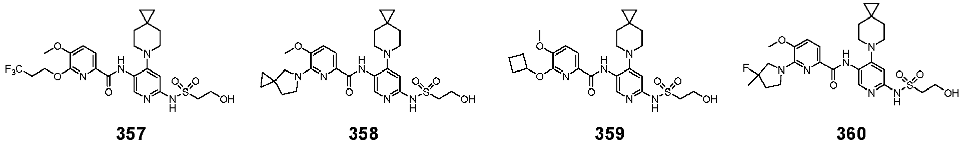

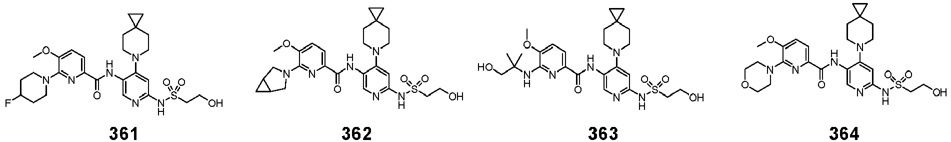

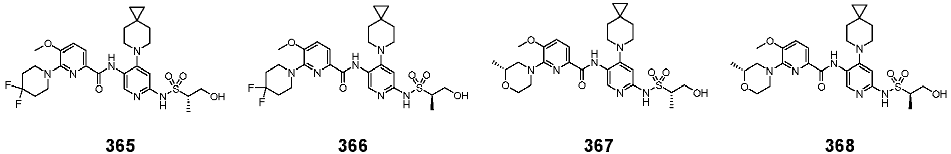

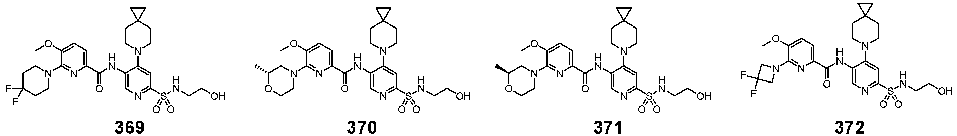

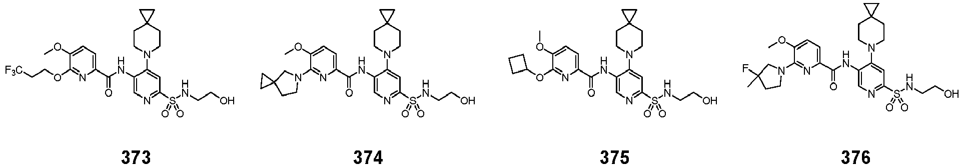

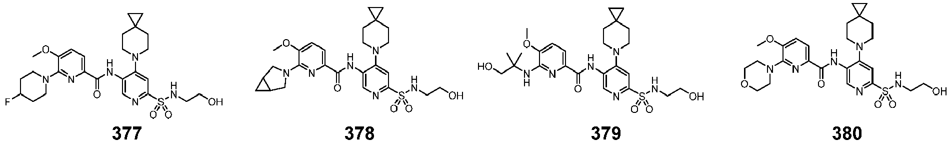

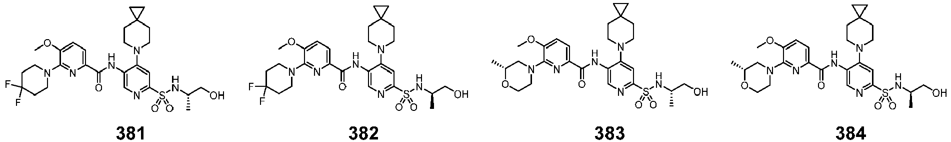

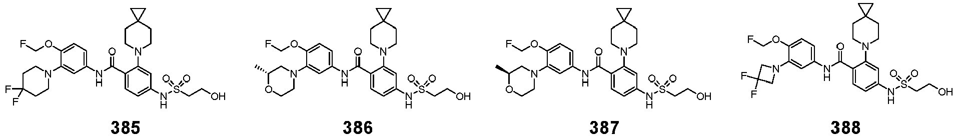

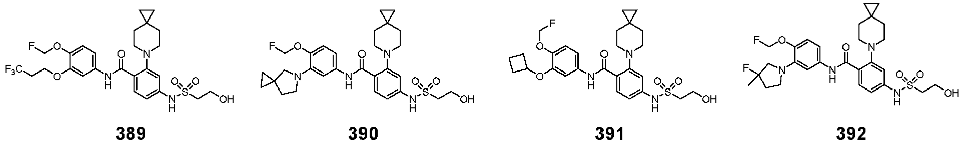

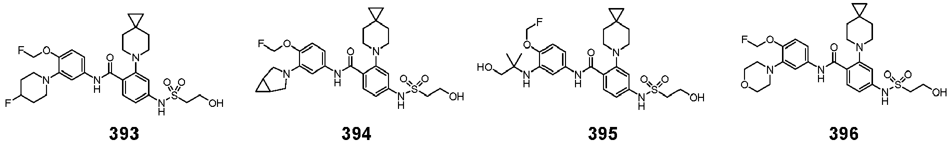

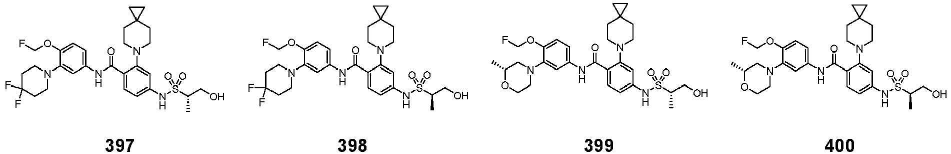

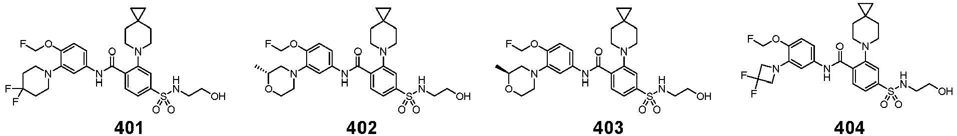

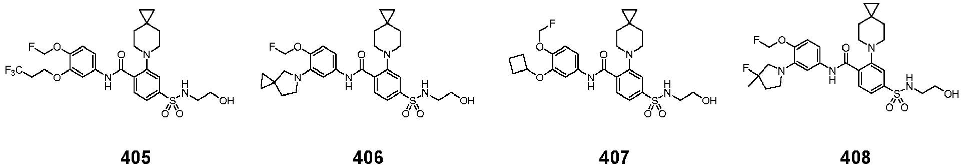

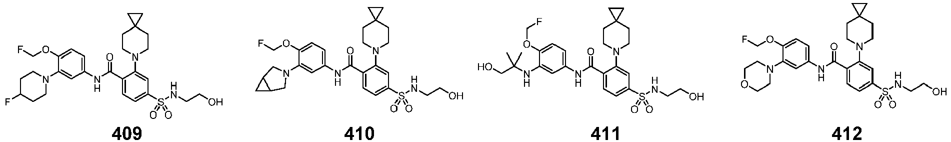

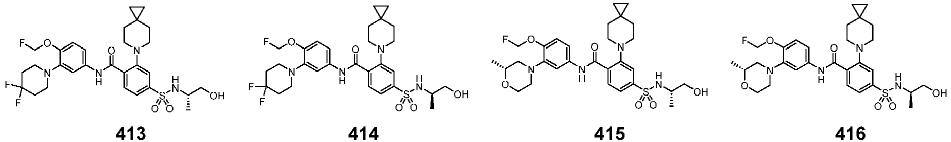

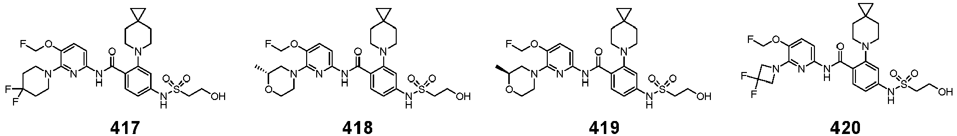

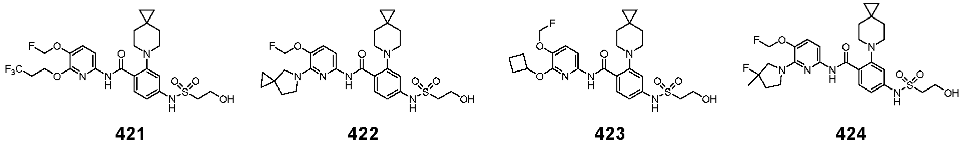

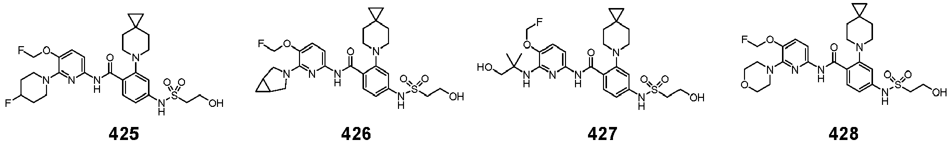

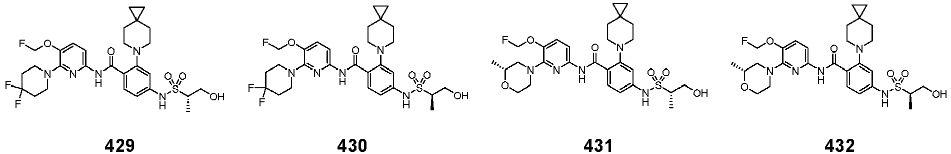

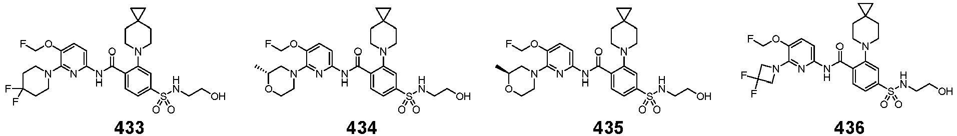

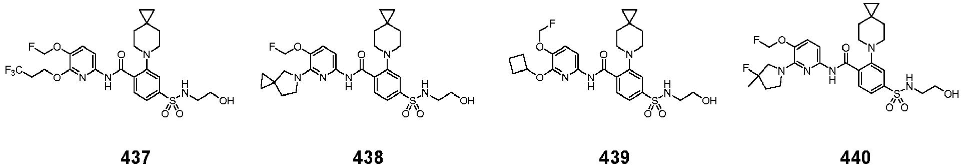

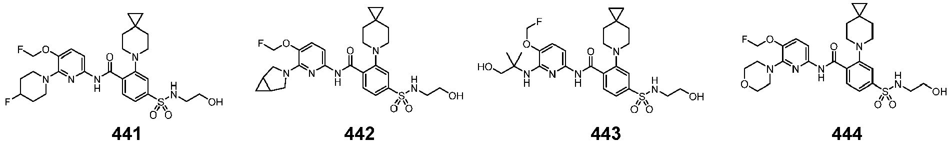

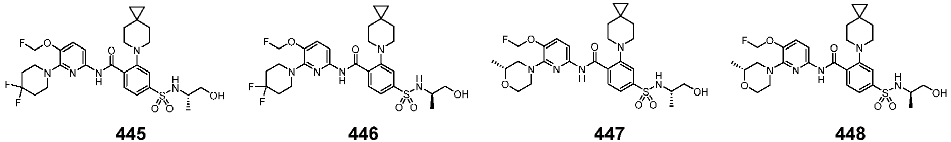

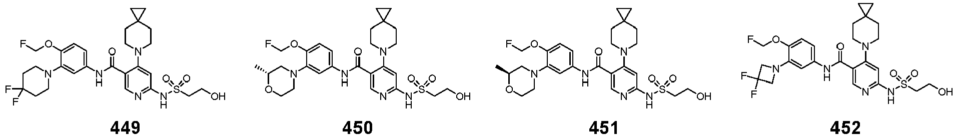

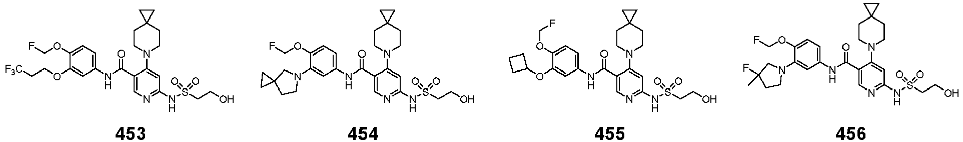

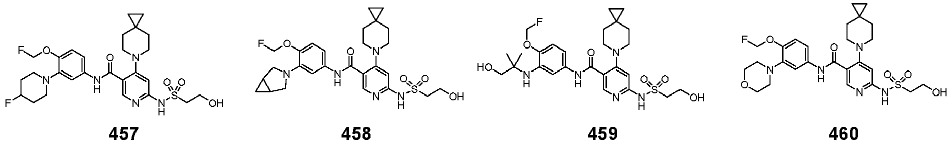

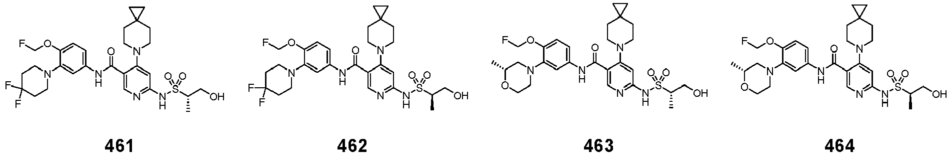

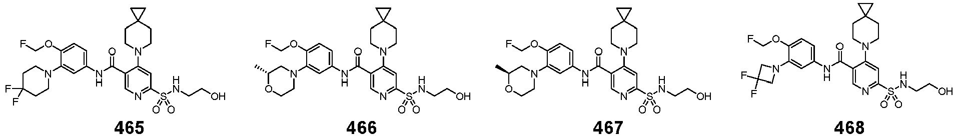

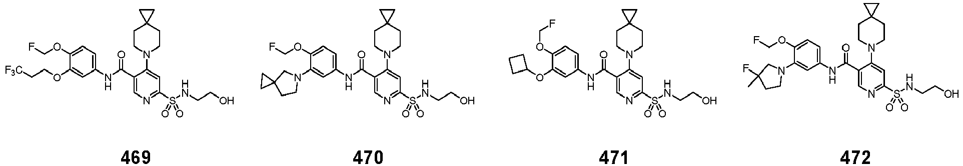

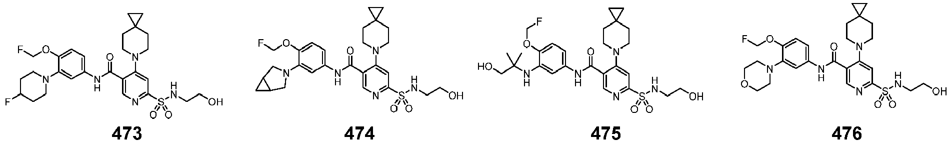

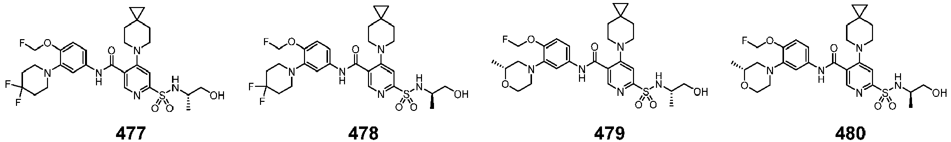

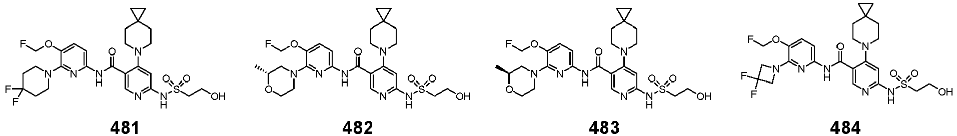

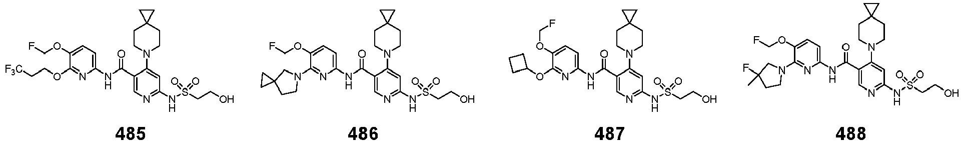

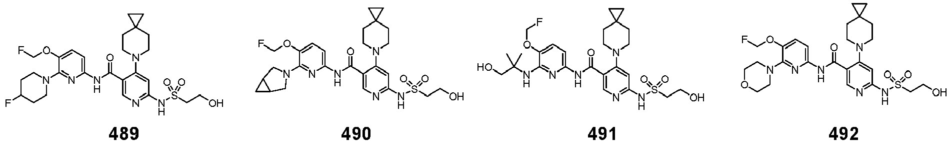

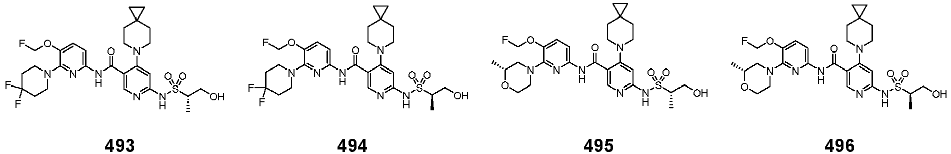

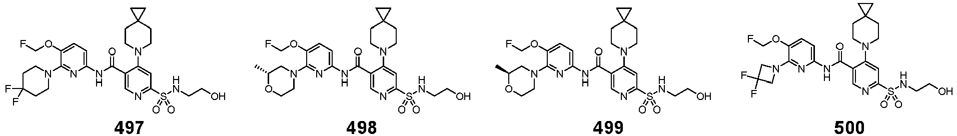

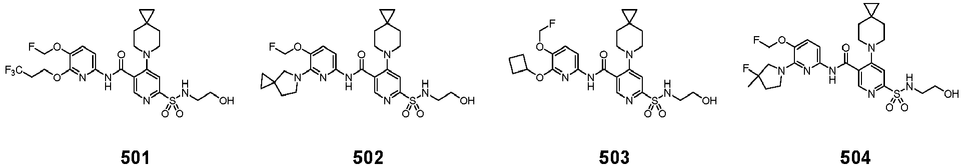

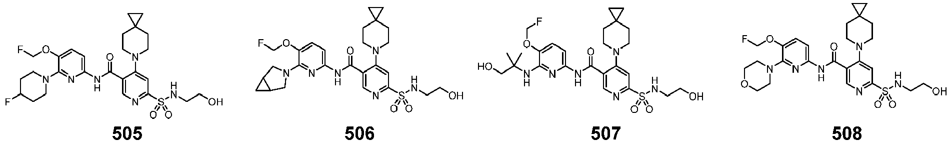

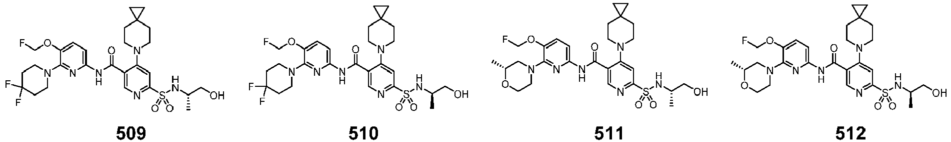

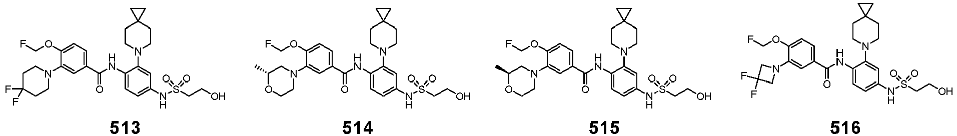

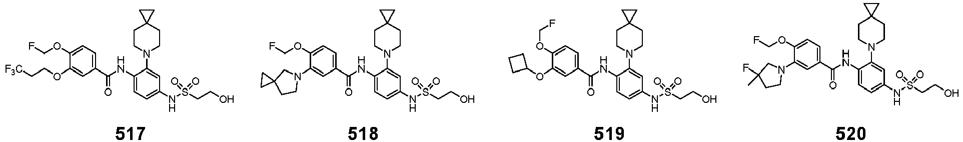

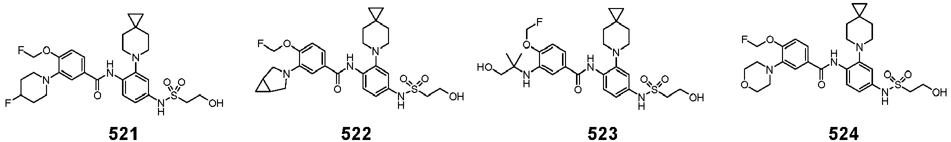

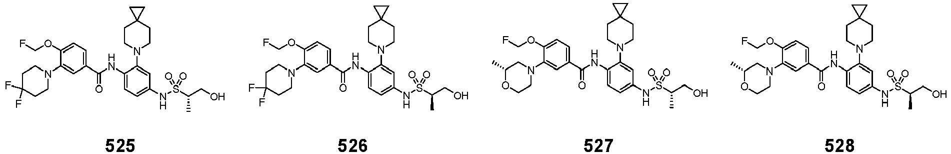

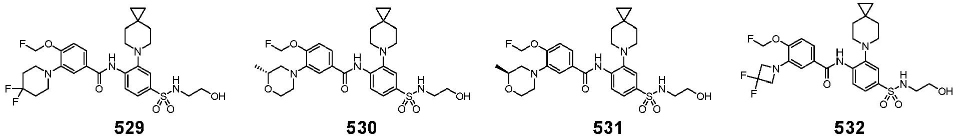

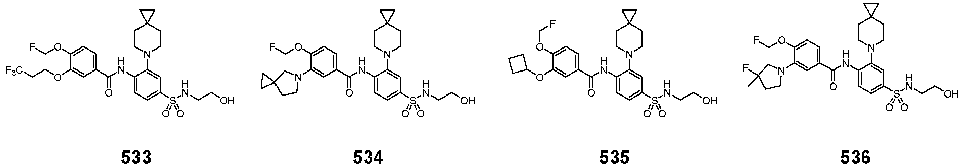

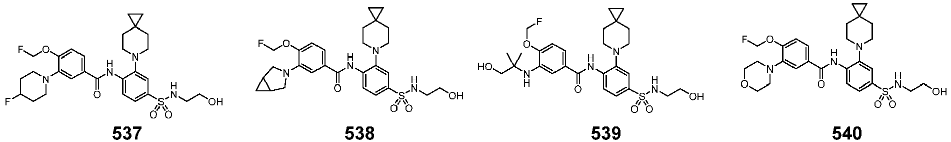

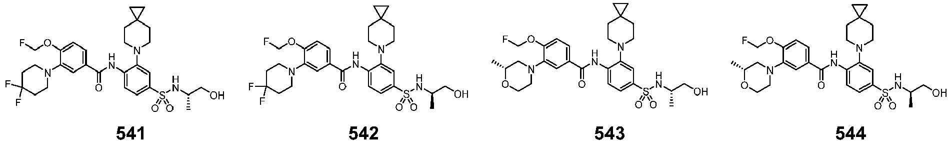

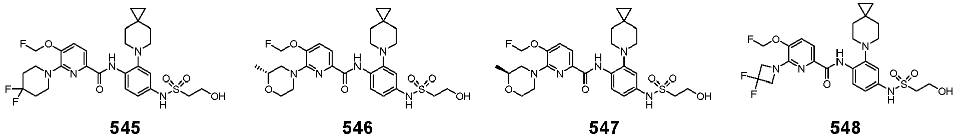

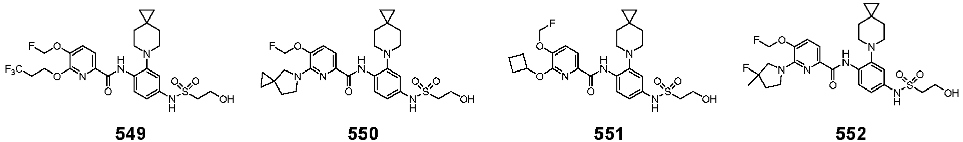

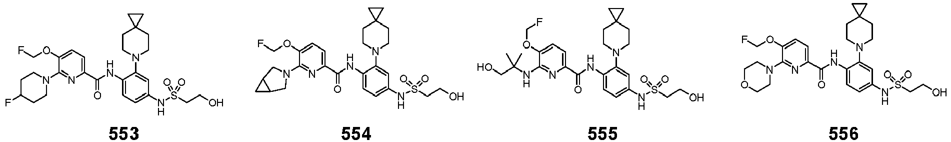

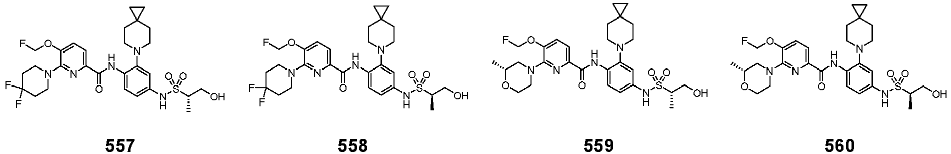

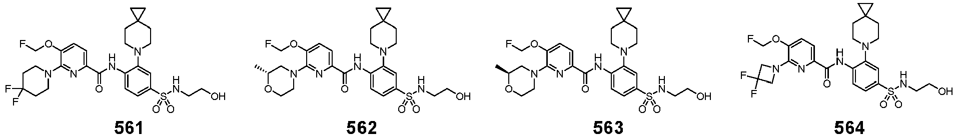

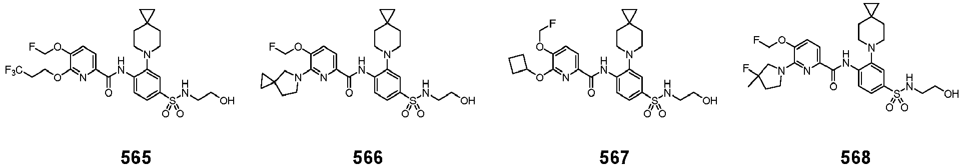

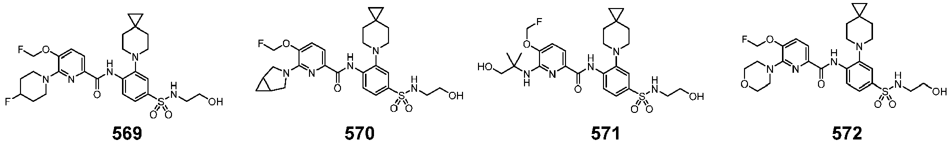

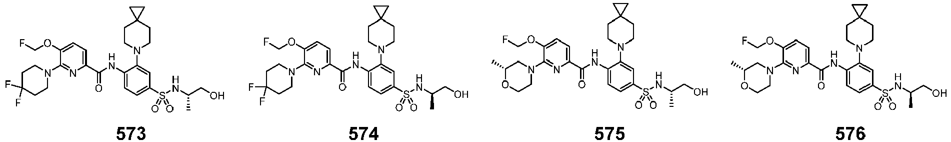

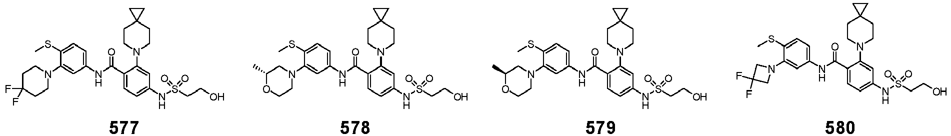

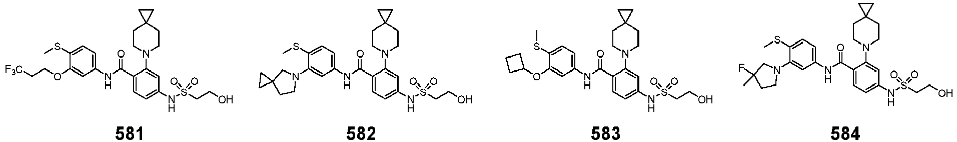

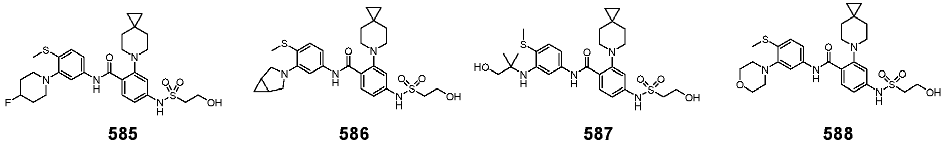

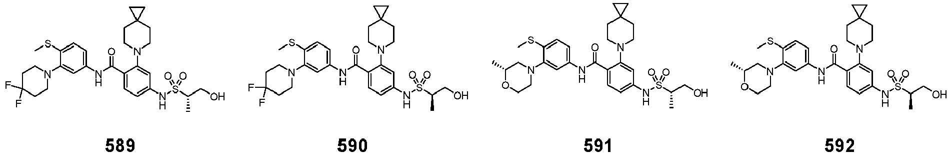

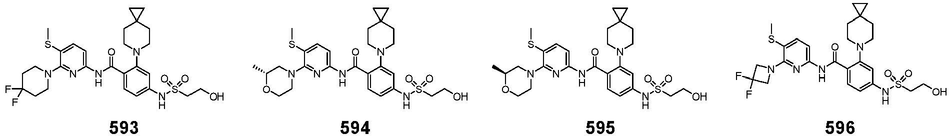

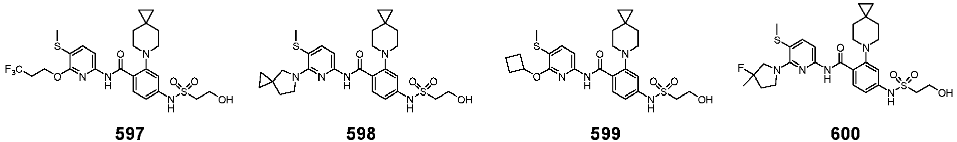

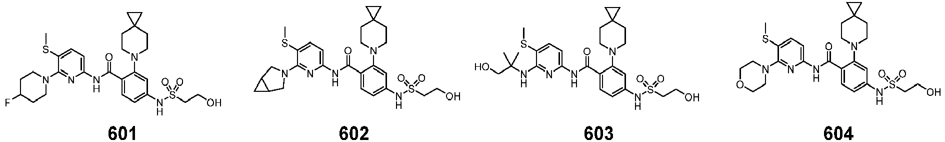

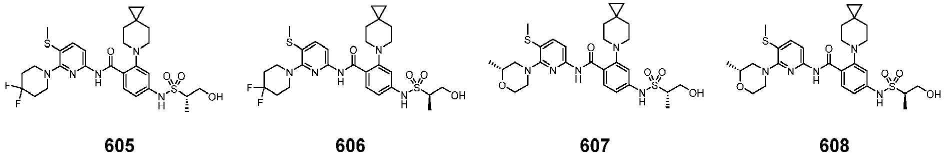

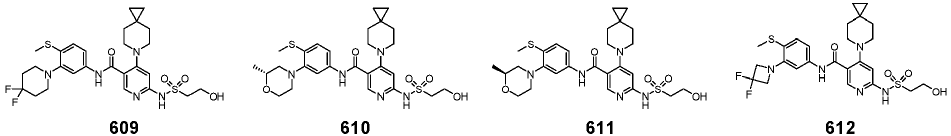

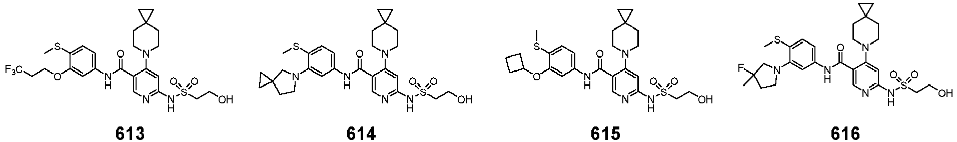

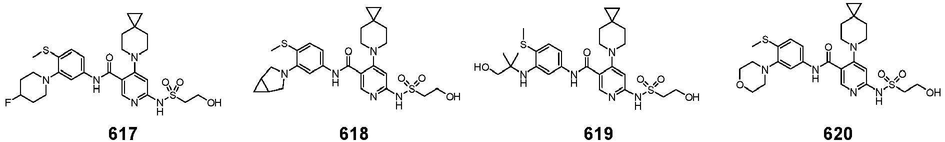

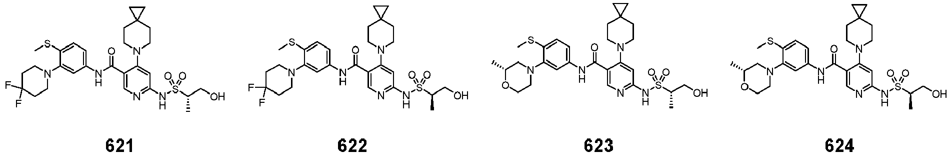

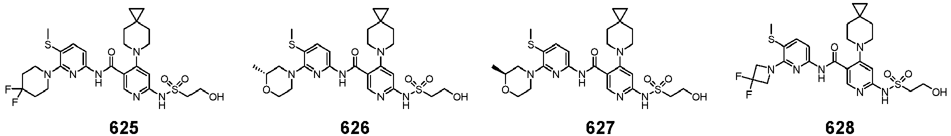

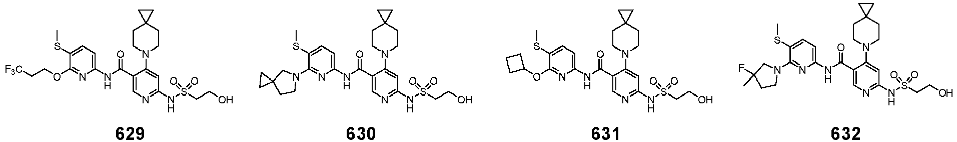

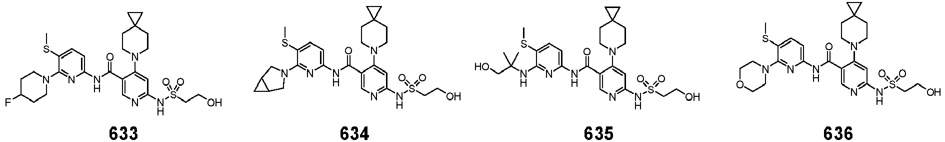

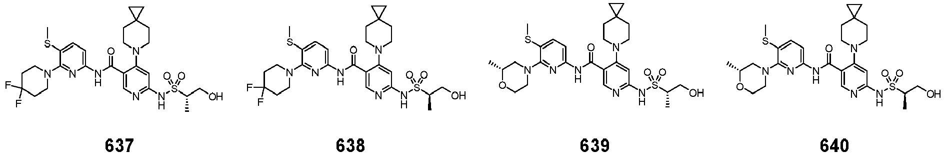

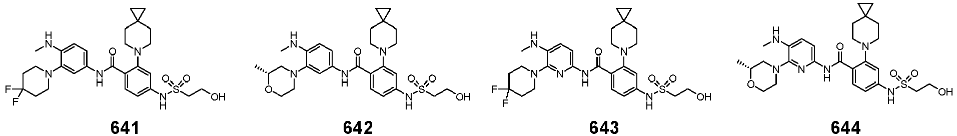

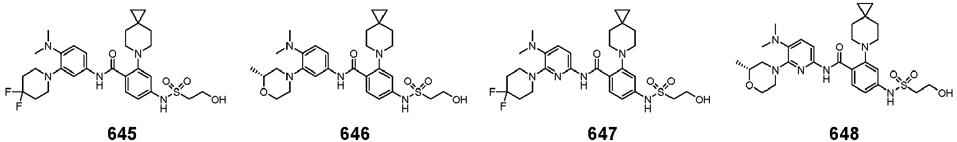

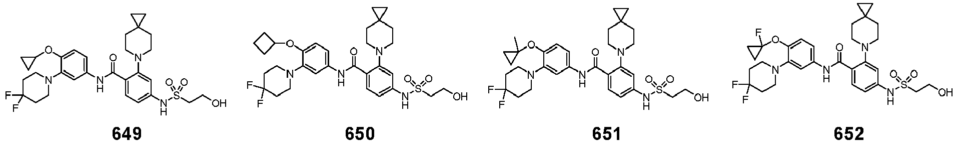

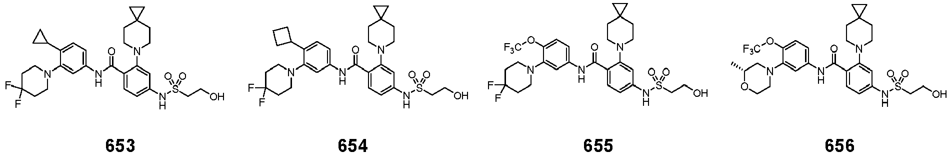

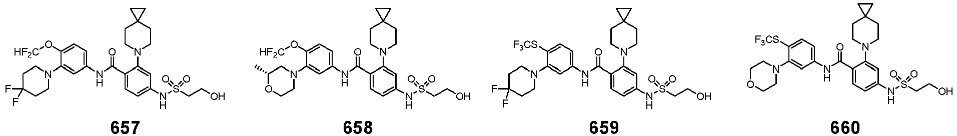

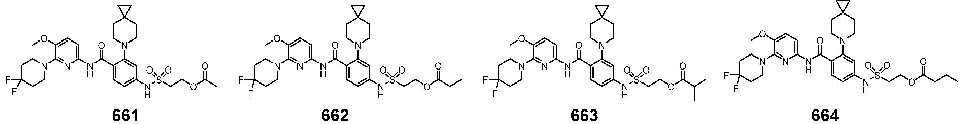

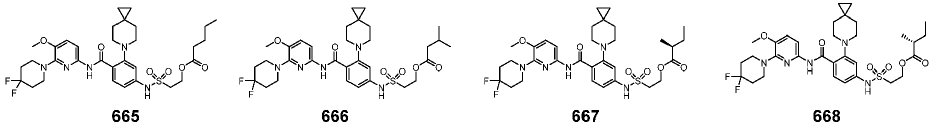

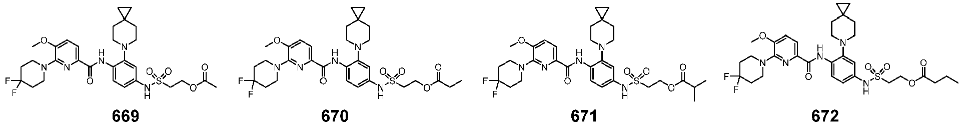

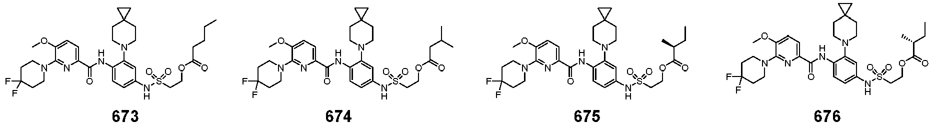

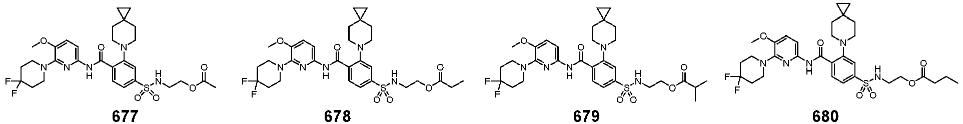

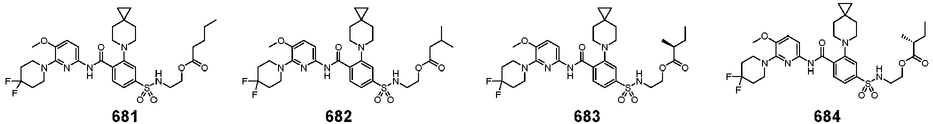

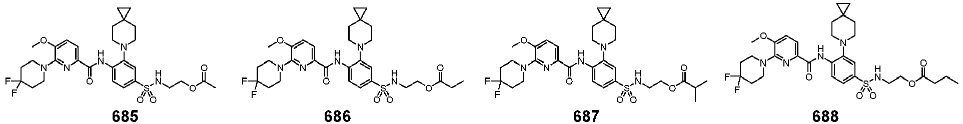

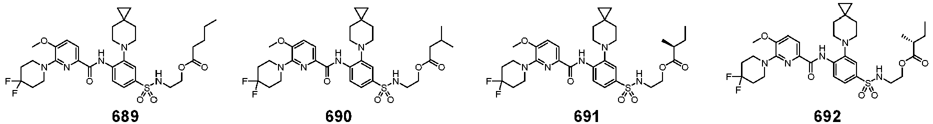

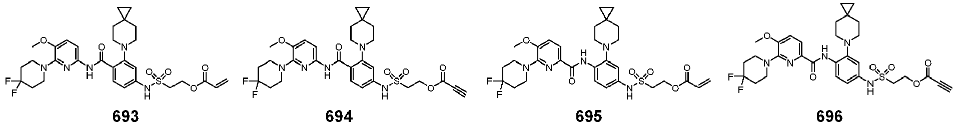

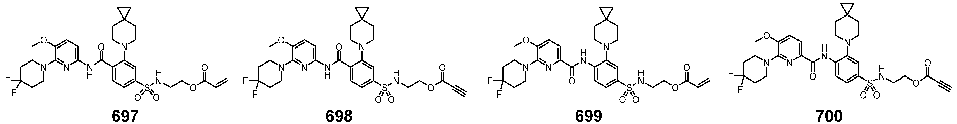

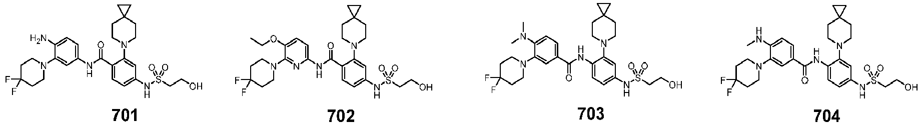

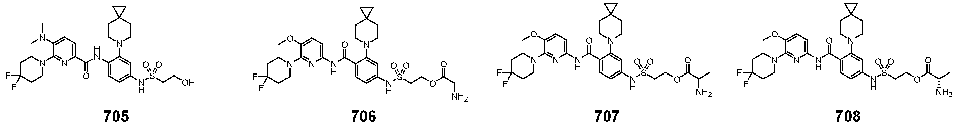

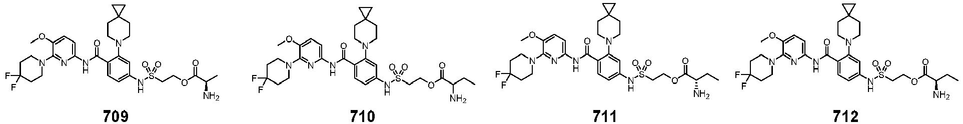

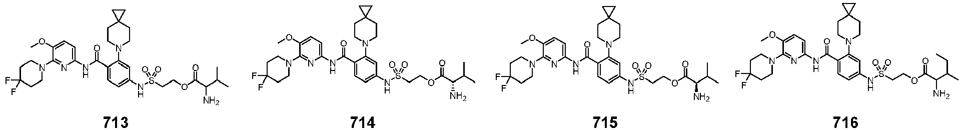

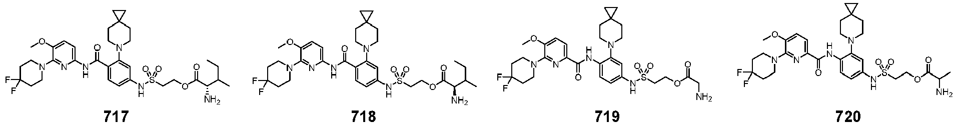

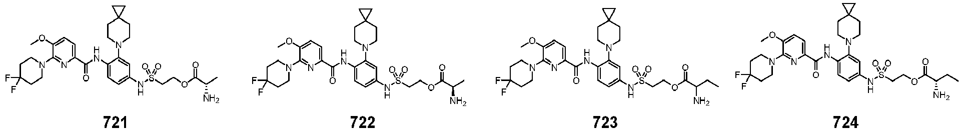

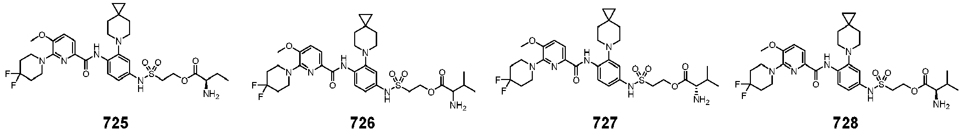

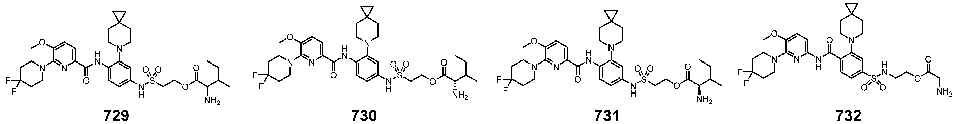

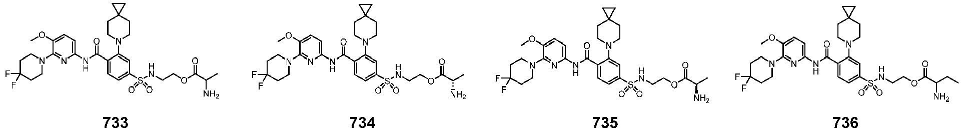

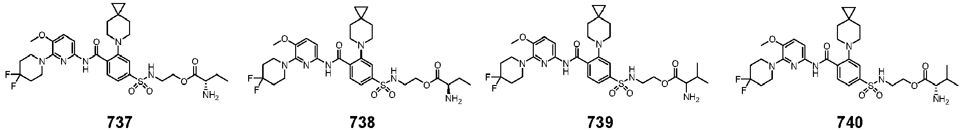

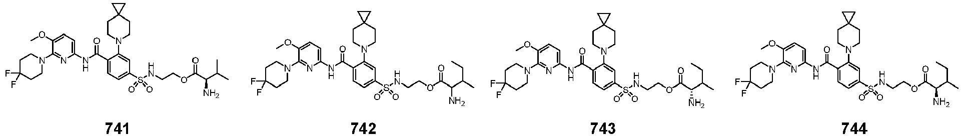

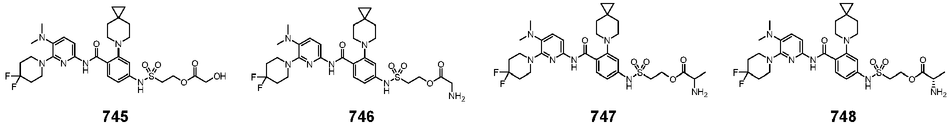

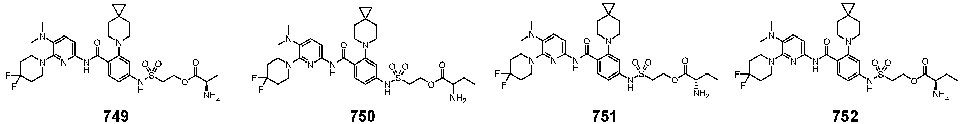

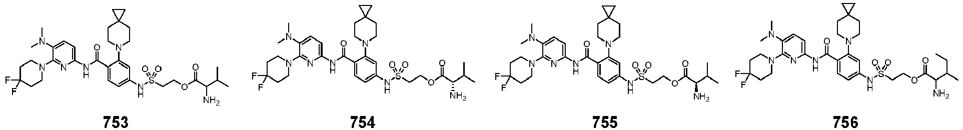

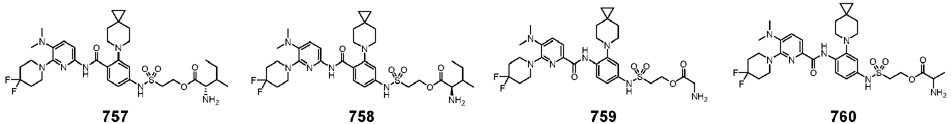

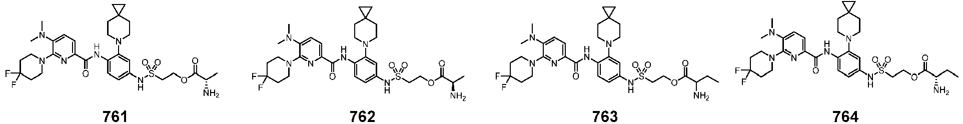

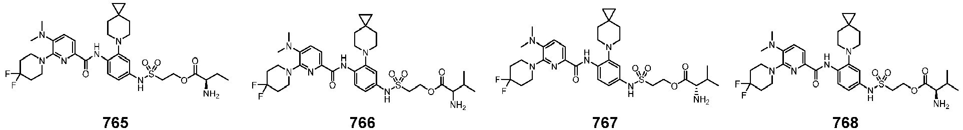

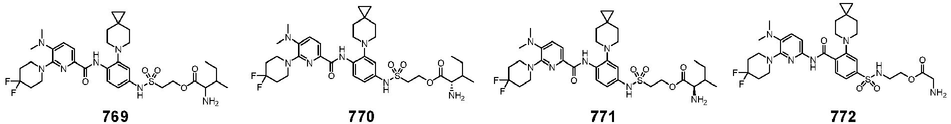

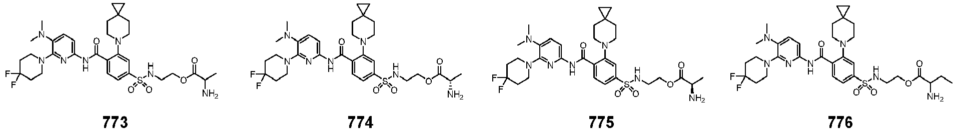

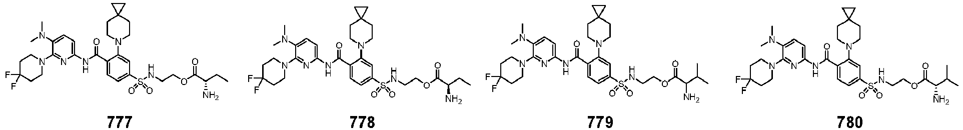

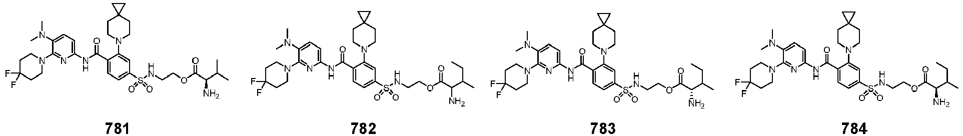

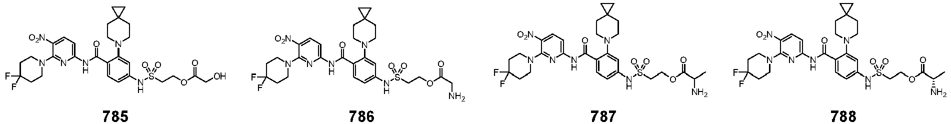

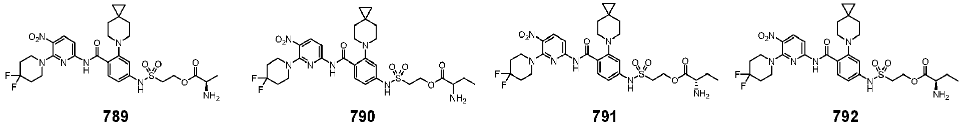

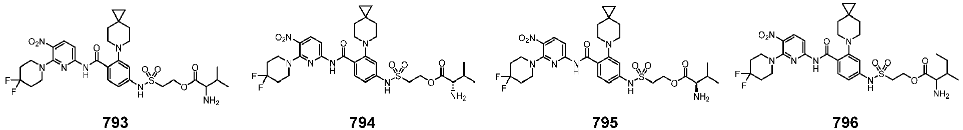

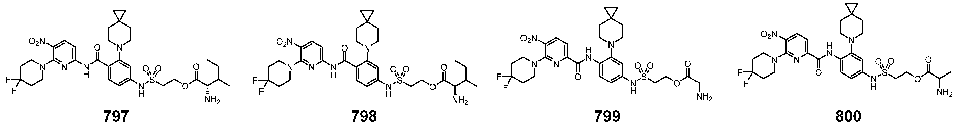

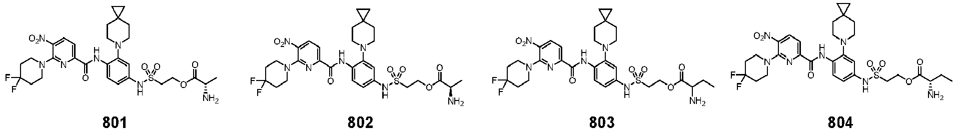

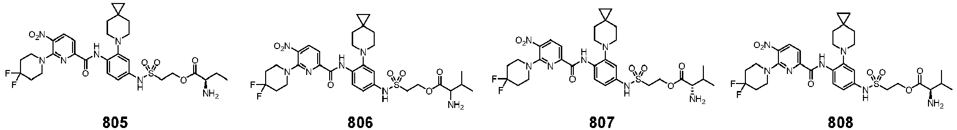

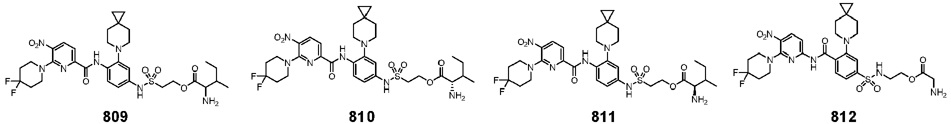

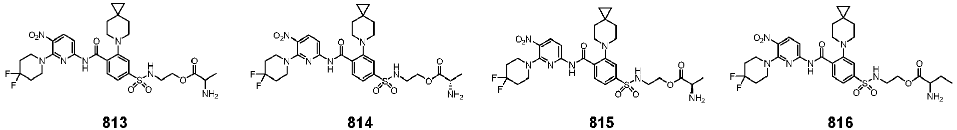

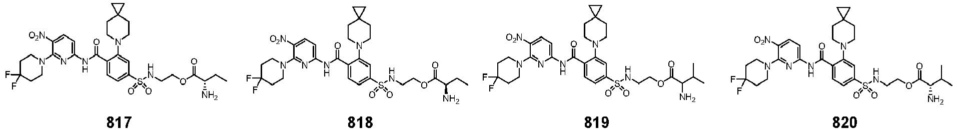

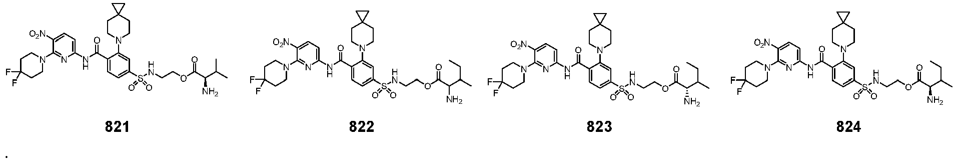

33. The compound, or the isomer, the crystalline form, the pharmaceutically acceptable salt, the hydrate or the solvate thereof according to any one of claims 1-31, wherein the compound has one of the following structures: 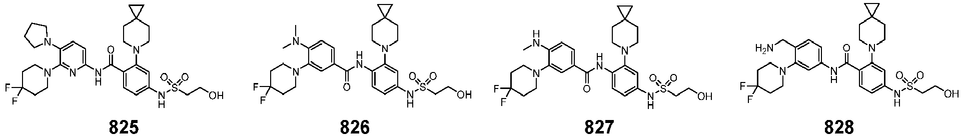 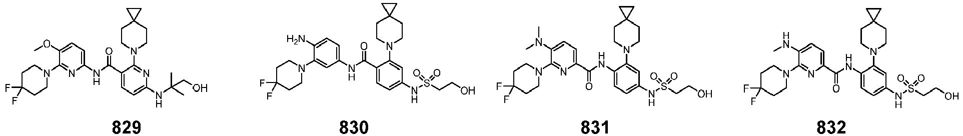 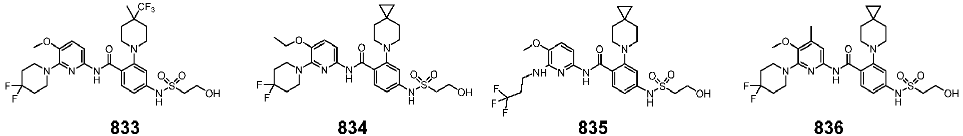 or

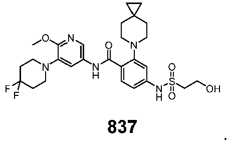

34. A pharmaceutical composition, comprising a pharmaceutically acceptable excipient or carrier, and the compound, or the isomer, the crystalline form, the pharmaceutically acceptable salt, the hydrate or the solvate thereof according to any one of claims 1-33 as an active ingredient.

35. Use of the compound, or the isomer, the crystalline form, the pharmaceutically acceptable salt, the hydrate or the solvate thereof according to any one of claims 1-33, or the pharmaceutical composition according to claim 34 in preparing a medicament for treating a related disease mediated by KIF18A protein.

36. The use according to claim 35, wherein the disease is a cancer, and the cancer is a hematologic cancer or a solid tumor.
